(19) Europäisches Patentamt
European Patent Office
Office européen des brevets

(11) EP 1 968 963 B1

(12) **EUROPÄISCHE PATENTSCHRIFT**

(45) Veröffentlichungstag und Bekanntmachung des Hinweises auf die Patenterteilung:
**05.10.2011 Patentblatt 2011/40**

(21) Anmeldenummer: **06829854.6**

(22) Anmeldetag: **22.12.2006**

(51) Int Cl.:
***C07D 307/66*** $^{(2006.01)}$ ***C07D 233/88*** $^{(2006.01)}$
***C07D 277/42*** $^{(2006.01)}$ ***C07D 285/12*** $^{(2006.01)}$
***A61K 31/495*** $^{(2006.01)}$ ***A61K 31/41*** $^{(2006.01)}$
***A61P 29/00*** $^{(2006.01)}$

(86) Internationale Anmeldenummer:
**PCT/EP2006/012481**

(87) Internationale Veröffentlichungsnummer:
**WO 2007/079959 (19.07.2007 Gazette 2007/29)**

(54) **SUBSTITUIERTE PROPIOLSÄUREAMIDE UND IHRE VERWENDUNG ZUR HERSTELLUNG VON ANALGETIKA**

SUBSTITUTED PROPIOLIC ACID AMIDES AND THEIR USE FOR PRODUCING ANALGESICS

AMIDES DE L'ACIDE PROPIOLIQUE SUBSTITUES ET LEUR UTILISATION POUR PRODUIRE DES ANALGESIQUES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **28.12.2005 DE 102005062986**

(43) Veröffentlichungstag der Anmeldung:
**17.09.2008 Patentblatt 2008/38**

(73) Patentinhaber: **Grünenthal GmbH**
**52078 Aachen (DE)**

(72) Erfinder:
- **HAURAND, Michael**
**52078 Aachen (DE)**
- **SCHIENE, Klaus**
**40227 Düsseldorf (DE)**
- **KÜHNERT, Sven**
**52355 Düren (DE)**
- **REICH, Melanie**
**52072 Aachen (DE)**
- **ZEMOLKA, Saskia**
**78465 Konstanz (DE)**

(74) Vertreter: **Brosch, Oliver et al**
**Kutzenberger & Wolff**
**Theodor-Heuss-Ring 23**
**50668 Köln (DE)**

(56) Entgegenhaltungen:
**WO-A-03/093236**
**WO-A-2004/014903**
**WO-A-2004/029044**
**WO-A-2005/016323**
**WO-A1-2006/002981**

EP 1 968 963 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft substituierte Propiolsäureamide, Verfahren zu ihrer Herstellung, Arzneimittel enthaltend diese Verbindungen sowie deren Verwendung zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufrieden stellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nozizeption in letzter Zeit erschienen sind.

Klassische Opioide, wie beispielsweise Morphin, sind bei der Therapie starker bis sehr starker Schmerzen wirksam, führen jedoch oftmals zu unerwünschten Begleiterscheinungen wie beispielsweise Atemdepression, Erbrechen, Sedierung, Obstipation oder Toleranzentwicklung. Des Weiteren sind sie bei neuropathischen Schmerzen, unter denen insbesondere Tumorpatienten leiden, häufig nicht ausreichend wirksam.

Aus WO 2005/016323 und WO 2004/014903 sind Propiolsäureamide bekannt. Aus WO 2004/029044 und WO 03/093236 sind weitere Propiolsäureamide bekannt, die sich als Anlagetika eignen.

Eine Aufgabe der vorliegenden Erfindung bestand daher darin, neue Verbindungen zur Verfügung zu stellen, die sich insbesondere als pharmazeutische Wirkstoffe in Arzneimitteln eignen, bevorzugt in Arzneimitteln zur Behandlung von Schmerzen.

Es wurde nun überraschenderweise gefunden, dass sich die substituierten Propiolsäureamide der nachstehend angegebenen allgemeinen Formel I zur mGluR5-Rezeptor-Regulation eignen und daher insbesondere als pharmazeutische Wirkstoffe in Arzneimitteln zur Prophylaxe und/oder Behandlung von mit diesen Rezeptoren bzw. Prozessen in Verbindung stehenden Störungen oder Erkrankungen eingesetzt werden können.

Ein Gegenstand der vorliegenden Erfindung sind daher substituierte Propiolsäureamide der allgemeinen Formel I,

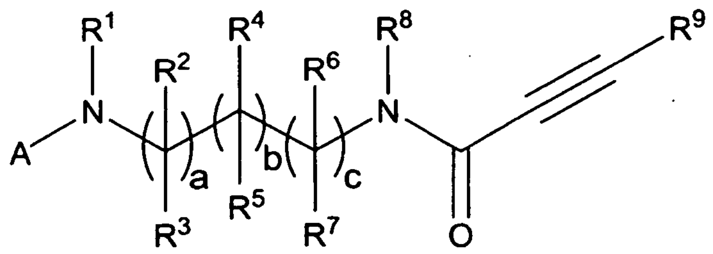

**I,**

worin

a, b und c, unabhängig voneinander, jeweils für 0 oder 1 stehen, wobei die Summe aus a, b und c gleich 1, 2 oder 3 ist;

A für einen der folgenden Reste

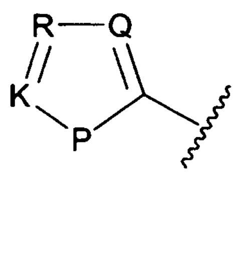

oder

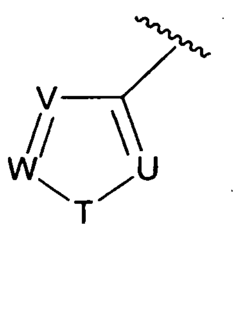

steht;

Q und U jeweils für $CR^{10}$ oder N stehen;

R und V jeweils für $CR^{11}$ oder N stehen;

K und W jeweils für $CR^{12}$ oder N stehen;
P und T jeweils für O, S oder $NR^{13}$ stehen;

mit der Maßgabe, dass Verbindungen ausgeschlossen sind, worin P für S steht, Q für N steht, R für $CR^{11}$ steht und K für $CR^{12}$ oder N steht;
$R^1$ und $R^8$, unabhängig voneinander, jeweils für H; -C(=O)-$R^{28}$; -C(=O)-O-$R^{29}$; - C(=O)-$NH_2$; -C(=O)-NH-$R^{30}$; -C(=O)-$NR^{31}R^{32}$; -S(=O)-$R^{33}$; -$S(=O)_2$-$R^{34}$; unsubstituiertes oder substituiertes Alkyl, Alkenyl oder Alkinyl; unsubstituiertes oder substituiertes Heteroalkyl-, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes - (Alkylen)-Cycloalkyl, -(Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes -(Heteroalkylen)-Cycloalkyl, -(Heteroalkenylen)-Cycloalkyl, -(Heteroalkylen)-Cycloalkenyl oder -(Heteroalkenylen)- Cycloalkenyl; unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl,-(Alkenylen)-Heterocycloalkenyl oder -(Alkinylen)-Heterocycloalkenyl; unsubstituiertes oder substituiertes -(Heteroalkylen)-Heterocycloalkyl, -(Heteroalkenylen)-Heterocycloalkyl, -(Heteroalkylen)-Heterocycloalkenyl oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, - (Alkenylen)-Aryl, -(Alkinylen)-Aryl, -(Heteroalkylen)-Aryl oder -(Heteroalkenylen)-Aryl; oder unsubstituiertes oder substituiertes -(Alkylen)-Heteroaryl, -(Alkenylen)-Heteroaryl, -(Alkinylen)-Heteroaryl, -(Heteroalkylen)-Heteroaryl oder - (Heteroalkenylen)-Heteroaryl stehen;
$R^2$, $R^3$, $R^4$, $R^5$, $R^6$, $R^7$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ und $R^{27}$, unabhängig voneinander, jeweils für H; F; Cl; Br; I; -$NO_2$; -CN; -$NH_2$; -OH; -SH; - C(=O)-OH; -C(=O)-H; -NH-C(=O)-H; -C(=O)-$R^{28}$; -C(=O)-O-$R^{29}$; -C(=O)-$NH_2$; -C(=O)-NH-$R^{30}$ ; -C(=O)-$NR^{31}R^{32}$; -S(=O)-$R^{33}$; -$S(=O)_2$-$R^{34}$; -NH-$R^{35}$; -$NR^{36}R^{37}$; -O-C(=O)-$R^{38}$; -NH-C(=O)-$R^{39}$; -$NR^{40}$-C(=O)-$R^{41}$; -O-$R^{42}$; -S-$R^{43}$; -NH-C(=O)-NH-$R^{44}$; -NH-C(=S)-NH-$R^{45}$; -NH-$S(=O)_2$-$R^{46}$; -$NR^{47}$-$S(=O)_2$-$R^{48}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes -(Alkylen)-Cycloalkyl, - (Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder wenigstens einfach substituiertes -(Heteroalkylen)-Cycloalkyl, -(Heteroalkenylen)-Cycloalkyl, - (Heteroalkylen)-Cycloalkenyl oder -(Heteroalkenylen)-Cycloalkenyl; unsubstituiertes oder wenigstens einfach substituiertes -(Alkylen)-Heterocycloalkyl, -(Alkenylen)-Heterocycloalkyl, -(Alkinylen)-Heterocycloalkyl, -(Alkylen)-Heterocycloalkenyl, - (Alkenylen)-Heterocycloalkenyl oder -(Alkinylen)-Heterocycloalkenyl; unsubstituiertes oder wenigstens einfach substituiertes -(Heteroalkylen)-Heterocycloalkyl, - (Heteroalkenylen)-Heterocycloalkyl, -(Heteroalkylen)-Heterocycloalkenyl oder-(Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, - (Alkenylen)-Aryl, -(Alkinylen)-Aryl, -(Heteroalkylen)-Aryl oder -(Heteroalkenylen)-Aryl; oder unsubstituiertes oder wenigstens einfach substituiertes -(Alkylen)-Heteroaryl, - (Alkenylen)-Heteroaryl, -(Alkinylen)-Heteroaryl, -(Heteroalkylen)-Heteroaryl oder-(Heteroalkenylen)-Heteroaryl stehen;
oder $R^2$ und $R^3$ oder $R^4$ und $R^5$ oder $R^6$ und $R^7$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$, unabhängig voneinander, zusammen jeweils für einen Rest ausgewählt aus der Gruppe bestehend aus einer Oxo-Gruppe (=O) und einer Thioxo-Gruppe (=S) stehen;
oder $R^1$ und $R^8$ zusammen mit der sie verbindenden -N-$CR^2R^3(CR^4R^5)_b$-$(CR^6R^7)_c$-Gruppe einen Rest der allgemeinen Formel B,

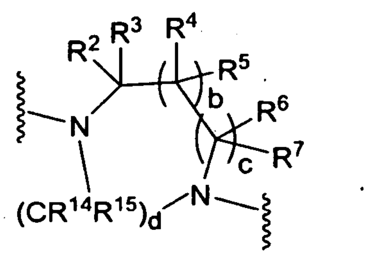

B,

bilden; wobei d für 1, 2 oder 3 steht und b für 0 oder 1 steht und c für 0 oder 1 steht;
oder $R^1$ und $R^2$ zusammen mit der sie verbindenden -N-$CR^3$-Gruppe einen Rest der allgemeinen Formel C,

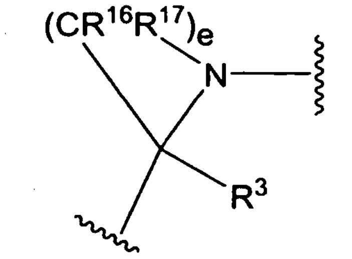

C,

bilden; wobei e für 1, 2, 3 oder 4 steht und, in diesem Fall, b für 0 oder 1 steht und c für 0 steht;
oder $R^1$ und $R^4$ zusammen mit der sie verbindenden -N-$CR^2R^3$-$CR^5$-Gruppe einen Rest der allgemeinen Formel D,

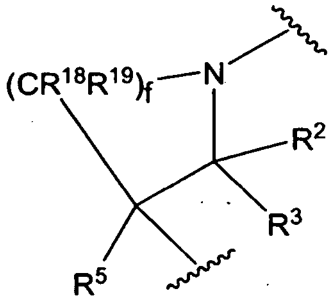

D,

bilden; wobei f für 1, 2, 3 oder 4 steht, und, in diesem Fall, c für 0 steht;
oder $R^1$ und $R^6$ zusammen mit der sie verbindenden -N-$CR^2R^3$-$CR^4R^5$-$CR^7$- Gruppe einen Rest der allgemeinen Formel E,

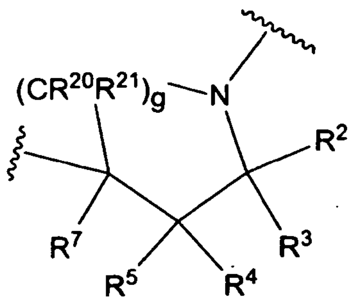

E,

bilden; wobei g für 1, 2 oder 3 steht;
oder $R^6$ und $R^8$ zusammen mit der sie verbindenden -N-$CR^7$-Gruppe einen Rest der allgemeinen Formel F

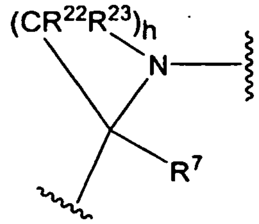

F,

bilden; wobei h für 1, 2, 3 oder 4 steht und, in diesem Fall, b für 0 oder 1 steht und a für 0 steht;
oder $R^4$ und $R^8$ zusammen mit der sie verbindenden -N-$CR^6R^7$-$CR^5$-Gruppe einen Rest der allgemeinen Formel G,

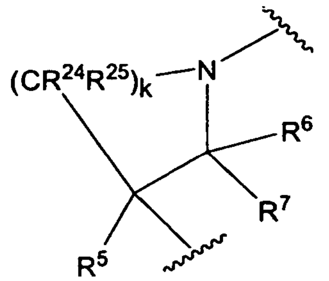

G,

bilden; wobei k für 1, 2, 3 oder 4 steht, und, in diesem, Fall a für 0 steht;
oder $R^2$ und $R^8$ zusammen mit der sie verbindenden -N-$CR^6R^7$-$CR^4R^5$-$CR^3$-Gruppe einen Rest der allgemeinen Formel H,

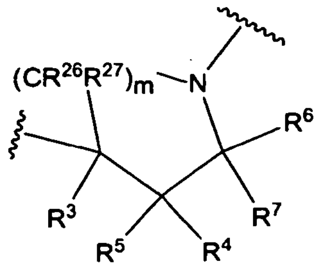

H,

bilden; wobei m für 1, 2 oder 3 steht;
$R^9$ für unsubstituiertes oder substituiertes Aryl oder unsubstituiertes oder substituiertes Heteroaryl steht;
$R^{10}$, $R^{11}$ und $R^{12}$, unabhängig voneinander, jeweils für H; F; Cl; Br; I; $-NO_2$; -CN;$-NH_2$; -OH; -SH; -C(=O)-OH; -C(=O)-H; -NH-C(=O)-H; -C(=O)-$R^{28}$; -C(=O)-O-$R^{29}$;-C(=O)-$NH_2$; -C(=O)-NH-$R^{30}$; -C(=O)-$NR^{31}R^{32}$; -S(=O)-$R^{33}$; $-S(=O)_2$-$R^{34}$; -NH-$R^{35}$;-$NR^{36}R^{37}$; -O-C(=O)-$R^{38}$; -NH-C(=O)-$R^{39}$; -$NR^{40}$-C(=O)-$R^{41}$; -O-$R^{42}$; -S-$R^{43}$; -NH-C(=O)-NH-$R^{44}$; -NH-C (=S)-NH-$R^{45}$; -NH-$S(=O)_2$-$R^{46}$; -$NR^{47}$-$S(=O)_2$-$R^{48}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes -(Alkylen)-Cycloalkyl, -(Alkenylen)-Cycloalkyl, -(Alkinylen)-Cycloalkyl, - (Alkylen)-Cycloalkenyl, -(Alkenylen)-Cycloalkenyl oder -(Alkinylen)-Cycloalkenyl; unsubstituiertes oder wenigstens einfach substituiertes -(Heteroalkylen)-Cycloalkyl, - (Heteroalkenylen)-Cycloalkyl, -(Heteroalkylen)-Cycloalkenyl oder -(Heteroalkenylen)-Cycloalkenyl; unsubstituiertes oder wenigstens einfach substituiertes -(Alkylen)-Heterocycloalkyl, -(Alkenylen)-Heterocycloalkyl, -(Alkinylen)-Heterocycloalkyl, - (Alkylen)-Heterocycloalkenyl, -(Alkenylen)-Heterocycloalkenyl oder -(Alkinylen)-Heterocycloalkenyl; unsubstituiertes oder wenigstens einfach substituiertes - (Heteroalkylen)-Heterocycloalkyl, -(Heteroalkenylen)-Heterocycloalkyl,-(Heteroalkylen)-Heterocycloalkenyl oder -(Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder wenigstens einfach substituiertes -(Alkylen)-Heteroaryl, -(Alkenylen)-Heteroaryl, - (Alkinylen)-Heteroaryl, -(Heteroalkylen)-Heteroaryl oder -(Heteroalkenylen)-Heteroaryl stehen;

$R^{13}$ für H; -C(=O)-OH; -C(=O)-H; -C(=O)-$R^{28}$; -C(=O)-O-$R^{29}$; -C(=O)-$NH_2$; -C(=O)-NH-$R^{30}$; -C(=O)-N$R^{31}R^{32}$; -S(=O)-$R^{33}$; -$S(=O)_2$-$R^{34}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes -(Alkylen)-Cycloalkyl, - (Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder wenigstens einfach substituiertes -(Heteroalkylen)-Cycloalkyl, -(Heteroalkenylen)-Cycloalkyl, - (Heteroalkylen)-Cycloalkenyl oder -(Heteroalkenylen)-Cycloalkenyl; unsubstituiertes oder wenigstens einfach substituiertes -(Alkylen)-Heterocycloalkyl, -(Alkenylen)-Heterocycloalkyl, -(Alkinylen)-Heterocycloalkyl, -(Alkylen)-Heterocycloalkenyl, (Alkenylen)-Heterocycloalkenyl oder -(Alkinylen)-Heterocycloalkenyl; unsubstituiertes oder wenigstens einfach substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, - (Alkenylen)-Aryl, -(Alkinylen)-Aryl, -(Heteroalkylen)-Aryl oder -(Heteroalkenylen)-Aryl; oder unsubstituiertes oder wenigstens einfach substituiertes -(Alkylen)-Heteroaryl, - (Alkenylen)-Heteroaryl, -(Alkinylen)-Heteroaryl, -(Heteroalkylen)-Heteroaryl oder - (Heteroalkenylen)Heteroaryl steht;

und $R^{28}$, $R^{29}$, $R^{30}$, $R^{31}$, $R^{32}$, $R^{33}$, $R^{34}$, $R^{35}$, $R^{36}$, $R^{37}$ $R^{38}$, $R^{39}$, $R^{40}$, $R^{41}$, $R^{42}$, $R^{43}$, $R^{44}$, $R^{45}$, $R^{46}$, $R^{47}$und $R^{48}$, unabhängig voneinander, jeweils für unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl, - (Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes - (Heteroalkylen)-Cycloalkyl, -(Heteroalkenylen)-Cycloalkyl, -(Heteroalkylen)-Cycloalkenyl oder -(Heteroalkenylen)-Cycloalkenyl; unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder substituiertes -(Heteroalkylen)-Heterocycloalkyl,- (Heteroalkenylen)-Heterocycloalkyl, -(Heteroalkylen)-Heterocycloalkenyl; oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, -(Heteroalkylen)-Aryl oder -(Heteroalkenylen)-Aryl; oder unsubstituiertes oder substituiertes -(Alkylen)-Heteroaryl, -(Alkenylen)-Heteroaryl, -(Alkinylen)-Heteroaryl, - (Heteroalkylen)-Heteroaryl oder -(Heteroalkenylen)-Heteroaryl stehen;

wobei "substituiert" im Zusammenhang mit den Resten "Alkyl", "Alkenyl", "Alkinyl", "Heteroalkyl", "Heteroalkenyl" und "Heteroalkinyl" für einen entsprechenden Rest steht, der einfach oder mehrfach mit Substituenten unabhängig voneinander ausgewählt aus der Gruppe bestehend aus F, Cl, Br, I, -$NO_2$, -CN, -OH, -SH, -$NH_2$,-N($C_{1-5}$-Alkyl)$_2$, -N($C_{1-5}$-Alkyl)(Phenyl), -N($C_{1-5}$-Alkyl)($CH_2$-Phenyl), -N($C_{1-5}$-Alkyl)($CH_2$-$CH_2$-Phenyl), -C(=O)-H, -C(=O)-$C_{1-5}$-Alkyl, -C(=O)-Phenyl, -C(=S)-$C_{1-5}$-Alkyl, -C(=S)-Phenyl, -C(=O)-OH, -C(=O)-O-$C_{1-5}$-Alkyl, -C(=O)-O-Phenyl, -C(=O)-$NH_2$, -C(=O)-NH-$C_{1-5}$-Alkyl, -C(=O)-N($C_{1-5}$-Alkyl)$_2$, -S(=O)-$C_{1-5}$-Alkyl, -S(=O)-Phenyl, -$S(=O)_2$-$C_{1-5}$-Alkyl, -$S(=O)_2$-Phenyl, -$S(=O)_2$-$NH_2$ und -$SO_3H$ substituiert ist, wobei die vorstehend genannten $C_{1-5}$-Alkyl-Reste jeweils linear oder verzweigt sein können und die vorstehend genannten Phenyl-Reste mit 1, 2, 3, 4 oder 5 Substituenten unabhängig voneinander ausgewählt aus der Gruppe bestehend aus F, Cl, Br, I, -CN, -$CF_3$, -OH, -$NH_2$, -O-$CF_3$, -SH, -O-$CH_3$, -O-$C_2H_5$, -O-$C_3H_7$, Methyl, Ethyl, n-Propyl, Isopropyl, n-Butyl, 2-Butyl, Isobutyl und tert-Butyl substituiert sein können,

wobei "substituiert" im Zusammenhang mit den Resten "Cycloalkyl", "Heterocycloalkyl", "Cycloalkenyl" und "Heterocycloalkenyl" für einen entsprechenden Rest steht, der einfach oder mehrfach mit Substituenten unabhängig voneinander ausgewählt aus der Gruppe bestehend aus F, Cl, Br, I, -CN, -$CF_3$, -OH, -$NH_2$, -O-$CF_3$, -SH, -O-$C_{1-5}$-Alkyl, -O-Phenyl, -O-$CH_2$-Phenyl, -($CH_2$)-O-$C_{1-5}$-Alkyl, -S-$C_{1-5}$-Alkyl, -S-Phenyl, -S-$CH_2$-Phenyl, -$C_{1-5}$-Alkyl, -$C_{2-5}$-Alkenyl, -$C_{2-5}$-Alkinyl, -C=C-Si($CH_3$)3, -C≡C-Si($C_2H_5$)$_3$, -C(=O)-O-$C_{1-5}$-Alkyl, -C(=O)-$CF_3$, -$S(=O)_2$-$C_{1-5}$-Alkyl, - S(=O)-$C_{1-5}$-Alkyl, -$S(=O)_2$-Phenyl, Oxo (=O), Thioxo (=S), -N($C_{1-5}$-Alkyl)$_2$, -N(H)($C_{1-5}$-Alkyl), -$NO_2$, -S-$CF_3$, -C(=O)-OH, -NH-$S(=O)_2$-$C_{1-5}$-Alkyl, -NH-C(=O)-$C_{1-5}$-Alkyl, - C(=O)-H, -C(=O)-$C_{1-5}$-Alkyl, -C(=O)-$NH_2$, -C(=O)-N($C_{1-5}$-Alkyl)$_2$, -C(=O)-N(H)($C_{1-5}$-Alkyl) und Phenyl substituiert ist, wobei die vorstehend genannten $C_{1-5}$-Alkyl-Reste jeweils linear oder verzweigt sein können und die Phenyl-Reste jeweils unsubstituiert oder mit 1, 2, 3, 4 oder 5 unabhängig voneinander ausgewählt aus der Gruppe bestehend aus F, Cl, Br, I, -CN, -$CF_3$, -OH, -$NH_2$, -O-$CF_3$, -SH, -O-$C_{1-5}$-Alkyl, -0-Phenyl, -O-$CH_2$-Phenyl, -($CH_2$)-O-$C_{1-5}$-Alkyl, -S-$C_{1-5}$-Alkyl, -S-Phenyl, -S-$CH_2$-Phenyl, -$C_{1-5}$-Alkyl, -$C_{2-5}$-Alkenyl, -$C_{2-5}$-Alkinyl, -C≡C-Si($CH_3$)$_3$, -C≡C-Si($C_2H_5$)$_3$, -C(=O)-O-$C_{1-5}$-Alkyl und -C(=O)-$CF_3$ substituiert sein können,

wobei "substituiert" im Zusammenhang mit den Resten "Aryl" und "Heteroaryl" für einen entsprechenden Rest steht, der einfach oder mehrfach mit Substituenten unabhängig voneinander ausgewählt aus der Gruppe bestehend aus F, Cl, Br, I,-CN, $-NO_2$, -OH, -SH, $-NH_2$, -C(=O)-OH, $-C_{1-5}$-Alkyl, $-(CH_2)-O-C_{1-5}$-Alkyl, $-C_{2-5}$-Alkenyl, $-C_{2-5}$-Alkinyl, -C=C-Si $(CH_3)_3$, $-C=C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$, $-0-CHF_2$, $-O-CH_2F$, $-C(=O)-CF_3$, $-S-CF_3$, $-S-CHF_2$, $-S-CH_2F$, $-S(=O)_2$-Phenyl, $-S(=O)_2-C_{1-5}$-Alkyl, $-S(=O)-C_{1-5}$-Alkyl. $-NH-C_{1-5}$-Alkyl, $N(C_{1-5}Alkyl)_2$, $-C(=O)-O-C_{1-5}$-Alkyl, -C(=O)-O-Phenyl, -C(=O)-H; $-C(=O)-C_{1-5}$-Alkyl, $-CH_2-O-C(=O)$-Phenyl, $-O-C(=O)-C_{1-5}$-Alkyl,-O-C(=O)-Phenyl, $-NH-S(=O)_2-C_{1-5}$-Alkyl, $-NH-C(=O)-C_{1-5}$-Alkyl, -C (=O)-$NH_2$, $-C(=O)-NH-C_{1-5}$-Alkyl, $-C(=O)-N(C_{1-5}\text{-Alkyl})_2$, $-C(=O)-N(C_{1-5}$-Alkyl)(Phenyl), -C(=O)-NH-Phenyl, Cyclopropyl, Cyclobutyl, Cyclopentyl, Cyclohexyl, Pyrazolyl, Phenyl, Furyl (Furanyl), Thiazolyl, Thiadiazolyl, Thiophenyl (Thienyl), Benzyl und Phenethyl substituiert ist, wobei die vorstehend genannten $C_{1-5}$-Alkyl-Reste jeweils linear oder verzweigt sein können und die zyklischen Substituenten bzw. die zyklischen Reste dieser Substituenten selbst mit ggf. 1, 2, 3, 4 oder 5 Substituenten unabhängig voneinander ausgewählt aus der Gruppe bestehend aus F, Cl, Br, I, -CN, $-NO_2$, -OH, -SH, $-NH_2$, -C(=O)-OH, $-C_{1-5}$-Alkyl, $-(CH_2)-O-C_{1-5}$-Alkyl, $-C_{2-5}$-Alkenyl, $-C_{2-5}$-Alkinyl,$-C\equiv C-Si(CH_3)_3$, $-C=C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$, -O-$CHF_2$, $-O-CH_2F$,$-C(=O)-CF_3$, $-S-CF_3$, $-S-CHF_2$ und $-S-CH_2F$ substituiert sein können,

wobei "substituiert" im Zusammenhang mit den Resten "Alkylen", "Alkenylen", "Alkinylen", "Heteroalkylen" und "Heteroalkenylen" für einen entsprechenden Rest steht, der einfach oder mehrfach mit Substituenten unabhängig voneinander ausgewählt aus der Gruppe bestehend aus Phenyl, F, Cl, Br, I, $-NO_2$, -CN, -OH, -O-Phenyl, $-O-CH_2$-Phenyl, -SH, -S-Phenyl, $-S-CH_2$-Phenyl, $-NH_2$, $-N(C_{1-5}\text{-Alkyl})_2$, -NH-Phenyl,- $N(C_{1-5}$-Alkyl)(Phenyl), $-N(C_{1-5}\text{-Alkyl})(CH_2\text{-Phenyl})$, -N $(C_{1-5}\text{-Alkyl})(CH_2\text{-}CH_2\text{-Phenyl})$, -C(=O)-H, $-C(=O)-C_{1-5}$-Alkyl, -C(=O)-Phenyl, $-C(=S)-C_{1-5}$-Alkyl, -C(=S)-Phenyl, -C (=O)-OH, $-C(=O)-O-C_{1-5}$-Alkyl, -C(=O)-O-Phenyl, $-C(=O)-NH_2$, $-C(=O)-NH-C_{1-5}$-Alkyl, $-C(=O)-N(C_{1-5}\text{-Alkyl})_2$, -S (=O)-$C_{1-5}$-Alkyl, -S(=O)-Phenyl, $-S(=O)_2-C_{1-5}$-Alkyl, $-S(=O)_2$-Phenyl, $-S(=O)_2-NH_2$ und $-SO_3H$ substituiert ist, wobei die vorstehend genannten $C_{1-5}$-Alkyl-Reste jeweils linear oder verzweigt sein können und die vorstehend genannten Phenyl-Reste mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$, -C(=O)-OH, $-C_{1-5}$-Alkyl, $-(CH_2)-O-C_{1-5}$-Alkyl, $-C_{2-5}$-Alkenyl, $-C_{2-5}$-Alkinyl,$-C=C-Si(CH_3)_3$, $-C\equiv C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$, $-O-CHF_2$, $-O-CH_2F$,$-C(=O)-CF_3$, $-S-CF_3$, $-S-CHF_2$ und $-S-CH_2F$ substituiert sein können,

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Der Begriff "Alkyl" umfaßt im Sinne der vorliegenden Erfindung azyklische gesättigte Kohlenwasserstoffreste, die verzweigt oder geradkettig sowie unsubstituiert oder wenigstens einfach substituiert sein können mit wie im Fall von $C_{1-12}$-Alkyl 1 bis 12 (d. h. 1, 2, 3, 4, 5, 6, 7, 8, 9, 10, 11 oder 12) C-Atomen bzw. mit wie im Fall von $C_{1-6}$-Alkyl 1 bis 6 (d. h. 1, 2, 3, 4, 5 oder 6) C-Atomen. Sofern einer oder mehrere der Substituenten für einen Alkyl-Rest stehen oder einen Alkyl-Rest aufweisen, der einfach oder mehrfach substituiert ist, ist dieser mit 1, 2, 3, 4 oder 5, besonders bevorzugt mit 1, 2 oder 3, Substituenten unabhängig voneinander ausgewählt aus der Gruppe bestehend aus F, Cl, Br, I, $-NO_2$, -CN, -OH, -SH, $-NH_2$, $-N(C_{1-5}\text{-Alky})_2$,$-N(C_{1-5}$-Alkyl)(Phenyl), $-N(C_{1-5}\text{-Alkyl})(CH_2\text{-Phenyl})$, $-N(C_{1-5}\text{-Alkyl})(CH_2\text{-}CH_2\text{-Phenyl})$,-C (=O)-H, $-C(=O)-C_{1-5}$-Alkyl, -C(=O)-Phenyl, $-C(=S)-C_{1-5}$-Alkyl, -C(=S)-Phenyl,-C(=O)-OH, $-C(=O)-O-C_{1-5}$-Alkyl, -C (=O)-O-Phenyl, $-C(=O)-NH_2$, $-C(=O)-NH-C_{1-5}$-Alkyl, $-C(=O)-N(C_{1-5}\text{-Alkyl})_2$, $-S(=O)-C_{1-5}$-Alkyl, -S(=O)-Phenyl, -S $(=O)_2-C_{1-5}$-Alkyl,$-S(=O)_2$-Phenyl, $-S(=O)_2-NH_2$ und $-SO_3H$ substituiert, wobei die vorstehend genannten $C_{1-5}$-Alkyl-Reste jeweils linear oder verzweigt sein können und die vorstehend genannten Phenyl-Reste bevorzugt mit 1, 2, 3, 4 oder 5 Substituenten unabhängig voneinander ausgewählt aus der Gruppe bestehend aus F, Cl, Br, I, -CN, $-CF_3$, -OH, $-NH_2$, $-O-CF_3$, -SH, $-O-CH_3$, $-O-C_2H_5$, $-O-C_3H_7$, Methyl, Ethyl, n-Propyl, Isopropyl, n-Butyl, 2-Butyl, Isobutyl und tert-Butyl substituiert sein können. Besonders bevorzugte Substituenten können unabhängig voneinander ausgewählt werden aus der Gruppe bestehend aus F, Cl, Br, I, $-NO_2$, -CN, -OH, -SH, $-NH_2$,$-N(CH_3)_2$, $-N(C_2H_5)_2$ und $-N(CH_3)(C_2H_5)$.

Als geeignete $C_{1-12}$-Alkyl-Reste, die unsubstituiert oder einfach oder mehrfach substituiert sein können, seien beispielsweise Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, 2-Hexyl, 3-Hexyl, n-Heptyl, n-Octyl, $-C(H)(C_2H_5)_2$, $-C(H)(n\text{-}C_3H_7)_2$ und $-CH_2-CH_2-C(H)(CH_3)-(CH_2)_3-CH_3$ genannt. Als geeignete $C_{1-6}$-Alkyl-Reste seien beispielsweise Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, 2-Hexyl und 3-Hexyl genannt.

Unter mehrfach substituierten Alkyl-Resten sind solche Alkyl-Reste zu verstehen, die entweder an verschiedenen oder an gleichen C-Atomen mehrfach, bevorzugt zwei- oder dreifach, substituiert sind, beispielsweise dreifach am gleichen C-Atom wie im Fall von $-CF_3$ oder an verschiedenen Stellen wie im Fall von $-(CHCl)-(CH_2F)$. Die Mehrfachsubstitution kann mit dem gleichen oder mit verschiedenen Substituenten erfolgen. Als geeignete substituierte Alkyl-Reste seien beispielsweise $-CF_3$, $-CF_2H$, - $CFH_2$, $-(CH_2)-OH$, $-(CH_2)-NH_2$, $-(CH_2)-CN$, $-(CH_2)-(CF_3)$, $-(CH_2)-(CHF_2)$, $-(CH_2)-(CH_2F)$, $-(CH_2)-(CH_2)-OH$, $-(CH_2)-(CH_2)-NH_2$, $-(CH_2)-(CH_2)-CN$, $-(CF_2)-(CF_3)$, $-(CH_2)-(CH_2)-(CF_3)$ und

$-(CH_2)-(CH_2)-(CH_2)-OH$ genannt.

Der Begriff "Alkenyl" umfaßt im Sinne der vorliegenden Erfindung azyklische ungesättigte Kohlenwasserstoffreste, die verzweigt oder geradkettig sowie unsubstituiert oder wenigstens einfach substituiert sein können und wenigstens eine Doppelbindung, bevorzugt 1, 2 oder 3 Doppelbindungen, aufweisen, mit wie im Fall von $C_{2-12}$-Alkenyl 2 bis 12 (d. h. 2, 3, 4, 5, 6, 7, 8, 9, 10, 11 oder 12) C-Atomen bzw. mit wie im Fall von $C_{2-6}$-Alkenyl 2 bis 6 (d. h. 2, 3, 4, 5 oder 6) C-Atomen. Sofern einer oder mehrere der Substituenten für einen Alkenyl-Rest stehen oder einen Alkenyl-Rest aufweisen, der einfach oder mehrfach substituiert ist, ist dieser mit 1, 2, 3, 4 oder 5, besonders bevorzugt mit 1, 2 oder 3, Substituenten unabhängig voneinander ausgewählt aus der Gruppe bestehend aus F, Cl, Br, I, $-NO_2$, -CN, -OH, -SH, $-NH_2$, $-N(C_{1-5}$-Alkyl$)_2$,$-N(C_{1-5}$-Alkyl)(Phenyl), $-N(C_{1-5}$-Alkyl)($CH_2$-Phenyl), $-N(C_{1-5}$-Alkyl)($CH_2$-$CH_2$-Phenyl), -C(=O)-H, $-C(=O)-C_{1-5}$-Alkyl,-C(=O)-Phenyl,- C(=S)-$C_{1-5}$-Alkyl, -C(=S)-Phenyl, -C(=O)-OH, -C(=O)-O-$C_{1-5}$-Alkyl, - C(=O)-O-Phenyl, $-C(=O)-NH_2$, -C(=O)-NH-$C_{1-5}$-Alkyl, -C(=O)-N($C_{1-5}$-Alkyl$)_2$, -S(=O)-$C_{1-5}$-Alkyl, -S(=O)-Phenyl, $-S(=O)_2-C_{1-5}$-Alkyl, $-S(=O)_2$-Phenyl, $-S(=O)_2-NH_2$ und - $SO_3H$ substituiert, wobei die vorstehend genannten $C_{1-5}$-Alkyl-Reste jeweils linear oder verzweigt sein können und die vorstehend genannten Phenyl-Reste bevorzugt mit 1, 2, 3, 4 oder 5 Substituenten unabhängig voneinander ausgewählt aus der Gruppe bestehend aus F, Cl, Br, I, -CN, $-CF_3$, -OH, $-NH_2$, $-O-CF_3$, -SH, $-0-CH_3$, $-O-C_2H_5$, $-O-C_3H_7$, Methyl, Ethyl, n-Propyl, Isopropyl, n-Butyl, 2-Butyl, Isobutyl und tert-Butyl substituiert sein können. Besonders bevorzugte Substituenten können unabhängig voneinander ausgewählt werden aus der Gruppe bestehend aus F, Cl, Br, I, $-NO_2$, -CN, -OH, -SH, $-NH_2$, $-N(CH_3)_2$, $-N(C_2H_5)_2$ und $-N(CH_3)(C_2H_5)$.

Als geeignete $C_{2-12}$-Alkenyl-Reste seien beispielsweise Ethenyl, 1-Propenyl, 2-Propenyl, 1-Butenyl, 2-Butenyl, 3-Butenyl, 1-Pentenyl, 2-Pentenyl, 3-Pentenyl, 4-Pentenyl, Hexenyl, $-CH=CH-CH=CH-CH_3$ und $-CH_2-CH_2-CH=CH_2$ genannt.

Unter mehrfach substituierten Alkenyl-Resten sind solche Alkenyl-Reste zu verstehen, die entweder an verschiedenen oder an gleichen C-Atomen mehrfach, bevorzugt zweifach, substituiert sind, beispielsweise zweifach am gleichen C-Atom wie im Fall von $-CH=CCl_2$ oder an verschiedenen Stellen wie im Fall von $-CCl=CH-(CH_2)-NH_2$. Die Mehrfachsubstitution kann mit dem gleichen oder mit verschiedenen Substituenten erfolgen. Als geeignete substituierte Alkenyl-Reste seien beispielsweise $-CH=CH-(CH_2)-OH$, $-CH=CH-(CH_2)-NH_2$ und -CH=CH-CN genannt.

Der Begriff "Alkinyl" umfaßt im Sinne der vorliegenden Erfindung azyklische ungesättigte Kohlenwasserstoffreste, die verzweigt oder geradkettig sowie unsubstituiert oder wenigstens einfach substituiert sein können und wenigstens eine Dreifachbindung, bevorzugt 1 oder 2 Dreifachbindungen, aufweisen, mit wie im Fall von $C_{2-12}$-Alkinyl 2 bis 12 (d. h. 2, 3, 4, 5, 6, 7, 8, 9, 10, 11 oder 12) C-Atomen bzw. mit wie im Fall von $C_{2-6}$-Alkinyl 2 bis 6 (d. h. 2, 3, 4, 5 oder 6) C-Atomen. Sofern einer oder mehrere der Substituenten für einen Alkinyl-Rest stehen oder einen Alkinyl-Rest aufweisen, der einfach oder mehrfach substituiert ist, ist dieser mit 1, 2, 3, 4 oder 5, besonders bevorzugt mit ggf. 1 oder 2, Substituenten unabhängig voneinander ausgewählt aus der Gruppe bestehend aus F, Cl, Br, I, $-NO_2$, -CN, -OH,- SH, $-NH_2$, $-N(C_{1-5}$-Alkyl$)_2$,$-N(C_{1-5}$-Alkyl)(Phenyl), $-N(C_{1-5}$-Alkyl)($CH_2$-Phenyl), $-N(C_{1-5}$-Alkyl)($CH_2$-$CH_2$-Phenyl), -C(=O)-H, $-C(=O)-C_{1-5}$-Alkyl, -C(=O)-Phenyl, -C(=S)-$C_{1-5}$-Alkyl, -C(=S)-Phenyl, -C(=O)-OH, -C(=O)-O-$C_{1-5}$-Alkyl, -C(=O)-O-Phenyl, $-C(=O)-NH_2$, -C(=O)-NH-$C_{1-5}$-Alkyl, -C(=O)-N($C_{1-5}$-Alkyl$)_2$, -S(=O)-$C_{1-5}$-Alkyl, -S(=O)-Phenyl,$-S(=O)_2-C_{1-5}$-Alkyl, -S (=O$)_2$-Phenyl, $-S(=O)_2-NH_2$ und $-SO_3H$ substituiert, wobei die vorstehend genannten $C_{1-5}$-Alkyl-Reste jeweils linear oder verzweigt sein können und die vorstehend genannten Phenyl-Reste bevorzugt mit 1, 2, 3, 4 oder 5 Substituenten unabhängig voneinander ausgewählt aus der Gruppe bestehend aus F, Cl, Br, I, -CN, $-CF_3$, -OH, $-NH_2$, $-O-CF_3$, -SH, $-O-CH_3$, $-O-C_2H_5$, $-O-C_3H_7$, Methyl, Ethyl, n-Propyl, Isopropyl, n-Butyl, 2-Butyl, Isobutyl und tert-Butyl substituiert sein können. Besonders bevorzugte Substituenten können unabhängig voneinander ausgewählt werden aus der Gruppe bestehend aus F, Cl, Br, I, $-NO_2$, -CN, -OH, -SH, $-NH_2$, $-N(CH_3)_2$, $-N(C_2H_5)_2$ und $-N(CH_3)(C_2H_5)$.

Als geeignete $C_{2-12}$-Alkinyl-Reste seien beispielsweise Ethinyl, 1-Propinyl, 2-Propinyl, 1-Butinyl, 2-Butinyl, 3-Butinyl, 1-Pentinyl, 2-Pentinyl, 3-Pentinyl, 4-Pentinyl und Hexinyl genannt.

Unter mehrfach substituierten Alkinyl-Resten sind solche Alkinyl-Reste zu verstehen, die entweder an verschiedenen C-Atomen mehrfach substituiert sind, beispielsweise zweifach an verschiedenen C-Atomen wie im Fall von $-CHCl-C\equiv CCl$. Als geeignete substituierte Alkinyl-Reste seien beispielsweise $-C\equiv C-F$, $-C\equiv C-Cl$ und $-C\equiv C-I$ genannt.

Der Begriff "Heteroalkyl" bezeichnet einen wie vorstehend beschriebenen Alkyl-Rest, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkyl-Reste können bevorzugt 1, 2 oder 3 Heteroatom(e) unabhängig voneinander ausgewählt aus der Gruppe bestehend aus Sauerstoff, Schwefel und Stickstoff (NH) als Kettenglied(er) aufweisen. Heteroalkyl-Reste können bevorzugt 2- bis 12-gliedrig, besonders bevorzugt 2- bis 6-gliedrig, sein.

Als geeignete Heteroalkyl-Reste, die unsubstituiert oder einfach oder mehrfach substituiert sein können, seien beispielsweise $-CH_2-O-CH_3$, $-CH_2-O-C_2H_5$, $-CH_2-O-CH(CH_3)_2$, $-CH_2-O-C(CH_3)_3$, $-CH_2-S-CH_3$, $-CH_2-S-C_2H_5$, $-CH_2-S-CH(CH_3)_2$, $-CH_2-S-C(CH_3)_3$, $-CH_2-NH-CH_3$, $-CH_2-NH-C_2H_5$, $-CH_2-NH-CH(CH_3)_2$, $-CH_2-NH-C(CH_3)_3$, $-CH_2-CH_2-O-CH_3$, $-CH_2-CH_2-O-C_2H_5$, $-CH_2-CH_2-O-CH(CH_3)_2$, $-CH_2-CH_2-O-C(CH_3)_3$, $-CH_2-CH_2-S-CH_3$, $-CH_2-CH_2-S-C_2H_5$, $-CH_2-CH_2-S-CH(CH_3)_2$, $-CH_2-CH_2-S-C(CH_3)_3$, $-CH_2-CH_2-NH-CH_3$, $-CH_2-CH_2-NH-C_2H_5$, $-CH_2-CH_2-NH-CH(CH_3)_2$, $-CH_2-CH_2-NH-C(CH_3)_3$, - $CH_2-S-CH_2-O-CH_3$, $-CH_2-O-CH_2-O-C_2H_5$, $-CH_2-O-CH_2-O-CH(CH_3)_2$, $-CH_2-S-CH_2-O-C(CH_3)_3$, $-CH_2-O-CH_2-S-CH_3$, $-CH_2-O-CH_2-S-C_2H_5$, $-CH_2-O-CH_2-S-CH(CH_3)_2$, $-CH_2-NH-CH_2-S-C(CH_3)_3$, $-CH_2-O-CH_2-NH-CH_3$,

$-CH_2-O-CH_2-NH-C_2H_5$, $-CH_2-O-CH_2-NH-CH(CH_3)_2$, $-CH_2-S-CH_2-NH-C(CH_3)_3$ und $-CH_2-CH_2-C(H)(CH_3)-(CH_2)_3-CH_3$ genannt.

Als geeignete substituierte Heteroalkyl-Reste seien beispielsweise $-(CH_2)-O-(CF_3)$, $-(CH_2)-O-(CHF_2)$, $-(CH_2)-O-(CH_2F)$, $-(CH_2)-S-(CF_3)$, $-(CH_2)-S-(CHF_2)$, $-(CH_2)-S-(CH_2F)$, $-(CH_2)-(CH_2)-O-(CF_3)$, $-(CF_2)-O-(CF_3)$, $-(CH_2)-(CH_2)-S-(CF_3)$ und $-(CH_2)-(CH_2)-(CH_2)-O-(CF_3)$ genannt.

Der Begriff "Heteroalkenyl" bezeichnet einen wie vorstehend beschriebenen Alkenyl-Rest, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkenyl-Reste können bevorzugt 1, 2 oder 3 Heteroatom (e) unabhängig voneinander ausgewählt aus der Gruppe bestehend aus Sauerstoff, Schwefel und Stickstoff (NH) als Kettenglied(er) aufweisen. Heteroalkenyl-Reste können bevorzugt 2- bis 12-gliedrig, besonders bevorzugt 2- bis 6-gliedrig, sein.

Als geeignete Heteroalkenyl-Reste seien beispielsweise $-CH_2-O-CH=CH_2$, $-CH=CH-O-CH=CH-CH_3$, $-CH_2-CH_2-O-CH=CH_2$, $-CH_2-S-CH=CH_2$, $-CH=CH-S-CH=CH-CH_3$, $-CH_2-CH_2-S-CH=CH_2$, $-CH_2-NH-CH=CH_2$, $-CH=CH-NH-CH=CH-CH_3$ und $-CH_2-CH_2-NH-CH=CH_2$ genannt.

Als geeignete substituierte Heteroalkenyl-Reste seien beispielsweise $-CH_2-O-CH=CH-(CH_2)-OH$, $-CH_2-S-CH=CH-(CH_2)-NH_2$ und $-CH_2-NH-CH=CH-CN$ genannt. Der Begriff "Heteroalkinyl" bezeichnet einen wie vorstehend beschriebenen Alkinyl-Rest, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkinyl-Reste können bevorzugt 1, 2 oder 3 Heteroatom(e) unabhängig voneinander ausgewählt aus der Gruppe bestehend aus Sauerstoff, Schwefel und Stickstoff (NH) als Kettenglied(er) aufweisen. Heteroalkinyl-Reste können bevorzugt 2- bis 12-gliedrig, besonders bevorzugt 2- bis 6-gliedrig, sein.

Als geeignete Heteroalkinyl-Reste seien beispielsweise $-CH_2-O-C\equiv CH$, $-CH_2-CH_2-O-C\equiv CH$, $-CH_2-O-C\equiv C-CH_3$, $-CH_2-CH_2-O-C\equiv C-CH_3$, $-CH_2-S-C\equiv CH$, $-CH_2-CH_2-S-CH_2\equiv CH$, $-CH_2-S-C\equiv C-CH_3$, $-CH_2-CH_2-S-C\equiv C-CH_3$ genannt.

Als geeignete substituierte Heteroalkinyl-Reste seien beispielsweise $-CH_2-O-C\equiv C-Cl$, $-CH_2-CH_2-O-C\equiv C-I$, $-CHF-O-C\equiv C-CH_3$, $-CHF-CH_2-O-C\equiv C-CH_3$, $-CH_2-S-C\equiv C-Cl$,$-CH_2-CH_2-S-C\equiv C-Cl$, $-CHF-S-C\equiv C-CH_3$, $-CHF-CH_2-S-C\equiv C-CH_3$ genannt.

Der Begriff "Cycloalkyl" bedeutet im Sinne der vorliegenden Erfindung einen zyklischen gesättigten Kohlenwasserstoff-Rest mit bevorzugt 3, 4, 5, 6, 7, 8 oder 9 C-Atomen, besonders bevorzugt mit 3, 4, 5, 6 oder 7 C-Atomen, ganz besonders bevorzugt mit 5 oder 6 C-Atomen, wobei der Rest unsubstituiert oder einfach substituiert oder mehrfach gleich oder verschieden substituiert sein kann.

Als geeignete $C_{3-9}$-Cycloalkyl-Reste, die unsubstituiert oder einfach oder mehrfach substituiert sein können, seien beispielsweise Cyclopropyl, Cyclobutyl, Cyclopentyl, Cyclohexyl, Cycloheptyl, Cyclooctyl und Cyclononyl genannt. Als geeignete $C_{3-7}$-Cycloalkyl-Reste seien Cyclopropyl, Cyclobutyl, Cyclopentyl, Cyclohexyl und Cycloheptyl genannt.

Der Begriff "Cycloalkenyl" bedeutet im Sinne der vorliegenden Erfindung einen zyklischen ungesättigten Kohlenwasserstoff-Rest mit bevorzugt 3, 4, 5, 6, 7, 8 oder 9 C-Atomen, besonders bevorzugt mit 3, 4, 5, 6 oder 7 C-Atomen, ganz besonders bevorzugt mit 5 oder 6 C-Atomen, der wenigstens eine Doppelbindung, vorzugsweise eine Doppelbindung, aufweist und unsubstituiert oder einfach substituiert oder mehrfach gleich oder verschieden substituiert sein kann.

Als geeignete $C_{3-9}$-Cycloalkenyl-Reste, die unsubstituiert oder einfach oder mehrfach substituiert sein können, seien Cyclobutenyl, Cyclopentenyl, Cyclohexenyl, Cycloheptenyl, Cyclononenyl und Cyclooctenyl genannt. Als geeignete $C_{5-6}$- Cycloalkenyl-Reste seien Cyclopentenyl und Cyclohexenyl genannt.

Der Begriff "Heterocycloalkyl" bedeutet im Sinne der vorliegenden Erfindung einen zyklischen gesättigten Kohlenwasserstoff-Rest mit bevorzugt 3, 4, 5, 6, 7, 8 oder 9 C-Atomen, besonders bevorzugt mit 3, 4, 5, 6 oder 7 C-Atomen, ganz besonders bevorzugt mit 5 oder 6 C-Atomen, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heterocycloalkyl-Reste können bevorzugt 1, 2 oder 3 Heteroatom(e) unabhängig voneinander ausgewählt aus der Gruppe bestehend aus Sauerstoff, Schwefel und Stickstoff (NH) als Ringglied(er) aufweisen. Ein Heterocycloalkyl-Rest kann unsubstituiert oder einfach substituiert oder mehrfach gleich oder verschieden substituiert sein. Heterocycloalkyl-Reste können bevorzugt 3- bis 9-gliedrig, besonders bevorzugt 3- bis 7-gliedrig, ganz besonders bevorzugt 5- bis 7-gliedrig, sein.

Als geeignete 3- bis 9-gliedrige Heterocycloalkyl-Reste, die unsubstituiert oder einfach oder mehrfach substituiert sein können, seien beispielsweise Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Oxetanyl, Azepanyl, Azocanyl, Diazepanyl, Dithiolanyl, (1,3)-Dioxolan-2-yl, Isoxazolidinyl, Isothioazolidinyl, Pyrazolidinyl, Oxazolidinyl, (1,2,4)-Oxadiazolidinyl, (1,2,4)-Thiadiazolidinyl, (1,2,4)-Triazolidin-3-yl, (1,3,4)-Thiadiazolidin-2-yl, (1,3,4)-Triazolidin-1-yl, (1,3,4)-Triazoldidin-2-yl, Tetrahydropyridazinyl, Tetrahydropyrimidinyl, Tetrahydropyrazinyl, (1,3,5)-Tetrahydrotriazinyl, (1,2,4)-Tetrahydrotriazin- 1-yl, (1,3)-Dithian-2-yl und (1,3)-Thiazolidinyl genannt. Als geeignete 5- bis 7-gliedrige Heterocycloalkyl-Reste seien beispielsweise Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Oxetanyl, Azepanyl, Diazepanyl und (1,3)-Dioxolan-2-yl genannt.

Der Begriff "Heterocycloalkenyl" bedeutet im Sinne der vorliegenden Erfindung einen zyklischen ungesättigten Kohlenwasserstoff-Rest mit bevorzugt 4, 5, 6, 7, 8 oder 9 C-Atomen, besonders bevorzugt mit 4, 5, 6 oder 7 C-Atomen, ganz besonders bevorzugt mit 5 oder 6 C-Atomen, der wenigstens eine Doppelbindung, vorzugsweise eine Doppelbindung, aufweist und in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heterocycloalkenyl-Reste können bevorzugt 1, 2 oder 3 Heteroatom(e) unabhängig voneinander ausgewählt aus der Gruppe bestehend aus Sauerstoff, Schwefel und Stickstoff (NH) als Ringglied(er) aufweisen. Ein Heterocycloalkenyl-Rest kann unsubstituiert oder einfach substituiert oder mehrfach gleich oder verschieden substituiert sein. Heterocycloalkenyl-Reste können bevorzugt 4- bis 9-gliedrig, besonders bevorzugt 4-bis 7-gliedrig, ganz besonders bevorzugt 5- bis 7-gliedrig, sein.

Als geeignete Heterocycloalkenyl-Reste bzw. als geeignete 5- bis 7-gliedrige Heterocycloalkenyl-Reste, die unsubstituiert oder einfach oder mehrfach substituiert sein können, seien beispielsweise (2,3)-Dihydrofuranyl, (2,5)-Dihydrofuranyl, (2,3)-Dihydrothienyl, (2,5)-Dihydrothienyl, (2,3)-Dihydropyrrolyl, (2,5)-Dihydropyrrolyl, (2,3)-Dihydroisoxazolyl, (4,5)-Dihydroisoxazolyl, (2,5)-Dihydroisothiazolyl, (2,3)-Dihydropyrazolyl, (4,5)-Dihydropyrazolyl, (2,5)-Dihydropyrazolyl, (2,3)-Dihydrooxazolyl, (4,5)-Dihydrooxazolyl, (2,5)-Dihydrooxazolyl, (2,3)-Dihydrothiazolyl, (4,5)-Dihydrothiazolyl, (2,5)-Dihydrothiazolyl, (2,3)-Dihydroimidazolyl, (4,5)-Dihydroimidazolyl, (2,5)-Dihydroimidazolyl, (3,4,5,6)-Tetrahydropyridin-2-yl, (1,2,5,6)-Tetrahydropyridin-1-yl, (1,2)-Dihydropyridin-1-yl, (1,4)-Dihydropyridin-1-yl, Dihydropyranyl und (1,2,3,4)-Tetrahydropyridin-1-yl genannt.

Cycloalkyl-Rest, Heterocycloalkyl-Rest, Cycloalkenyl-Rest oder Heterocyclalkenyl-Rest können im Sinne der vorliegenden Erfindung mit einem unsubstituierten oder wenigstens einfach substituierten mono- bzw. bizyklischem Ringsystem kondensiert (anneliert) sein. Unter einem mono- bzw. bizyklischem Ringsystem werden im Sinne der vorliegenden Erfindung mono- bzw. bizyklische Kohlenwasserstoffreste verstanden, die gesättigt, ungesättigt oder aromatisch sein und ggf. eines oder mehrere Heteroatome als Ringglieder aufweisen können. Vorzugsweise sind die Ringe der vorstehend genannten mono- oder bizyklischen Ringsysteme jeweils 4-, 5-oder 6-gliedrig und können jeweils bevorzugt ggf. 0, 1, 2, 3, 4 oder 5 Heteroatom(e), besonders bevorzugt ggf. 0, 1 oder 2 Heteroatom(e) als Ringglied(er) aufweisen, die unabhängig voneinander aus der Gruppe bestehend aus Sauerstoff, Stickstoff und Schwefel ausgewählt sind. Sofern ein bizyklisches Ringsystem vorliegt, können die verschiedenen Ringe, jeweils unabhängig voneinander, einen unterschiedlichen Sättigungsgrad aufweisen, d.h. gesättigt, ungesättigt oder aromatisch sein.

Sofern einer oder mehrere der Substituenten ein monozyklisches oder bizyklisches Ringsystem aufweisen, das einfach oder mehrfach substituiert ist, kann dieses bevorzugt mit ggf. 1, 2, 3, 4 oder 5, besonders bevorzugt mit ggf. 1, 2 oder 3, Substituenten substituiert sein, die unabhängig voneinander ausgewählt werden können aus der Gruppe bestehend aus F, Cl, Br, I, -CN, $-NO_2$, -OH, -SH, $-NH_2$, Oxo (=O), Thioxo (=S), -C(=O)-OH, $C_{1-5}$-Alkyl, $-C_{2-5}$-Alkenyl, $-C_{2-5}$-Alkinyl, $-C{\equiv}C-Si(CH_3)_3$,$-C{\equiv}C-Si(C_2H_5)_3$, $-(CH_2)-O-C_{1-5}$-Alkyl, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$, $-O-CHF_2$, $-O-CH_2F$,$-C(=O)-CF_3$, $-S-CF_3$, $-S-CHF_2$, $-S(=O)_2$-Phenyl, $-S=(O)_2-C_{1-5}$-Alkyl, $C_{1-5}$-Alkyl, $-NH-C_{1-5}$-Alkyl, $-N(C_{1-5}Alkyl)(C_{1-5}$-Alkyl), $-C(=O)-O-C_{1-5}$-Alkyl, -C(=O)-H,$-C(=O)-C_{1-5}$-Alkyl, $-CH_2-O-C(=O)$-Phenyl, -O-C(=O)-Phenyl, $-NH-S(=O)_2-C_{1-5}$-Alkyl,$-NH-C(=O)-C_{1-5}$-Alkyl, $-C(=O)-NH_2$, $-C(=O)-NH-C_{1-5}$-Alkyl, $-C(=O)-N(C_{1-5}\text{-Alkyl})_2$, Pyrazolyl, Phenyl, Furyl (Furanyl), Thiadiazolyl, Thiophenyl (Thienyl) und Benzyl, wobei die vorstehend genannten $C_{1-5}$-Alkyl-Reste jeweils linear oder verzweigt sein können und die zyklischen Substituenten bzw. die zyklischen Reste dieser Substituenten selbst jeweils mit ggf. 1, 2, 3, 4 oder 5, bevorzugt mit ggf. 1, 2, 3 oder 4, Substituenten unabhängig voneinander ausgewählt aus der Gruppe bestehend aus F, Cl, Br, I, -CN, $-CF_3$, -OH, $-NH_2$, $-O-CF_3$, -SH, $-O-C_{1-5}$-Alkyl, -O-Phenyl, $-O-CH_2$-Phenyl, $-(CH_2)-O-C_{1-5}$-Alkyl, $-S-C_{1-5}$-Alkyl, -S-Phenyl, $-S-CH_2$-Phenyl, $-C_{1-5}$-Alkyl, $-C_{2-5}$-Alkenyl, $-C_{2-5}$-Alkinyl, $-C{\equiv}C-Si(CH_3)_3$, $-C{\equiv}C-Si(C_2H_5)_3$, $-C(=O)-O-C_{1-5}$-Alkyl und$-C(=O)-CF_3$ substituiert sein können.

Besonders bevorzugt können die Substituenten, jeweils unabhängig voneinander, ausgewählt werden aus der Gruppe bestehend aus F, Cl, Br, I, -CN, $-NO_2$, -OH, -SH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, $-C{\equiv}C-Si(CH_3)_3$, $-C{\equiv}C-Si(C_2H_5)_3$, $-C{\equiv}C-Si(CH_3)_3$, $-C{\equiv}C-Si(C_2H_5)_3$, $-CH_2-O-CH_3$, $-CH_2-O-C_2H_5$, -OH, -SH, $-NH_2$, Oxo (=O),-C(=O)-OH,- $S-CH_3$, $-S-C_2H_5$, $-S(=O)-CH_3$, $-S(=O)_2-CH_3$, $-S(=O)-C_2H_5$, $-S(=O)_2-C_2H_5$,$-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-S(=O)_2$-Phenyl, Pyrazolyl, Phenyl,$-N(CH_3)_2$, $-N(C_2H_5)_2$, $-NH-CH_3$, $-NH-C_2H_5$, $-CH_2-O-C(=O)$-Phenyl, $-NH-S(=O)_2-CH_3$,$-C(=O)-O-CH_3$, $-C(=O)-O-C_2H_5$, $-C(=O)-O-C(CH_3)_3$, -C(=O)-H, $-C(=O)-CH_3$, $-C(=O)-C_2H_5$, $-NH-C(=O)-CH_3$, $-NH-C(=O)-C_2H_5$, -O-C(=O)-Phenyl, $-C(=O)-NH_2$, $-C(=O)-NH-CH_3$, $-C(=O)-N(CH_3)_2$, Phenyl, Furyl (Furanyl), Thiadiazolyl, Thiophenyl (Thienyl) und Benzyl, wobei die zyklischen Substituenten bzw. die zyklischen Reste dieser Substituenten selbst mit ggf. 1, 2, 3, 4 oder 5, bevorzugt mit ggf. 1, 2, 3 oder 4, Substituenten unabhängig voneinander ausgewählt aus der Gruppe bestehend aus F, Cl, Br, I, -CN, $-CF_3$, -OH, $-NH_2$, $-O-CF_3$, -SH, $-O-CH_3$, $-O-C_2H_5$, $-O-C_3H_7$, Methyl, Ethyl, n-Propyl, Isopropyl, n-Butyl, 2-Butyl, Isobutyl, tert-Butyl, Ethenyl, Allyl, Ethinyl, Propinyl, $-C{=}{-}C-Si(CH_3)_3$, $-C{\equiv}C-Si(C_2H_5)_3$, $-C(=O)-O-C_{1-5}$-Alkyl und $-C(=O)-CF_3$ substituiert sein können.

Als geeignete Cycloalkyl-Rest, Heterocycloalkyl-Rest, Cycloalkenyl-Rest oder Heterocyclalkenyl-Rest, die unsubstituiert oder einfach oder mehrfach substituiert sein können, und mit einem mono- bzw. bizyklischem Ringsystem kondensiert sind, seien beispielhaft (1,2,3,4)-Tetrahydrochinolinyl, (1,2,3,4)-Tetrahydroisochinolinyl, (2,3)-Dihydro-1H-isoindolyl, (1,2,3,4)-Tetrahydronaphthyl, (2,3)-Dihydrobenzo[1.4]dioxinyl, Benzo[1.3]dioxolyl, (3,4)-Dihydro-2H-benzo [1.4]oxazinyl und Octahydro-pyrrolo[3,4-c]pyrrolyl genannt.

Sofern einer oder mehrere der Substituenten für einen Cycloalkyl-Rest, Heterocycloalkyl-Rest, Cycloalkenyl-Rest oder Heterocyclalkenyl-Rest stehen oder einen solchen Rest aufweisen, der einfach oder mehrfach substituiert ist, ist dieser mit 1, 2, 3, 4 oder 5, besonders bevorzugt mit ggf. 1, 2 oder 3, Substituenten unabhängig voneinander ausgewählt aus der Gruppe bestehend aus F, Cl, Br, I, -CN, $-CF_3$, -OH, $-NH_2$, $-O-CF_3$, -SH, $-O-C_{1-5}$-Alkyl, -O-Phenyl, $-O-CH_2$-Phenyl, $-(CH_2)-O-C_{1-5}$-Alkyl, $-S-C_{1-5}$-Alkyl, -S-Phenyl, $-S-CH_2$-Phenyl, $-C_{1-5}$-Alkyl, $-C_{2-5}$-Alkenyl, $-C_{2-5}$-Alkinyl, $-C{\equiv}C-Si(CH_3)_3$, $-C{\equiv}C-Si(C_2H_5)_3$, $-C(=O)-O-C_{1-5}$-Alkyl, $-C(=O)-CF_3$, $-S(=O)_2-C_{1-5}$-Alkyl, $-S(=O)-C_{1-5}$-Alkyl, $-S(=O)_2$-Phenyl, Oxo (=O), Thioxo (=S), - $N(C_{1-5}$-Alkyl$)_2$, $-N(H)(C_{1-5}$-Alkyl), $-NO_2$, $-S-CF_3$, -C(=O)-OH, $-NH-S(=O)_2-C_{1-5}$-Alkyl, - NH-C(=O)-$C_{1-5}$-Alkyl, -C(=O)-H, $-C(=O)-C_{1-5}$-Alkyl, $-C(=O)-NH_2$, $-C(=O)-N(C_{1-5}$-Alkyl$)_2$,- $C(=O)-N(H)(C_{1-5}$-Alkyl) und Phenyl substituiert, wobei die vorstehend genannten $C_{1-5}$-Alkyl-Reste jeweils linear oder verzweigt sein können und die Phenyl-Reste jeweils unsubstituiert oder mit 1, 2, 3, 4 oder 5, bevorzugt mit 1, 2, 3 oder 4, Substituenten unabhängig voneinander ausgewählt aus der Gruppe bestehend aus F, Cl, Br, I, -CN, $-CF_3$, -OH, $-NH_2$, $-O-CF_3$, -SH, $-O-C_{1-5}$-Alkyl, -O-Phenyl, $-O-CH_2$-Phenyl, $-(CH_2)-O-C_{1-5}$-Alkyl, $-S-C_{1-5}$-Alkyl, -S-Phenyl, $-S-CH_2$-Phenyl, $-C_{1-5}$-Alkyl, $-C_{2-5}$-Alkenyl, $-C_{2-5}$-Alkinyl, $-C{\equiv}C-Si(CH_3)_3$, $-C{\equiv}C-Si(C_2H_5)_3$, $-C(=O)-O-C_{1-5}$-Alkyl und-$C(=O)-CF_3$ substituiert sein können.

Besonders bevorzugt können die Substituenten, jeweils unabhängig voneinander, ausgewählt werden aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, $-C{\equiv}-C-Si(CH_3)_3$, $-C{\equiv}C-Si(C_2H_5)_3$, -OH, Oxo, Thioxo, $-O-CH_3$, $-O-C_2H_5$, $-O-C_3H_7$,$-(CH_2)-O-CH_3$, $-(CH_2)-O-C_2H_5$, $-NH_2$, $-N(CH_3)_2$, $-N(C_2H_5)_2$, $-NH-CH_3$, $-NH-C_2H_5$, $-NO_2$,$-CF_3$, $-O-CF_3$, $-S-CF_3$, -SH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-NH-S(=O)_2-CH_3$, -C(=O)-OH, -C(=O)-H; $-C(=O)-CH_3$, -C (=O)-$C_2H_5$,$-C(=O)-N(CH_3)_2$, $-C(=O)-NH-CH_3$, $-C(=O)-NH_2$, $-NH-C(=O)-CH_3$, $-NH-C(=O)-C_2H_5$,$-C(=O)-O-CH_3$, -C (=O)-O-$C_2H_5$, $-C(=O)-O-C(CH_3)_3$ und Phenyl, wobei der Phenyl-Rest mit 1, 2, 3, 4 oder 5, bevorzugt 1, 2 oder 3, Substituenten unabhängig voneinander ausgewählt aus der Gruppe bestehend aus F, Cl, Br, I, -CN, $-CF_3$, -OH, $-NH_2$, $-O-CF_3$, -SH, $-O-CH_3$, $-O-C_2H_5$, $-O-C_3H_7$, Methyl, Ethyl, n-Propyl, Isopropyl, n-Butyl, 2-Butyl, Isobutyl, tert-Butyl, Ethenyl, Allyl, Ethinyl, Propinyl, $-C{\equiv}C-Si(CH_3)_3$,$-C{\equiv}C-Si(C_2H_5)_3$, $-C(=O)-O-C_{1-5}$-Alkyl und $-C(=O)-CF_3$ substituiert sein kann.

Der Begriff "Aryl" bedeutet im Sinne der vorliegenden Erfindungen einen mono- oder polyzyklischen, bevorzugt einen mono- oder bizyklischen, aromatischen Kohlenwasserstoff-Rest mit bevorzugt 6, 10 oder 14 C-Atomen. Ein Aryl-Rest kann unsubstituiert oder einfach substituiert oder mehrfach gleich oder verschieden substituiert sein. Als geeignete Aryl-Reste seien beispielsweise Phenyl-, 1-Naphthyl, 2-Naphthyl und Anthracenyl genannt. Besonders bevorzugt ist ein Aryl-Rest ein Phenyl-Rest.

Der Begriff "Heteroaryl" bedeutet im Sinne der vorliegenden Erfindung einen monozyklischen oder polyzyklischen, bevorzugt einen mono-, bi- oder trizyklischen, aromatischen Kohlenwasserstoff-Rest mit bevorzugt 5, 6, 7, 8, 9, 10, 11, 12, 13 oder 14 C-Atomen, besonders bevorzugt mit 5, 6, 9, 10, 13 oder 14 C-Atomen, ganz besonders bevorzugt mit 5 oder 6 C-Atomen, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroaryl-Reste können bevorzugt 1, 2, 3, 4 oder 5, besonders bevorzugt 1, 2 oder 3, Heteroatom(e) unabhängig voneinander ausgewählt aus der Gruppe bestehend aus Sauerstoff, Schwefel und Stickstoff (NH) als Ringglied(er) aufweisen. Ein Heteroaryl-Rest kann unsubstituiert oder einfach substituiert oder mehrfach gleich oder verschieden substituiert sein.

Als geeignete Heteroaryl-Reste seien beispielsweise Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Pyrimidinyl, Indazolyl, Chinoxalinyl, Chinazolinyl, Chinolinyl, Naphthridinyl und Isochinolinyl genannt.

Aryl- oder Heteroaryl-Reste können im Sinne der vorliegenden Erfindung mit einem mono- bzw. bizyklischem Ringsystem kondensiert (anneliert) sein.

Beispielhaft für Aryl-Reste, die mit einem mono- bzw. bizyklischen Ringsystem kondensiert sind, seien (2,3)-Dihydrobenzo[b]thiophenyl, (2,3)-Dihydro-1H-indenyl, Indolinyl, (2,3)-Dihydrobenzofuranyl, (2,3)-Dihydrobenzo[d]oxazolyl, Benzo[d][1,3]dioxolyl, Benzo[d][1,3]oxathiolyl, Isoindolinyl, (1,3)-Diyhydroisobenzofuranyl, (1,3)-Dihydrobenzo[c] thiophenyl, (1,2,3,4)-Tetrahydronaphthyl, (1,2,3,4)-Tetrahydrochinolinyl, Chromanyl, Thiochromanyl, (1,2,3,4)-Tetrahydroisochinolinyl, (1,2,3,4)-Tetrahydrochinoxalinyl, (3,4)-Dihydro-2H-benzo[b][1,4]oxazinyl, (3,4)-Dihydro-2H-benzo[b] [1,4]thiazinyl, (2,3)-Dihydrobenzo[b][1,4]dioxinyl, (2,3)-Dihydrobenzo[b][1,4]oxathiinyl, (6,7,8,9)-Tetrahydro-5H-benzo [7]annulenyl, (2,3.4,5)-Tetrahydro-1H-benzo[b]azepinyl und (2,3,4,5)-Tetrahydro-1H-benzo[c]azepinyl genannt. Falls nicht anders angegeben, können, sofern einer oder mehrere der Substituenten für einen Aryl- oder Heteroaryl-Rest stehen oder einen Aryl- oder Heteroaryl-Rest aufweisen, der einfach oder mehrfach substituiert ist, diese Aryl- oder Heteroaryl-Reste mit 1, 2, 3, 4 oder 5, besonders bevorzugt mit ggf. 1, 2 oder 3, Substituenten unabhängig voneinander ausgewählt aus der Gruppe bestehend aus F, Cl, Br, I, -CN, $-NO_2$, -OH, -SH, $-NH_2$, -C(=O)-OH, $-C_{1-5}$-Alkyl, $-(CH_2)-O-C_{1-5}$-Alkyl,$-C_{2-5}$-Alkenyl, $-C_{2-5}$-Alkinyl, $-C{\equiv}C-Si(CH_3)_3$, $-C{\equiv}C-Si(C_2H_5)_3$, $-S-C_{1-5}$-Alkyl, -S-Phenyl, - $S-CH_2$-Phenyl, $-O-C_{1-5}$-Alkyl, -O-Phenyl, $-O-CH_2$-Phenyl, $-CF_3$, $-CHF_2$, $-CH_2F$, $-0-CF_3$, $-O-CHF_2$, $-O-CH_2F$, $-C(=O)-CF_3$, $-S-CF_3$, $-S-CHF_2$,

$-S-CH_2F$, $-S(=O)_2$-Phenyl,$-S(=O)_2-C_{1-5}$-Alkyl, $-S(=O)-C_{1-5}$-Alkyl, $-NH-C_{1-5}$-Alkyl, $N(C_{1-5}Alkyl)_2$, $-C(=O)-O-C_{1-5}$-Alkyl, -C (=O)-O-Phenyl, -C(=O)-H; $-C(=O)-C_{1-5}$-Alkyl, $-CH_2-O-C(=O)$-Phenyl, $-O-C(=O)-C_{1-5}$-Alkyl, -O-C(=O)-Phenyl, -NH-S $(=O)_2-C_{1-5}$-Alkyl, $-NH-C(=O)-C_{1-5}$-Alkyl,$-C(=O)-NH_2$, $-C(=O)-NH-C_{1-5}$-Alkyl. $-C(=O)-N(C_{1-5}\text{-Alkyl})_2$, $-C(=O)-N(C_{1-5}$-Alkyl)(Phenyl), -C(=O)-NH-Phenyl, Cyclopropyl, Cyclobutyl, Cyclopentyl, Cyclohexyl, Pyrazolyl, Phenyl, Furyl (Furanyl), Thiazolyl, Thiadiazolyl, Thiophenyl (Thienyl), Benzyl und Phenethyl substituiert sein, wobei die vorstehend genannten $C_{1-5}$-Alkyl-Reste jeweils linear oder verzweigt sein können und die zyklischen Substituenten bzw. die zyklischen Reste dieser Substituenten selbst mit ggf. 1, 2, 3, 4 oder 5, bevorzugt mit ggf. 1, 2, 3 oder 4, Substituenten unabhängig voneinander ausgewählt aus der Gruppe bestehend aus F, Cl, Br, I, -CN, $-NO_2$, -OH, -SH, $-NH_2$, -C(=O)-OH,$-C_{1-5}$-Alkyl, $-(CH_2)-O-C_{1-5}$-Alkyl, $-C_{2-5}$-Alkenyl, $-C_{2-5}$-Alkinyl, $-C{\equiv}C-Si(CH_3)_3$, $-C{=}C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ und $-S-CH_2F$ substituiert sein können.

Besonders bevorzugt können die Substituenten, jeweils unabhängig voneinander ausgewählt werden aus der Gruppe bestehend aus F, Cl, Br, I, -CN, $-NO_2$, -OH, -SH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, $-C{\equiv}C-Si(CH_3)_3$, $-C{\equiv}C-Si(C_2H_5)_3$, $-CH_2-O-CH_3$, $-CH_2-O-C_2H_5$, -OH, -SH, $-NH_2$, -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $- CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $- S(=O)_2$-Phenyl, Pyrazolyl, Phenyl, $-N(CH_3)_2$, $-N(C_2H_5)_2$, $-NH-CH_3$, $-NH-C_2H_5$, $-CH_2-O-C(=O)$-Phenyl, -NH-S $(=O)_2-CH_3$, $-C(=O)-O-CH_3$, $-C(=O)-O-C_2H_5$, $-C(=O)-O-C(CH_3)_3$, - C(=O)-H, $-C(=O)-CH_3$, $-C(=O)-C_2H_5$, $-NH-C(=O)-CH_3$, $-NH-C(=O)-C_2H_5$, -O-C(=O)-Phenyl, $-C(=O)-NH_2$; $-C(=O)-NH-CH_3$, $-C(=O)-N(CH_3)_2$, $-C(=O)-O-CH(CH_3)_2$, -C $(=O)-O-(CH2)_3-CH_3$, -C(=O)-O-Phenyl, $-O-C(=O)-CH_3$, $-O-C(=O)-C_2H_5$, $-C(=O)-NH-C_2H_5$, $- C(=O)-NH-C(CH_3)_3$, -C $(=O)-N(C_2H_5)_2$, -C(=O)-NH-Phenyl, $-C(=O)-N(CH_3)$-Phenyl, $- C(=O)-N(C_2H_5)$-Phenyl, Cyclopropyl, Cyclobutyl, Cyclopentyl, Cyclohexyl, Phenyl, Furyl (Furanyl), Thiadiazolyl, Thiophenyl (Thienyl) und Benzyl, wobei die zyklischen Substituenten bzw. die zyklischen Reste dieser Substituenten selbst jeweils mit ggf. 1, 2, 3, 4, oder 5, bevorzugt mit ggf. 1, 2, 3 oder 4, Substituenten unabhängig voneinander ausgewählt aus der Gruppe bestehend aus F, Cl, Br, I, -CN, $-NO_2$, -OH, -SH, $-NH_2$, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, $-C{\equiv}C-Si(CH_3)_3$, $- C{\equiv}C-Si(C_2H_5)_3$, $-CH_2-O-CH_3$, $-CH_2-O-C_2H_5$, $-S-CH_3$, $-S-C_2H_5$, -S $(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $- CHF_2$, $-CH_2F$, $-O-CF_3$, $-O-CHF_2$, $-O-CH_2F$, $-C(=O)-CF_3$, $-S-CF_3$, $-S-CHF_2$ und $-S-CH_2F$ substituiert sein können.

Ganz besonders bevorzugt kann ein substituierter Aryl-Rest aus der Gruppe bestehend aus 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyano-phenyl, 4-Cyano-phenyl, 2-Hydroxy-phenyl, 3-Hydroxy-phenyl, 4-Hydroxy-phenyl, 2-Amino-phenyl, 3-Amino-phenyl, 4-Amino-phenyl, 2-Dimethylamino-phenyl, 3-Dimethylamino-phenyl, 4-Dimethylamino-phenyl, 2-Methylamino-phenyl, 3-Methylamino-phenyl, 4-Methylamino-phenyl, 2-Acetyl-phenyl, 3-Acetyl-phenyl, 4-Acetyl-phenyl, 2-Methylsulfinyl-phenyl, 3-Methylsulfinyl-phenyl, 4-Methylsulfinyl-phenyl, 2-Methylsulfonyl-phenyl, 3-Methylsulfonyl-phenyl, 4-Methylsulfonyl-phenyl, 2-Methoxy-phenyl, 3-Methoxy-phenyl, 4-Methoxy-phenyl, 2-Chlor-phenyl, 3-Chlor-phenyl, 4-Chlor-phenyl, 2-Ethoxy-phenyl, 3-Ethoxy-phenyl, 4-Ethoxyphenyl, 2-Trifluormethyl-phenyl, 3-Trifluormethyl-phenyl, 4-Trifluormethyl-phenyl, 2-Difluormethyl-phenyl, 3-Difluormethyl-phenyl, 4-Difluormethyl-phenyl, 2-Fluormethyl-phenyl, 3-Fluormethyl-phenyl, 4-Fluormethyl-phenyl, 2-Nitro-phenyl, 3-Nitro-phenyl, 4-Nitro-phenyl, 2-Ethyl-phenyl, 3-Ethyl-phenyl, 4-Ethyl-phenyl, 2-Propyl-phenyl, 3-Propyl-phenyl, 4-Propyl-phenyl, 2-Isopropyl-phenyl, 3-Isopropyl-phenyl, 4-Isopropyl-phenyl, 2-tert-Butyl-phenyl, 3-tert-Butyl-phenyl, 4-tert-Butyl-phenyl, 2-Carboxyphenyl, 3-Carboxy-phenyl, 4-Carboxyphenyl, 2-Ethenyl-phenyl, 3-Ethenyl-phenyl, 4-Ethenyl-phenyl, 2-Ethinyl-phenyl, 3-Ethinyl-phenyl, 4-Ethinyl-phenyl, 2-Allyl-phenyl, 3-Allyl-phenyl, 4-Allyl-phenyl, 2-Trimethylsilanylethinyl-phenyl, 3-Trimethylsilanylethinyl-phenyl, 4-Trimethylsilanylethinyl-phenyl, 2-Formyl-phenyl, 3-Formyl-phenyl, 4-Formyl-phenyl, 2-Acetamino-phenyl, 3-Acetamino-phenyl, 4-Acetamino-phenyl, 2-Dimethylaminocarbonyl-phenyl, 3-Dimethylaminocarbonyl-phenyl, 4-Dimethylaminocarbonyl-phenyl, 2-Methoxymethyl-phenyl, 3-Methoxymethyl-phenyl, 4-Methoxymethyl-phenyl, 2-Ethoxymethyl-phenyl, 3-Ethoxymethyl-phenyl, 4-Ethoxymethyl-phenyl, 2-Aminocarbonyl-phenyl, 3-Aminocarbonyl-phenyl, 4-Aminocarbonyl-phenyl, 2-Methylaminocarbonyl-phenyl, 3-Methylaminocarbonyl-phenyl, 4-Methylaminocarbonyl-phenyl, 2-Carboxymethylester-phenyl, 3-Carboxymethylester-phenyl, 4-Carboxymethylester-phenyl, 2-Carboxyethylester-phenyl, 3-Carboxyethylester-phenyl, 4-Carboxyethylester-phenyl, 2-Carboxy-tert-butylester-phenyl, 3-Carboxy-tert-butylester-phenyl, 4-Carboxy-tert-butylester-phenyl, 2-Methylmercapto-phenyl, 3-Methylmercapto-phenyl, 4-Methylmercapto-phenyl, 2-Ethylmercapto-phenyl, 3-Ethylmercapto-phenyl, 4-Ethylmercaptophenyl, 2-Biphenyl, 3-Biphenyl, 4-Biphenyl, 2-Bromphenyl, 3-Bromphenyl, 4-Bromphenyl, 2-Iod-phenyl, 3-Iodphenyl, 4-Iodphenyl, 2-Trifluormethoxy-phenyl, 3-Trifluormethoxy-phenyl, 4-Trifluormethoxy-phenyl, 2-Fluor-3-trifluormethylphenyl, 2-Fluor-4-methyl-phenyl, (2,3)-Difluorphenyl, (2,3)-Dimethyl-phenyl, (2,3)-Dichlorphenyl, 3-Fluor-2-trifluormethylphenyl, (2,4)-Dichlor-phenyl, (2,4)-Difluorphenyl, 4-Fluor-2-trifluormethyl-phenyl, (2,4)-Dimethoxyphenyl, 2-Chlor-4-fluor-phenyl, 2-Chlor-4-nitro-phenyl, 2-Chlor-4-methyl-phenyl, 2-Chlor-5-trifluormethyl-phenyl, 2-Chlor-5-methoxy-phenyl, 2-Brom-5-trifluormethyl-phenyl, 2-Brom-5-methoxy-phenyl, (2,4)-Dibrom-phenyl, (2,4)-Dimethyl-phenyl, 2-Fluor-4-trifluormethyl-phenyl, (2,5)-Difluor-phenyl, 2-Fluor-5-trifluormethyl-phenyl, 5-Fluor-2-trifluormethyl-phenyl, 5-Chlor-2-trifluormethyl-phenyl, 5-Brom-2-trifluormethyl-phenyl, (2,5)-Dimethoxy-phenyl, (2,5)-Bis-trifluormethyl-phenyl, (2,5)-Dichlor-phenyl, (2,5)-Dibrom-phenyl, 2-Methoxy-5-nitro-phenyl, 2-Fluor-6-trifluormethyl-phenyl, (2,6)-Dimethoxy-phenyl, (2,6)-Dimethyl-phenyl, (2,6)-Dichlorphenyl, 2-Chlor-6-fluor-phenyl, 2-Brom-6-chlor-phenyl, 2-Brom-6-fluor-phenyl, (2,6)-Difluor-phenyl, (2,6)-Difluor-3-methyl-phenyl, (2,6)-Dibrom-phenyl, (2,6)-Dichlorphenyl, 3-Chlor-2-fluor-phenyl, 3-Chlor-5-methyl-phenyl, (3,4)-Dichlorphenyl, (3,4)-Dimethyl-phenyl, 3-Methyl-4-methoxy-phenyl, 4-Chlor-3-nitro-phenyl, (3,4)-Dimethoxy-phenyl, 4-Fluor-3-trifluormethylphenyl, 3-Fluor-4-trifluormethyl-phenyl, (3,4)-Difluor-phenyl, 3-Cyano-4-fluor-phenyl, 3-Cyano-4-methyl-phenyl, 3-Cyano-4-methoxy-phenyl, 3-Brom-4-fluor-phenyl, 3-Brom-4-methyl-phenyl, 3-Brom-4-methoxy-phenyl, 4-Chlor-2-fluor-phenyl, 4-Chlor-3-trifluormethyl, 4-Brom-3-methyl-phenyl, 4-Brom-5-methyl-phenyl, 3-Chlor-4-fluor-phenyl, 4-Fluor-3-nitro-phenyl, 4-Brom-3-nitro-phenyl, (3,4)-Dibrom-phenyl; 4-Chlor-3-methyl-phenyl, 4-Brom-3-methyl-phenyl, 4-Fluor-3-methyl-phenyl, 3-Fluor-4-methyl-phenyl, 3-Fluor-5-methyl-phenyl, 2-Fluor-3-methyl-phenyl, 4-Methyl-3-nitro-phenyl, (3,5)-Dimethoxy-phenyl, (3,5)-Dimethylphenyl, (3,5)-Bis-trifluormethyl-phenyl, (3,5)-Difluor-phenyl, (3,5)-Dinitro-phenyl, (3,5)-Dichlor-phenyl, 3-Fluor-5-trifluormethyl-phenyl, 5-Fluor-3-trifluormethyl-phenyl, (3,5)-Dibrom-phenyl, 5-Chlor-4-fluor-phenyl, 5-Chlor-4-fluor-phenyl, 5-Brom-4-methylphenyl, (2,3,4)-Trifluorphenyl, (2,3,4)-Trichlorphenyl, (2,3,6)-Trifluor-phenyl, 5-Chlor-2-methoxy-phenyl, (2,3)-Difluor-4-methyl, (2,4,5)-Trifluor-phenyl, (2,4,5)-Trichlorphenyl, (2,4)-Dichlor-5-fluor-phenyl, (2,4,6)-Trichlor-phenyl, (2,4,6)-Trimethylphenyl, (2,4,6)-Trifluor-phenyl, (2,4,6)-Trimethoxy-phenyl, (3,4,5)-Trimethoxy-phenyl, (2,3,4,5)-Tetrafluor-phenyl, 4-Methoxy-(2,3,6)-trimethyl-phenyl, 4-Methoxy-(2,3,6)-trimethyl-phenyl, 4-Chlor-2,5-dimethyl-phenyl, 2-Chlor-6-fluor-3-methyl-phenyl, 6-Chlor-2-fluor-3-methyl-, (2,4,6)-Trimethylphenyl und (2,3,4,5,6)-Pentafluor-phenyl ausgewählt werden.

Ganz besonders bevorzugt kann ein substituierter Heteroaryl-Rest aus der Gruppe bestehend aus 3-Methyl-pyrid-2-yl, 4-Methyl-pyrid-2-yl, 5-Methyl-pyrid-2-yl, 6-Methylpyrid-2-yl, 2-Methyl-pyrid-3-yl, 4-Methyl-pyrid-3-yl, 5-Methyl-pyrid-3-yl, 6-Methylpyrid-3-yl, 2-Methyl-pyrid-4-yl, 3-Methyl-pyrid-4-yl, 3-Fluor-pyrid-2-yl, 4-Fluor-pyrid-2-yl, 5-Fluor-pyrid-2-yl, 6-Fluor-pyrid-2-yl, 3-Chlor-pyrid-2-yl, 4-Chlor-pyrid-2-yl, 5-Chlorpyrid-2-yl, 6-Chlor-pyrid-2-yl, 3-Trifluormethyl-pyrid-2-yl, 4-Trifluormethyl-pyrid-2-yl, 5-Trifluormethyl-pyrid-2-yl, 6-Trifluormethyl-pyrid-2-yl, 3-Methoxy-pyrid-2-yl, 4-Methoxypyrid-2-yl, 5-Methoxy-pyrid-2-yl, 6-Methoxy-pyrid-2-yl, 4-Methyl-thiazol-2-yl, 5-Methylthiazol-2-yl, 4-Trifluormethyl-thiazol-2-yl, 5-Trifluormethyl-thiazol-2-yl, 4-Chlor-thiazol-2-yl, 5-Chlor-thiazol-2-yl, 4-Brom-thiazol-2-yl, 5-Brom-thiazol-2-yl, 4-Fluor-thiazol-2-yl, 5-Fluor-thiazol-2-yl, 4-Cyano-thiazol-2-yl, 5-Cyano-thiazol-2-yl, 4-Methoxy-thiazol-2-yl, 5-Methoxy-thiazol-2-yl, 4-Methyl-oxazol-2-yl, 5-Methyl-oxazol-2-yl, 4-Trifluormethyl-oxazol-2-yl, 5-Trifluormethyl-oxazol-2-yl, 4-Chlor-oxazol-2-yl, 5-Chloroxazol-2-yl, 4-Brom-oxazol-2-yl, 5-Brom-oxazol-2-yl, 4-Fluor-oxazol-2-yl, 5-Fluoroxazol-2-yl, 4-Cyano-oxazol-2-yl, 5-Cyano-oxazol-2-yl, 4-Methoxy-oxazol-2-yl, 5-Methoxy-oxazol-2-yl, 2-Methyl-(1,2,4)-thiadiazol-5-yl, 2-Trifluormethyl-(1,2,4)-thiadiazol-5-yl, 2-Chlor-(1,2,4)-thiadiazol-5-yl, 2-Fluor-(1,2,4)-thiadiazol-5-yl, 2-Methoxy-(1,2,4)-thiadiazol-5-yl, 2-Cyano-(1,2,4)-thiadiazol-5-yl, 2-Methyl-(1,2,4)-oxadiazol-5-yl, 2-Trifluormethyl-(1,2,4)-oxadiazol-5-yl, 2-Chlor-(1,2,4)-oxadiazol-5-yl, 2-Fluor-(1,2,4)-oxadiazol-5-yl, 2-Methoxy-(1,2,4)-oxadiazol-5-yl und 2-Cyano-(1,2,4)-oxadiazol-5-yl ausgewählt werden.

Der Begriff "Alkylen" umfaßt im Sinne der vorliegenden Erfindung acyclische gesättigte Kohlenwasserstoffketten, die einen Aryl-, Heteroaryl-, Cycloalkyl-, Heterocyloalkyl-, Cycloalkenyl- oder Heterocycloalkenyl-Rest mit den Verbindungen der allgemeinen Formel I bzw. mit einem anderen Substituenten verbinden. AlkylenKetten können verzweigt oder geradkettig sowie unsubstituiert oder wenigstens einfach substituiert sein mit wie im Fall von $C_{1-12}$-Alkylen 1 bis 12 (d. h. 1, 2, 3, 4, 5, 6, 7, 8, 9, 10, 11 oder 12) C-Atomen, mit wie im Fall von $C_{1-6}$-Alkylen 1 bis 6 (d. h. 1, 2, 3, 4, 5 oder 6) C-Atomen bzw. mit wie im Fall von $C_{1-3}$-Alkylen 1 bis 3 (d. h. 1, 2 oder 3) C-Atomen. Beispielhaft seien $C_{1-6}$-Alkylen-Gruppen wie $-(CH_2)-$, $-(CH_2)_2-$, - $C(H)(CH_3)-$, $-(CH_2)_3-$, $-(CH_2)_4-$, $-(CH_2)_5-$, $-(CH_3)_2-$, $-(CH)(CH_3)-$, $-C(H)(C(H)(CH_3)_2)-$ und $C(C_2H_5)(H)-$ genannt. Als geeignete $C_{1-3}$-Alkylen-Gruppe seien beispielhaft - $(CH_2)-$, $-(CH_2)_2-$ und $-(CH_2)_3-$ genannt.

Der Begriff "Alkenylen" umfaßt im Sinne der vorliegenden Erfindung azyklische ungesättigte Kohlenwasserstoffketten, die einen Aryl-, Heteroaryl-, Cycloalkyl-, Heterocyloalkyl-, Cycloalkenyl- oder Heterocycloalkenyl-Rest mit den Verbindungen der allgemeinen Formel I bzw. mit einem anderen Substituenten verbinden. Alkenylen-Ketten weisen wenigstens eine Doppelbindung, bevorzugt 1, 2 oder 3 Doppelbindungen, auf und können verzweigt oder geradkettig sowie unsubstituiert oder wenigstens einfach substituiert sein mit wie im Fall von $C_{2-12}$-Alkenylen 2 bis 12 (d. h. 2, 3, 4, 5, 6, 7, 8, 9, 10, 11 oder 12) C-Atomen, mit wie im Fall von $C_{2-6}$-Alkenylen 2 bis 6 (d. h. 2, 3, 4, 5 oder 6) C-Atomen bzw. mit wie im Fall von $C_{2-3}$-Alkenylen 2 bis 3 (d. h. 2 oder 3) C-Atomen. Beispielhaft seien $C_{2-3}$-Alkenylen-Gruppen wie $-CH=CH-$ und $-CH_2-CH=CH-$ genannt.

Der Begriff "Alkinylen" umfaßt im Sinne der vorliegenden Erfindung azyklische ungesättigte Kohlenwasserstoffketten, die einen Aryl-, Heteroaryl-, Cycloalkyl-, Heterocyloalkyl-, Cycloalkenyl- oder Heterocycloalkenyl-Rest mit den Verbindungen der allgemeinen Formel I bzw. mit einem anderen Substituenten verbinden. Alkinylen-Ketten weisen wenigstens eine Dreifachbindung, bevorzugt 1 oder 2 Dreifachbindungen, auf und können verzweigt oder geradkettig sowie unsubstituiert oder wenigstens einfach substituiert sein mit wie im Fall von $C_{2-12}$-Alkinylen 2 bis 12 (d. h. 2, 3, 4, 5, 6, 7, 8, 9, 10, 11 oder 12) C-Atomen, mit wie im Fall von $C_{2-6}$-Alkinylen 2 bis 6 (d. h. 2, 3, 4, 5 oder 6) C-Atomen bzw. mit wie im Fall von $C_{2-3}$-Alkinylen 2 bis 3 (d. h. 2 oder 3) C-Atomen. Beispielhaft seien $C_{2-3}$-Alkinylen-Gruppen wie $-C\equiv C-$ und $-CH_2-C\equiv C-$ genannt.

Der Begriff "Heteroalkylen" bezeichnet eine wie vorstehend beschriebenen AlkylenKette, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkylen-Gruppen können bevorzugt 1, 2 oder 3 Heteroatom (e), besonders bevorzugt ein Heteroatom, ausgewählt aus der Gruppe bestehend aus Sauerstoff, Schwefel und Stickstoff (NH) als Kettenglied(er) aufweisen. Heteroalkylen-Gruppen können bevorzugt 2- bis 12-gliedrig, besonders bevorzugt 2- bis 6-gliedrig, ganz besonders bevorzugt 2- oder 3-gliedrig, sein.

Beispielhaft seien Heteroalkylen-Gruppen wie $-(CH_2)-O-$, $-(CH_2)_2-O-$, $-(CH_2)_3-O-$,$-(CH_2)_4-O-$, $-O-(CH_2)-$, $-O-(CH_2)_2-$, $-O-(CH_2)_3-$, $-O-(CH_2)_4-$, $-C(C_2H_5)(H)-O-$, $-O-C(C_2H_5)(H)-$, $-CH_2-O-CH_2-$, $-CH_2-S-CH_2-$, $-CH_2-NH-CH_2-$, $-CH_2-NH-$ und $-CH_2-CH_2-NH-CH_2-CH_2$ genannt.

Der Begriff "Heteroalkenylen" bezeichnet eine wie vorstehend beschriebene Alkenylen-Kette, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkenylen-Gruppen können bevorzugt 1, 2 oder 3 Heteroatom(e), besonders bevorzugt 1 Heteroatom, ausgewählt aus der Gruppe bestehend aus Sauerstoff, Schwefel und Stickstoff (NH) als Kettenglied(er) aufweisen. Heteroalkenylen-Gruppen können bevorzugt 2- bis 12-gliedrig, besonders bevorzugt 2- bis 6-gliedrig, ganz besonders bevorzugt 2- oder 3-gliedrig, sein. Beispielhaft seien Heteroalkenylen-Gruppen wie -CH=CH-NH-,-CH=CH-O- und -CH=CH-S- genannt.

Sofern einer oder mehrere der Substituenten für eine Alkylen-, Alkenylen-, Alkinylen-, Heteroalkylen- oder Heteroalkenylen-Gruppe stehen oder eine solche Gruppe aufweisen, die einfach oder mehrfach substituiert ist, ist diese mit 1, 2, 3, 4 oder 5, besonders bevorzugt mit ggf. 1, 2 oder 3, Substituenten unabhängig voneinander ausgewählt aus der Gruppe bestehend aus Phenyl, F, Cl, Br, I, $-NO_2$,-CN, -OH, -O-Phenyl, $-O-CH_2$-Phenyl, -SH, -S-Phenyl, $-S-CH_2$-Phenyl, $-NH_2$, $-N(C_{1-5}\text{-Alkyl})_2$, -NH-Phenyl, $-N(C_{1-5}$-Alkyl)(Phenyl), $-N(C_{1-5}$-Alkyl)($CH_2$-Phenyl), $-N(C_{1-5}$-Alkyl)($CH_2$-$CH_2$-Phenyl), -C(=O)-H, $-C(=O)-C_{1-5}$-Alkyl, -C(=O)-Phenyl, $-C(=S)-C_{1-5}$-Alkyl, -C(=S)-Phenyl, -C(=O)-OH, -C(=O)-O-$C_{1-5}$-Alkyl, -C(=O)-O-Phenyl, $-C(=O)-NH_2$, -C(=O)-NH-$C_{1-5}$-Alkyl, $-C(=O)-N(C_{1-5}\text{-Alkyl})_2$, $-S(=O)-C_{1-5}$-Alkyl, -S(=O)-Phenyl,$-S(=O)_2-C_{1-5}$-Alkyl, $-S(=O)_2$-Phenyl, $-S(=O)_2-NH_2$ und $-SO_3H$ substituiert, wobei die vorstehend genannten $C_{1-5}$-Alkyl-Reste jeweils linear oder verzweigt sein können und die vorstehend genannten Phenyl-Reste mit 1, 2, 3, 4 oder 5, bevorzugt mit 1, 2, 3 oder 4, Substituenten unabhängig voneinander ausgewählt aus der Gruppe bestehend aus F, Cl, Br, I, -CN, $-NO_2$, -OH, -SH, $-NH_2$, -C(=O)-OH, $-C_{1-5}$-Alkyl,$-(CH_2)-O-C_{1-5}$-Alkyl, $-C_{2-5}$-Alkenyl, $-C_{2-5}$-Alkinyl, $-C{\equiv}C-Si(CH_3)_3$, $-C{\equiv}C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$,$-CHF_2$, $-CH_2F$, $-O-CF_3$, $-O-CHF_2$, $-O-CH_2F$, $-C(=O)-CF_3$, $-S-CF_3$, $-S-CHF_2$ und $-S-CH_2F$ substituiert sein können.

Besonders bevorzugt können Alkylen-, Alkenylen-, Alkinylen-, Heteroalkylen- oder Heteroalkenylen-Gruppen mit 1, 2 oder 3 Substituenten unabhängig voneinander ausgewählt werden aus der Gruppe bestehend aus Phenyl, F, Cl, Br, I, $-NO_2$, -CN,-OH, -O-Phenyl, -SH, -S-Phenyl, $-NH_2$, $-N(CH_3)_2$, $-N(C_2H_5)_2$ und $-N(CH_3)(C_2H_5)$ substituiert sein, wobei der Phenyl-Rest mit 1, 2, 3, 4 oder 5 Substituenten unabhängig voneinander ausgewählt aus der Gruppe bestehend aus F, Cl, Br, I,-OH, -SH, $-NO_2$, -CN, $-O-CH_3$, $-O-CF_3$ und $-O-C_2H_5$ substituiert sein kann.

Sofern Verbindungen der allgemeinen Formel I mehrere Substituenten ausgewählt aus der Gruppe bestehend aus $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ und $R^{27}$ mit der gleichen Bezeichnung aufweisen, kann jeder dieser Substituenten jeweils unabhängig von anderen Substituenten mit der gleichen Bezeichnung des Substituenten ausgewählt werden.

Beispielhaft kann der folgende Rest,

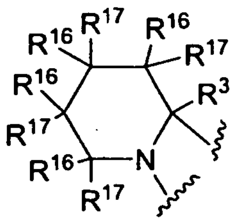

nach Auswahl der entsprechenden Substituenten für diesen Rest stehen

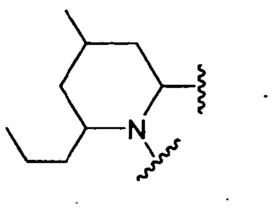

Bevorzugt sind Propiolsäureamide der vorstehend angegebenen allgemeinen Formel I, worin a, b und c, unabhängig voneinander, jeweils für 0 oder 1 stehen, wobei die Summe aus a, b und c gleich 1, 2 oder 3 ist; A für einen der folgenden Reste

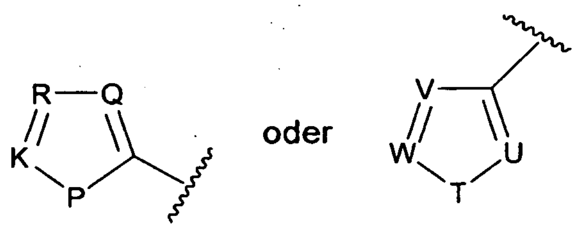

steht;

| | |
|---|---|
| Q und U | jeweils für $CR^{10}$ oder N stehen; |
| R und V | jeweils für $CR^{11}$ oder N stehen; |
| K und W | jeweils für $CR^{12}$ oder N stehen; |
| P und T | jeweils für O, S oder $NR^{13}$ stehen; |

mit der Maßgabe, dass Verbindungen ausgeschlossen sind, worin P für S steht, Q für N steht, R für $CR^{11}$ steht und K für $CR^{12}$ oder N steht;

$R^1$ und $R^8$, unabhängig voneinander, jeweils für H; -C(=O)-$R^{28}$; -C(=O)-O-$R^{29}$; - C(=O)-$NH_2$; -C(=O)-NH-$R^{30}$; -C (=O)-$NR^{31}R^{32}$; -S(=O)-$R^{33}$; -$S(=O)_2$-$R^{34}$; unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes - (Alkylen)-Cycloalkyl, -(Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes -(Heteroalkylen)-Cycloalkyl, -(Heteroalkenylen)-Cycloalkyl, -(Heteroalkylen)-Cycloalkenyl oder -(Heteroalkenylen)- Cycloalkenyl; unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl, - (Alkenylen)-Heterocycloalkenyl oder -(Alkinylen)-Heterocycloalkenyl; unsubstituiertes oder substituiertes -(Hetercalkylen)-Heterocycloalkyl, -(Heteroalkenylen)-Heterocycloalkyl, -(Heteroalkylen)-Heterocycloalkenyl oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, - (Alkenylen)-Aryl, -(Alkinylen)-Aryl, -(Heteroalkylen)-Aryl oder -(Heteroalkenylen)-Aryl; oder unsubstituiertes oder substituiertes -(Alkylen)-Heteroaryl, -(Alkenylen)-Heteroaryl, -(Alkinylen)-Heteroaryl, -(Heteroalkylen)-Heteroaryl oder - (Heteroalkenylen)-Heteroaryl stehen;

$R^2$, $R^3$, $R^4$, $R^5$, $R^6$, $R^7$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ und $R^{27}$, unabhängig voneinander, jeweils für H; F; Cl; Br; I; -$NO_2$; -CN; -$NH_2$; -OH; -SH;-C(=O)-OH; -C(=O)-H; -NH-C(=O)-H; -C(=O)-$R^{28}$; -C(=O)-O-$R^{29}$; -C(=O)-$NH_2$; -C(=O)-NH-$R^{30}$; -C(=O)-$NR^{31}R^{32}$; -S(=O)-$R^{33}$; -$S(=O)_2$-$R^{34}$; -NH-$R^{35}$; -$NR^{36}R^{37}$; -O-C(=0)-Rsa; -NH-C (=O)-$R^{39}$; -$NR^{40}$-C(=O)-$R^{41}$; -O-$R^{42}$; -S-$R^{43}$; -NH-C(=O)-NH-$R^{44}$; -NH-C(=S)-NH-$R^{45}$; -NH-$S(=O)_2$-$R^{46}$; -$NR^{47}$-S $(=O)_2$-$R^{48}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl;

unsubstituiertes oder wenigstens einfach substituiertes -(Alkylen)-Cycloalkyl, - (Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder wenigstens einfach substituiertes -(Heteroalkylen)-Cycloalkyl, -(Heteroalkenylen)-Cycloalkyl, - (Heteroalkylen)-Cycloalkenyl oder -(Heteroalkenylen)-Cycloalkenyl; unsubstituiertes oder wenigstens einfach substituiertes -(Alkylen)-Heterocycloalkyl, -(Alkenylen)-Heterocycloalkyl, -(Alkinylen)-Heterocycloalkyl, -(Alkylen)-Heterocycloalkenyl,-(Alkenylen)-Heterocycloalkenyl oder -(Alkinylen)-Heterocycloatkenyl; unsubstituiertes oder wenigstens einfach substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, - (Alkenylen)-Aryl, -(Alkinylen)-Aryl, -(Heteroalkylen)-Aryl oder -(Heteroalkenylen)-Aryl; oder unsubstituiertes oder wenigstens einfach substituiertes -(Alkylen)-Heteroaryl, - (Alkenylen)-Heteroaryl, -(Alkinylen)-Heteroaryl, -(Heteroalkylen)-Heteroaryl oder-(Heteroalkenylen)-Heteroaryl stehen;

oder $R^2$ und $R^3$ oder $R^4$ und $R^5$ oder $R^6$ und $R^7$ oder $R^{14}$ und $R^{15}$ oder $R^{16}$ und $R^{17}$ oder $R^{18}$ und $R^{19}$ oder $R^{20}$ und $R^{21}$

oder $R^{22}$ und $R^{23}$ oder $R^{24}$ und $R^{25}$ oder $R^{26}$ und $R^{27}$, unabhängig voneinander, zusammen jeweils für einen Rest ausgewählt aus der Gruppe bestehend aus einer Oxo-Gruppe (=O) und einer Thioxo-Gruppe (=S) stehen;
oder $R^1$ und $R^8$ zusammen mit der sie verbindenden $-N-CR^2R^3-(CR^4R^5)_b-(CR^6R^7)_c$-Gruppe einen Rest der allgemeinen Formel B,

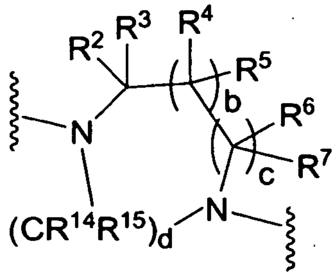

B,

bilden; wobei d für 1, 2 oder 3 steht und b für 0 oder 1 steht und c für 0 oder 1 steht;
oder $R^1$ und $R^2$ zusammen mit der sie verbindenden $-N-CR^3$-Gruppe einen Rest der allgemeinen Formel C,

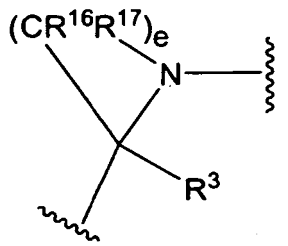

C,

bilden; wobei e für 1, 2 , 3 oder 4 steht und, in diesem Fall, b für 0 oder 1 steht und c für 0 steht;
oder $R^1$ und $R^4$ zusammen mit der sie verbindenden $-N-CR^2R^3-CR^5$-Gruppe einen Rest der allgemeinen Formel D,

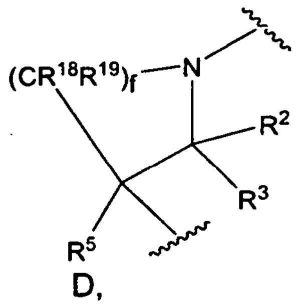

D,

bilden; wobei f für 1, 2, 3 oder 4 steht, und, in diesem Fall, c für 0 steht;
oder $R^1$ und $R^6$ zusammen mit der sie verbindenden $-N-CR^2R^3-CR^4R^5-CR^7$- Gruppe einen Rest der allgemeinen Formel E,

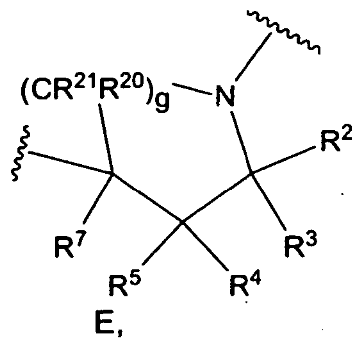

E,

bilden; wobei g für 1, 2 oder 3 steht;
oder $R^6$ und $R^8$ zusammen mit der sie verbindenden -N-$CR^7$-Gruppe einen Rest der allgemeinen Formel F

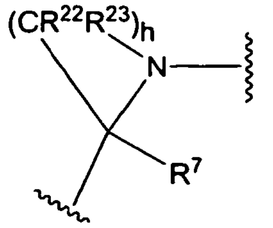

F,

bilden; wobei h für 1, 2 , 3 oder 4 steht und, in diesem Fall, b für 0 oder 1 steht und a für 0 steht;
oder $R^4$ und $R^8$ zusammen mit der sie verbindenden -N-$CR^6R^7$-$CR^5$-Gruppe einen Rest der allgemeinen Formel G,

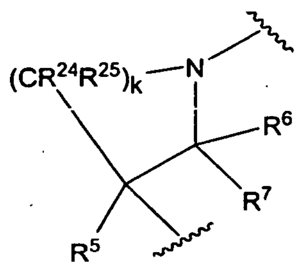

G,

bilden; wobei k für 1, 2, 3 oder 4 steht, und, in diesem, Fall a für 0 steht;
oder $R^2$ und $R^8$ zusammen mit der sie verbindenden -N-$CR^6R^7$-$CR^4R^5$-$CR^3$-Gruppe einen Rest der allgemeinen Formel H,

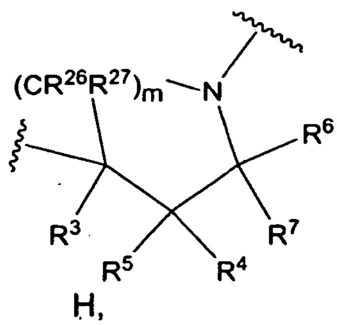

H,

bilden; wobei m für 1, 2 oder 3 steht;

$R^9$ für unsubstituiertes oder substituiertes Aryl oder unsubstituiertes oder substituiertes Heteroaryl steht;

$R^{10}$, $R^{11}$ und $R^{12}$, unabhängig voneinander, jeweils für H; F; Cl; Br; I; $-NO_2$; -CN;$-NH_2$; -OH; -SH; -C(=O)-OH; -C(=O)-H; -NH-C(=O)-H; -C(=O)-$R^{28}$; -C(=O)-O-$R^{29}$; - C(=O)-$NH_2$; -C(=O)-NH-$R^{30}$; -C(=O)-$NR^{31}R^{32}$; -S(=O)-$R^{33}$; -S(=O)$_2$-$R^{34}$; -NH-$R^{35}$; $NR^{36}R^{37}$; -O-C(=O)-$R^{38}$; -NH-C(=O)-$R^{39}$; -$NR^{40}$-C(=O)-$R^{41}$; -O-$R^{42}$; -S-$R^{43}$; -NH-C(=O)-NH-$R^{44}$; -NH-C (=S)-NH-$R^{45}$; -NH-S(=O)$_2$-$R^{46}$; -$NR^{47}$-S(=O)$_2$-$R^{48}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes -(Alkylen)-Cycloalkyl, -(Alkenylen)-Cycloalkyl, -(Alkinylen)-Cycloalkyl, - (Alkylen)-Cycloalkenyl, -(Alkenylen)-Cycloalkenyl oder -(Alkinylen)-Cycloalkenyl; unsubstituiertes oder wenigstens einfach substituiertes -(Heteroalkylen)-Cycloalkyl,-(Heteroalkenylen)-Cycloalkyl, -(Heteroalkylen)-Cycloalkenyl oder -(Heteroalkenylen)-Cycloalkenyl; unsubstituiertes oder wenigstens einfach substituiertes -(Alkylen)-Heterocycloalkyl, -(Alkenylen)-Heterocycloalkyl, -(Alkinylen)-Heterocycloalkyl, - (Alkylen)-Heterocycloalkenyl, -(Alkenylen)-Heterocycloalkenyl oder -(Alkinylen)-Heterocycloalkenyl; unsubstituiertes oder wenigstens einfach substituiertes - (Heteroalkylen)-Heterocycloalkyl, -(Heteroalkenylen)-Heterocycloalkyl,-(Heteroalkylen)-Heterocycloalkenyl oder -(Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder wenigstens einfach substituiertes -(Alkylen)-Heteroaryl, -(Alkenylen)-Heteroaryl, - (Alkinylen)-Heteroaryl, -(Heteroalkylen)-Heteroaryl oder -(Heteroalkenylen)-Heteroaryl stehen;

$R^{13}$ für H; -C(=O)-OH; -C(=O)-H; -C(=O)-$R^{28}$; -C(=O)-O-$R^{29}$; -C(=O)-$NH_2$; -C(=O)-NH-$R^{30}$; -C(=O)-$NR^31 1R^{32}$; -S (=O)-$R^{33}$; -S(=O)$_2$-$R^{34}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder-wenigstens einfach substituiertes Heterocycloalkyl oder Heterocycloalkenyl; unsubstituiertes oder wenigstens einfach substituiertes -(Alkylen)-Cycloalkyl, - (Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder wenigstens einfach substituiertes -(Heteroalkylen)-Cycloalkyl, -(Heteroalkenylen)-Cycloalkyl, - (Heteroalkylen)-Cycloalkenyl oder -(Heteroalkenylen)-Cycloalkenyl; unsubstituiertes oder wenigstens einfach substituiertes -(Alkylen)-Heterocycloalkyl, -(Alkenylen)-Heterocycloalkyl, -(Alkinylen)-Heterocycloalkyl, -(Alkylen)-Heterocycloalkenyl, - (Alkenylen)-Heterocycloalkenyl oder -(Alkinylen)-Heterocycloalkenyl; unsubstituiertes oder wenigstens einfach substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl,-(Alkenylen)-Aryl, -(Alkinylen)-Aryl, -(Heteroalkylen)-Aryl oder -(Heteroalkenylen)-Aryl; oder unsubstituiertes oder wenigstens einfach substituiertes -(Alkylen)-Heteroaryl,-(Alkenylen)-Heteroaryl, -(Alkinylen)-Heteroaryl, -(Heteroalkylen)-Heteroaryl oder-(Heteroalkenylen)-Heteroaryl steht;

und $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$ und $R^{48}$, unabhängig voneinander, jeweils für unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl, - (Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes - (Heteroalkylen)-Cycloalkyl, -(Heteroalkenylen)-Cycloalkyl, -(Heteroalkylen)-Cycloalkenyl oder -(Heteroalkenylen)-Cycloalkenyl; unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder substituiertes -(Heteroalkylen)-Heterocycloalkyl, -(Heteroalkenylen)-Heterocycloalkyl, -(Heteroalkylen)-Heterocycloalkenyl; oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, -(Heteroalkylen)-Aryl oder -(Heteroalkenylen)-Aryl; oder unsubstituiertes oder substituiertes -(Alkylen)-Heteroaryl, -(Alkenylen)-Heteroaryl, -(Alkinylen)-Heteroaryl, - (Heteroalkylen)-Heteroaryl oder -(Heteroalkenylen)-Heteroaryl stehen;

wobei

die vorstehend genannten Alkyl-Reste jeweils verzweigt oder geradkettig sind und 1, 2, 3, 4, 5, 6, 7, 8, 9, 10, 11 oder 12 Kohlenstoffatome als Kettenglieder aufweisen; die vorstehend genannten Alkenyl-Reste jeweils verzweigt oder geradkettig sind und 2, 3, 4, 5, 6, 7, 8, 9, 10, 11 oder 12 Kohlenstoffatome als Kettenglieder aufweisen; die vorstehend genannten Alkinyl-Reste jeweils verzweigt oder geradkettig sind und 2, 3, 4, 5, 6, 7, 8, 9, 10, 11 oder 12 Kohlenstoffatome als Kettenglieder aufweisen; die vorstehend genannten Heteroalkyl-Reste, Heteroalkenyl-Reste und Heteroalkinyl-Reste jeweils 2-, 3-, 4-, 5-, 6-, 7-, 8-, 9-, 10-, 11- oder 12-gliedrig sind; die vorstehend genannten Heteroalkyl-Reste, Heteroalkenyl-Reste und Heteroalkinyl-Reste jeweils ggf. 1, 2 oder 3 Heteroatom(e) unabhängig voneinander ausgewählt aus

der Gruppe bestehend aus Sauerstoff, Schwefel und Stickstoff als Kettenglied(er) aufweisen;
die vorstehend genannten Alkyl-Reste, Alkenyl-Reste, Alkinyl-Reste, Heteroalkyl-Reste, Heteroalkenyl-Reste und Heteroalkinyl-Reste jeweils mit ggf. 1, 2, 3, 4 oder 5 Substituenten unabhängig voneinander ausgewählt aus der Gruppe bestehend aus F, Cl, Br, I, $-NO_2$, -CN, -OH, -SH, $-NH_2$, $-N(C_{1-5}\text{-Alkyl})_2$, $-N(C_{1-5}$-Alkyl)(Phenyl), $-N(C_{1-5}$-Alkyl)($CH_2$-Phenyl), $-N(C_{1-5}$-Alkyl)($CH_2$-$CH_2$-Phenyl), -C(=O)-H, -C(=O)-$C_{1-5}$-Alkyl, - C(=O)-Phenyl, -C(=S)-$C_{1-5}$-Alkyl, -C(=S)-Phenyl, -C(=O)-OH, -C(=O)-O-$C_{1-5}$-Alkyl, - C(=O)-O-Phenyl, -C(=O)-$NH_2$, -C(=O)-NH-$C_{1-5}$-Alkyl, -C(=O)-$N(C_{1-5}\text{-Alkyl})_2$, -S (=O)-$C_{1-5}$-Alkyl, -S(=O)-Phenyl, $-S(=O)_2$-$C_{1-5}$-Alkyl, $-S(=O)_2$-Phenyl, $-S(=O)_2-NH_2$ und-$SO_3H$ substituiert sein können, wobei die Phenyl-Reste mit 1, 2, 3, 4 oder 5 Substituenten unabhängig voneinander ausgewählt aus der Gruppe bestehend aus F, Cl, Br, I, -CN, $-CF_3$, -OH, $-NH_2$, $-O-CF_3$, -SH, $-O-CH_3$, $-O-C_2H_5$, $-O-C_3H_7$, Methyl, Ethyl, n-Propyl, Isopropyl, n-Butyl, 2-Butyl, Isobutyl und tert-Butyl substituiert sein können;
die vorstehend genannten Cycloalkyl-Reste jeweils 3, 4, 5, 6, 7, 8 oder 9 Kohlenstoffatome als Ringglieder aufweisen;
die vorstehend genannten Cycloalkenyl-Reste jeweils 3, 4, 5, 6, 7, 8 oder 9 Kohlenstoffatome als Ringglieder aufweisen;
die vorstehend genannten Heterocycloalkyl-Reste jeweils 3-, 4-, 5-, 6-, 7-, 8- oder 9-gliedrig sind;
die vorstehend genannten Heterocycloalkenyl-Reste jeweils 4-, 5-, 6-, 7-, 8- oder 9-gliedrig sind;
die vorstehend genannten Heterocycloalkyl-Reste und Heterocycloalkenyl-Resten jeweils ggf. 1, 2 oder 3 Heteroatom (e) unabhängig voneinander ausgewählt aus der Gruppe bestehend aus Sauerstoff, Schwefel und Stickstoff (NH) als Ringglied(er) aufweisen;
die vorstehend genannten Cycloalkyl-Reste, Heterocycloalkyl-Reste, Cycloalkenyl-Reste oder Heterocycloalkenyl-Reste jeweils mit ggf. 1, 2, 3, 4 oder 5 Substituenten unabhängig voneinander, ausgewählt aus der Gruppe bestehend aus F, cl, Br, I,-CN, $-CF_3$, -OH, $-NH_2$, $-O-CF_3$, -SH, -O-$C_{1-5}$-Alkyl, -O-Phenyl, -O-$CH_2$-Phenyl, -($CH_2$)-O-$C_{1-5}$-Alkyl, -S-$C_{1-5}$-Alkyl, -S-Phenyl, -S-$CH_2$-Phenyl, -$C_{1-5}$-Alkyl, -$C_{2-5}$-Alkenyl, -$C_{2-5}$-Alkinyl, -C≡C-$Si(CH_3)_3$, -C≡C-$Si(C_2H_5)_3$, -C(=O)-O-$C_{1-5}$-Alkyl, -C(=O)-$CF_3$, $-S(=O)_2$-$C_{1-5}$-Alkyl, -S(=O)-$C_{1-5}$-Alkyl, $-S(=O)_2$-Phenyl, Oxo (=O), Thioxo (=S), $-N(C_{1-5}\text{-Alkyl})_2$, - N(H)($C_{1-5}$-Alkyl), $-NO_2$, $-S-CF_3$, -C(=O)-OH, -NH-C(=O)-$C_{1-5}$-Alkyl, -C(=O)-H, -C(=O)-$C_{1-5}$-Alkyl, -C(=O)-$NH_2$, -C (=O)-$N(C_{1-5}\text{-Alkyl})_2$, -C(=O)-N(H)($C_{1-5}$-Alkyl) und Phenyl substituiert sein können, wobei die Phenyl-Reste jeweils unsubstituiert oder mit 1, 2, 3, 4 oder 5 Substituenten unabhängig voneinander ausgewählt aus der Gruppe bestehend aus F, Cl, Br, I, -CN, $-CF_3$, -OH, $-NH_2$, $-O-CF_3$, -SH, -O-$C_{1-5}$-Alkyl, -O-Phenyl, -O-$CH_2$-Phenyl, -($CH_2$)-O-$C_{1-5}$-Alkyl, -S-$C_{1-5}$-Alkyl, -S-Phenyl, -S-$CH_2$-Phenyl, -$C_{1-5}$-Alkyl, -$C_{2-5}$-Alkenyl, -$C_{2-5}$-Alkinyl, -C≡C-$Si(CH_3)_3$, -C≡C-$Si(C_2H_5)_3$, -C (=O)-O-$C_{1-5}$-Alkyl und -C(=O)-$CF_3$ substituiert sein können, wobei die vorstehend genannten Phenyl-Reste bevorzugt mit 1, 2, 3, 4 oder 5 Substituenten unabhängig voneinander ausgewählt aus der Gruppe bestehend aus F, Cl, Br, I, -CN, $-CF_3$, -OH, $-NH_2$, $-0-CF_3$, -SH, $-O-CH_3$, $-O-C_2H_5$, $-O-C_3H_7$, Methyl, Ethyl, n-Propyl, Isopropyl, n-Butyl, 2-Butyl, Isobutyl und tert-Butyl substituiert sein können;
die vorstehend genannten Alkylen-Reste jeweils verzweigt oder geradkettig sind und 1, 2, 3, 4, 5, 6, 7, 8, 9, 10, 11 oder 12 Kohlenstoffatome als Kettenglieder aufweisen;
die vorstehend genannten Alkenylen-Reste jeweils verzweigt oder geradkettig sind und 2, 3, 4, 5, 6, 7, 8, 9, 10, 11 oder 12 Kohlenstoffatome als Kettenglieder aufweisen;
die vorstehend genannten Alkinylen-Reste jeweils verzweigt oder geradkettig sind und 2, 3, 4, 5, 6, 7, 8, 9, 10, 11 oder 12 Kohlenstoffatome als Kettenglieder aufweisen;
die vorstehend genannten Heteroalkylen-Reste und Heteroalkenylen-Reste jeweils 2-, 3-, 4-, 5-, 6-, 7-, 8-, 9-, 10-, 11- oder 1 2-gliedrig sind;
die vorstehend genannten Heteroalkylen- und Heteroalkenylen-Gruppen jeweils ggf. 1, 2 oder 3 Heteroatom(e) unabhängig voneinander ausgewählt aus der Gruppe bestehend aus Sauerstoff, Schwefel und Stickstoff (NH) als Kettenglied (er) aufweisen;
die vorstehend genannten Alkylen-, Alkenylen-, Alkinylen-, Heteroalkylen- oder Heteroalkenylen-Gruppe jeweils unsubstituiert oder mit ggf. 1, 2, 3, 4 oder 5 Substituenten unabhängig voneinander ausgewählt aus der Gruppe bestehend aus Phenyl, F, Cl, Br, I, $-NO_2$, -CN, -OH, -O-Phenyl, -O-$CH_2$-Phenyl, -SH, -Q-phenyl, -S-$CH_2$-Phenyl, $NH_2$, $-N(C_{1-5}\text{-Alkyl})_2$, -NH-Phenyl, $-N(C_{1-5}$-Alkyl)(Phenyl), $-N(C_{1-5}$-Alkyl)($CH_2$-Phenyl), $-N(C_{1-5}$-Alkyl)($CH_2$-$CH_2$-Phenyl), -C(=O)-H, -C (=O)-$C_{1-5}$-Alkyl, - C(=O)-Phenyl, -C(=S)-$C_{1-5}$-Alkyl, -C(=S)-Phenyl, -C(=O)-OH, -C(=O)-O-$C_{1-5}$-Alkyl, - C(=O)-O-Phenyl, -C(=O)-$NH_2$, -C(=O)-NH-$C_{1-5}$-Alkyl, -C(=O)-$N(C_{1-5}\text{-Alkyl})_2$, -S(=O)-$C_{1-5}$-Alkyl, -S(=O)-Phenyl, $-S(=O)_2$-$C_{1-5}$-Alkyl, -S (=O)$_2$Phenyl, $-S(=O)_2-NH_2$ und -$SO_3H$ substituiert sein können, wobei die Phenyl-Reste mit 1, 2, 3, 4 oder 5 Substituenten unabhängig voneinander ausgewählt aus der Gruppe bestehend aus F, Cl, Br, I, - CN, $-NO_2$, -OH, -SH, $-NH_2$, -C(=O)-OH, -$C_{1-5}$-Alkyl, -($CH_2$)-O-$C_{1-5}$-Alkyl, -$C_{2-5}$-Alkenyl, -$C_{2-5}$-Alkinyl, -C≡C-$Si(CH_3)_3$, -C≡C-$Si(C_2H_5)_3$, -S-$C_{1-5}$-Alkyl, -S-Phenyl, -S-$CH_2$-Phenyl, -O-$C_{1-5}$-Alkyl, -O-Phenyl, -O-$CH_2$-Phenyl, $-CF_3$, $-CHF_2$, $-CH_2F$, $-O-CF_3$, $-O-CHF_2$, $-O-CH_2F$, -C (=O)-$CF_3$, $-S-CF_3$, $-S-CHF_2$ und $-S-CH_2F$ substituiert sein können; die vorstehend genannten Aryl-Reste mono- oder bizyklisch sind und 6, 10 oder 14-Kohlenstoffatome aufweisen;
die vorstehend genannten Heteroaryl-Reste mono-, bi- oder trizyklisch und 5-, 6-, 7-, 8-, 9-, 10-, 11-, 12-, 13- oder 14-gliedrig sind;
die vorstehend genannten 5- bis 14-gliedriges Heteroaryl-Reste ggf. 1, 2, 3, 4 oder 5 Heteroatom(e) unabhängig voneinander ausgewählt aus der Gruppe bestehend aus Sauerstoff, Schwefel und Stickstoff (NH) als Ringglied(er) aufweisen;

und die vorstehend genannten Aryl-Reste und Heteroaryl-Reste jeweils mit ggf. 1, 2, 3, 4 oder 5 Substituenten unabhängig voneinander ausgewählt aus der Gruppe bestehend aus F, Cl, Br, I, -CN, $-NO_2$, -OH, -SH, $-NH_2$, -C(=O)-OH, $-C_{1-5}$-Alkyl,$-(CH_2)-O-C_{1-5}$-Alkyl, $-C_{2-5}$-Alkenyl, $-C_{2-5}$-Alkinyl, $-C{\equiv}C-Si(CH_3)_3$, $-C{\equiv}C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$,-CHF2, $-CH_2F$, $-O-CF_3$, $-O-CHF_2$, $-O-CH_2F$, $-C(=O)-CF_3$, $-S-CF_3$, $-S-CHF_2$, $-S-CH_2F$,$-S(=O)_2$-Phenyl, $-S(=O)_2-C_{1-5}$-Alkyl, $-S(=O)-C_{1-5}$-Alkyl, $-NH-C_{1-5}$-Alkyl, $N(C_{1-5}Alkyl)_2$,-C(=O)-O-$C_{1-5}$-Alkyl, -C(=O)-O-Phenyl, -C(=O)-H, $-C(=O)-C_{1-5}$-Alkyl, $-CH_2$-O-C(=O)-Phenyl, -O-C(=O)-Phenyl, -O-C(=O)-$C_{1-5}$-Alkyl, -NH-C(=O)-$C_{1-5}$-Alkyl, $-C(=O)-NH_2$,-C(=O)-NH-$C_{1-5}$-Alkyl, $-C(=O)-N(C_{1-5}\text{-Alkyl})_2$, -C(=O)-N($C_{1-5}$-Alkyl)(Phenyl), -C(=O)-NH-Phenyl, Cyclopropyl, Cyclobutyl, Cyclopentyl, Cyclohexyl, Pyrazolyl, Phenyl, Furyl (Furanyl), Thiazolyl, Thiadiazolyl, Thiophenyl (Thienyl) , Benzyl und Phenethyl substituiert sein können, wobei die zyklischen Substituenten bzw. die zyklischen Reste dieser Substituenten selbst mit ggf. 1, 2, 3, 4 oder 5, Substituenten unabhängig voneinander ausgewählt aus der Gruppe bestehend aus F, Cl, Br, I,-CN, $-NO_2$. -OH, -SH, $-NH_2$, -C(=O)-OH, $-C_{1-5}$-Alkyl, $-(CH_2)-O-C_{1-5}$-Alkyl,$-C_{2-5}$-Alkenyl,$-C_{2-5}$-Alkinyl,$-C{\equiv}C-Si(CH_3)_3$,$-C{\equiv}C-Si(C_2H_5)_3$,$-S-C_{1-5}$-Alkyl,-S-Phenyl,$-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ und $-S-CH_2F$ substituiert sein können;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind Propiolsäureamide der vorstehend angegebenen allgemeinen Formel I, worin A für einen Rest ausgewählt aus der Gruppe bestehend aus

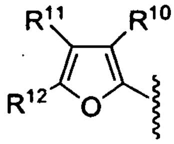 , 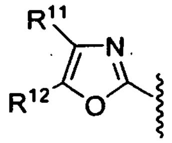 , 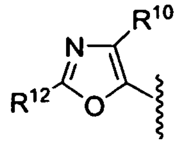 , 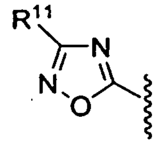 ,

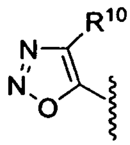 , 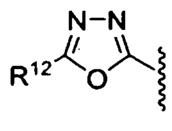 , 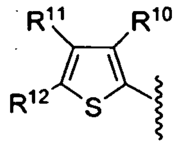 , 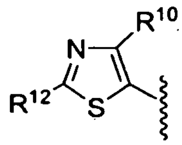 ,

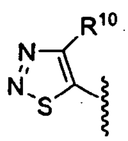 , 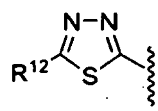 , 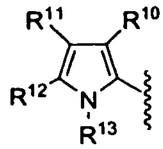 , 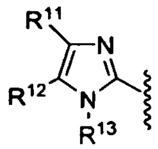 ,

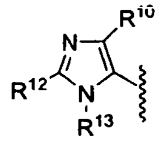 , 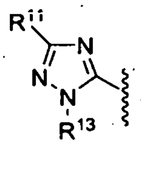 , 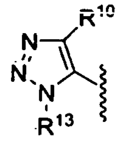 , 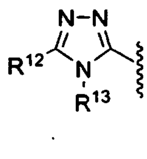 , 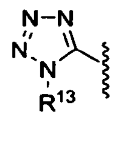 ,

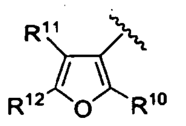 , 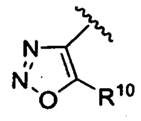 , 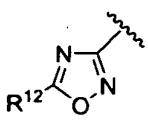 , 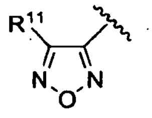 , 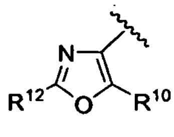 ,

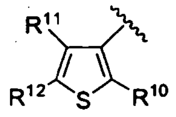 , 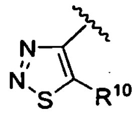 , 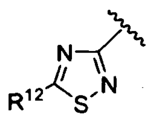 , 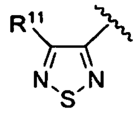 , 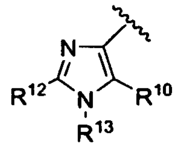 ,

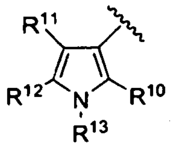 , 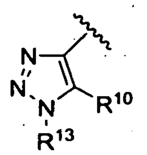 , 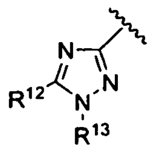 , 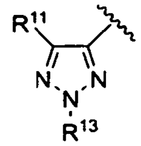 , 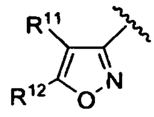 ,

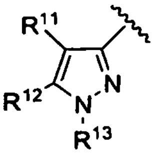 , 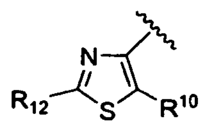 und 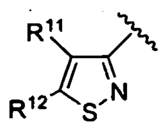

steht;
und jeweils die übrigen Reste die vorstehend genannte. Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ebenfalls besonders bevorzugt sind Propiolsäureamide der vorstehend angegebenen allgemeinen Formel I, worin

$R^1$ und $R^8$, unabhängig voneinander, jeweils für H; -C(=O)-$R^{28}$; -C(=O)-O-$R^{29}$; C(=O)-$NH_2$; -C(=O)-NH-$R^{30}$; -C (=O)-$NR^{31}R^{32}$; -S(=O)-$R^{33}$; -S(=O)$_2$-$R^{34}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, -$NO_2$, -CN, -OH, -SH und-$NH_2$ substituiert ist; $C_{3-8}$-Cycloalkyl, das unsubstituiert oder mit ggf. 1, 2, 3, 4 oder 5 Substituenten unabhängig voneinander ausgewählt aus der Gruppe bestehend aus F, Cl, Br, I, -$NO_2$, -CN, -OH, -SH und -$NH_2$ substituiert ist; oder für einen Phenyl-Rest stehen, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit 1, 2, 3; 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, -O-$CH_3$, -O-$C_2H_5$ und -O-$C_3H_7$ substituiert ist;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Weiterhin besonders bevorzugt sind Propiolsäureamide der vorstehend angegebenen allgemeinen Formel I, worin $R^2$, $R^3$, $R^4$, $R^5$, $R^6$, $R^7$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ und $R^{27}$, unabhängig voneinander, jeweils für H; F; Cl; Br; I; -$NO_2$; -CN; -$NH_2$; -OH; -SH; -C(=O)-OH; -C(=O)-$R^{28}$; -C(=O)-O-$R^{29}$; -NH-$R^{35}$; -$NR^{36}R^{37}$; -O-$R^{42}$; -S-$R^{43}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, -$NO_2$, -CN, -OH, -SH und -$NH_2$ substituiert ist; $C_{3-7}$-Cycloalkyl, $C_{5-6}$-Cycloalkenyl, 5- bis 7-gliedriges Heterocycloalkyl oder 5- bis 7-gliedriges Heterocycloalkenyl, das jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br,

I, -CN, Methyl, Ethyl, n-Propyl, Isopropyl, n-Butyl, 2-Butyl, Isobutyl, tert-Butyl, -OH, Oxo, Thioxo, $-O-CH_3$, $-O-C_2H_5$, $-O-C_3H_7$, $-NH_2$, $-N(CH_3)_2$,$-N(C_2H_5)_2$, $-NH-CH_3$, $-NH-C_2H_5$, $-NO_2$, $-CF_3$, $-O-CF_3$, $-S-CF_3$, -SH, $-S-CH_3$ und $-S-C_2H_5$ substituiert ist; oder für einen Rest ausgewählt aus der Gruppe bestehend aus Phenyl, Naphthyl, Anthracenyl, Pyrrolyl, Indolyl, Furanyl, Benzo[b]furanyl, Thiophenyl, Benz[b]thiophenyl, Benzo[d]thiazolyl, Pyrazolyl, Imidazolyl, Thiazolyl, Thiadiazolyl, Triazolyl, Oxazolyl, Oxadiazolyl, Isoxazolyl, Pyridinyl, Pyridazinyl, Pyrimidinyl, Pyrazinyl, Pyranyl, Indazolyl, Chinolinyl, Isochinolinyl und Chinazolinyl stehen, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 der 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist;
oder $R^2$ und $R^3$ oder $R^4$ und $R^5$ oder $R^6$ und $R^7$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$, unabhängig voneinander, zusammen jeweils für einen Rest ausgewählt aus der Gruppe bestehend aus einer Oxo-Gruppe (=O) und einer Thioxo-Gruppe (=S) stehen;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ebenfalls besonders bevorzugt sind Propiolsäureamide der vorstehend angegebenen allgemeinen Formel I, worin $R^1$ und $R^8$ zusammen mit der sie verbindenden $-N-CR^2R^3-(CR^4R^5)_b-(CR^6R^7)_c$-Gruppe einen Rest ausgewählt aus der Gruppe bestehend aus

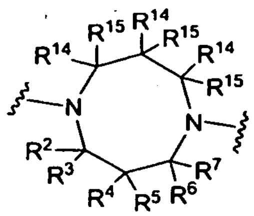 , 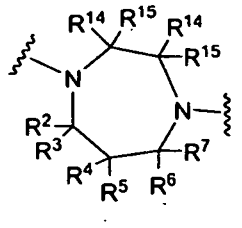 , 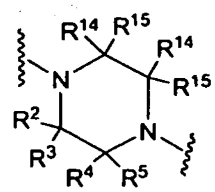 ,

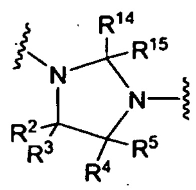 und 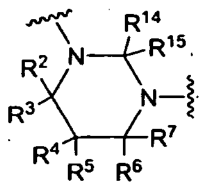

bilden;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Weiterhin besonders bevorzugt sind Propiolsäureamide der vorstehend angegebenen allgemeinen Formel I, worin $R^1$ und $R^2$ zusammen mit der sie verbindenden $-N-CR^3$-Gruppe einen Rest ausgewählt aus der Gruppe bestehend aus

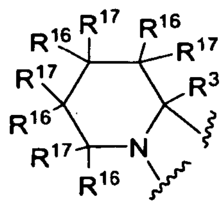 , 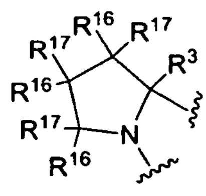 und 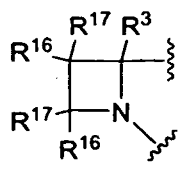

bilden, wobei in diesem Fall b für steht und c für 0 steht;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ebenfalls besonders bevorzugt sind Propiolsäureamide der vorstehend angegebenen allgemeinen Formel I, worin $R^1$ und $R^4$ zusammen mit der sie verbindenden -N-$CR^2R^3$-$CR^5$-Gruppe einen Rest ausgewählt aus der Gruppe bestehend aus

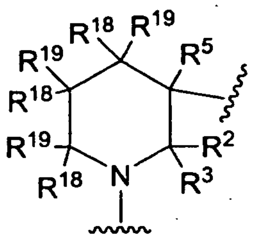 , 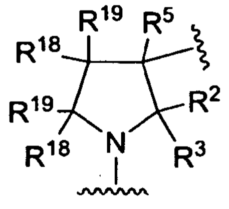 und 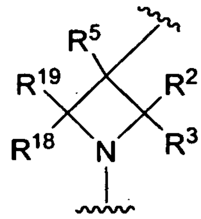

bilden, wobei in diesem Fall c für 0 steht;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Weiterhin besonders bevorzugt sind Propiolsäureamide der vorstehend angegebenen allgemeinen Formel I, worin $R^1$ und $R^6$ zusammen mit der sie verbindenden -N-$CR^2R^3$-$CR^4R^5$-$CR^7$-Gruppe einen Rest ausgewählt aus der Gruppe bestehend aus

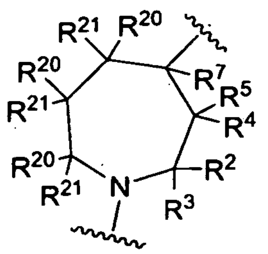 , 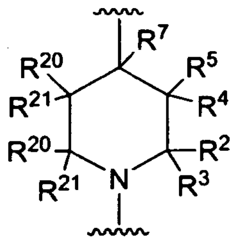 und 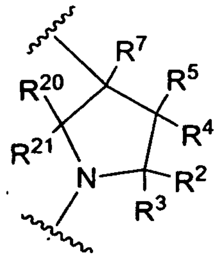

bilden;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen

Mischungsverhältnis, oder jeweils in Form entsprechender Salze oder jeweils in Form entsprechender Solvate.

Ebenfalls besonders bevorzugt sind Propiolsäureamide der vorstehend angegebenen allgemeinen Formel I, worin $R^6$ und $R^8$ zusammen mit der sie verbindenden -N-$CR^7$-Gruppe einen Rest ausgewählt aus der Gruppe bestehend aus

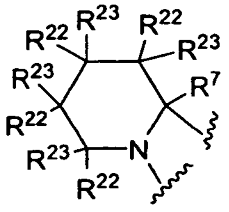 , 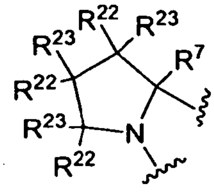 und 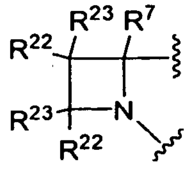

bilden, wobei in diesem Fall b für 1 steht und a für 0 steht;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsvefiältnis, oder jeweils in Form entsprechender Salze oder jeweils in Form entsprechender Solvate.

Weiterhin besonders bevorzugt sind Propiolsäureamide der vorstehend angegebenen allgemeinen Formel I, worin $R^4$ und $R^8$ zusammen mit der sie verbindenden $-N-CR^6R^7-CR^5$-Gruppe einen Rest ausgewählt aus der Gruppe bestehend aus

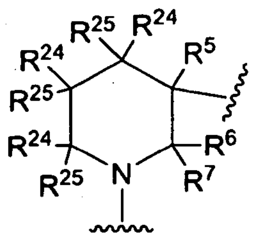
,
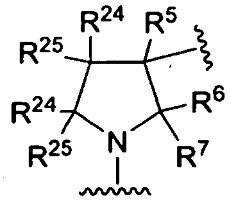
und
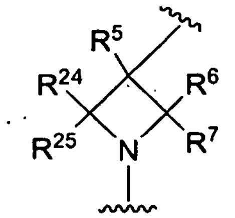

bilden, wobei in diesem Fall a für 0 steht;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ebenfalls besonders bevorzugt sind Propiolsäureamide der vorstehend angegebenen allgemeinen Formel I, worin $R^2$ und $R^8$ zusammen mit der sie verbindenden $-N-CR^6R^7-CR^4R^5-CR^3$-Gruppe einen Rest ausgewählt aus der Gruppe bestehend aus

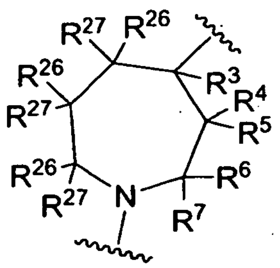
,
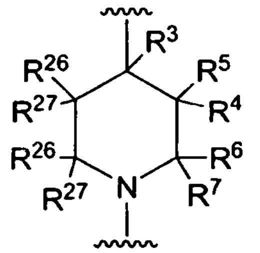
und
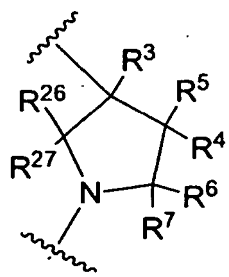

bilden;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Weiterhin besonders bevorzugt sind Propiolsäureamide der vorstehend angegebenen allgemeinen Formel I, worin

$R^9$ für einen Rest ausgewählt aus der Gruppe bestehend aus Phenyl, Naphthyl, Anthracenyl, Furyl, Thienyl, Pyrazolyl, Pyrazinyl, Pyrimidinyl, Pyridazinyl, Pyridinyl, Pyrrolyl, Oxazolyl, Isoxazolyl, Thiazolyl, Thiadiazolyl, Oxadiazolyl, Triazolyl, Imidazolyl, Indolyl, Benz[b]thiophenyl, Benzo[d]thiazolyl, Benzo[b]furanyl, Chinolinyl, Isochinolinyl und Chinazolinyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, Cyclopropyl, Cyclobutyl, Cyclopentyl, Cyclohexyl, $-C{\equiv}C-Si(CH_3)_3$, $-C{\equiv}C-Si(C_2H_5)_3$, $-CH_2-O-CH_3$, $-CH_2-O-C_2H_5$, -OH, $-O-CH_3$, $-O-C_2H_5$, $-O-C_3H_7$, $-S-CH_3$, $-S-C_2H_5$, $-S(=O)-CH_3$, $-S(=O)_2-CH_3$, $-S(=O)-C_2H_5$, $-S(=O)_2C_2H_5$, $-NH_2$, $-N(CH_3)_2$, $-N(C_2H_5)_2$, $-NH-CH_3$, $-NH-C_2H_5$, $-NO_2$, $-CF_3$, $-CH_2F$, $-CHF_2$, $-O-CF_3$, $-S-CF_3$, -SH, $-NH-S(=O)_2-CH_3$, -C(=O)-OH, -C(=O)-H; $-C(=O)-CH_3$, $-C(=O)-C_2H_5$, $-C(=O)-NH_2$, $-C(=O)-N(CH_3)_2$, $-C(=O)-NH-CH_3$, $-NH-C(=O)-CH_3$, $-NH-C(=O)-C_2H_5$. $-C(=O)-O-CH_3$, $-C(=O)O-C_2H_5$, $-C(=O)-O-C(CH_3)_3$ und Phenyl substituiert ist;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ebenfalls besonders bevorzugt sind Propiolsäureamide der vorstehend angegebenen allgemeinen Formel I, worin

$R^{10}$, $R^{11}$ und $R^{12}$, unabhängig voneinander, jeweils für H; F; Cl; Br; I; $-NO_2$; -CN; $-NH_2$; -OH; -SH; -C(=O)-OH; -C(=O)-H; -NH-C(=O)-H; -C(=O)-$R^{28}$; -C(=O)-O-$R^{29}$; -C(=O)-$NH_2$; -C(=O)-NH-$R^{30}$; -C(=O)-$NR^{31}R^{32}$; -S(=O)=$R^{33}$; $-S(=O)_2$-$R^{34}$; -NN-$R^{35}$; -$NR^{36}R^{37}$;-O-C(=O)$R^{38}$; -NH-C(=O)-$R^{39}$; -$NR^{40}$-C(=O)-$R^{41}$; -O-$R^{42}$; -S-$R^{43}$-NH-C(=O)-NH-$R^{44}$; - NH-C (=S)-NH-$R^{45}$; -NH-$S(=O)_2$-$R^{46}$; -$NR^{47}$-$S(=O)_2$-$R^{48}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, $-NO_2$, -CN, -OH, -SH und $-NH_2$ substituiert ist; $C_{2-6}$-Alkenyl; $C_{2-6}$-Alkinyl; $C_{3-7}$-Cycloalkyl, $C_{5-6}$-Cycloalkenyl, 5- bis 7-gliedriges Heterocycloalkyl oder 5- bis 7-gliedriges Heterocycloalkenyl, das jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, -O-$CH_3$, -O-$C_2H_5$, -O-$C_3H_7$, $-NH_2$, $-N(CH_3)_2$, $-N(C_2H_5)_2$, -NH-$CH_3$, -NH-$C_2H_5$, $-NO_2$, $-CF_3$,-O-$CF_3$, -S-$CF_3$, -SH, -S-$CH_3$ und -S-$C_2H_5$ substituiert ist; oder für einen Rest ausgewählt aus der Gruppe bestehend aus Phenyl, Naphthyl, Anthracenyl, Thienyl, Furyl, Pyridinyl, Thiazolyl, Thiadiazolyl, Oxazolyl, Oxadiazolyl und Isoxazolyl stehen, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl,- OH, -SH, $-NH_2$, -C(=O)-OH, -S-$CH_3$, -S-$C_2H_5$, -S(=O)-$CH_3$, $-S(=O)_2$-$CH_3$, - S(=O)-$C_2H_5$, $-S(=O)_2$-$C_2H_5$, -O-$CH_3$, -O-$C_2H_5$, -O-$C_3H_7$, $-CF_3$, $-CHF_2$, $-CH_2F$ und -0-$CF_3$ substituiert ist;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Weiterhin besonders bevorzugt sind Propiolsäureamide der vorstehend angegebenen allgemeinen Formel I, worin

$R^{13}$ für H; -C(=O)-$R^{28}$; -C(=O)-H; -C(=O)-O-$R^{29}$; -C(=O)-$NH_2$; -C(=O)-NH-$R^{30}$; -C(=O)-$NR^{31}R^{32}$; -S(=O)-$R^{33}$; -S $(=O)_2$-$R^{34}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, $-NO_2$. -CN, -OH, -SH und $-NH_2$ substituiert ist; $C_{3-7}$-Cycloalkyl, $C_{5-6}$-Cycloalkenyl, 5- bis 7-gliedriges Heterocycloalkyl oder 5- bis 7-gliedriges Heterocycloalkenyl, das jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählte aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, -O-$CH_3$, -O-$C_2H_5$, -O-$C_3H_7$, $-NH_2$, -N $(CH_3)_2$,$-N(C_2H_5)_2$, -NH-$CH_3$, -NH-$C_2H_5$, $-NO_2$, $-CF_3$, -O-$CF_3$; -S-$CF_3$, -SH, -S-$CH_3$ und -S-$C_2H_5$ substituiert ist; oder für einen Rest ausgewählt aus der Gruppe bestehend aus Phenyl, Naphthyl, Anthracenyl, Thienyl, Furyl, Pyridinyl, Thiazolyl, Thiadiazolyl, Oxazolyl, Oxadiazolyl und Isoxazolyl steht, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl, -OH, -SH, $-NH_2$, -C(=O)-OH, -S-$CH_3$, -S-$C_2H_5$,-S(=O)-$CH_3$, $-S(=O)_2$-$CH_3$, -S(=O)-$C_2H_5$, -S $(=O)_2$-$C_2H_5$, -O-$CH_3$, -O-$C_2H_5$, -O-$C_3H_7$,$-CF_3$, $-CHF_2$, $-CH_2F$ und -O-$CF_3$ substituiert ist;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ebenfalls besonders bevorzugt sind Propiolsäureamide der vorstehend angegebenen allgemeinen Formel I, worin $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$ und $R^{48}$, unabhängig voneinander, jeweils für $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I,$-NO_2$, -CN, -OH, -SH und $-NH_2$ substituiert ist; $C_{3-7}$-Cycloalkyl, $C_{5-6}$-Cycloalkenyl, 5-bis 7-gliedriges Heterocycloalkyl oder 5- bis 7-gliedriges Heterocycloalkenyl, das jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I,-CN, Methyl, Ethyl, n-Propyl, Isopropyl, n-Butyl, 2-butyl, isobutyl, tert-Butyl, -OH, Oxo, Thioxo, -O-$CH_3$, -O-$C_2H_5$, -O-$C_3H_7$, $-NH_2$, -N $(CH_3)_2$, $-N(C_2H_5)_2$, -NH-$CH_3$, -NH-$C_2H_5$, - $NO_2$, $-CF_3$, -O-$CF_3$, -S-$CF_3$, -SH, -S-$CH_3$ und -S-$C_2H_5$ substituiert ist; oder für einen Rest ausgewählt aus der Gruppe bestehend aus Phenyl, Naphthyl, Anthracenyl, Pyrrolyl, Indolyl, Furanyl, Benzo[b]furanyl, Thiophenyl, Benz[b]thiophenyl, Benzo[d]thiazolyl, Pyrazolyl, Imidazolyl, Thiazolyl, Thiadiazolyl, Triazolyl, Oxazolyl, Oxadiazolyl, Isoxazolyl, Pyridinyl, Pyridazinyl, Pyrimidinyl, Pyrazinyl, Pyranyl, Indazolyl, Chinolinyl, Isochinolinyl und Chinazolinyl stehen, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH, $-NH-S(=O)_2-CH_3$, -C(=O)-OH, $-C(=O)-CH_3$, $-C(=O)-C_2H_5$, $-C(=O)-N(CH_3)_2$, $-C(=O)-NH-CH_3$, $-NH-C(=O)-CH_3$, $-NH-C(=O)-C_2H_5$, $-C(=O)-O-CH_3$ und $-C(=O)-O-C_2H_5$ substituiert ist;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.Besonders bevorzugt sind substituierte Propiolsäureamide der vorstehend angegebenen allgemeinen Formel I, worin

a, b und c, unabhängig voneinander, jeweils für 0 oder 1 stehen, wobei die Summe aus a, b und c gleich 1, 2 oder 3 ist;

A für einen Rest ausgewählt aus der Gruppe bestehend aus

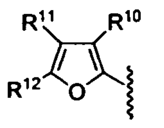 , 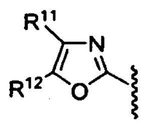 , 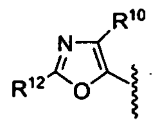 , 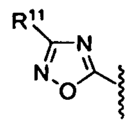 ,

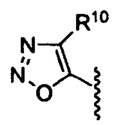 , 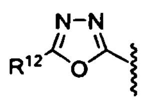 , 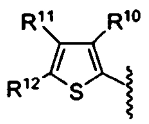 , 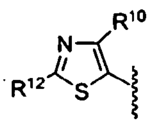 ,

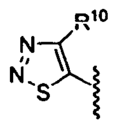 , 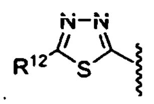 , 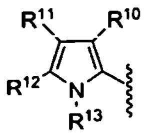 , 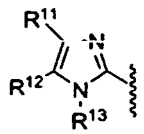 ,

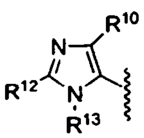 , 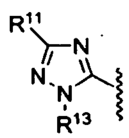 , 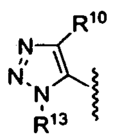 , 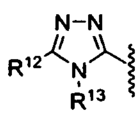 , 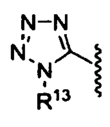 ,

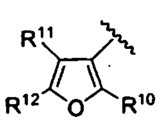 , 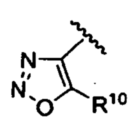 , 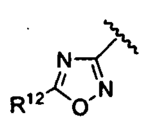 , 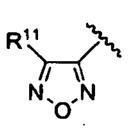 , 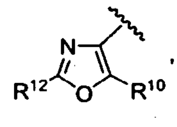 ,

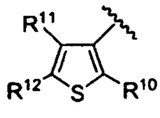, 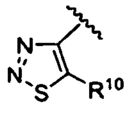, 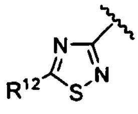, 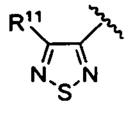, 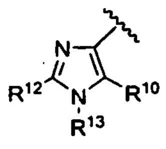,

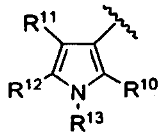, 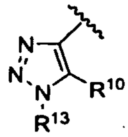, 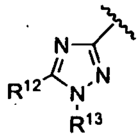, 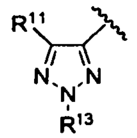, 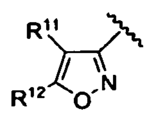,

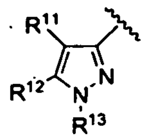, 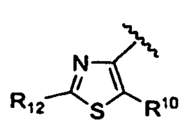 und 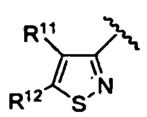

steht;

$R^1$ und $R^8$, unabhängig voneinander, jeweils für H; -C(=O)-$R^{28}$; -C(=O)-O-$R^{29}$; - C(=O)-$NH_2$; -C(=O)-NH-$R^{30}$; -C(=O)-N$R^{31}R^{32}$; -S(=O)-$R^{33}$; -S$(=O)_2$-$R^{34}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, -$NO_2$, -CN, -OH, -SH und-$NH_2$ substituiert ist;

$C_{3-8}$-Cycloalkyl, das unsubstituiert oder mit ggf. 1, 2, 3, 4 oder 5 Substituenten unabhängig voneinander ausgewählt aus der Gruppe bestehend aus F, Cl, Br, I,-$NO_2$, -CN, -OH, -SH und -$NH_2$ substituiert ist;

oder für einen Phenyl-Rest stehen, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, -O-$CH_3$, -O-$C_2H_5$ und -O-$C_3H_7$ substituiert ist;

$R^2$, $R^3$, $R^4$, $R^5$, $R^6$, $R^7$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ und $R^{27}$ unabhängig voneinander jeweils für H; F; Cl; Br; I; -$NO_2$; -CN; -$NH_2$; -OH; -SH:-C(=O)-OH; -C(=O)-$R^{28}$; -C(=O)-O-$R^{29}$; -NH-$R^{35}$ ; -N$R^{36}R^{37}$; -O-$R^{42}$; -S-$R^{43}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, -$NO_2$, -CN, -OH, -SH und -$NH_2$ substituiert ist; $C_{3-7}$-Cycloalkyl, $C_{5-6}$-Cycloalkenyl, 5- bis 7-gliedriges Heterocycloalkyl oder 5- bis 7-gliedriges Heterocycloalkenyl, das jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, -O-$CH_3$, -O-$C_2H_5$, -O-$C_3H_7$, -$NH_2$, -N$(CH_3)_2$, -N$(C_2H_5)_2$, -NH-$CH_3$, -NH-$C_2H_5$, -$NO_2$, -$CF_3$, - O-$CF_3$, -S-$CF_3$, -SH, -S-$CH_3$ und -S-$C_2H_5$ substituiert ist; oder für einen Rest ausgewählt aus der Gruppe bestehend aus Phenyl, Naphthyl, Anthracenyl, Pyrrolyl, Indolyl, Furanyl, Benzo[b]furanyl, Thiophenyl, Benz[b]thiophenyl, Benzo[d]thiazolyl, Pyrazolyl, Imidazolyl, Thiazolyl, Thiadiazolyl, Triazolyl, Oxazolyl, Oxadiazolyl, Isoxazolyl, Pyridinyl, Pyridazinyl, Pyrimidinyl, Pyrazinyl, Pyranyl, Indazolyl, Chinolinyl, Isochinolinyl und Chinazolinyl stehen, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, -O-$CH_3$, -O-$C_2H_5$ und -O-$C_3H_7$ substituiert ist;

oder $R^2$ und $R^3$ oder $R^4$ und $R^5$ oder $R^6$ und $R^7$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$, unabhängig voneinander, zusammen jeweils für einen Rest ausgewählt aus der Gruppe bestehend aus einer Oxo-Gruppe (=O) und einer Thioxo-Gruppe (=S) stehen;

oder $R^1$ und $R^8$ zusammen mit der sie verbindenden -N-C$R^2R^3$-$(CR^4R^5)_b$-$(CR^6R^7)_c$-Gruppe einen Rest ausgewählt aus der Gruppe bestehend aus

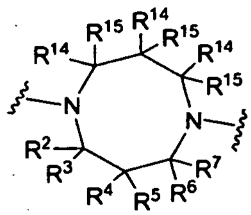,
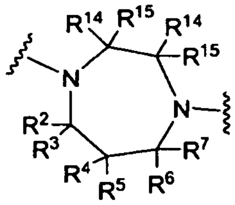,
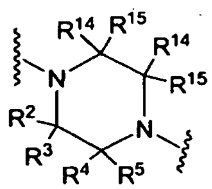,

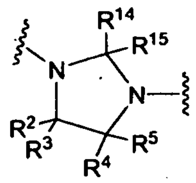 und 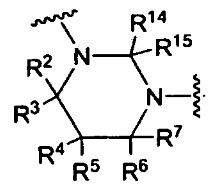

bilden;

oder $R^1$ und $R^2$ zusammen mit der sie verbindenden -N-$CR^3$-Gruppe einen Rest ausgewählt aus der Gruppe bestehend aus

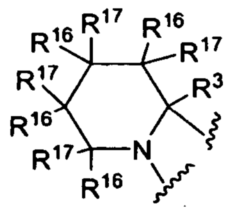,
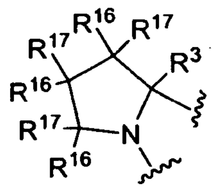 und 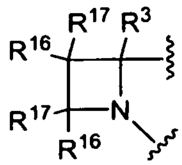

bilden, wobei in diesem Fall b für 1 steht und c für 0 steht; oder $R^1$ und $R^4$ zusammen mit der sie verbindenden -N-$CR^2R^3$-$CR^5$-Gruppe einen Rest ausgewählt aus der Gruppe bestehend aus

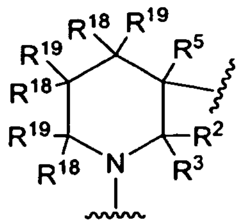,
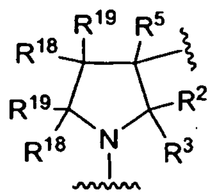 und 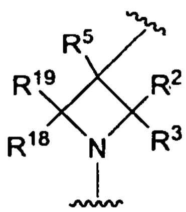

bilden, wobei in diesem Fall c für 0 steht;

$R^1$ und $R^6$ zusammen mit der sie verbindenden -N-$CR^2R^3$-$CR^4R^5$-$CR^7$-Gruppe einen Rest ausgewählt aus der Gruppe bestehend aus

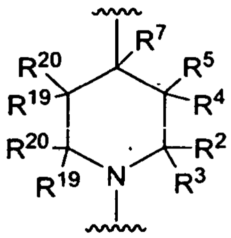 und 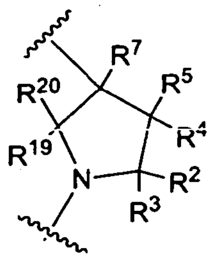

bilden;
oder $R^6$ und $R^8$ zusammen mit der sie verbindenden -N-$CR^7$-Gruppe einen Rest ausgewählt aus der Gruppe bestehend aus

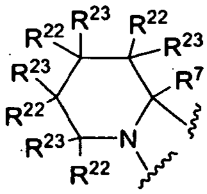 , 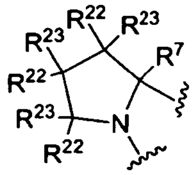 und 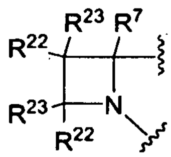

bilden, wobei in diesem Fall b für 1 steht und a für 0 steht;
oder $R^4$ und $R^8$ zusammen mit der sie verbindenden -N-$CR^6R^7$-$CR^5$-Gruppe einen Rest ausgewählt aus der Gruppe bestehend aus

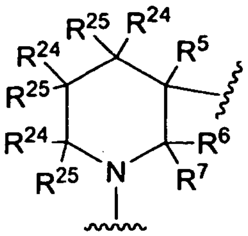 , 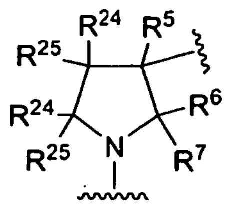 und 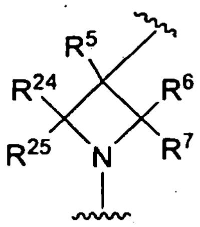

bilden, wobei in diesem Fall a für 0 steht;
oder $R^2$ und $R^8$ zusammen mit der sie verbindenden -N-$CR^6R^7$-$CR^4R^5$-$CR^3$-Gruppe einen Rest ausgewählt aus der Gruppe bestehend aus

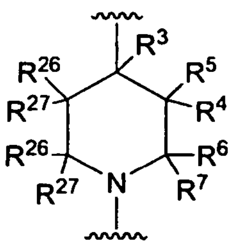 und 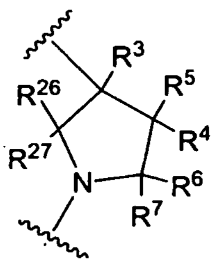

bilden;
$R^9$ für einen Rest ausgewählt aus der Gruppe bestehend aus Phenyl, Naphthyl, Anthracenyl, Furyl, Thienyl, Pyrazolyl, Pyrazinyl, Pyrimidinyl, Pyridazinyl, Pyridinyl, Pyrrolyl, Oxazolyl, Isoxazolyl, Thiazolyl, Thiadiazolyl, Oxadiazolyl, Triazolyl,

Imidazolyl, Indolyl, Benz[b]thiophenyl, Benzo[d]thiazolyl, Benzo[b]furanyl, Chinolinyl, Isochinolinyl und Chinazolinyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, Cyclopropyl, Cyclobutyl, Cyclopentyl, $-C{\equiv}C-Si(CH_3)_3$, $-C{\equiv}C-Si(C_2H_5)_3$, $-CH_2-O-CH_3$, $-CH_2-O-C_2H_5$, -OH, $-0-CH_3$, $-O-C_2H_5$, $-O-C_3H_7$, $-S-CH_3$, $-S-C_2H_5$, $-S(=O)-CH_3$, $-S(=O)_2-CH_3$, $-S(=O)-C_2H_5$, - $S(=O)_2-C_2H_5$, $-NH_2$, $-N(CH_3)_2$, $-N(C_2H_5)_2$, $-NH-CH_3$, $-NH-C_2H_5$, $-NO_2$, $-CF_3$, $-CH_2F$, - $CHF_2$, $-O-CF_3$, $-S-CF_3$, -SH, $-NH-S(=O)_2-CH_3$, -C (=O)-OH, -C(=O)-H; $-C(=O)-CH_3$, - $C(=O)-C_2H_5$. $-C(=O)-NH_2$, $-C(=O)-N(CH_3)_2$, $-C(=O)-NH-CH_3$, $-NH-C(=O)-CH_3$, $-NH-C(=O)-C_2H_5$, $-C(=O)-O-CH_3$, $-C(=O)-O-C_2H_5$, $-C(=O)-O-C(CH_3)_3$ und Phenyl substituiert ist;

$R^{10}$, $R^{11}$ und $R^{12}$, unabhängig voneinander, jeweils für H; F; Cl; Br; I; $-NO_2$; -CN; $-NH_2$; -OH; -SH; -C(=O)-OH; -C(=O)-H; -NH-C(=O)-H; $-C(=O)-R^{28}$; $-C(=O)-O-R^{29}$; $-C(=O)-NH_2$; $-C(=O)-NH-R^{30}$; $-C(=O)-NR^{31}R^{32}$; $-S(=O)-R^{33}$; $-S(=O)_2-R^{34}$; $-NH-R^{35}$; $-NR^{36}R^{37}$; - $O-C(=O)-R^{38}$; $-NH-C(=O)-R^{39}$; $-NR^{40}-C(=O)-R^{41}$; $-O-R^{42}$; $-S-R^{43}-NH-C(=O)-NH-R^{44}$; - NH-C (=S)-NH-$R^{45}$; $-NH-S(=O)_2-R^{46}$; $-NR^{47}-S(=O)_2-R^{48}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, $-NO_2$, -CN, -OH, -SH und $-NH_2$ substituiert ist; $C_{2-6}$-Alkenyl; $C_{2-6}$-Alkinyl; $C_{3-7}$-Cycloalkyl, $C_{5-6}$-Cycloalkenyl, 5- bis 7-gliedriges Heterocycloalkyl oder 5- bis 7-gliedriges Heterocycloalkenyl, das jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, $-0-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, - $O-CF_3$, $-S-CF_3$, -SH, $-S-CH_3$ und $-S-C_2H_5$ substituiert ist; oder für einen Rest ausgewählt aus der Gruppe bestehend aus Phenyl, Naphthyl, Anthracenyl, Thienyl, Furyl, Pyridinyl, Thiazolyl, Thiadiazolyl, Oxazolyl, Oxadiazolyl und Isoxazolyl stehen, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n=Butyl, Isobutyl, 2-Butyl, tert-Butyl, -OH, -SH, $-NH_2$, -C(=O)-OH, $-S-CH_3$, $-S-C_2H_5$, $-S(=O)-CH_3$, $-S(=O)_2-CH_3$,$-S(=O)-C_2H_5$, $-S(=O)_2-C_2H_5$, $-O-CH_3$, $-O-C_2H_5$, $-O-C_3H_7$, $-CF_3$, $-CHF_2$, $-CH_2F$ und $-0-CF_3$ substituiert ist;

$R^{13}$ für H; $-C(=O)-R^{28}$; -C(=O)-H; $-C(=O)-O-R^{29}$; $-C(=O)-NH_2$; $-C(=O)-NH-R^{30}$; $-C(=O)-NR^{31}R^{32}$; $-S(=O)-R^{33}$; -S $(=O)_2-R^{34}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, $-NO_2$, -CN, -OH, -SH und $-NH_2$ substituiert ist; $C_{3-7}$-Cycloalkyl, $C_5$-$_6$-Cycloalkenyl, 5- bis 7-gliedriges Heterocycloalkyl oder 5- bis 7-gliedriges Heterocycloalkenyl, das jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, $-O-CH_3$, $-O-C_2H_5$, $-O-C_3H_7$, $-NH_2$, -N $(CH_3)_2$, - $N(C_2H_5)_2$, $-NH-CH_3$, $-NH-C_2H_5$, $-NO_2$, $-CF_3$, $-O-CF_3$, $-S-CF_3$, -SH, $-S-CH_3$ und $-S-C_2H_5$ substituiert ist; oder für einen Rest ausgewählt aus der Gruppe bestehend aus Phenyl, Naphthyl, Anthracenyl, Thienyl, Furyl, Pyridinyl, Thiazolyl, Thiadiazolyl, Oxazolyl, Oxadiazolyl und Isoxazolyl steht, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl, -OH, -SH, $-NH_2$, -C(=O)-OH, $-S-CH_3$, $-S-C_2H_5$, - $S(=O)-CH_3$, $-S(-O)_2-CH_3$, -S $(=O)-C_2H_5$, $-S(=O)_2-C_2H_5$, $-O-CH_3$, $-O-C_2H_5$, $-O-C_3H_7$, - $CF_3$, $-CHF_2$, $-CH_2F$ und $-O-CF_3$ substituiert ist;

und $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$ und $R^{48}$, unabhängig voneinander, jeweils für $C_1$-$_6$-Alkyl, das unsubstituiert oder mit ggf. 1, 2, 3, 4 oder 5 Substituenten unabhängig voneinander ausgewählt aus der Gruppe betstehend aus F, Cl, Br, I, $-NO_2$, -CN, -OH, -SH und-$NH_2$ substituiert ist; $C_{3-7}$-Cycloalkyl, $C_{5-6}$-Cycloalkenyl, 5- bis 7-gliedriges Heterocycloalkyl oder 5- bis 7-gliedriges Heterocycloalkenyl, das jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, - $O-CF_3$, $-S-CF_3$, -SH, $-S-CH_3$ und $-S-C_2H_5$ substituiert ist; oder für einen Rest ausgewählt aus der Gruppe bestehend aus Phenyl, Naphthyl, Anthracenyl, Pyrrolyl, Indolyl, Furanyl, Benzo[b]furanyl, Thiophenyl, Benz[b]thiophenyl, Benzo[d]thiazolyl, Pyrazolyl, Imidazolyl, Thiazolyl, Thiadiazolyl, Triazolyl, Oxazolyl, Oxadiazolyl, Isoxazolyl, Pyridinyl, Pyridazinyl, Pyrimidinyl, Pyrazinyl, Pyranyl, Indazolyl, Chinolinyl, Isochinolinyl und Chinazolinyl stehen, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, $-O-CH_3$, $-O-C_2H_5$, $-O-C_3H_7$, $-NH_2$, $-N(CH_3)_2$, - $N(C_2H_5)_2$, $-NH-CH_3$, $-NH-C_2H_5$, $-NO_2$, $-CF_3$, $-O-CF_3$, $-S-CF_3$, -SH, $-NH-S(=O)_2-CH_3$, - C(=O)-OH, $-C(=O)-CH_3$, $-C(=O)-C_2H_5$, $-C(=O)-N(CH_3)_2$, $-C(=O)-NH-CH_3$, $-NH-C(=O)-CH_3$, $-NH-C(=O)-C_2H_5$, $-C(=O)-O-CH_3$ und $-C(=O)-O-C_2H_5$ substituiert ist;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in

einem beliebigen Mischungsverhältnis, oder jeweils in Form entsprechender Salze, oder jeweils in Form entsprechender Solvate.

Ebenfalls besonders bevorzugt sind substituierte Propiolsäureamide der vorstehend angegebenen allgemeinen Formel I, worin

a, b und c, unabhängig voneinander, jeweils für 0 oder 1 stehen, wobei die Summe aus a, b und c gleich 1, 2 oder 3 ist;

A für einen Rest ausgewählt aus der Gruppe bestehend aus

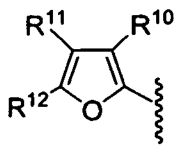 , 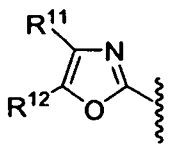 , 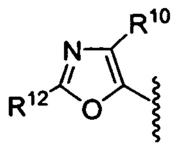 , 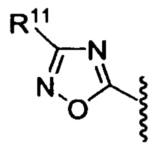 ,

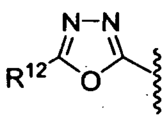 , 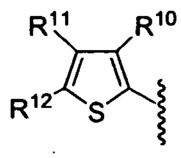 , 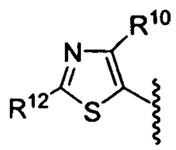 , 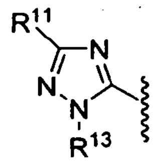 ,

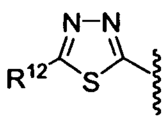 , 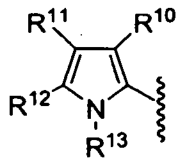 , 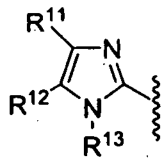 , 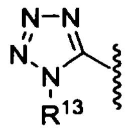 ,

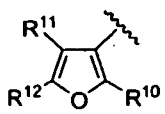 , 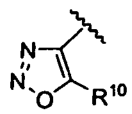 , 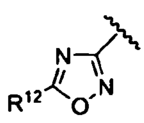 , 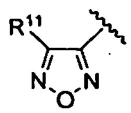 , 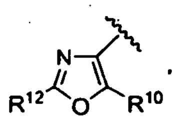 ,

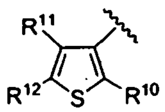 , 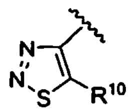 , 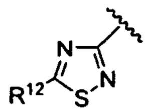 , 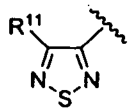 , 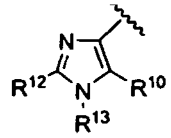 ,

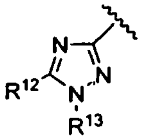 , 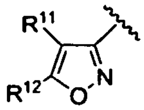 , 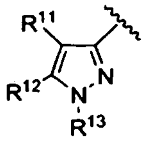 , 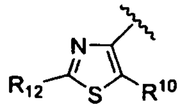 und 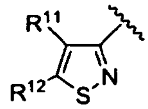

steht;

$R^1$ und $R^8$, unabhängig voneinander, jeweils für H; $-C(=O)-R^{28}$; $-C(=O)-O-R^{29}$; $S(=O)-R^{33}$; $-S(=O)_2-R^{34}$; einen Alkyl-

Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; einen Cycloalkyl-Rest ausgewählt aus der Gruppe bestehend aus Cyclopropyl, Cyclobutyl, Cyclopentyl und Cyclohexyl; oder für einen Benzyl- oder Phenethyl-Rest stehen, der unsubstituiert oder mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, $-O-CH_3$, $- O-C_2H_5$ und $-O-C_3H_7$ substituiert ist;
$R^2$, $R^3$, $R^4$, $R^5$, $R^6$, $R^7$ $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$ , $R^{26}$ und $R^{27}$, unabhängig voneinander, jeweils für H; F; Cl; Br; I; $-NH_2$; -OH; -SH; -CN; $-NO_2$; $- CF_3$; $-NH-R^{35}$; $-NR^{36}R^{37}$; $-O-R^{42}$; $-S-R^{43}$; für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; oder für einen Rest ausgewählt aus der Gruppe bestehend aus Cyclopropyl, Cyclobutyl, Cyclopentyl und Cyclohexyl stehen;
oder $R^2$ und $R^3$ oder $R^4$ und $R^5$ oder $R^6$ und $R^7$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$, unabhängig voneinander, zusammen jeweils für einen Rest ausgewählt aus der Gruppe bestehend aus einer Oxo-Gruppe (=O) und einer Thioxo-Gruppe (=S) stehen;
oder $R^1$ und $R^8$ zusammen mit der sie verbindenden $-N-CR^2R^3-(CR^4R^5)_b-(CR^6R^7)_c$-Gruppe einen Rest ausgewählt aus der Gruppe bestehend aus

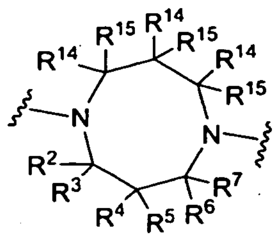
,
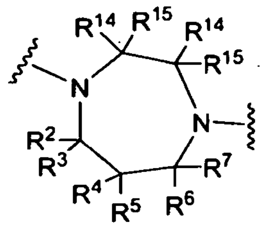
,
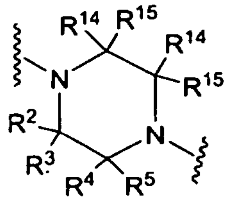
,

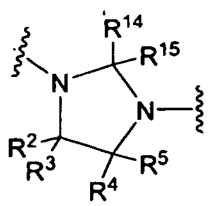
und
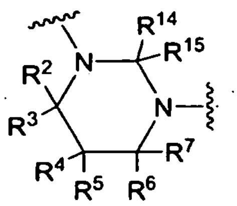

bilden;
oder $R^1$ und $R^2$ zusammen mit der sie verbindenden $-N-CR^3$-Gruppe einen Rest ausgewählt aus der Gruppe bestehend aus

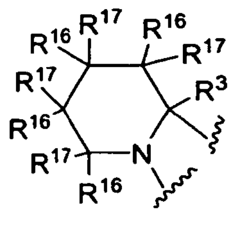
,
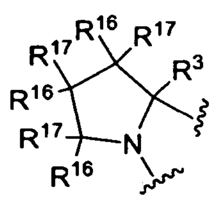
und
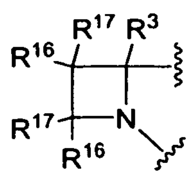

bilden, wobei in diesem Fall b für 1 steht und c für 0 steht;
oder $R^1$ und $R^4$ zusammen mit der sie verbindenden $-N-CR^2R^3-CR^5$-Gruppe einen Rest ausgewählt aus der Gruppe bestehend aus

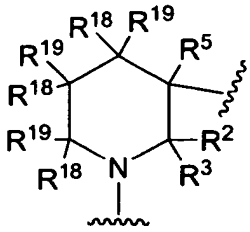
,
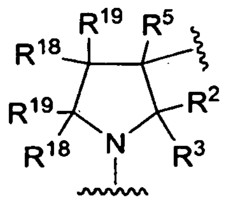
und
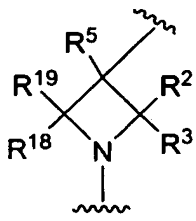

bilden, wobei in diesem Fall c für 0 steht;
$R^1$ und $R^6$ zusammen mit der sie verbindenden $-N-CR^2R^3-CR^4R^5-CR^7-$Gruppe einen Rest ausgewählt aus der Gruppe bestehend aus

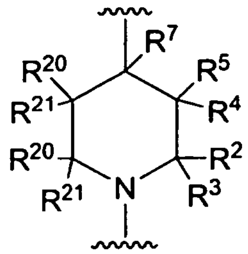
und
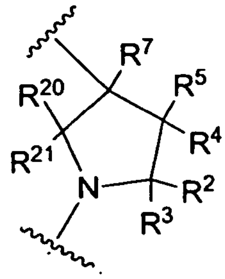

bilden;
oder $R^6$ und $R^8$ zusammen mit der sie verbindenden $-N-CR^7-$Gruppe einen Rest ausgewählt aus der Gruppe bestehend aus

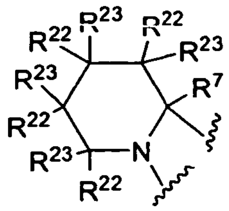
,
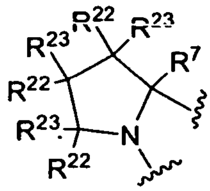
und
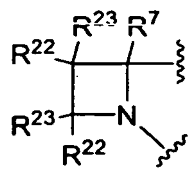

bilden, wobei in diesem Fall b für 1 steht und a für 0 steht;
oder $R^4$ und $R^8$ zusammen mit der sie verbindenden $-N-CR^6R^7-CR^5-$Gruppe einen Rest ausgewählt aus der Gruppe bestehend aus

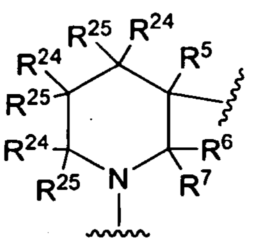
,
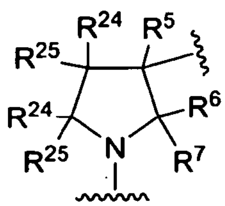
und
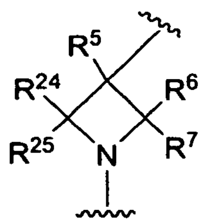

bilden, wobei in diesem Fall a für 0 steht;
der $R^2$ und $R^8$ zusammen mit der sie verbindenden $-N-CR^6R^7-CR^4R^5-CR^3-$Gruppe einen Rest ausgewählt aus der Gruppe bestehend aus

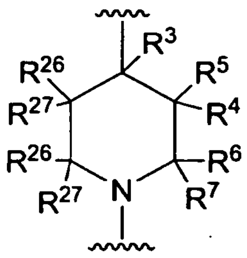

und

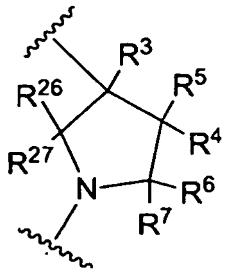

bilden;

$R^9$ für einen Rest ausgewählt aus der Gruppe bestehend aus Phenyl, Furyl, Thienyl, Pyrazolyl, Pyrazinyl, Pyridazinyl, Pyrimidinyl, Pyridinyl, Pyrrolyl, Oxazolyl, Isoxazolyl, Thiazolyl, Thiadiazolyl, Oxadiazolyl, Triazolyl und Imidazolyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, Cyclopropyl, Cyclobutyl, $-C{\equiv}C-Si(CH_3)_3$, $-C{\equiv}C-Si(C_2H_5)_3$, $-CH_2-O-CH_3$, $-CH_2-O-C_2H_5$, -OH, $-O-CH_3$, $-O-C_2H_5$, $-O-C_3H_7$, $-S-CH_3$, $-S-C_2H_5$, $-S(=O)-CH_3$, $-S(=O)_2-CH_3$, $-S(=O)-C_2H_5$, $-S(=O)_2-C_2H_5$, $-NH_2$, $-N(CH_3)_2$, $-N(C_2H_5)_2$, $-NH-CH_3$, $-NH-C_2H_5$, $-NO_2$, $-CF_3$, $-CH_2F$, $-CHF_2$, $-O-CF_3$, $-S-CF_3$, -SH, $-NH-S(=O)_2-CH_3$, -C(=O)-OH, -C(=O)-H; $-C(=O)-CH_3$, $-C(=O)-C_2H_5$, $-C(=O)-NH_2$, $-C(=O)-N(CH_3)_2$, $-C(=O)-NH-CH_3$, $-NH-C(=O)-CH_3$,$-NH-C(=O)-C_2H_5$, $-C(=O)-O-CH_3$, $-C(=O)-O-C_2H_5$, $-C(=O)-O-C(CH_3)_3$ und Phenyl substituiert ist;

$R^{10}$, $R^{11}$ und $R^{12}$, unabhängig voneinander, jeweils für H; F; Cl; Br; I; $-CF_3$; $-NO_2$; -CN; -C(=O)-OH; $-C(=O)-O-R^{29}$; $-C(=O)-NH_2$; $-C(=O)-NH-R^{30}$; $-C(=O)-NR^{31}R^{32}$; $-S(=O)-R^{33}$; $-S(=O)_2-R^{34}$; $-O-R^{42}$; $-S-R^{43}$; einen Alkyl-Rest ausgewählt aus der Gruppe bestehend aus Methyl, Ethyl, n-Propyl, Isopropyl, n-Butyl, 2-Butyl, Isobutyl und tert-Butyl; einen Rest ausgewählt aus der Gruppe bestehend aus Ethenyl, Ethinyl, Allyl und Propinyl; für einen Rest ausgewählt aus der Gruppe bestehend aus Cyclopropyl, Cyclobutyl und Cyclopentyl; oder für einen Rest ausgewählt aus der Gruppe bestehend aus Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist, stehen;

$R^{13}$ für H; $-C(=O)-R^{28}$; -C(=O)-H; $-C(=O)-O-R^{29}$; $-S(=O)-R^{33}$; $-S(=O)_2-R^{34}$; einen Alkyl-Rest ausgewählt aus der Gruppe bestehend aus Methyl, Ethyl, n-Propyl, Isopropyl, n-Butyl, 2-Butyl, Isobutyl und tert-Butyl; einen Rest ausgewählt aus der Gruppe bestehend Cyclopropyl, Cyclobutyl, Cyclopentyl und Cyclohexyl; oder für einen Rest ausgewählt aus der Gruppe bestehend aus Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils über eine $C_{1-3}$-Alkylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist, steht;

und $R^{28}$, $R^{29}$, $R^{30}$, $R^{31}$, $R^{32}$, $R^{33}$, $R^{34}$, $R^{35}$, $R^{36}$, $R^{37}$, $R^{42}$ und $R^{43}$, unabhängig voneinander, jeweils für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; $-CF_3$; $-C_2F_5$; $-CH_2-CF_3$; oder für einen Phenyl-, Benzyl- oder Phenethyl-Rest stehen, der unsubstituiert oder mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, $-O-CH_3$, $-O-C_2H_5$ und $-0-C_3H_7$ substituiert ist;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind substituierte Propiolsäureamide der vorstehend angegebenen allgemeinen Formel I, worin a, b und c, unabhängig voneinander, jeweils für 0 oder 1 stehen, wobei die Summe aus a, b und c gleich 1, 2 oder 3 ist;

A für einen Rest ausgewählt aus der Gruppe bestehend aus

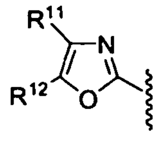, 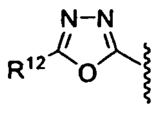, 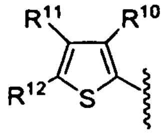, 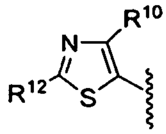,

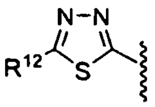, 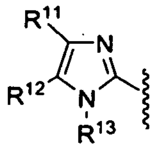, 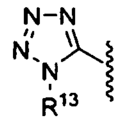, 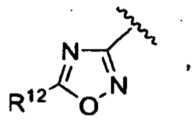,

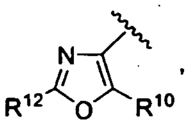, 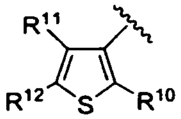, 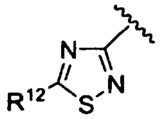, 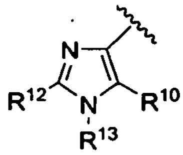,

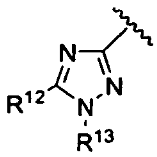, 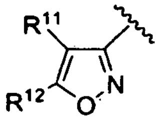, 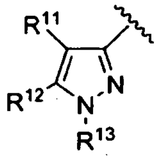 und 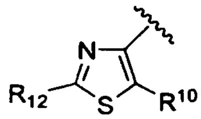

steht;

$R^1$ und $R^8$, unabhängig voneinander, jeweils für H; -C(=O)-$R^{28}$; -C(=O)-O-$R^{29}$; für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; oder für einen Cyclopropyl-Rest; stehen;

$R^2$, $R^3$, $R^4$, $R^5$, $R^6$, $R^7$, $R^{14}$, $R^{15}$, $R^{16}$, $R^{17}$, $R^{18}$, $R^{19}$, $R^{22}$, $R^{23}$, $R^{24}$ und $R^{25}$, unabhängig voneinander, jeweils für H; F; Cl; Br; I; $-NH_2$; -OH; -SH; -CN; $-NO_2$; $-CF_3$; -NH-$R^{35}$;-N$R^{36}R^{37}$; -O-$R^{42}$; -S-$R^{43}$; für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl oder für einen Cyclopropyl-Rest stehen;

oder $R^1$ und $R^8$ zusammen mit der sie verbindenden $-N-CR^2R^3-(CR^4R^5)_b-(CR^6R^7)_c-$Gruppe einen Rest ausgewählt aus der Gruppe bestehend aus

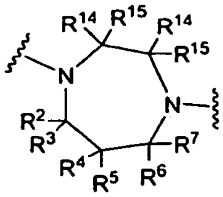, 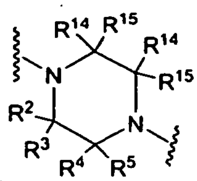, 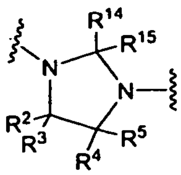 und 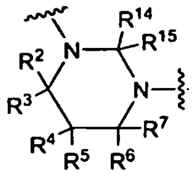

bilden;

oder $R^1$ und $R^2$ zusammen mit der sie verbindenden -N-C$R^3$-Gruppe einen Rest ausgewählt aus der Gruppe bestehend aus

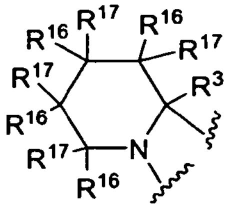, 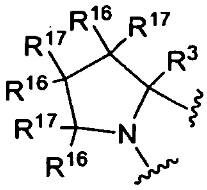 und 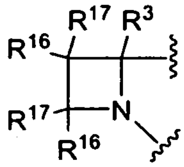

bilden, wobei in diesem Fall b für 1 steht und c für 0 steht;
oder $R^1$ und $R^4$ zusammen mit der sie verbindenden -N-$CR^2R^3$-$CR^5$-Gruppe einen Rest ausgewählt aus der Gruppe bestehend aus

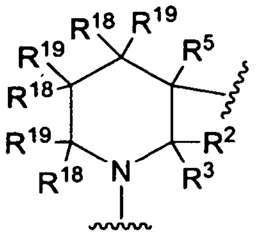, 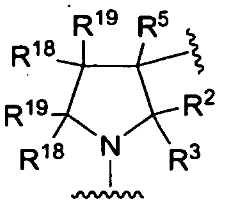 und 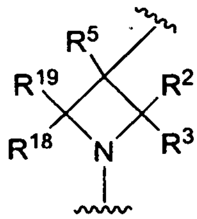

bilden, wobei in diesem Fall c für 0 steht;
oder $R^6$ und $R^8$ zusammen mit der sie verbindenden -N-$CR^7$-Gruppe einen Rest ausgewählt aus der Gruppe bestehend aus

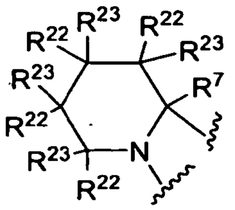, 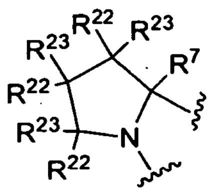 und 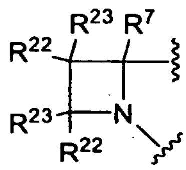

bilden, wobei in diesem Fall b für 1 steht und a für 0 steht;
oder $R^4$ und $R^8$ zusammen mit der sie verbindenden -N-$CR^6R^7$-$CR^5$-Gruppe einen Rest ausgewählt aus der Gruppe bestehend aus

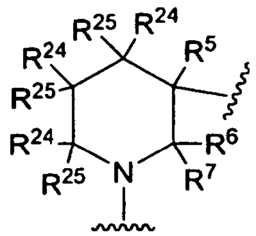, 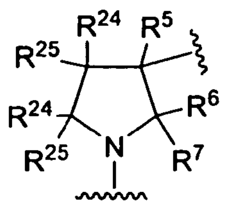 und 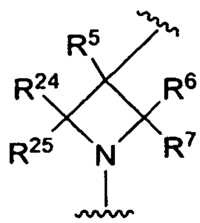

bilden, wobei in diesem Fall a für 0 steht;
$R^9$ für einen Rest ausgewählt aus der Gruppe bestehend aus Phenyl, Thienyl, Pyrazolyl, Pyrazinyl, Pyridazinyl, Pyrimidinyl, Pyridinyl, Thiazolyl und Thiadiazolyl steht, der jeweils unsubstituiert oder mit ggf. 1, 2, 3, 4 oder 5 Substituenten

unabhängig voneinander ausgewählt aus der Gruppe bestehend aus F, Cl, Br, I,-CN, Methyl, Ethyl, n-Propyl, Isopropyl, n-Butyl, 2-Butyl, Isobutyl, tert-Butyl, Ethenyl, Allyl, Ethinyl, Propinyl, Cyclopropyl, Cyclobutyl, $-C\equiv C-Si(CH_3)_3$, $-C\equiv C-Si(C_2H_5)_3$, $-CH_2-O-CH_3$, $-CH_2-O-C_2H_5$, -OH, $-O-CH_3$, $-O-C_2H_5$, $-O-C_3H_7$, $-S-CH_3$, $-S-C_2H_5$, $-S(=O)-CH_3$, $-S(=O)_2-CH_3$, $-S(=O)-C_2H_5$, $-S(=O)_2-C_2H_5$, $-NH_2$, $-N(CH_3)_2$, $-N(C_2H_5)_2$, $-NH-CH_3$, $-NH-C_2H_5$, $-NO_2$, $-CF_3$, $-CH_2F$, $-CHF_2$, $-O-CF_3$, $-S-CF_3$, -SH, $-NH-S(=O)_2-CH_3$, -C(=O)-OH, -C(=O)-H; $-C(=O)-CH_3$, $-C(=O)-C_2H_5$, $-C(=O)-NH_2$, $-C(=O)-N(CH_3)_2$, $-C(=O)-NH-CH_3$, $-NH-C(=O)-CH_3$, $-NH-C(=O)-C_2H_5$, $-C(=O)-O-CH_3$, $-C(=O)-O-C_2H_5$, $-C(=O)-O-C(CH_3)_3$ und Phenyl substituiert ist;

$R^{10}$, $R^{11}$ und $R^{12}$, unabhängig voneinander, jeweils für H; F; Cl; Br; I; $-CF_3$; $-NO_2$; -CN; -C(=O)-OH; $-C(=O)-O-R^{29}$; $-C(=O)-NH_2$; $-O-R^{42}$; $-S-R^{43}$; Ethenyl; Ethinyl; Cyclopropyl oder für einen Alkyl-Rest ausgewählt aus der Gruppe bestehend aus Methyl, Ethyl, n-Propyl, Isopropyl, n-Butyl, 2-Butyl, Isobutyl und tert-Butyl stehen;

$R^{13}$ für H; $-C(=O)-R^{28}$; -C(=O)-H; $-C(=O)-O-R^{29}$; $-S(=O)-R^{33}$; $-S(=O)_2-R^{34}$; Cyclopropyl; Cyclobutyl; oder für einen Alkyl-Rest ausgewählt aus der Gruppe bestehend aus Methyl, Ethyl, n-Propyl, Isopropyl, n-Butyl, 2-Butyl, Isobutyl und tert-Butyl steht;

und $R^{28}$, $R^{29}$, $R^{33}$, $R^{34}$, $R^{35}$, $R^{36}$, $R^{37}$, $R^{42}$ und $R^{43}$, unabhängig voneinander, jeweils für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; $-CF_3$; $-C_2F_5$; $-CH_2-CF_3$; oder für einen Phenyl-, Benzyl- oder Phenethyl-Rest stehen, der unsubstituiert oder mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls ganz besonders bevorzugt sind substituierte Propiolsäureamide der vorstehend angegebenen allgemeinen Formel I, worin

a, b und c, unabhängig voneinander, jeweils für 0 oder 1 stehen, wobei die Summe aus a, b und c gleich 1 oder 2 ist;

A für einen Rest ausgewählt aus der Gruppe bestehend aus

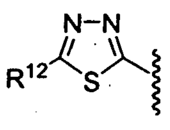 , 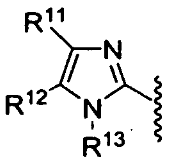 , 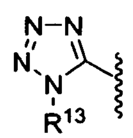 , 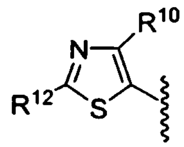 ,

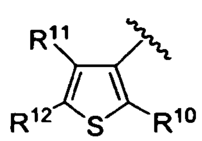 und 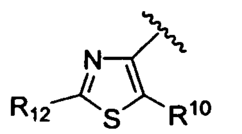

steht;

$R^1$ und $R^8$, unabhängig voneinander, jeweils für H; für $-C(=O)-R^{28}$; für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; oder für einen Cyclopropyl-Rest stehen;

$R^2$, $R^3$, $R^4$, $R^5$, $R^6$, $R^7$, $R^{14}$, $R^{15}$, $R^{16}$, $R^{17}$, $R^{22}$, $R^{23}$, $R^{24}$ und $R^{25}$, unabhängig voneinander, jeweils für H; F; Cl; Br; I; -CN; $-NO_2$; $-CF_3$; $-O-R^{42}$; für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl oder für einen Cyclopropyl-Rest stehen;

oder $R^1$ und $R^8$ zusammen mit der sie verbindenden $-N-CR^2R^3-CR^4R^5$ -Gruppe den folgenden Rest

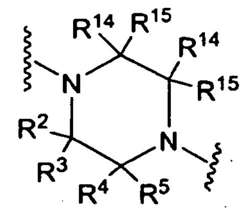

bilden;
oder $R^1$ und $R^2$ zusammen mit der sie verbindenden -N-$CR^3$-Grüppe einen Rest ausgewählt aus der Gruppe bestehend aus

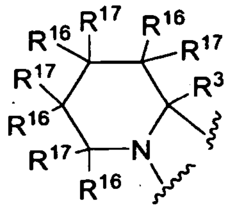 , 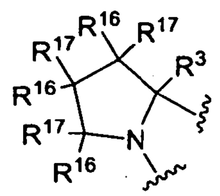 und 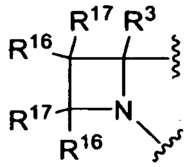

bilden, wobei in diesem Fall b für 1 steht und c für 0 steht;
oder $R^6$ und $R^8$ zusammen mit der sie verbindenden -N-$CR^7$-Gruppe einen Rest ausgewählt aus der Gruppe bestehend aus

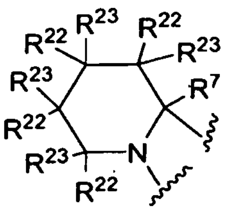 , 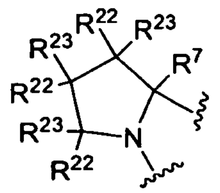 und 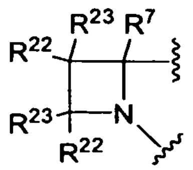

bilden, wobei in diesem Fall b für 1 steht und a für 0 steht;
$R^9$ für einen Rest ausgewählt aus der Gruppe bestehend aus Phenyl und Thienyl steht, der jeweils unsubstituiert oder mit 1 oder 2 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Ethinyl, Cyclopropyl, -O-$CH_3$, -O-$C_2H_5$, -O-$C_3H_7$,-$NO_2$, -$CF_3$ und -O-$CF_3$ substituiert ist;
$R^{10}$, $R^{11}$ und $R^{12}$, unabhängig voneinander, jeweils für H; F; Cl; Br; -$CF_3$; -$NO_2$; -CN;-O-$R^{42}$; für einen Alkyl-Rest ausgewählt aus der Gruppe bestehend aus Methyl, Ethyl, n-Propyl, Isopropyl, Isobutyl und tert-Butyl; Ethenyl; Ethinyl oder Cyclopropyl stehen;
$R^{13}$ für H; -C(=O)-H; -C(=O)-$R^{28}$; Cyclopropyl; oder für einen Alkyl-Rest ausgewählt aus der Gruppe bestehend aus Methyl, Ethyl, n-Propyl, Isopropyl, n-Butyl, 2-Butyl, Isobutyl und tert-Butyl steht;
und $R^{28}$ und $R^{42}$, unabhängig voneinander, jeweils für einen Alkyl-Rest ausgewählt aus der Gruppe bestehend aus Methyl, Ethyl, n-Propyl, Isopropyl, n-Butyl, 2-Butyl, Isobutyl und tert-Butyl; oder -$CF_3$ stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin ganz besonders bevorzugt sind substituierte Propiolsäureamide der vorstehend angegebenen allgemeinen Formel I, worin
a, b und c, unabhängig voneinander, jeweils für 0 oder 1 stehen, wobei die Summe aus a, b und c gleich 2 ist;
A für einen Rest ausgewählt aus der Gruppe bestehend aus

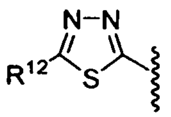 , 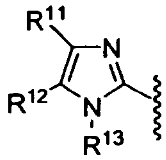 , 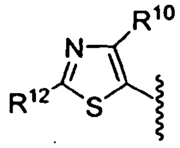 ,

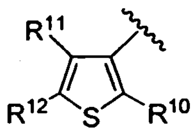 und 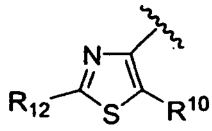

steht;

$R^1$ und $R^8$, unabhängig voneinander, jeweils für H, $-C(=O)-CH_3$, Methyl, Isopropyl oder Cyclopropyl stehen;

$R^2$, $R^3$, $R^4$, $R^5$, $R^6$, $R^7$, $R^{14}$, $R^{15}$, $R^{16}$, $R^{17}$, $R^{22}$ und $R^{23}$, unabhängig voneinander, jeweils für H oder für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl stehen;

oder $R^1$ und $R^8$ zusammen mit der sie verbindenden $-N-CR^2R^3-CR^4R^5$ -Gruppe den folgenden Rest

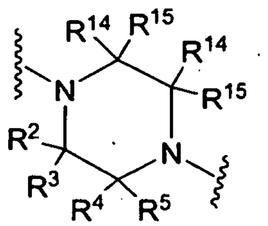

bilden;

oder $R^1$ und $R^2$ zusammen mit der sie verbindenden $-N-CR^3$-Gruppe den folgenden Rest

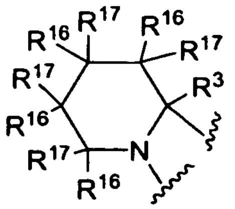

bilden, wobei in diesem Fall b für 1 steht und c für 0 steht;

oder $R^6$ und $R^8$ zusammen mit der sie verbindenden $-N-CR^7$-Gruppe den folgenden Rest

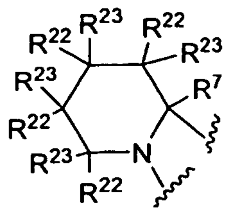

bilden, wobei in diesem Fall b für 1 steht und a für 0 steht;

$R^9$ für einen Phenyl-Rest, der jeweils unsubstituiert oder mit 1 oder 2 Substituenten unabhängig voneinander ausgewählt aus der Gruppe bestehend aus F, Cl, Br, I, - CN, Methyl, Ethyl, n-Propyl, Isopropyl, n-Butyl, 2-Butyl, Isobutyl, tert-Butyl, Ethenyl, Ethinyl, Cyclopropyl, $-O-CH_3$, $-O-C_2H_5$, $-O-C_3H_7$,$-NO_2$, $-CF_3$ und $-O-CF_3$ substituiert ist;

$R^{10}$, $R^{11}$ und $R^{12}$, unabhängig voneinander, jeweils für H; F; Cl; Br; $-CF_3$; $-NO_2$; -CN;$-O-CF_3$; für einen Alkyl-Rest ausgewählt aus der Gruppe bestehend aus Methyl, Ethyl, n-Propyl, Isopropyl, Isobutyl und tert-Butyl; Ethenyl; Ethinyl oder Cyclopropyl stehen;

und $R^{13}$ für H oder für einen Alkyl-Rest ausgewählt aus der Gruppe bestehend aus Methyl, Ethyl, n-Propyl, Isopropyl, n-Butyl, 2-Butyl, Isobutyl und tert-Butyl steht;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin ganz besonders bevorzugt sind substituierte Propiolsäureamide der vorstehend angegebenen allgemeinen Formel I, worin

a, b und c, unabhängig voneinander, jeweils für 0 oder 1 stehen, wobei die Summe aus a, b und c gleich 2 ist;

A für einen Rest ausgewählt aus der Gruppe bestehend aus

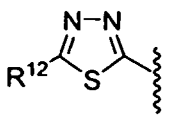 , 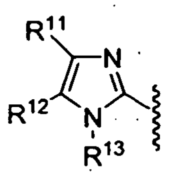 , 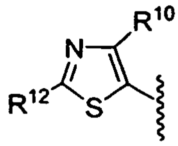 ,

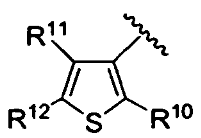 und 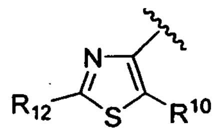

steht;

$R^1$ und $R^8$, unabhängig voneinander, jeweils für H, Methyl oder Isopropyl stehen;

$R^2$ $R^3$, $R^4$, $R^5$, $R^6$, $R^7$, $R^{14}$ und $R^{15}$, unabhängig voneinander, jeweils für H oder für einen Alkyl-Rest ausgewählt aus der Gruppe bestehend aus Methyl, und Ethyl stehen;

oder $R^1$ und $R^8$ zusammen mit der sie verbindenden $-N-CR^2R^3-CR^4R^5$ -Gruppe den folgenden Rest

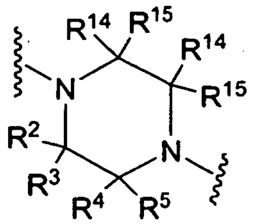

bilden;

$R^9$ für einen Phenyl-Rest, der jeweils unsubstituiert oder mit 1 oder 2 Substituenten unabhängig voneinander ausgewählt aus der Gruppe bestehend aus F, Cl, Br, I und - CN substituiert ist;

$R^{10}$, $R^{11}$ und $R^{12}$, unabhängig voneinander, jeweils für H; oder für einen Alkyl-Rest ausgewählt aus der Gruppe bestehend aus Methyl, Ethyl, n-Propyl, Isopropyl, Isobutyl und tert-Butyl;

und $R^{13}$ für H oder für einen Alkyl-Rest ausgewählt aus der Gruppe bestehend aus Methyl, Ethyl, n-Propyl, Isopropyl, n-Butyl, 2-Butyl, Isobutyl und tert-Butyl steht;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Am weitesten bevorzugt sind substituierte Propiolsäureamide der vorstehend angegebenen allgemeinen Formel I ausgewählt aus der Gruppe bestehend aus Verbindungen gemäß einem oder mehreren der Ansprüche 1 bis 21 ausgewählt aus der Gruppe bestehend aus

[1] 1-(3-Phenyl-propiolyl)-4-(thiophen-3-yl)-piperazin,
[2] 1-(3-Phenyl-propiolyl)-4-(thiazol-4-yl)-piperazin,
[3] 1-(3-Phenyl-propiolyl)-4-(1-methyl-imidazol-2-yl)-piperazin,
[4] (R)-2-Methyl-1-(3-(3-chloro-phenyl)-propiolyl)-4-(thiazol-4-yl)-piperazin,
[5] 2-Methyl-1-(3-(3-chloro-phenyl)-propiolyl)-4-(1,3,4-thiadiazol-2-yl)-piperazin,
[6] 3-(3-Chlorphenyl)-N-(2-(methyl(1,3,4-thiadiazol-2-yl)amino)ethyl)propiolamid,
[7] 3-(3-Chlorphenyl)-N-(2-(methyl(thiazol-5-yl)amino)ethyl)propiolamid und
[8] 3-(3-Chlorphenyl)-N-(2-(methyl(1-methyl-1H-imidazol-2-yl)amino)ethyl)-propiolamid;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.
Ebenfalls besonders bevorzugt sind substituierte Propiolsäureamide der vorstehend angegebenen allgemeinen Formel I, die nach 60 Minuten Inkubation in 450 μg Protein aus Schweinehimhomogenat bei einer Temperatur zwischen 20 °C und 25 °C in einer Konzentration kleiner 2000 nM, bevorzugt kleiner 1000 nM, besonders bevorzugt kleiner 700 nM, ganz besonders bevorzugt kleiner 100 nM, noch weiter bevorzugt kleiner 30 nM, eine 50-prozentige Verdrängung von [$^3$H]-2-Methyl-6-(3-methoxyphenyl)-ethinylpyridin bewirken, dass in einer Konzentration von 5 nM vorliegt.

Die Bestimmung der Verdrängung von [$^3$H]-2-Methyl-6-(3-methoxyphenyl)-ethinylpyridin erfolgt dabei wie im Abschnitt Pharmakologische Methoden, I. Methode zur Bestimmung der Hermmung der [$^3$H]-MPEP-Bindung im mGluR5-Rezeptor-Bindungsassay beschrieben.

Ein weiterer Gegenstand der vorliegenden Erfindung ist ein Verfahren zur Herstellung von Verbindungen der vorstehend angegebenen allgemeinen Formel I gemäß dem wenigstens eine Verbindung der allgemeinen Formel II,

A-X,

worin A die vorstehend genannte Bedeutung hat und X für eine Abgangsgruppe, vorzugsweise für einen Halogen-Rest oder einen Sulfonsäureester steht, mit wenigstens einer Verbindung der allgemeinen Formel III,

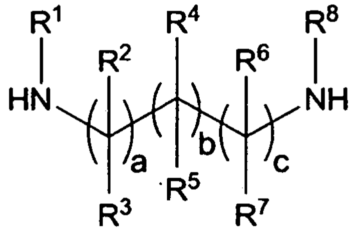

III,

worin a, b, c, $R^1$, $R^2$, $R^3$, $R^4$, $R^5$, $R^6$, $R^7$ und $R^8$ die vorstehend genannte Bedeutung haben, ggf. in einem Reaktionsmedium, ggf. in Gegenwart wenigstens einer Base, vorzugsweise in Gegenwart wenigstens einer Base ausgewählt aus der Gruppe bestehend aus Triethylamin, Natriumhydrogencarbonat, Dimethylaminopyridin, Kaliumcarbonat und Natriumhydroxid, und/oder wenigstens einer metallorganischen Verbindung und/oder wenigstens eines Metallhydrid-Reagenzes, vorzugsweise in Gegenwart wenigstens einer metallorganischen Verbindung oder eines Metallhydrid-Reagenzes ausgewählt aus der Gruppe bestehend aus n-Butyllithium, Phenyllithium, Natriumhydrid, Kaliumhydrid und Natriumamid, oder in Gegenwart wenigstens eines Kupfersalzes, bevorzugt in Gegenwart wenigstens eines Kupfersalzes ausgewählt aus der Gruppe bestehend aus Kupfer(I)chlorid und Kupfer(I)iodid, und ggf. in Gegenwart wenigstens eines Metalls, vorzugsweise in Gegenwart von Kupfer, vorzugsweise bei einer Temperatur von - 70 °C bis 300 °C, besonders bevorzugt von - 70 °C bis 150 °C, in wenigstens eine entsprechende Verbindung der allgemeinen Formel IV, ggf. in

Form eines entsprechenden Salzes,

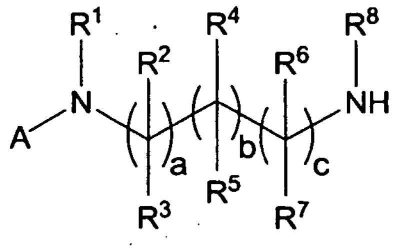

IV,

worin a, b, c, A, $R^1$, $R^2$, $R^3$, $R^4$, $R^5$, $R^6$, $R^7$ und $R^8$, die vorstehend genannte Bedeutung haben, überführt wird und diese ggf. gereinigt und/oder isoliert wird;
oder wenigstens eine Verbindung der allgemeinen Formel II mit wenigstens einer Verbindung der allgemeinen Formel V,

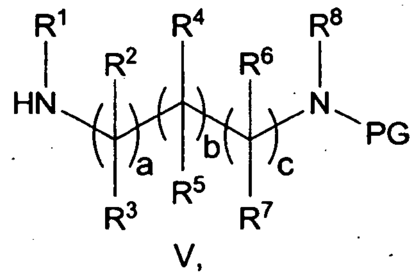

V,

worin a, b, c, $R^1$, $R^2$, $R^3$, $R^4$, $R^5$, $R^6$, $R^7$ und $R^8$, die vorstehend genannte Bedeutung haben und PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht, ggf. in einem Reaktionsmedium, ggf. in Gegenwart wenigstens einer Base, vorzugsweise in Gegenwart wenigstens einer Base ausgewählt aus der Gruppe bestehend aus Triethylamin, Natriumhydrogencarbonat, Dimethylaminopyridin, Kaliumcarbonat und Natriumhydroxid, und/oder wenigstens einer metallorganischen Verbindung und/oder wenigstens eines Metallhydrid-Reagenzes, vorzugsweise in Gegenwart wenigstens einer metallorganischen Verbindung oder eines Metallhydrid-Reagenzes ausgewählt aus der Gruppe bestehend aus n-Butyllithium, Phenyllithium, Natriumhydrid, Kaliumhydrid und Natriumamid, oder in Gegenwart-wenigstens eines Kupfersalzes, bevorzugt in Gegenwart wenigstens eines Kupfersalzes ausgewählt aus der Gruppe bestehend aus Kupfer (I)chlorid und Kupfer(I)iodid, und ggf. in Gegenwart wenigstens eines Metalls, vorzugsweise in Gegenwart von Kupfer, oder in Gegenwart wenigstens eines Katalysators, bevorzugt in Gegenwart wenigstens eines Palladium-Katalysators ausgewählt aus der Gruppe bestehend aus Bis(dibenzylidenaceton)palladium $[Pd(dba)_2]$; Tris(dibenzylidenaceton)dipalladium $[Pd_2dba_3]$; Dichlorobis(tri-*o*-tolylphosphin)palladium $[Pd(P(o\text{-}Tol)_3)_2Cl_2]$; Palladiumchlorid $[PdCl_2]$; Palladiumacetat $[Pd(OAc)_2]$; Palladiumtrifluoroacetat $[Pd(O_2CCF_3)_2]$; Tetrakistriphenylphosphinpalladium $[Pd(PPh_3)_4]$; Bistriphenylphosphinpalladium-dichlorid $[Pd(PPh_3)_2Cl_2]$; Bistriphenylphosphinpalladiumacetat $[Pd(PPh_3)_2(OAc)_2]$ und Dichloro[1,1'-bis(diphenylphosphino)ferrocen]palladium $[Pd(dppf)Cl_2]$, ggf. in Gegenwart wenigstens eines Liganden, bevorzugt in Gegenwart wenigstens eines Liganden ausgewählt aus der Gruppe bestehend aus (±)-2,2'-Bis(diphenylphosphino)-1,1'-binaphthalen (BINAP), Diphenylphosphinoferrocen (dppf), tri-*tert.*-Butylphosphin $[P(tBu)_3]$, Triphenylphosphin $[P(Ph)_3]$, tri-*ortho*-Tolylphosphin $[P(oTol)_3]$, tri-2-Furylphosphin $[P(2\text{-}furyl)_3]$, Dicyclohexyl-(2',6'-dimethoxybiphenyl-2-yl)phosphin, Dicyclohexyl-(2',6'-diisopropoxybiphenyl-2-yl)phosphin und Biphenyl-2-yldicyclohexylphosphin, ggf. in Gegenwart wenigstens einer organischen oder anorganischen Base gewählt aus der Gruppe Kalium-*tert*-Butylat, Natrium-*tert*-Butylat, tri-Kaliumphosphat, Cäsiumcarbonat, Lithiumbis(trimethylsilyl)amid, Natriumbis(trimethylsilyl)amid und Kaliumbis(trimethylsilyl)amid, vorzugsweise bei einer Temperatur von - 70 °C bis 300 °C in wenigstens eine entsprechende Verbindung der allgemeinen Formel VI,

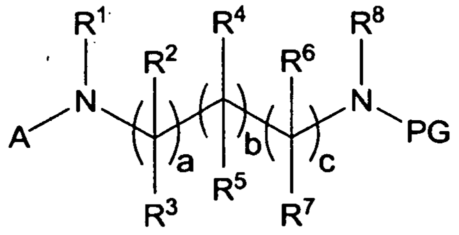

VI,

worin a, b, c, A, $R^1$, $R^2$, $R^3$, $R^4$, $R^5$, $R^6$, $R^7$, $R^8$ und PG die vorstehend genannte Bedeutung haben, überführt wird und diese ggf. gereinigt und/oder isoliert wird;
oder wenigstens eine Verbindung der allgemeinen Formel VII,

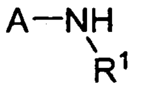

VII,

worin $R^1$ und A die vorstehend genannte Bedeutung haben, mit wenigstens einer Verbindung der allgemeinen Formel VIII,

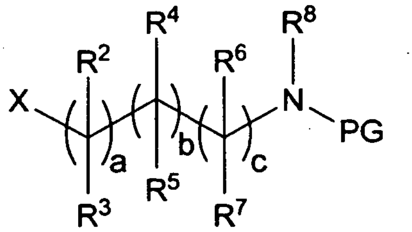

VIII,

worin a, b, c, $R^2$, $R^3$, $R^4$, $R^5$, $R^6$, $R^7$, $R^8$ und PG die vorstehend genannte Bedeutung haben und X für eine Abgangsgruppe, vorzugsweise für einen Halogen-Rest oder für einen Sulfonsäureester, besonders bevorzugt für einen Chlor- oder Brom-Rest steht, ggf. in einem Reaktionsmedium, ggf. in Gegenwart wenigstens einer Base, vorzugsweise in Gegenwart wenigstens einer Base ausgewählt aus der Gruppe bestehend aus Kalium-tert-butylat, Natriumhydroxid, Kaliumhydroxid, Dimethylamin und Triethylamin, besonders bevorzugt in Gegenwart von Diethylamin, oder ggf. in Gegenwart wenigstens einer metallorganischen Verbindung, bevorzugt in Gegenwart wenigstens einer metallorganischen Verbindung ausgewählt aus der Gruppe bestehend aus Methyllithium und Butyllithium oder ggf. in Gegenwart wenigstens einer Metallhydridverbindung, besonders bevorzugt in Gegenwart von Natriumhydrid, vorzugsweise bei einer Temperatur von - 70 °C bis 300 °C, besonders bevorzugt von - 70°C bis 150°C, in wenigstens eine entsprechende Verbindung der allgemeinen Formel VI überführt wird und diese ggf. gereinigt und/oder isoliert wird;
und wenigstens eine Verbindung der allgemeinen Formel VI für den Fall, dass PG für eine tert-Butoxycarbonyl- oder 9-Fluorenylmethyloxycarbonyl-Gruppe steht, in einem Reaktionsmedium, in Gegenwart wenigstens einer Säure, vorzugsweise in Gegenwart wenigstens einer Säure ausgewählt aus der Gruppe bestehend aus Salzsäure und Trifluoressigsäure, vorzugsweise bei einer Temperatur zwischen -70 °C bis 100°C oder für den Fall, dass PG für eine Benzyl-Gruppe oder Benzyloxycarbonyl-Gruppe steht, in einem Reaktionsmedium, in Gegenwart von Wasserstoff und in Gegenwart wenigstens eines Katalysators, vorzugsweise in Gegenwart von Palladium auf Kohle, vorzugsweise bei einer Temperatur zwischen - 70 °C bis 100°C in wenigstens eine entsprechende Verbindung der allgemeinen Formel IV, ggf. in Form eines entsprechenden Salzes überführt wird, und diese ggf. gereinigt und/oder isoliert wird;
und wenigstens eine Verbindung der allgemeinen Formel IV durch Umsetzung mit wenigstens einer Verbindung der allgemeinen Formel $R^9$-C≡C-C(=O)-OH, worin $R^9$ die vorstehend genannte Bedeutung hat, in einem Reaktionsmedium,

ggf. in Gegenwart wenigstens eines geeigneten Kopplungsmittels, ggf. in Gegenwart wenigstens einer Base, vorzugsweise in Gegenwart wenigstens einer Base ausgewählt aus der Gruppe bestehend aus Triethylamin, Pyridin, Dimethylaminopyridin, Diisopropylethylamin und N-Methyl-morpholin, vorzugsweise bei einer Temperatur von - 70°C bis 100°C, oder durch Umsetzung mit wenigstens einer Verbindung der allgemeinen Formel $R^9$-C≡C-C(=O)-X, worin $R^9$ die vorstehend genannte Bedeutung hat und X für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise in Gegenwart wenigstens einer Base ausgewählt aus der Gruppe bestehend aus Triethylamin; Dimethylaminopyridin, Pyridin und Diisopropylethylamin, vorzugsweise bei einer Temperatur von - 70 °C bis 100°C, in wenigstens eine entsprechende Verbindung der allgemeinen Formel I, ggf. in Form eines entsprechenden Salzes überführt wird,

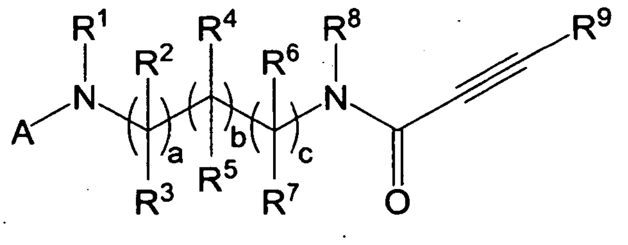

I,

worin a, b, c, A, $R^1$, $R^2$, $R^3$, $R^4$, $R^5$, $R^6$, $R^7$, $R^8$ und $R^9$ die vorstehend genannte Bedeutung haben, und diese ggf. gereinigt und/oder isoliert wird;

oder wenigstens eine Verbindung der allgemeinen Formel IV durch Umsetzung mit Propinsäure [HC≡C-C(=O)-OH] in einem Reaktionsmedium, ggf. in Gegenwart wenigstens eines geeigneten Kopplungsmittels, ggf. in Gegenwart wenigstens einer Base, vorzugsweise in Gegenwart wenigstens einer Base ausgewählt aus der Gruppe bestehend aus Triethylamin, Pyridin, Dimethylaminopyridin, Diisopropylethylamin und N-Methyl-morpholin, vorzugsweise bei einer Temperatur von - 70 °C bis 100 °C, oder durch Umsetzung mit wenigstens einer Verbindung der allgemeinen Formel HC≡C-C(=O)-X, worin X für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise in Gegenwart wenigstens einer Base ausgewählt aus der Gruppe bestehend aus Triethylamin, Dimethylaminopyridin, Pyridin und Diisopropylethylamin, vorzugsweise bei einer Temperatur von - 70 °C bis 100°C, in wenigstens eine entsprechende Verbindung der allgemeinen Formel IX, ggf. in Form eines entsprechenden Salzes überführt wird,

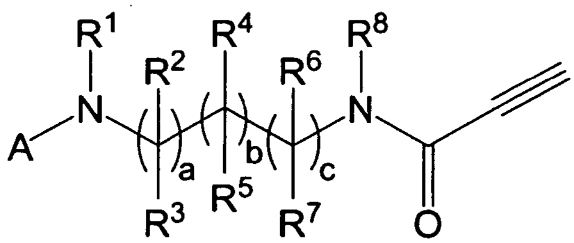

IX,

worin a, b, c, A, $R^1$, $R^2$, $R^3$, $R^4$, $R^5$, $R^6$, $R^7$ und $R^8$, die vorstehend genannte Bedeutung haben, und diese ggf. gereinigt und/oder isoliert wird,

und wenigstens eine Verbindung der allgemeinen Formel IX durch Umsetzung mit wenigstens einer Verbindung der allgemeinen Formel $R^9$-X, worin $R^9$ die vorstehend genannte Bedeutung und X für eine Abgangsgruppe, vorzugsweise für einen Halogen-Rest oder einen Sulfonsäureester, besonders bevorzugt für Iod, Brom oder Triflat, steht, in einem Reaktionsmedium, ggf. in Gegenwart wenigstens eines Katalysators, bevorzugt in Gegenwart wenigstens eines Palladium-Katalysators ausgewählt aus der Gruppe bestehend aus Palladiumchlorid [$PdCl_2$], Palladiumacetat [$Pd(OAc)_2$], Tetrakistriphenylphosphinpalladium [$Pd(PPh_3)_4$], Bistriphenylphosphinpalladiumdichlorid [$Pd(PPh_3)_2Cl_2$] und Bistriphenylphosphinpalladiumacetat [$Pd(PPh_3)_2(OAc)_2$], ggf. in Gegenwart wenigstens eines Liganden, bevorzugt in Gegenwart

wenigstens eines Liganden ausgewählt aus der Gruppe bestehend aus Triphenylphosphin, Triphenylarsin und Tri-2-furyl-phosphin, ggf. in Gegenwart wenigstens eines anorganischen Salzes, bevorzugt in Gegenwart wenigstens eines anorganischen Salzes ausgewählt aus der Gruppe bestehend aus Lithiumchlorid und Zinkchlorid, ggf. in Gegenwart wenigstens eines Kupfersalzes, bevorzugt in Gegenwart von Kupferiodid, ggf. in Gegenwart wenigstens einer organischen oder anorganischen Base, vorzugsweise in Gegenwart wenigstens einer Base ausgewählt aus der Gruppe bestehend aus Triethylamin, [1,4]-Diazabicyclo-[2.2.2]-octan, Diisopropylamin, Diisopropylethylamin, Kaliumcarbonat und Natriumhydrogencarbonat, vorzugsweise bei einer Temperatur zwischen -70 °C bis 300 °C in wenigstens eine entsprechende Verbindung der allgemeinen Formel I, ggf. in Form eines entsprechenden Salzes überführt wird, und diese ggf. gereinigt und/oder isoliert wird.

Ebenfalls ein Gegenstand der vorliegenden Erfindung ist ein Verfahren zur Herstellung einer Verbindung der allgemeinen Formel I, worin wenigstens eine Verbindung der allgemeinen Formel III,

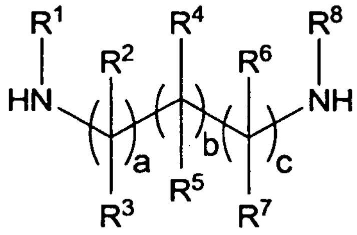

III,

worin a, b, c, $R^1$, $R^2$, $R^3$, $R^4$, $R^5$, $R^6$, $R^7$ und $R^8$ die vorstehend genannte Bedeutung haben,
durch Umsetzung mit wenigstens einer Verbindung der allgemeinen Formel $R^9$-C≡C-C(=O)-OH, worin $R^9$ die vorstehend genannte Bedeutung hat, in einem Reaktionsmedium, ggf. in Gegenwart wenigstens eines geeigneten Kopplungsmittels, ggf. in Gegenwart wenigstens einer Base, vorzugsweise in Gegenwart wenigstens einer Base ausgewählt aus der Gruppe bestehend aus Triethylamin, Pyridin, Dimethylaminopyridin, Diisopropylethylamin und N-Methyl-morpholin, vorzugsweise bei einer Temperatur von - 70°C bis 100°C, oder durch Umsetzung mit wenigstens einer Verbindung der allgemeinen Formel $R^9$-C≡C-C(=O)-X, worin $R^9$ die vorstehend genannte Bedeutung hat und X für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise in Gegenwart wenigstens einer Base ausgewählt aus der Gruppe bestehend aus Triethylamin, Dimethylaminopyridin, Pyridin und Diisopropylethylamin, vorzugsweise bei einer Temperatur von - 70 °C bis 100°C, in wenigstens eine entsprechende Verbindung der allgemeinen Formel XI, ggf. in Form eines entsprechenden Salzes,

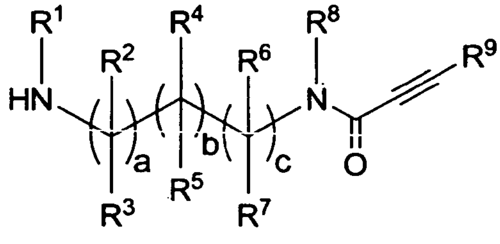

XI,

worin a, b, c, $R^1$, $R^2$, $R^3$, $R^4$, $R^5$, $R^6$, $R^7$, $R^8$ und $R^9$ die vorstehend genannte Bedeutung haben, überführt wird und diese ggf. gereinigt und/oder isoliert wird;
oder wenigstens eine Verbindung der allgemeinen Formel V,

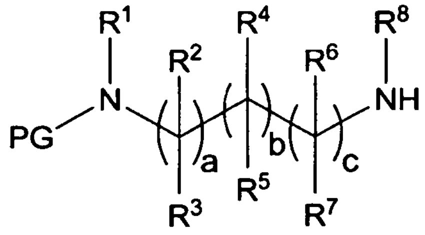

V,

worin a, b, c, $R^1$, $R^2$, $R^3$, $R^4$, $R^5$, $R^6$, $R^7$ und $R^8$ die vorstehend genannte Bedeutung haben und PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht,
durch Umsetzung mit wenigstens einer Verbindung der allgemeinen Formel $R^9$-C≡C-C(=O)-OH, worin $R^9$ die vorstehend genannte Bedeutung hat, in einem Reaktionsmedium, ggf. in Gegenwart wenigstens eines geeigneten Kopplungsmittels, ggf. in Gegenwart wenigstens einer Base, vorzugsweise in Gegenwart wenigstens einer Base ausgewählt aus der Gruppe bestehend aus Triethylamin, Pyridin, Dimethylaminopyridin, Diisopropylethylamin und N-Methyl-morpholin, vorzugsweise bei einer Temperatur von - 70°C bis 100°C, oder durch Umsetzung mit wenigstens einer Verbindung der allgemeinen Formel $R^9$-C≡C-C(=O)-X, worin $R^9$ die vorstehend genannte Bedeutung hat und X für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise in Gegenwart wenigstens einer Base ausgewählt aus der Gruppe bestehend aus Triethylamin, Dimethylaminopyridin, Pyridin und Diisopropylethylamin, vorzugsweise bei einer Temperatur von - 70 °C bis 100°C, in wenigstens eine entsprechende Verbindung der allgemeinen Formel XII, ggf. in Form eines entsprechenden Salzes,

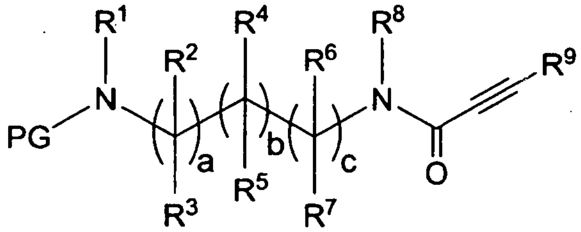

XII,

worin a, b, c, $R^1$, $R^2$, $R^3$, $R^4$, $R^5$, $R^6$, $R^7$, $R^8$, $R^9$ und PG die vorstehend genannte Bedeutung haben, überführt wird und diese ggf. gereinigt und/oder isoliert wird;
und wenigstens eine Verbindung der allgemeinen Formel XII für den Fall, dass PG für eine tert-Butoxycarbonyl- oder 9-Fluorenylmethyloxycarbonyl-Gruppe steht, in einem Reaktionsmedium, in Gegenwart wenigstens einer Säure, vorzugsweise in Gegenwart wenigstens einer Säure ausgewählt aus der Gruppe bestehend aus Salzsäure und Trifluoressigsäure, vorzugsweise bei einer Temperatur zwischen -70 °C bis 100°C oder für den Fall, dass PG für eine Benzyl- oder Benzyloxycarbonyl-Gruppe steht, in einem Reaktionsmedium, in Gegenwart von Wasserstoff und in Gegenwart wenigstens eines Katalysators, vorzugsweise in Gegenwart von Palladium auf Kohle, vorzugsweise bei einer Temperatur zwischen -70 °C bis 100°C in wenigstens eine entsprechende Verbindung der allgemeinen Formel XI, ggf. in Form eines entsprechenden Salzes überführt wird, und diese ggf. gereinigt und/oder isoliert wird;
und wenigstens eine Verbindung der allgemeinen Formel XI durch Umsetzung mit wenigstens einer Verbindung der allgemeinen Formel II,

A-X,

worin der Rest A die vorstehend genannte Bedeutung hat und X für eine Abgangsgruppe vorzugsweise für einen Halogen-Rest oder einen Sulfonsäureester, steht, in einem Reaktionsmedium, ggf. in Gegenwart wenigstens einer Base, vorzugsweise in Gegenwart wenigstens einer Base ausgewählt aus der Gruppe bestehend aus Triethylamin, Natriumhydrogencarbonat, Dimethylaminopyridin, Kaliumcarbonat und Natriumhydroxid, und/oder wenigstens einer metallorganischen Verbindung und/oder wenigstens eines Metallhydrid-Resgenzes, vorzugsweise in Gegenwart wenigstens einer metallorganischen Verbindung oder eines Metallhydrid-Reagenzes ausgewählt aus der Gruppe bestehend aus n-Butyllithium, Phenyllithium, Natriumhydrid, Kaliumhydrid und Natriumamid, oder in Gegenwart wenigstens eines Kupfersalzes, bevorzugt in Gegenwart wenigstens eines Kupfersalzes ausgewählt aus der Gruppe bestehend aus Kupfer(I) chlorid und Kupfer(I)iodid, und ggf. in Gegenwart wenigstens eines Metalls, vorzugsweise in Gegenwart von Kupfer, oder in Gegenwart wenigstens eines Katalysators, bevorzugt in Gegenwart wenigstens eines Palladium-Katalysators ausgewählt aus der Gruppe bestehend aus Bis(dibenzylidenaceton)palladium [$Pd(dba)_2$]; Tris(dibenzylidenaceton)dipalladium [$Pd_2dba_3$]; Dichlorobis(tri-*o*-tolylphosphin)palladium [$Pd(P(o\text{-}Tol)_3)_2Cl_2$]; Palladiumchlorid [$PdCl_2$]; Palladiumacetat [$Pd(OAc)_2$]; Palladiumtrifluoroacetat [$Pd(O_2CCF_3)_2$]; Tetrakistriphenylphosphinpalladium [$Pd(PPh_3)_4$]; Bistriphenylphosphinpalladium-dichlorid [$Pd(PPh_3)_2Cl_2$]; Bistriphenylphosphinpalladiumacetat [$Pd(PPh_3)_2(OAc)_2$] und Dichloro[1,1'-bis(diphenylphosphino)ferrocen]palladium [$Pd(dppf)Cl_2$], ggf. in Gegenwart wenigstens eines Liganden, bevorzugt in Gegenwart wenigstens eines Liganden ausgewählt aus der Gruppe bestehend aus (±)-2,2'-Bis(diphenylphosphino)-1,1'-binaphthalen (BINAP), Diphenylphosphinoferrocen (dppf), tri-*tert*.-Butylphosphin [$P(tBu)_3$], Triphenylphosphin [$P(Ph)_3$], tri-*ortho*-Tolylphosphin [$P(oTol)_3$], tri-2-Furylphosphin [$P(2\text{-}furyl)_3$], Dicyclohexyl-(2',6'-dimethoxybiphenyl-2-yl)phosphin, Dicyclohexyl-(2',6'-diisopropoxybiphenyl-2-yl)phosphin und Biphenyl-2-yldicyclohexylphosphin, ggf. in Gegenwart wenigstens einer organischen oder anorganischen Base gewählt aus der Gruppe Kalium-*tert*-Butylat, Natrium-*tert*-Butylat, tri-Kaliumphosphat, Cäsiumcarbonat, Lithiumbis(trimethylsilyl)amid, Natriumbis(trimethylsilyl)amid und Kaliumbis(trimethylsilyl)amid, vorzugsweise bei einer Temperatur von - 70 °C bis 300 °C in wenigstens eine entsprechende Verbindung der allgemeinen Formel I, ggf. in Form eines entsprechenden Salzes überführt wird,

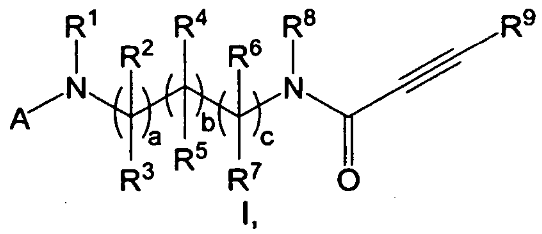

I,

worin a, b, c. A, $R^1$, $R^2$, $R^3$, $R^4$, $R^5$, $R^6$, $R^7$, $R^8$ und $R^9$ die vorstehend genannte Bedeutung haben, und diese ggf. gereinigt und/oder isoliert wird.

Ein erfindungsgemäßes Verfahren zur Herstellung substituierter Propiolsäureamide der vorstehend angegebenen allgemeinen Formel I ist auch im folgenden Schema 1 angegeben.

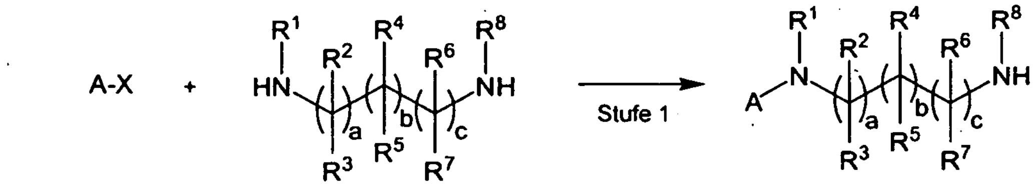


II III IV

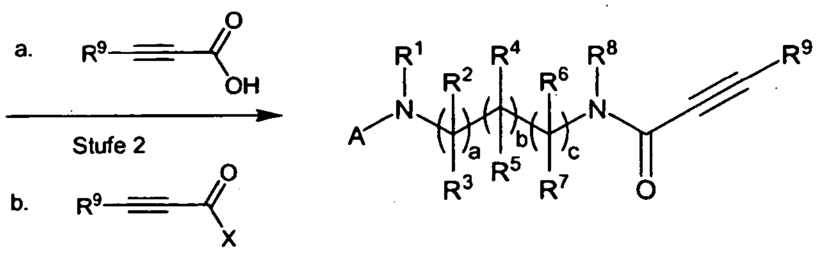


I

Schema 1.

In Stufe 1 werden Verbindungen der vorstehend angegebenen allgemeinen Formel II, worin X für eine Abgangsgruppe, vorzugsweise für einen Halogen-Rest oder einen Sulfonsäureester ausgewählt aus der Gruppe bestehend aus Mesylat, Triflat und Tosylat, besonders bevorzugt für ein Chlor- oder Bromatom steht, mit Verbindungen der vorstehend angegebenen allgemeinen Formel III, ggf. in einem Reaktionsmedium, vorzugsweise ausgewählt aus der Gruppe bestehend aus Methanol, Ethanol, Isopropanol, n-Butanol, Diethylether, Tetrahydrofuran, Dichlormethan, Chloroform, Dimethylformamid, Acetonitril, Pyridin, Dioxan, Ethylacetat, Dimethylsulfoxid, Toluol und entsprechenden Mischungen, besonders bevorzugt in einem Reaktionsmedium ausgewählt aus der Gruppe bestehend aus Methanol, Ethanol und n-Butanol, ggf. in Gegenwart wenigstens einer organischen oder anorganischen Base, vorzugsweise ausgewählt aus der Gruppe bestehend aus Triethylamin, Natriumhydrogencarbonat, Dimethylaminopyridin, Kaliumcarbonat und Natriumhydroxid, und/oder ggf. in Gegenwart wenigstens eines Metallsalzes, vorzugsweise eines Kupfersalzes, besonders bevorzugt Kupfer(I)-jodid oder Kupfer(I)chlorid, und ggf. in Gegenwart wenigstens eines Metalls, bevorzugt Kupfer, und/oder ggf. in Gegenwart wenigstens einer metallorganischen Verbindung oder eines Metallhydrid-Reagenzes, vorzugsweise ausgewählt aus der Gruppe bestehend aus n-Butyllithium, Phenyllithium, Natriumhydrid, Kaliumhydrid und Natriumamid, vorzugsweise bei Temperaturen von -70 °C bis 300 °C, besonders bevorzugt bei Temperaturen von -70 °C bis 150 °C, zu Verbindungen der allgemeinen Formel IV umgesetzt.

In Stufe 2 werden Verbindungen der vorstehend angegebenen allgemeinen Formel IV mit Carbonsäuren der vorstehend angegebenen allgemeinen Formel $R^9$-C≡C-C(=O)-OH in einem Reaktionsmedium, vorzugsweise ausgewählt aus der Gruppe bestehend aus Diethylether, Tetrahydrofuran, Acetonitril, Methanol, Ethanol, (1,2)-Dichlorethan, Dimethylformamid, Dichlormethan und entsprechenden Mischungen, ggf. in Gegenwart wenigstens eines Kopplungsmittels, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), PL-EDC (polymergebundenes N-Benzyl-3-((ethylimino)methylenamino)-N,N-dimethylpropan-1-aminium chlorid), 1,1'-Carbonyl-diimidazol (CDI), N-[(Dimethyamino)-1H-1,2.3-triazolo[4,5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU), O-(Benzotriazol-1-yl)-N,N,N',N'-tetramethyluronium-tetrafluoroborat (TBTU), 1-Hydroxy-7-azabenzotriazol (HOAt) und polermergebundenes Carbodiimid Harz (PS-carbodiimid resing, PSii), besonders bevorzugt in Gegenwart eines Kopplungsmittels ausgewählt aus der Gruppe bestehend aus TBTU, EDCI und PL-EDC, ggf. in Gegenwart wenigstens einer organischen Base, vorzugsweise ausgewählt aus der Gruppe bestehend aus Triethylamin, Pyridin, Dimethylaminopyridin, N-Methylmorpholin, [1,4]-Diazabicyclo-[2.2.2]-octan (DABCO), 1,8-Diazabicyclo[5.4.0]undec-7-en (DBU), 1,5-Diazabicyclo[4.3.0]non-5-en (DBN) und Diisopropylethylamin, bevorzugt in Gegenwart von Diisopropylethylamin oder Triethylamin, vorzugsweise bei Temperaturen von -70°C bis 250°C zu Verbindungen der allgemeinen Formel I umgesetzt.

Alternativ werden Verbindungen der vorstehend angegebenen allgemeinen Formel IV mit Carbonsäurederivaten der vorstehend angegebenen allgemeinen Formel $R^9$-C≡C-C(=O)-X, worin X für eine Abgangsgruppe, bevorzugt

einen Halogen-Rest, besonders bevorzugt Chlor oder Brom, steht, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan, 1,2-Dichlorethan und entsprechenden Mischungen, ggf. in Gegenwart einer organischen oder anorganischen Base, vorzugsweise ausgewählt aus der Gruppe bestehend aus Triethylamin, Dimethylaminopyridin, Pyridin und Diisopropylamin, bei Temperaturen von -70°C bis 250°C zu Verbindungen der allgemeinen Formel I umgesetzt.

Ein weiteres erfindungsgemäßes Verfahren zur Herstellung von Verbindungen der allgemeinen Formel IV ist auch im folgenden Schema 2 angegeben.

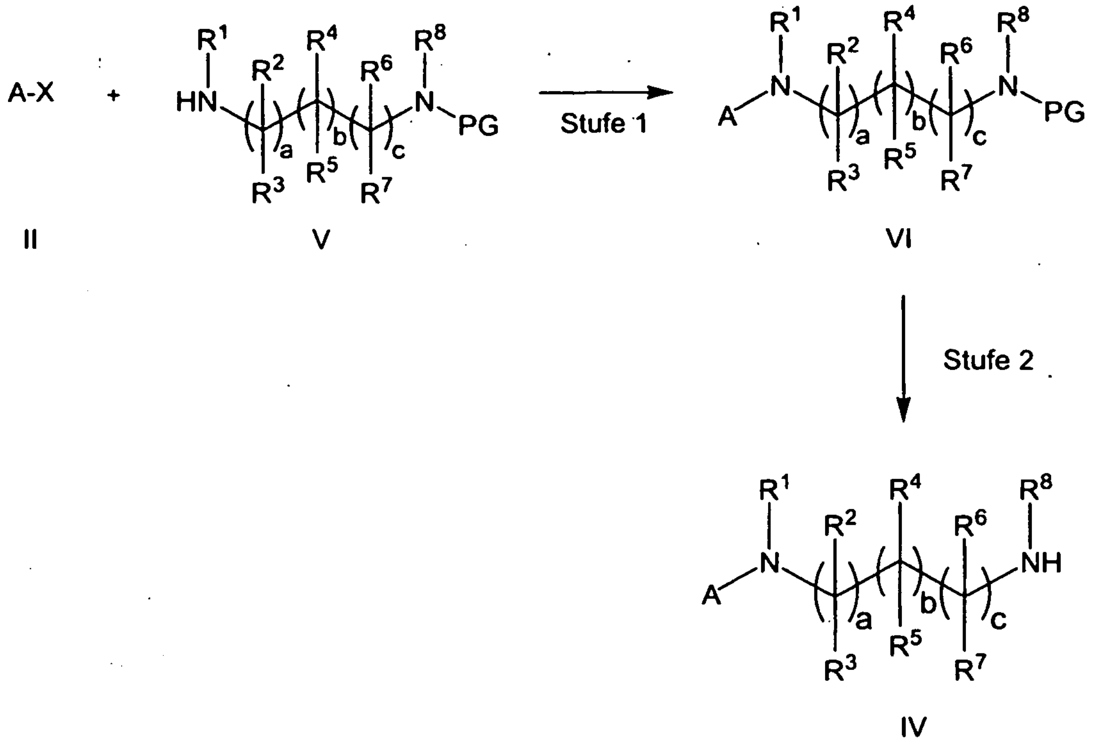


II V VI IV

Schema 2.

In Stufe 1 werden Verbindungen der vorstehend angegebenen allgemeinen Formel II, worin X für eine Abgangsgruppe, vorzugsweise für einen Halogen-Rest oder einen Sulfonsäureester ausgewählt aus der Gruppe bestehend aus Mesylat, Triflat und Tosylat, besonders bevorzugt für ein Chlor- oder Bromatom steht, mit Verbindungen der vorstehend angegebenen allgemeinen Formel V, worin PG für eine Schutzgruppe, bevorzugt für eine Schutzgruppe ausgewählt aus der Gruppe bestehend aus tert-Butyloxy-carbonyl, Benzyloxycarbonyl, Benzyl und 9-Fluorenylmethyloxycarbonyl steht, ggf. in einem Reaktionsmedium, vorzugsweise ausgewählt aus der Gruppe bestehend aus Methanol, Ethanol, Isopropanol, n-Butanol, Diethylether, Tetrahydrofuran, Dichlormethan, Chloroform, Dimethylformamid, Acetonitril, Pyridin, Dioxan, Ethylacetat, Dimethylsulfoxid, Toluol und entsprechenden Mischungen, besonders bevorzugt in einem Reaktionsmedium ausgewählt aus der Gruppe bestehend aus Methanol, Ethanol und n-Butanol, ggf. in Gegenwart wenigstens einer organischen oder anorganischen Base, vorzugsweise ausgewählt aus der Gruppe bestehend aus Triethylamin, Natriumhydrogencarbonat, Dimethylaminopyridin, Kaliumcarbonat und Natriumhydroxid, und/oder ggf. in Gegenwart wenigstens eines Metallsalzes, vorzugsweise eines Kupfersalzes, besonders bevorzugt Kupfer(I) jodid oder Kupfer(I)chlorid, und ggf. in Gegenwart wenigstens eines Metalls, bevorzugt Kupfer, und/oder ggf. in Gegenwart wenigstens einer metallorganischen Verbindung oder eines Metallhydrid-Reagenzes, vorzugsweise ausgewählt aus der Gruppe bestehend aus n-Butyllithium, Phenyllithium, Natriumhydrid, Kaliumhydrid und Natriumamid, vorzugsweise bei Temperaturen von -70°C bis 300 °C, besonders bevorzugt bei Temperaturen von - 70°C bis 150°C, zu Verbindungen der allgemeinen Formel VI umgesetzt.

Alternativ werden in Stufe 1 Verbindungen der allgemeinen Formel II, worin X für eine Abgangsgruppe, vorzugsweise für einen Halogen-Rest oder einen Sulfonsäureester ausgewählt aus der Gruppe bestehend aus Mesylat, Triflat und Tosylat, besonders bevorzugt für ein Chlor-, Brom-, Iodatom oder Triflat, steht, mit Verbindungen der vorstehend angegebenen allgemeinen Formel V, ggf. in einem Reaktionsmedium, bevorzugt in einem Reaktionsmedium

ausgewählt aus der Gruppe bestehend aus Benzol, Toluol, Xylol, Trimethylbenzol, Tetrahydrofuran, Dimethoxyethan, Dioxan und entsprechenden Mischungen, besonders bevorzugt in einem Reaktionsmedium ausgewählt aus der Gruppe bestehend aus Toluol, Xylol und Trimethylbenzol, ggf. in Gegenwart wenigstens eines Katalysators, bevorzugt in Gegenwart wenigstens eines Katalysators ausgewählt aus der Gruppe bestehend aus Bis(dibenzylidenaceton)palladium [$Pd(dba)_2$], Tris(dibenzylidenaceton)dipalladium [$Pd_2dba_3$], Dichlorobis(tri-*o*-tolylphosphin)palladium [Pd(P(*o*-Tol)$_3$)$_2$Cl$_2$], Palladiumchlorid [$PdCl_2$], Palladiumacetat [$Pd(OAc)_2$], Palladiumtrifluoroacetat [$Pd(O_2CCF_3)_2$], Tetrakistriphenylphosphinpalladium [$Pd(PPh_3)_4$], Bistriphenylphosphinpalladiumdichlorid [$Pd(PPh_3)_2Cl_2$], Bistriphenylphosphinpalladiumacetat [$Pd(PPh_3)_2(OAc)_2$] und Dichloro[1,1'-bis(diphenylphosphino)ferrocen]palladium [Pd(dppf)$Cl_2$], besonders bevorzugt in Gegenwart eines Katalysators ausgewählt aus der Gruppe bestehend aus [$Pd(dba)_2$], [$Pd_2dba_3$], [Pd (P(*o*-Tol)$_3$)$_2$Cl$_2$], [$Pd(O_2CCF_3)_2$], [$Pd(PPh_3)_4$] und [$Pd(OAc)_2$], ggf. in Gegenwart wenigstens eines Liganden, bevorzugt in Gegenwart wenigstens eines Liganden ausgewählt aus der Gruppe bestehend aus (±)-2,2'-Bis(diphenylphosphino)-1,1'-binaphthalen (BINAP), Diphenylphosphinoferrocen (dppf), tri-*tert*.-Butylphosphin [P(*t*Bu)$_3$], Triphenylphosphin [P (Ph)$_3$], tri-*ortho*-Tolylphosphin [P(*o*Tol)$_3$], tri-2-Furylphosphin [P(2-furyl)$_3$], sterisch anspruchsvollen Biarylphosphinliganden (Buchwald-Liganden) wie Dicyclohexyl(2',6'-dimethoxybiphenyl-2-yl)phosphin, Dicyclohexyl(2',6'-diisopropoxybiphenyl-2-yl)phosphin und Biphenyl-2-yldicyclohexylphosphin, besonders bevorzugt in Gegenwart wenigstens eines Liganden ausgewählt aus der Gruppe bestehend aus [P(*t*Bu)$_3$], Dicyclohexyl(2',6'-dimethoxybiphenyl-2-yl)phosphin und Dicyclohexyl(2',6'-düsopropoxybiphenyl-2-yl)phosphin, ggf. in Gegenwart wenigstens einer organischen oder anorganischen Base gewählt aus der Gruppe bestehend aus Kalium-*tert*-Butylat (KO*t*Bu), Natrium-*tert*-Butylat (NaO*t*Bu), tri-Kaliumphosphat ($K_3PO_4$), Cäsiumcarbonat ($Cs_2CO_3$), Lithiumbis(trimethylsilyl)amid [$LiN(SiMe_3)_2$], Natriumbis(trimethylsilyl)amid [$NaN(SiMe_3)_2$] und Kaliumbis(trimethylsilyl)amid [$KN(SiMe_3)_2$], vorzugsweise bei einer Temperatur zwischen -70°C bis 300°C in eine Verbindung der allgemeinen Formel IV umgesetzt. Besonders bevorzugt werden Verbindungen der allgemeinen Formel II mit Verbindungen der allgemeinen Formel V in 1 ,2,4-Trimethylbenzol in Gegenwart von Pd (dba)$_2$Cl$_2$, [P(*t*Bu)$_3$] und NaO*t*Bu zu Verbindungen der allgemeinen Formel VI umgesetzt.

In Stufe 2 werden Verbindungen der allgemeinen Formel VI für den Fall, dass PG für eine tert-Butoxycarbonyl- oder 9-Fluorenylmethyloxycarbonyl-Gruppe steht, in einem Reaktionsmedium, vorzugsweise in einem Reaktionsmedium ausgewählt aus der Gruppe bestehend aus Methanol, Ethylacetat, Ethanol, Isopropanol, n-Butanol, Diethylether, Dioxan, Tetrahydrofuran, Chloroform, Dichlormethan, Dimethylformamid, Acetonitril, Pyridin, Dimethylsulfoxid, Toluol und entsprechenden Mischungen, in Gegenwart wenigstens einer Säure, vorzugsweise in Gegenwart wenigstens einer Säure ausgewählt aus der Gruppe bestehend aus Salzsäure und Trifluoressigsäure, vorzugsweise bei einer Temperatur zwischen -70°C bis 100 °C oder für den Fall, dass PG für eine Benzyl-Gruppe oder Benzyloxycarbonyl-Gruppe steht, in einem Reaktionsmedium, vorzugsweise in einem Reaktionsmedium ausgewählt aus der Gruppe bestehend aus Methanol, Ethylacetat, Ethanol, Isopropanol, n-Butanol, Diethylether, Dioxan, Tetrahydrofuran, Chloroform, Dichlormethan, Dimethylformamid, Acetonitril, Pyridin, Dimethylsulfoxid, Toluol und entsprechenden Mischungen, in Gegenwart von Wasserstoff und in Gegenwart wenigstens eines Katalysators, vorzugsweise in Gegenwart von Palladium auf Kohle, vorzugsweise bei einer Temperatur zwischen -70 °C bis 100 °C in eine entsprechende Verbindung der allgemeinen Formel IV überführt.

Geeignete Methoden zum Entfernen der vorstehend genannten Schutzgruppen lassen sich auch den Monografien Protective Groups in Organic Synthesis, T. W. Greene et al., 3rd edition, 1999, Wiley, New York und Protecting Groups, P. J. Kocienski, 3rd edition, 2004, Georg Thieme Verlag, Stuttgart 2004 entnehmen. Die entsprechenden Teile der Referenzen gelten hiermit als Teil der Offenbarung.

Ein weiteres erfindungsgemäßes Verfahren zur Herstellung substituierter Propiolsäureamide der vorstehend angegebenen allgemeinen Formel I ist auch im folgenden Schema 3 angegeben.

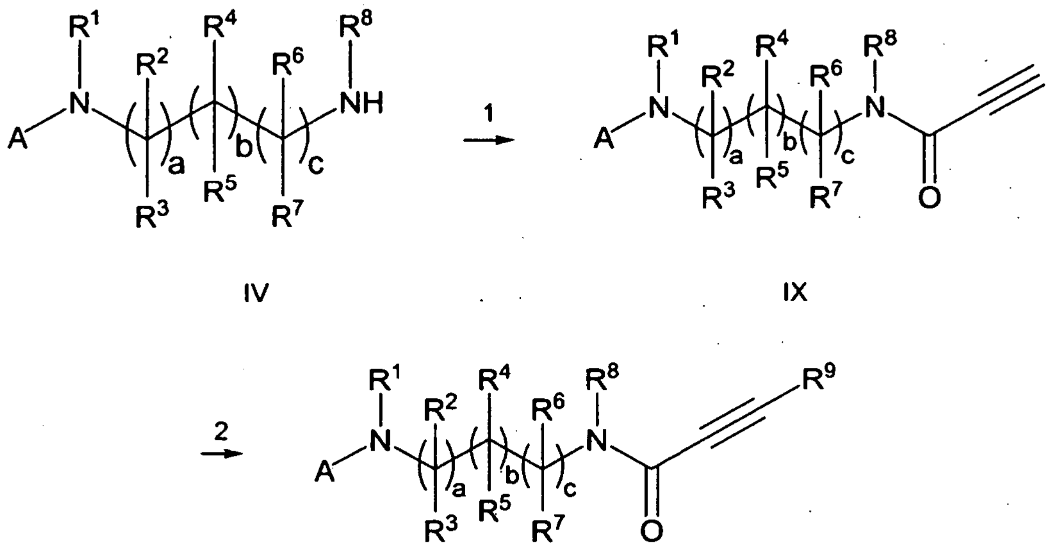


IV

IX

I

Schema 3.

In Stufe 1 werden Verbindungen der vorstehend angegebenen allgemeinen Formel IV mit Propiolsäure H-C≡C-C(=O)-OH oder mit Carbonsäurederivaten der allgemeinen Formel H-C≡C-C(=O)-X, worin X für eine Abgangsgruppe, bevorzugt einen Halogen-Rest, besonders bevorzugt Chlor oder Brom, steht, wie in Schema 1, Stufe 2, beschrieben, zu Verbindungen der allgemeinen Formel IX umgesetzt.

In Stufe 2 werden Verbindungen der vorstehend angegebenen allgemeinen Formel IX mit Verbindungen der allgemeinen Formel $R^9$-X, worin $R^9$ die vorstehend genannte Bedeutung mit Ausnahme von Wasserstoff hat und X für eine Abgangsgruppe, vorzugsweise für einen Halogen-Rest oder einen Sulfonsäureester, besonders bevorzugt für Iod, Brom oder Triflat, steht, in einem Reaktionsmedium, bevorzugt in einem Reaktionsmedium ausgewählt aus der Gruppe bestehend aus Methanol, Ethylacetat, Ethanol, Isopropanol, n-Butanol, Diethylether, Dioxan, Tetrahydrofuran, Chloroform, Dichlormethan, Dimethylformamid, Acetonitril, Pyridin, Dimethylsulfoxid, Wasser, Toluol und entsprechenden Mischungen, bevorzugt in Dimethylformamid, Wasser, Ethylacetat, Tetrahydrofuran und entsprechenden Mischungen, ggf. in Gegenwart wenigstens eines Katalysators, bevorzugt in Gegenwart wenigstens eines Katalysators ausgewählt aus der Gruppe bestehend aus Palladiumchlorid [$PdCl_2$], Palladiumacetat [$Pd(OAc)_2$], Tetrakistriphenylphosphinpalladium [$Pd(PPh_3)_4$], Bistriphenylphosphinpalladiumdichlorid [$Pd(PPh_3)_2Cl_2$] und Bistriphenylphosphinpalladiumacetat [$Pd(PPh_3)_2(OAc)_2$], besonders bevorzugt in Gegenwart eines Katalysators ausgewählt aus der Gruppe bestehend aus $Pd(PPh_3)_4$, $Pd(PPh_3)_2Cl_2$ und $Pd(PPh_3)_2(OAc)_2$, ggf. in Gegenwart wenigstens eines Liganden, bevorzugt in Gegenwart wenigstens eines Liganden ausgewählt aus der Gruppe bestehend aus Triphenylphosphin, Triphenylarsin und Tri-2-furyl-phosphin, bevorzugt in Gegenwart von Triphenylphosphin, ggf. in Gegenwart wenigstens eines anorganischen Salzes, bevorzugt in Gegenwart wenigstens eines anorganischen Salzes ausgewählt aus der Gruppe bestehend aus Lithiumchlorid und Zinkchlorid, ggf. in Gegenwart wenigstens eines Kupfersalzes, bevorzugt in Gegenwart von Kupferiodid, ggf. in Gegenwart wenigstens einer organischen oder anorganischen Base, vorzugsweise in Gegenwart wenigstens einer Base ausgewählt aus der Gruppe bestehend aus Triethylamin, [1,4]-Diazabicyclo-[2.2.2]-octan, Diisopropylamin, Diisopropylethylamin, Kaliumcarbonat und Natriumhydrogencarbonat, vorzugsweise bei einer Temperatur zwischen -70°C bis 300 °C in eine Verbindung der allgemeinen Formel I ungesetzt. Besonders bevorzugt werden Verbindungen der allgemeinen Formel $R^9$-I oder $R^9$-Br mit Verbindungen der allgemeinen Formel IX in Dimethylformamid in Gegenwart von $Pd(PPh_3)_2Cl_2$, Kupfer(I)iodid und Diisopropylamin oder Triethylamin umgesetzt.

Ein weiteres erfindungsgemäßes Verfahren zur Herstellung substituierter Propiolsäureamide vorstehend angegebenen allgemeinen Formel I ist auch im folgenden Schema 4 angegeben.

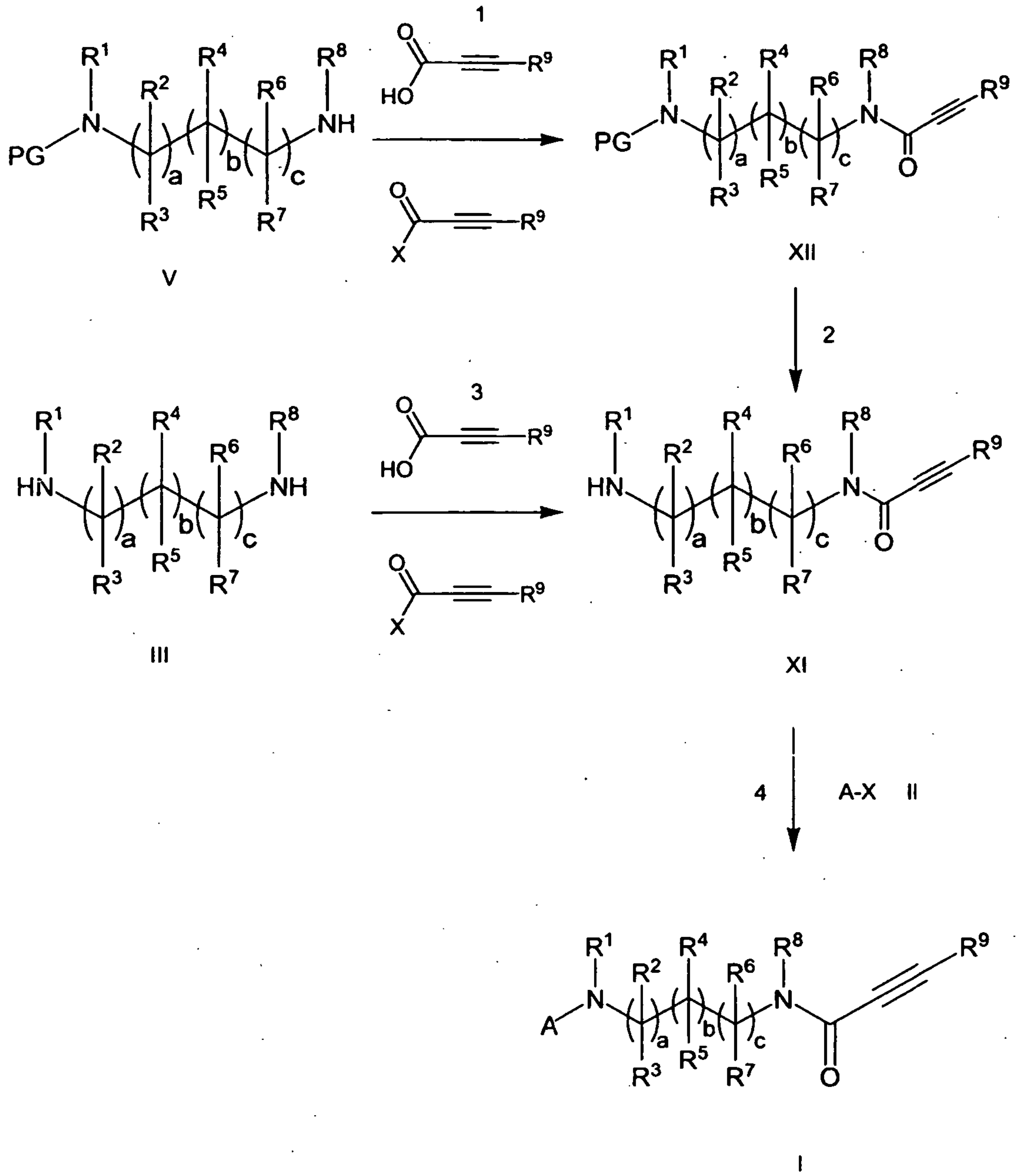


Schema 4.

In Stufe 1 werden Verbindungen der allgemeinen Formel V, worin PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht, mit Verbindungen der allgemeinen Formel $R^9$-C≡C-C(=O)-OH oder $R^9$-C≡C-C(=O)-X, worin X für eine Abgangsgruppe, bevorzugt einen Halogen-Rest, besonders bevorzugt Chlor oder Brom, steht, wie in Schema 1, Stufe 2, zu Verbindungen der allgemeinen Formel XII umgesetzt.

In Stufe 2 werden Verbindungen der allgemeinen Formel XII, worin PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht, wie in Schema 2, Stufe 2, beschrieben zu Verbindungen der allgemeinen Formel XI umgesetzt.

In Stufe 3 werden Verbindungen der vorstehend angegebenen allgemeinen Formel III mit Propiolsäure H-C≡C-C(=O)-OH oder mit Carbonsäurederivaten der vorstehend angegebenen allgemeinen Formel H-C≡C-C(=O)-X, worin X für eine Abgangsgruppe, bevorzugt einen Halogen-Rest, besonders bevorzugt Chlor oder Brom, steht, wie in Schema 1, Stufe 2, beschrieben, zu Verbindungen der allgemeinen Formel XI umgesetzt.

In Stufe 4 werden Verbindungen der allgemeinen Formel XI mit Verbindungen der allgemeinen Formel II wie in Schema 2, Stufe 1, zu Verbindungen der allgemeinen Formel I umgesetzt.

Verbindungen der allgemeinen Formel I, worin $R^1$ und $R^8$ jeweils für einen Wasserstoff-Rest stehen, im Folgenden als Verbindungen der allgemeinen Formel XV bezeichnet, lassen sich in Verbindungen der allgemeinen Formel I, worin $R^3$ und $R^8$ mit der sie verbindenden $-N-(CR^2R^3)_a-(CR^4R^5)_b-(CR^6R^7)_c-N-$Gruppe einen zyklischen Rest bilden, im Folgenden als Verbindungen der allgemeinen Formel XVI bezeichnet, überführen.

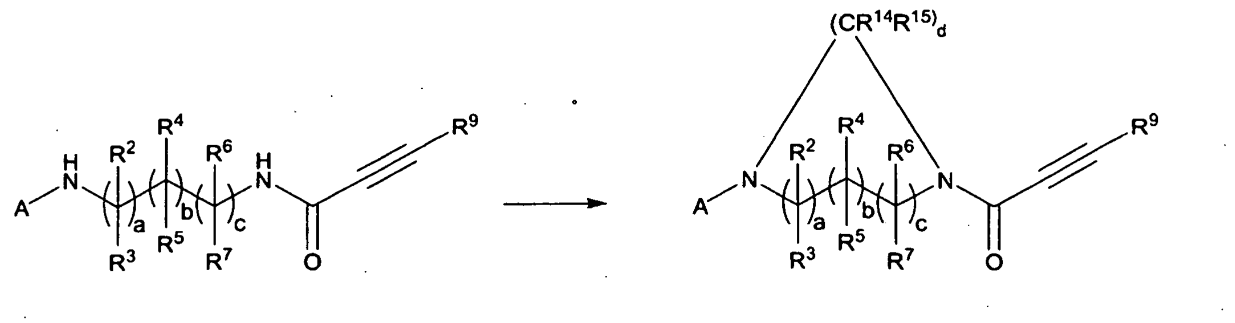

XV XVI

Verbindungen der allgemeinen Formel XV lassen sich mit Verbindungen der allgemeinen Formel $Y-(CR^{14}R^{15})_d-W$, worin Y und W unabhängig voneinander jeweils für eine Abgangsgruppe, vorzugsweise für einen Halogen-Rest oder einen Sulfonsäureester ausgewählt aus der Gruppe bestehend aus Mesylat, Triflat und Tosylat, besonders bevorzugt für ein Chlor-, oder Bromatom stehen, ggf. in einem Reaktionsmedium, vorzugsweise ausgewählt aus der Gruppe bestehend aus Methanol, Ethanol, Isopropanol, n-Butanol, Diethylether, Tetrahydrofuran, Dichlormethan, Chloroform, Dimethylformamid, Acetonitril, Pyridin, Dioxan, Ethylacetat, Dimethylsulfoxid, Toluol und entsprechenden Mischungen, besonders bevorzugt in einem Reaktionsmedium ausgewählt aus der Gruppe bestehend aus Acetontiril, Dichlorethan, Chloroform, Dimethylformamid, Tetrahydrofuran und Diethylether, ggf. in Gegenwart wenigstens einer organischen oder anorganischen Base, vorzugsweise ausgewählt aus der Gruppe bestehend aus Triethylamin, Natriumhydrogencarbonat, Dimethylaminopyridin, Kaliumcarbonat und Natriumhydroxid, und/oder ggf. in Gegenwart wenigstens einer metallorganischen Verbindung oder eines Metallhydrid-Reagenzes, vorzugsweise ausgewählt aus der Gruppe bestehend aus n-Butyllithium, Phenyllithium, Natriumhydrid, Kalium-tert.-Butanolat, Kaliumhydrid und Natriumamid, vorzugsweise bei Temperaturen von -70 °C bis 300 °C, besonders bevorzugt bei Temperaturen von -70 °C bis 150 °C, zu Verbindungen der allgemeinen Formel XVI, umsetzen.

Ebenfalls bevorzugt erfolgt die Umsetzung von Verbindungen der allgemeinen Formel XV mit Paraformaldehyd, zu Verbindungen der allgemeinen Formel XVI, worin $R^{14}$ und $R^{15}$ jeweils für H stehen und d gleich 1 ist, in einem Reaktionsmedium, besonders bevorzugt in einem Reaktionsmedium ausgewählt aus der Gruppe bestehend aus Acetonitril, Toluol, Dichlormethan, Tetrahydrofuran oder Diethylether, besonders bevorzugt in Toluol, und entsprechenden Mischungen, in Gegenwart von Pyridinium-4-toluolsulfonat , bevorzugt bei einer Temperatur von 0 °C bis 150°C.

Verbindungen der allgemeinen Formel XV lassen sich ebenfalls in Verbindungen der allgemeinen Formel I, worin wenigstens einer der Reste $R^1$ und $R^8$ nicht für einen Wasserstoff-Rest steht, überführen.

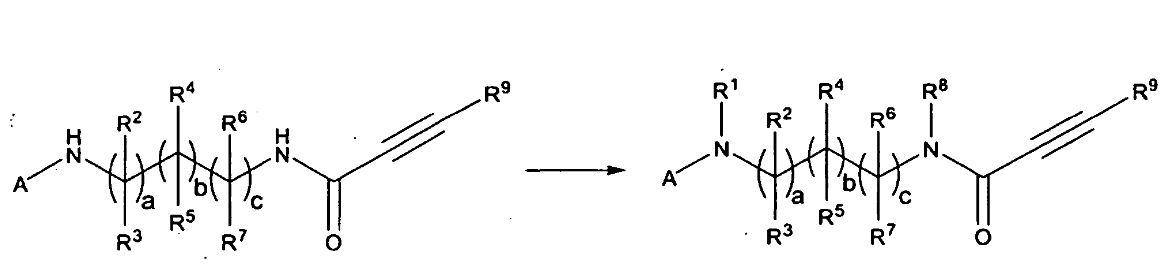

XV I, $R^1$ und/oder $R^8$ ungleich H

Verbindungen der allgemeinen Formel XV lassen sich mit Verbindungen der allgemeinen Formel $R^1$-X oder $R^8$-X, worin X für eine Abgangsgruppe, vorzugsweise für einen Halogen-Rest oder einen Sulfonsäureester ausgewählt aus der Gruppe bestehend aus Mesylat, Triflat und Tosylat, besonders bevorzugt für ein Chlor-, oder Bromatom steht, ggf. in einem Reaktionsmedium, vorzugsweise ausgewählt aus der Gruppe bestehend aus Methanol, Ethanol, Isopropanol, n-Butanol, Diethylether, Tetrahydrofuran, Dichlormethan, Chloroform, Dimethylformamid, Acetonitril, Pyridin, Dioxan, Ethylacetat, Dimethylsulfoxid, Toluol und entsprechenden Mischungen, besonders bevorzugt in einem Reaktionsmedium ausgewählt aus der Gruppe bestehend aus Acetontiril, Dichlorethan, Chloroform, Dimethylformamid, Tetrahydrofuran und Diethylether, ggf. in Gegenwart wenigstens einer organischen oder anorganischen Base, vorzugsweise ausgewählt aus der Gruppe bestehend aus Triethylamin, Natriumhydrogencarbonat, Dimethylaminopyridin, Kaliumcarbonat und Natriumhydroxid, und/oder ggf. in Gegenwart wenigstens einer metallorganischen Verbindung oder eines Metallhydrid-Reagenzes, vorzugsweise ausgewählt aus der Gruppe bestehend aus n-Butyllithium, Phenyllithium, Natriumhydrid, Kalium-tert-butanolat, Kaliumhydrid und Natriumamid, vorzugsweise bei Temperaturen von -70 °C bis 300 °C, besonders bevorzugt bei Temperaturen von -70 °C bis 150 °C, zu Verbindungen der allgemeinen Formel I, worin wenigstens einer der Reste $R^1$ und $R^8$ nicht für einen Wasserstoff-Rest steht, umsetzen.

Die Verbindungen der vorstehend angegebenen Formeln II, III, V, VII, VIII, sowie der allgemeinen Formeln Y-$(CR^{14}R^{15})_d$-W, $R^9$-C≡C-C(=O)-OH, $R^9$-X, $R^1$-X, $R^8$-X, $R^9$-C≡C-C(=O)-X und H-C≡C-C(=O)-X sind jeweils am Markt käuflich erhältlich und/oder können nach den üblichen, dem Fachmann bekannten Verfahren hergestellt werden.

Die vorstehend beschriebenen Umsetzungen können jeweils unter üblichen, dem Fachmann geläufigen Bedingungen, beispielsweise in Hinblick auf Druck oder Reihenfolge der Zugabe der Komponenten durchgeführt werden. Ggf. kann die gemäß den jeweiligen Bedingungen optimale Verfahrensführung vom Fachmann durch einfache Vorversuche ermittelt werden.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie.

Sämtliche der vorstehend beschriebenen Verfahrensschritte sowie jeweils auch die Reinigung und/oder Isolierung von Zwischen- oder Endprodukten können teilweise oder vollständig unter einer Inertgasatmossphäre, vorzugsweise unter Stickstoffatmossphäre, durchgeführt werden.

Sofern die erfindungsgemäßen substituierten Propiolsäureamide der vorstehend angegebenen allgemeinen Formel I, nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomere, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten Propiolsäureamide der vorstehend angegebenen allgemeinen Formel I sowie ggf. jeweils entsprechende Stereoisomere können nach üblichen, dem Fachmann bekannten Verfahren in Form entsprechender Salze, bevorzugt in Form entsprechender Hydrochloride, insbesondere in Form entsprechender physiologisch verträglicher Salze erhalten werden, erhalten werden, wobei das erfindungsgemäße Arzneimittel eines oder mehrere Salze einer oder mehrerer dieser Verbindungen aufweisen kann.

Die jeweiligen Salze der erfindungsgemäßen substituierten Propiolsäureamide der vorstehend angegebenen allgemeinen Formel I sowie entsprechender Stereoisomeren können beispielsweise durch Umsetzung mit einer oder mehreren anorganischen Säuren und/oder einer oder mehreren organischen Säuren erhalten werden. Geeignete Säuren können bevorzugt ausgewählt werden aus der Gruppe bestehend aus Perchlorsäure, Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Sacharinsäure, Cyclohexansulfamidsäure, Aspartam, Monomethylsebacinsäure, 5-Oxo-prolin, Hexan-1-suifonsäure, Nicotinsäure, 2-Aminobenzoesäure, 3-Aminobenzoesäure oder 4-Aminobenzoesäure, 2,4,6-Trimethylbenzoesäure, α-Liponsäure, Acetylglycin, Hippursäure, Phosphorsäure, Maleinsäure, Malonsäure und Asparaginsäure.

Die erfindungsgemäßen substituierten Propiolsäureamide der vorstehend angegebenen allgemeinen Formel I sowie ggf. entsprechende Stereoisomere und jeweils deren physiologisch verträgliche Salze können nach üblichen, dem Fachmann bekannten Verfahren auch in Form ihrer Solvate, insbesondere in Form ihrer Hydrate erhalten werden.

Es wurde überraschenderweise gefunden, dass sich die erfindungsgemäßen substituierten Propiolsäureamide der vorstehend angegebenen allgemeinen Formel I zur mGluR5-Rezeptor-Regulation eignen und daher insbesondere als pharmazeutische Wirkstoffe in Arzneimitteln zur Prophylaxe und/oder Behandlung von mit diesen Rezeptoren bzw. Prozessen in Verbindung stehenden Störungen oder Erkrankungen eingesetzt werden können.

Die erfindungsgemäßen substituierten Propiolsäureamide der vorstehend angegebenen allgemeinen Formel I und ggf. entsprechende Stereoisomere sowie jeweils die entsprechenden Salze und Solvate erscheinen toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens ein erfindungsgemäßes substituiertes Propiolsäureamid der vorstehend angegebenen allgemeinen Förmel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Das erfindungsgemäße Arzneimittel eignet sich zur mGluR5-Rezeptor-Regulation, insbesondere zur Inhibierung des mGluR5-Rezeptors.

Bevorzugt eignet sich das erfindungsgemäße Arzneimittel zur Prophylaxe und/oder Behandlung von Störungen und/oder Krankheiten, die zumindest teilweise durch mGluR5-Rezeptoren vermittelt werden.

Besonders bevorzugt eignet sich das erfindungsgemäße Arzneimittel daher zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Erkrankungen, vorzugsweise kognitiven Mangelzuständen, besonders bevorzugt des Aufmerksamkeit-Defizit-Syndroms (ADS); Angstzuständen; Panikattacken; Epilepsie; Husten; Harninkontinenz; Diarrhöe; Pruritus; Schizophrenie; cerebralen Ischämien; Muskelspasmen; Krämpfen; Lungenkrankheiten, vorzugsweise ausgewählt aus der Gruppe bestehend aus Asthma und Pseudokrupp; Regurgitation (Erbrechen); Schlaganfall; Dyskinesie; Retinopathie; Antriebslosigkeit; Kehlkopfentzündung (Laryngitis); Störungen der Nahrungsaufnahme, vorzugsweise ausgewählt aus der Gruppe bestehend aus Bulimie, Kachexie, Anorexie und Fettleibigkeit; Alkoholabhängigkeit; Medikamentenabhängigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen-(insbesondere Nikotin- und/oder Kokain-)abhängigkeit; Toleranzentwicklung gegenüber Medikamenten, vorzugsweise gegenüber natürlichen oder synthetischen Opioiden; Magen-Ösaphagus-Reflux-Syndrom; gastroösophageale Refluxkrankheit; Reizdarmsyndrom; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie.

Ganz besonders bevorzugt eignet sich das erfindungsgemäße Arzneimittel zur Prophylaxe von Schmerz, vorzugsweise von Schmerz ausgewählt aus der Gruppe bestehend aus akutem Schmerz, chronischem Schmerz, neuropathischem Schmerz und visceralem Schmerz; Angstzuständen; Panikattacken; Alkoholabhängigkeit; Medikamentenabhängigkeit; Störungen der Nahrungsaufnahme, vorzugsweise ausgewählt aus der Gruppe bestehend aus Bulimie, Kachexie, Anorexie und Fettleibigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen-(insbesondere Nikotin- und/oder Kokain-)abhängigkeit; Toleranzentwicklung gegenüber Medikamenten und/oder Drogen, insbesondere gegenüber natürlichen oder synthetischen Opioiden; Magen-Ösaphagus-Reflux-Syndrom, gastroösophageale Refluxkrankheit und Reizdarmsyndrom.

Noch weiter bevorzugt eignet sich das erfindungsgemäße Arzneimittel zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz, Angstzuständen und Panikattacken.

Am weitesten bevorzugt eignet sich das erfindungsgemäße Arzneimittel zur Prophylaxe und/oder Behandlung von Schmerzen, vorzugsweise von akuten Schmerzen, chronischen Schmerzen, neuropathischen Schmerzen oder visceralen Schmerzen.

Ein weiterer Gegenstand der vorliegenden Erfindung ist die Verwendung wenigstens eines erfindungsgemäßen substituierten Propiolsäureamids der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur mGluR5-Rezeptor-Regulation, vorzugsweise zur Inhibierung des mGluR5-Rezeptors.

Bevorzugt ist die Verwendung wenigstens eines erfindungsgemäßen substituierten Propiolsäureamids der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Störungen

und/oder Krankheiten, die zumindest teilweise durch mGluR5-Rezeptoren vermittelt werden.

Besonders bevorzugt ist die Verwendung wenigstens eines erfindungsgemäßen substituierten Propiolsäureamids der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Erkrankungen, vorzugsweise kognitiven Mangelzuständen, besonders bevorzugt des Aufmerksamkeit-Defizit-Syndroms (ADS); Angstzuständen; Panikattacken; Epilepsie; Husten; Harninkontinenz; Diarrhöe; Pruritus; Schizophrenie; cerebralen Ischämien; Muskelspasmen; Krämpfen; Lungenkrankheiten, vorzugsweise ausgewählt aus der Gruppe bestehend aus Asthma und Pseudokrupp; Regurgitation (Erbrechen); Schlaganfall; Dyskinesie; Retinopathie; Antriebslosigkeit; Kehlkopfentzündung (Laryngitis); Störungen der Nahrungsaufnahme, vorzugsweise ausgewählt aus der Gruppe bestehend aus Bulimie, Kachexie, Anorexie und Fettleibigkeit; Alkoholabhängigkeit; Medikamentenabhängigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen-(insbesondere Nikotin- und/oder Kokain-)abhängigkeit; Toleranzentwicklung gegenüber Medikamenten, insbesondere gegenüber natürlichen oder synthetischen Opioiden; Magen-Ösaphagus-Reflux-Syndrom; gastroösophageale Refluxkrankheit; Reizdarmsyndrom; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie.

Ganz besonders bevorzugt ist die Verwendung wenigstens eines erfindungsgemäßen substituierten Propiolsäureamids der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz; Angstzuständen; Panikattacken; Alkoholabhängigkeit; Medikamentenabhängigkeit; Störungen der Nahrungsaufnahme, vorzugsweise ausgewählt aus der Gruppe bestehend aus Bulimie, Kachexie, Anorexie und Fettleibigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen- (insbesondere Nikotin- und/oder Kokain-)abhängigkeit; Toleranzentwicklung gegenüber Medikamenten und/oder Drogen, insbesondere gegenüber natürlichen oder synthetischen Opioiden; Magen-Ösaphagus-Reflux-Syndrom, gastroösophageale Refluxkrankheit und Reizdarmsyndrom.

Noch weiter bevorzugt ist die Verwendung wenigstens eines erfindungsgemaßen substituierten Propiolsäureamids der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz, Angstzuständen und Panikattacken.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einem erfindungsgemäßen substituierten Propiolsäureamid der vorstehend angegebenen allgemeinen Formel I, ggf. in Form seiner reinen Stereoisomeren, insbesondere Enantiomeren oder Diastereomeren, seines Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden substituierten Propiolsäureamid der vorstehend angegebenen allgemeinen Formel I sind in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweiligen substituierten Propiolsäureamide der vorstehend angegebenen allgemeinen Formel I auch verzögert freisetzen.

Die Herstellung der erfindungsgemäßen Arzneimittel erfolgt mittels üblichen, aus dem Stande der Technik wohl bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge des jeweiligen substituierten Propiolsäureamids der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,05 bis 100 mg/kg, bevorzugt 0,05 bis 10 mg/kg, Körpergewicht des Patienten wenigstens einer solchen Verbindung appliziert.

## Pharmakologische Methoden:

### I. Methode zur Bestimmung der Hemmung der [$^3$H]-MPEP-Bindung im mGluR5-Rezeptor-Bindungsassay

Schweinehirnhomogenat wird durch Homogenisieren (Polytron PT 3000, Kinematica AG, 10.000 Umdrehungen pro Minute für 90 Sekunden) von Schweinehirnhälften ohne Medulla, Cerebellum und Pons in Puffer pH 8.0 (30mM Hepes, Sigma, Bestellnr.H3375 + 1 Tablette Complete auf 100ml, Roche Diagnostics, Bestellnr. 1836145) im Verhältnis 1:20 (Himgewicht/Volumen) und Differentialzentrifugation bei 900 x g und 40.000 x g hergestellt. In 250 µl Inkubationsansätzen in 96 Well Mikrotiterplatten werden jeweils 450 µg Protein aus Hirnhomogenat mit 5nM $^3$[H]-MPEP (Tocris, Bestellnr. R1212) (MPEP = 2-Methyl-6-(3-methoxyphenyl)-ethinylpyridin) und die zu untersuchenden Verbindungen (10 µM im Test) in Puffer (wie oben) bei Raumtemperatur für 60 min inkubiert.

Danach werden die Ansätze mit Hilfe eines Brandel Cell Harvesters (Brandel, TYP Robotic 9600) auf Unifilterplates mit Glasfaserfiltermatten (Perkin Elmer, Bestellnr. 6005177) filtriert und anschließend mit Puffer (wie oben) 3 mal mit je 250 µl pro Probe nach gewaschen. Die Filterplatten werden anschließend für 60 min bei 55°C getrocknet. Anschließend werden pro Well 30 µL Ultima Gold™ Szintillator (Packard BioScience, Bestellnr.6013159) zugegeben und nach 3 Stunden werden die Proben am β-Counter (Mikrobeta, Perkin Elmer) gemessen. Die unspezifische Bindung wird durch Zugabe von 10 µM MPEP (Tocris, Bestellnr.1212) bestimmt.

### II. Methode zur Bestimmung des $Ca^{2+}$-Influx im mGluR5-Rezeptor-Assay

Eine agonistische und/oder antagonistische Wirkung von Substanzen kann am mGluR5-Rezeptor der Spezies Ratte mit folgendem Assay bestimmt werden. Gemäß diesem Assay wird die intrazelluläre $Ca^{2+}$-Freisetzung nach Aktivierung des mGluR5-Rezeptors mit Hilfe eines $Ca^{2+}$-sensitiven Farbstoffs (Typ Fluo-4, Molecular Probes Europe BV, Leiden Niederlande) in der FlexStation (Molecular Devices, Sunnyvale, USA) quantifiziert.

## Präparation kortikaler Neurone:

Kortikale Neuronen werden unter sterilen Bedingungen aus postnatalen Ratten (P2-6) präpariert. Hierzu wird der Kortex entnommen und direkt in Kollagenase-Lösung (PAA Laboratories GmbH, Cölbe, Deutschland) überführt und 45 Minuten im Heizschüttler (37°C, 300 Umdrehungen pro Minute) inkubiert. Anschließend wird die Kollagenase-Lösung entnommen und das Gewebe mit Kulturmedium versetzt.

Kulturmedium (100 ml):

Neurobasalmedium (Gibco Invitrogen GmbH, Karlsruhe, Deutschland) 2 mM L-Glutamin (Sigma, Taufkirchen, Deutschland)
1 Vol-% Antibiotika/Antimycotika-Lösung (PAA Laboratories GmbH, Cölbe, Deutschland)
15 ng/ml NGF (Gibco Invitrogen GmbH, Karlsruhe, Deutschland) 1 ml B27 Supplement (Gibco Invitrogen GmbH, Karlsruhe, Deutschland) 1 ml ITS Supplement (Sigma, Taufkirchen, Deutschland)

Die Zellen werden durch Resuspendieren vereinzelt und nach Zugabe von 15 ml Neurobasalmedium durch einen 70 μm Filtereinsatz (BD Biosciences, Heidelberg, Deutschland) zentrifugiert. Das resultierende Zellpellet wird in Kulturmedium aufgenommen. Anschließend werden die Zellen auf Poly-D-Lysin-beschichtete, schwarze 96-Loch-Platten mit klarem Boden (BD Biosciences, Heidelberg, Deutschland), die zuvor zusätzlich mit Laminin (2 $\mu g/cm^2$, Gibco Invitrogen GmbH, Karlsruhe, Deutschland) beschichtet wurden, ausplattiert. Die Zelldichte beträgt 15.000 Zellen/Loch. Die Zellen werden bei 37°C und 5 % $CO_2$ inkubiert und am 2. oder 3. Tag nach Präparation erfolgt ein Mediumwechsel. Je nach Zellwachstum kann die funktionelle Untersuchung am 3.-7. Tag nach Präparation durchgeführt werden.

**Beschreibung des funktionellen $Ca^{2+}$-Influx assay**

20.000 CHO-hmGluR5 cells/ well (Euroscreen, Gosselies, Belgium) werden in 96 well Platten (BD Biosciences, Heidelberg, Deutschland, Ref 356640, clear bottom, 96 well, Poly-D-Lysine) auspipettiert und über Nacht in HBSS-Puffer (Gibco Nr. 14025-050) mit folgenden Zusätzen: 10% FCS (GIBCO, 10270-106) und Doxycyclin (BD Biosciences Clontech 631311 600ng/ml) inkubiert.

Zur funktionellen Untersuchung wurden die Zellen mit 2 μM Fluo-4 und 0,01 Vol% Pluronic F127 (Molecular Probes Europe BV, Leiden Niederlande) in HBSS-Puffer (Hank's buffered saline solution, Gibco Invitrogen GmbH, Karlsruhe, Deutschland) mit Probenicid (Sigma P8761, 0.69 mg/ml) für 30 min bei 37 °C beladen.

Die Zellen werden dann 3 mal mit Waschpuffer (HBSS-Puffer, Gibco Nr. 14025-050, mit Probenicid (Sigma P8761, 0.69 mg/ml) gewaschen und anschließend mit dem gleichen Puffer ad 100 μl aufgenommen. Nach 15 min. werden die Platten für die Bestimmung von $Ca^{2+}$-Messungen in Gegenwart von DHPG ((S)-3,5-Dihydroxyphenylglycine, Tocris Biotrend Chemikalien GmbH, Köln, Deutschland, finale DHPG-Konzentration: 10 μM) sowie in Gegenwart oder Abwesenheit von Testsubstanzen in einen Fuorometric Imaging Plate Reader (FLIPR, Molecular Devices, Sunnyvale, CA) transferiert.

Die $Ca^{2+}$-abhängige Fluoreszenz wird dabei vor und nach Zugabe von Testsubstanzen gemessen. Die Quantifizierung erfolgt durch die Messung der höchsten Fluoreszenzintensität über die Zeit.

Nach der Aufnahme einer Fluoreszenzbasislinie für 10 sec. werden 50 μl Testsubstanzlösung (verschiedene Testsubstanzkonzentrationen in HBSS-Puffer mit 1 % DMSO und 0.02% Tween 20, Sigma) zugegeben und das Fluoreszenzsignal wird für 6 min gemessen. Anschließend werden 50 μl DHPG-Lösung ((S)-3,5-Dihydroxyphenylglycine, Tocris Biotrend Chemikalien GmbH, Köln, Deutschland, finale DHPG-Konzentration: 10 μM) zugegeben und der Einstrom von $Ca^{2+}$ wird simultan für 60 sec gemessen. Die finale DMSO-Konzentration beträgt 0.25% und der finale Tween 20 Gehalt beträgt 0.005%. Die Daten werden mit Microsoft Excel und GraphPad Prism analysiert. Die Dosis-Wirkungskurven werden mit nicht-linearer Regression berechnet und $IC_{50}$-Werte ermittelt. Jeder Datenpunkt wird 3-fach bestimmt und $IC_{50}$-Werte werden aus minimal 2 unabhängigen Messungen gemittelt.

Ki-Werte werden nach folgender Formel berechnet: Ki = IC50/(1+($AG_{Konz.}$/EC50)).

$AG_{Konz.}$ = 10 μM; EC50 entspricht der DHPG-Konzentration, die für den halbmaximalen Einstrom von $Ca^{2+}$ notwendig ist.

**III. Formalintest an der Ratte:**

Der Formalintest (Dubuisson, D. and Dennis, S.G., 1977, Pain, 4,161 - 174) stellt ein Modell für den akuten sowie den chronischen Schmerz dar. Durch eine einmalige Formalin-Injektion in die dorsale Seite einer Hinterpfote wird bei freibeweglichen Versuchstieren eine biphasische nozizeptive Reaktion induziert, die durch Beobachtung von drei deutlich voneinander unterscheidbaren Verhaltensmustem erfasst wird. Die Reaktion ist zweiphasisch: Phase 1 = Sofortreaktion (Dauer bis 10 min; Pfotenschütteln, Lecken), Phase 2 = Spätreaktion (nach einer Ruhephase; ebenfalls Pfotenschütteln, Lecken; Dauer bis 60 min). Die 1. Phase reflektiert eine direkte Stimulation der peripheren Nozisensoren mit hohem spinalen nozizeptiven Input bzw. Glutamatfreisetzung (akute Schmerzphase); die 2. Phase reflektiert eine spinale und periphere Hypersensibilisierung (chronische Schmerzphase). In den hier vorgestellten Untersuchungen wurde die chronische Schmerzkomponente (Phase 2) ausgewertet.

Formalin wird mit dem Volumen von 50μl und einer Konzentration von 5 % subkutan in die dorsale Seite der rechten Hinterpfote jedes Tieres appliziert. Die zu testenden Substanzen werden 30 min vor der Formalininjektion oral (p.o.), intravenös (i.v.) oder intraperitoneal (i.p.) appliziert. Die spezifischen Verhaltensänderungen, wie Anheben und

Schütteln der Pfote, Gewichtsverlagerungen des Tieres sowie Beiss- und Leckreaktionen werden im Beobachtungszeitraum von 21 bis 27 min nach Formalininjektion beobachtet und registriert. Die Zusammenfassung der verschiedenen Verhaltensweisen erfolgt in der sogenannten Pain-Rate (PR), die, auf die Teilintervalle von 3 min bezogen, die Berechnung einer mittleren Nozizeptionsreaktion darstellt. Die Berechnung der PR erfolgt aufgrund einer numerischen Gewichtung (= jeweils Faktor 1, 2, 3) der beobachteten Verhaltensweisen (entsprechend Verhaltensscore 1, 2, 3) und wird mit folgender Formel berechnet:

$$PR = [(T_0 \times 0) + (T_1 \times 1) + (T_2 \times 2) + (T_3 \times 3)] / 180$$

wobei $T_0$, $T_1$, $T_2$, und $T_3$ jeweils der Zeit in Sekunden entspricht, in der das Tier die Verhaltensweisen 0, 1, 2 oder 3 zeigt. Die Gruppengröße beträgt 10 Tiere (n=10).

Die folgenden Beispiele dienen zur Erläuterung der Erfindung und schränken den allgemeinen Erfindungsgedanken nicht ein.

**Beispiele**

Die Ausbeuten der hergestellten Verbindungen sind nicht optimiert.

Alle Temperaturen sind unkorrigiert.

Die eingesetzten Chemikalien und Lösungsmittel wurden kommerziell bei den herkömmlichen Anbietern (Acros, Avocado, Aldrich, Bachem, Fluka, Lancaster, Maybridge,Merck, Sigma, TCI, etc.) bezogen oder nach dem Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Lösungsmitteln, Laufmitteln oder für chromatographische Untersuchungen sind stets in Volumen/Volumen angegeben.

Die Analytik erfolgte durch Massenspektroskopie und NMR.

**Abkürzungen:**

| | |
|---|---|
| aq. | wäßrig |
| Brine | gesättigte wässrige NaCl-Lösung |
| BuLi | n-Butyllithium |
| DCE | 1,2-Dichlorethan |
| DCM | Dichlormethan |
| DIC | N,N'-Diisopropylcarbodiimid |
| DIPE | Diisopropylether |
| DMF | N,N-Dimethylformamid |
| EE | Essigsäureethylester |
| Ether | Diethylether |
| HOBt | 1-Hydroxy-1H-Benzotriazol Hydrat |
| Lsg. | Lösung |
| M | molar |
| MeOH | Methanol |
| PL-EDC | polymergebundenes Carbodiimid mit folgender Struktur: |

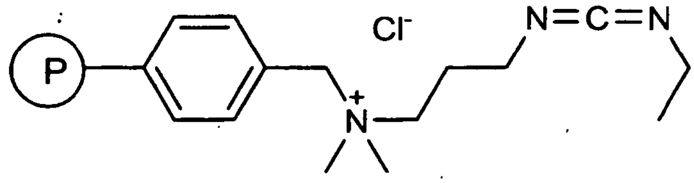

Beladung: ~ 1.4 mmol/g

Partikelgröße: 300-500 μm

PS- Carbodiimid ein polymergebundenes Carbodiimid mit folgender Struktur:

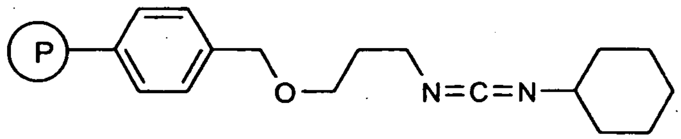

Beladung: 0.9-1.4 mmol/g
Partikelgröße: 75-150 μm

RT Raumtemperatur
SC Säulenchromatographie
TFA Trifluoressigsäure

**Beispiel 1:**

**1-(3-Phenyl-propiolyl)-4-(thiophen-3-yl)-piperazin Hydrochlorid**

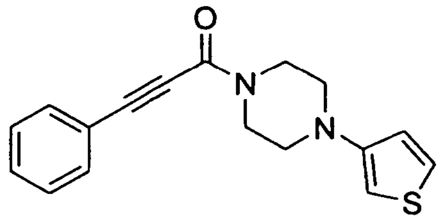

HCl

**a) Synthese von tert-Butyl 4-(3-phenylpropiolyl)piperazine-1-carboxylat**

Zu einer Lösung von 750 mg (5.1 mmol) Phenylpropiolsäure und 955 mg (5.1 mmol) tert-Butylpiperazin-1-carboxylat in DMF (4 ml) wurden 0.80 ml (5.1 mmol) DIC und 0.69 g (5.1 mmol) HOBt gegeben und die Reaktionslösung wurde 18 h bei RT gerührt-Anschließend wurde mit einer 1 molaren aq. $Na_2CO_3$-Lsg. (2 ml) versetzt und mit n-Hexan (5 ml) verdünnt. Der dabei ausgefallene Feststoff (1.7 g) wurde abgesaugt, mit n-Hexan gewaschen und ohne weitere Aufreinigung auf der nächsten Stufe eingesetzt.

**b) Synthese von 1-(Phenylpropiolyl)-piperazin**

Eine Lösung von 500 mg des bei Beispiel 1a) erhaltenen Rohprodukts in DCM (2 ml) wurde auf 0°C abgekühlt (Eisbad) und langsam mit 1.65 ml (21.5 mmol) TFA versetzt, wobei die Temperatur 5°C nicht überstieg. Nach beendeter Zugabe wurde 1 h bei RT gerührt. Anschließend wurde das Gemisch auf Eiswasser gegossen und mit einer 25%-igen aq. $NH_3$-Lsg. basisch (pH > 10) eingestellt. Dann wurde mit DCM (3 x 150 ml) extrahiert. Die gesammelten organischen Phasen wurden mit Brine gewaschen, über $MgSO_4$ getrocknet, filtriert und das Lösungsmittel wurde im Vakuum entfernt. Mit dem Rückstand wurde eine SC ($SiO_2$, MeOH/DCM 1:9) durchgeführt wobei 170 mg (0.8 mmol, 53% über 2 Stufen) 1-(Phenylpropiolyl)-piperazin erhalten wurden.

**c) Synthese von 1-(3-Phenyl-propiolyl)-4-(thiophen-3-yl)-piperazin**

Zu einer Lösung von 500 mg (2.33 mmol) 1-(Phenylpropiolyl)-piperazin in 1,2,4-Trimethylbenzol (2 ml) wurden nacheinander 246 mg (2.57 mmol) Natrium-tert.-butylat und 0.22 ml (2.33 mmol) 3-Bromthiophen gegeben. Zu der Reaktionslösung wurde eine Suspension aus 66 mg (0.12 mmol) Bis(dibenzylidenaceton)palladium(0) und 345 ml (0.12 mmol, 10% in Hexan) Tri-tert.-butylphosphin in 1,2,4-Trimethylbenzol (1.2 ml) gegeben. Anschließend wurde 18 h bei 120 °C gerührt, mit EE verdünnt und über Kieselgur filtriert. Das Filtrat wurde im Vakuum eingeengt und mit dem Rückstand wurde eine SC ($SiO_2$, DCM/DIPE 1:2) durchgeführt, wobei 300 mg (1.01 mmol, 43%) 1-(3-Phenyl-propiolyl)-4-(thiophen-3-yl)-piperazin erhalten wurden.

**d) Synthese von 1-(3-Phenyl-propiolyl)-4-(thiophen-3-yl)-piperazin Hydrochlorid**

300 mg (1.01 mmol) 1-(3-Phenyl-propiolyl)-4-(thiophen-3-yl)-piperazin wurden in Aceton (10 ml) gelöst und nacheinander mit 9 µl (0.51 mmol) Wasser und 128 µl (1.0 mmol) Trimethylchlorsilan versetzt. Der entstandene Niederschlag wurde abgesaugt und mit Aceton gewaschen. Dabei wurden 0.193 g (0.58 mmol, 57%) 1-(3-Phenylpropiolyl)-4-(thiophen-3-yl)-piperazin Hydrochlorid erhalten.
MS: $[MH^+]$ 297.1

**Beispiel 3:**

**1-(3-Phenyl-propiolyl)-4-(1-methyl-imidazol-2-yl)-piperazin Hydrochlorid**

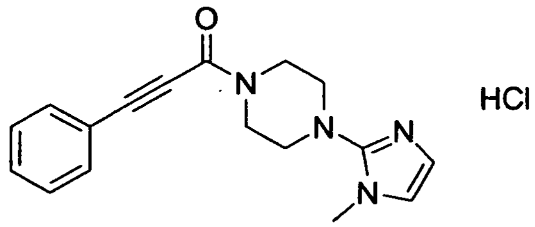

**a) Synthese von 1-(1-Methyl-1H-imidazol-2-yl)piperazin .**

1.0 g (6.2 mmol) 2-Brom-1-methyl-1*H*-imidazol wurden mit 3.2 ml (37.0 mmol) Piperazin bei 145°C geschmolzen und 18 h bei dieser Temperatur gerührt. Nach Abkühlen auf RT wurde der Rückstand in 10%-iger aq. Salzsäure aufgenommen und mit EE gewaschen. Anschließend wurde mit einer 10%-igen aq. NaOH-Lsg. basisch eingestellt (pH > 12) und mit DCM extrahiert. Die organische Phase wurde über $MgSO_4$ getrocknet, filtriert und im Vakuum eingeengt. Dabei wurden 780 mg (4.7 mmol, 76%) 1-(1-Methyl-1*H*-imidazol-2-yl)piperazin erhalten.

**b) Synthese von 1-(3-Phenyl-propiolyl)-4-(1-methyl-imidazol-2-yl)-piperazin**

Eine Lösung von 600 mg (4.1 mmol) Phenylpropiolsäure und 680 mg (4.1 mmol) 1-(1-Methyl-1*H*-imidazol-2-yl)piperazin in DMF (3.3 ml) wurde mit 0.64 ml (4.1 mmol) DIC und 0.55 g (4.1 mmol) HOBt versetzt und 18 h bei RT gerührt. Anschließend wurde eine 1 molare aq. $Na_2CO_3$-Lsg. (5 ml) zugegeben und mit DCM extrahiert. Die organische Phase wurde über $MgSO_4$ getrocknet, filtriert und das Lösungsmittel im Vakuum entfernt. Mit dem Rückstand wurde eine SC ($SiO_2$, EE/MeOH 9:1) durchgeführt, wobei 720 mg (2.4 mmol, 60%) 1-(3-Phenyl-propiolyl)-4-(1-methyl-imidazol-2-yl)-piperazin erhalten wurden.

**c) Synthese von 1-(3-Phenyl-propiolyl)-4-(1-methyl-imidazol-2-yl)-piperazin Hydrochlorid**

720 mg (2.40 mmol) 1-(3-Phenyl-propiolyl)-4-(1-methyl-imidazol-2-yl)-piperazin wurden in Aceton (20 ml) gelöst und nacheinander mit 22 µl (1.22 mmol) Wasser und 310 µl (2.45 mmol) Trimethylchlorsilan versetzt. Der entstandene Niederschlag wurde abgesaugt und mit Aceton gewaschen. Dabei wurden 440 mg (1.3 mmol, 32%) 1-(3-Phenyl-propiolyl)-4-(1-methyl-imidazol-2-yl)-piperazin Hydrochlorid erhalten.
MS: $[MH^+]$ 295.1

**Beispiel 4:**

**(*R*)-2-Methyl-1-(3-(3-chloro-phenyl)-propiolyl)-4-(thiazol-4-yl)-piperazin**

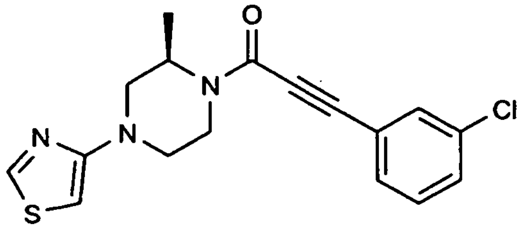

**a) Synthese von 4-Bromthiazol**

Eine Lösung von 10.0 g (41.2 mmol) 2,4-Dibromthiazol in Ether (210 ml) wurde auf-78 °C abgekühlt und bei dieser Temperatur tropfenweise mit 28.3 ml (45.3 mmol, 15%-ig in Hexan) n-Butyllithium versetzt. Nach 30 min Rühren wurden bei -78 °C zur Reaktionsmischung 3.3 ml (82.3 mmol) Methanol gegeben. Anschließend wurde über einen Zeitraum von 16 h auf RT erwärmt. Die Reaktionsmischung wurde über Kieselgel filtriert und es wurde mit einer n-Hexan/EE-Mischung (2:1) gewaschen. Das Filtrat wurde im Vakuum eingeengt, wobei 6.7 g (40.9 mmol, 99%) 4-Bromthiazol erhalten wurden.

**b) Synthese von (*R*)-4-(3-Methylpiperazin-1-yl)thiazol**

10.0 g (100 mmol) (*R*)-2-Methylpiperazin wurden bei 100°C geschmolzen. Zu dieser Schmelze wurden 2.7 g (16.8 mmol) 4-Bromthiazol über einen Zeitraum von 2 h portionsweise gegeben. Anschließend wurde 18 h bei 100°C gerührt. Nach Abkühlen auf RT wurde der Rückstand in 10%-iger aq. Salzsäure aufgenommen und mit EE gewaschen. Anschließend wurde mit einer 10%-igen aq. NaOH-Lsg. basisch eingestellt (pH > 12) und mit DCM extrahiert. Die organische Phase wurde über $MgSO_4$ getrocknet, filtriert und im Vakuum eingeengt. Mit dem Rückstand wurde eine SC ($SiO_2$, DCM/MeOH 9:1) durchgeführt, wobei 277 mg (1.5 mmol, 9%) (*R*)-4-(3-Methylpiperazin-1-yl)thiazol erhalten wurden.

**c) Synthese von (*R*)-2-Methyl 1-(3-(3-Chloro-phenyl)-propiolyl)-4-(thiazol-4-yl)-piperazin**

Eine Lösung von 270 mg (1.50 mmol) 3-(3-Chlor-phenyl)propiolsäure und 270 mg (1.50 mmol) (*R*)-4-(3-Methylpiperazin-1-yl)thiazol in DMF (1.2 ml) wurde mit 0.23 mi (1.50 mmol) DIC und 0.20 g (1.50 mmol) HOBt versetzt und 18 h bei RT gerührt. Anschließend wurde eine 1 molare $Na_2CO_3$-Lsg (3 ml) zugegeben und mit DCM extrahiert. Die organische Phase wurde über $MgSO_4$ getrocknet, filtriert und im Vakuum eingeengt. Mit dem Rückstand wurde eine SC ($SiO_2$, DCM/EE 19:1) durchgeführt, wobei 60 mg (0.17 mmol, 12%) (R)-2-Methyl 1-(3-(3-Chloro-phenyl)-propiolyl)-4-(thiazol-4-yl)-piperazin erhalten wurden.
MS: [$MH^+$] 346.1

**Beispiel 5:**

**2-Methyl 1-(3-(3-Chlor-phenyl)-propiolyl)-4-(1,3,4-thiadiazol-2-yl)-piperazin**

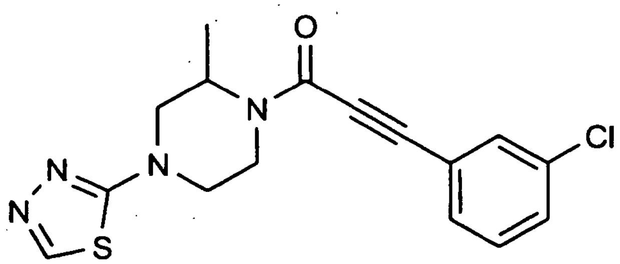

**a) Synthese von 2-(3-Methylpiperazin-1-yl)-1,3,4-thiadiazol**

2.0 g (12.1 mmol) 2-Brom-[1,3,4]-thiadiazol und 7.2 g (72.7 mmol) 2-Methylpiperazin wurden in n-Butanol (10

ml) 20 min bei 120 °C gerührt. Anschließend wurde im Vakuum eingeengt. Mit dem Rückstand wurde eine SC ($SiO_2$, DCE/EtOH/ konz. $NH_4OH$ 5:1:0.06) durchgeführt, wobei 1.7 g (9.3 mmol, 78%) 2-(3-Methylpiperazin-1-yl)-1,3,4-thiadiazol erhalten wurden.

**b) Synthese von 2-Methyl 1-(3-(3-chlor-phenyl)-propiolyl)-4-(1,3,4-thiadiazol-2-yl)-piperazin**

Zu einer Lösung von 276 mg (1.50 mmol) 2-(3-Methylpiperazin-1-yl)-1,3,4-thiadiazol und 406 mg (2.25 mmol) 3-(3-Chlor-phenyl)propiolsäure in DCM (30 ml) wurden 2.5 g (≅ 3.5 mmol) PL-EDC (polymergebundenes Carbodiimid Harz) gegeben und die Reaktionslösung wurde 16 h bei RT geschüttelt. Anschließend wurde das Harz abfiltriert und das Filtrat im Vakuum eingeengt. Mit dem Rückstand wurde eine SC ($SiO_2$, DCE/EtOH 10:1) durchgeführt, wobei 140 mg (0.45 mmol, 30%) 2-(3-Methylpiperazin-1-yl)-1,3,4-thiadiazol erhalten wurden.
MS: [$MH^+$] 347.1

Die Synthese von Beispiel 2 1-(3-Phenyl-propiolyl)-4-(thiazol-4-yl)-piperazin erfolgte nach den bei Beispiel 4b) und c) beschriebenen Verfahren.
MS: [$MH^+$] 298.1

Dem Fachmann ist dabei ersichtlich, welche Ausgangsverbindungen und Zwischenprodukte jeweils eingesetzt werden müssen um zum entsprechenden Beispiel zu gelangen.

**Beispiel 6:**

**3-(3-Chlorphenyl)-N-(2-(methyl(1,3,4-thiadiazol-2-yl)amino)ethyl)propiolamid**

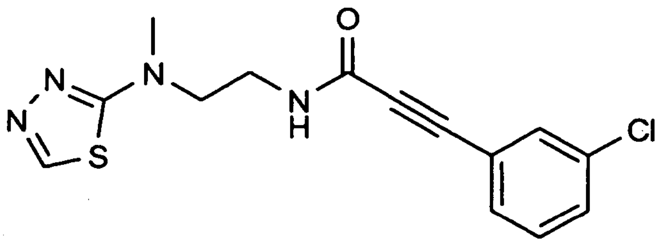

**a) Synthese von N'-Methyl-N'-(1,3,4-thiadiazol-2-yl)ethan-1,2-diamin**

Zu einer Lösung aus 3.3 g (20.0 mmol) 2-Bromthiadiazol und 10.6 ml (120.0 mmol) N-Methyl-ethylen-diamin in Isopropanol (114 ml) wurden 70 mg (1.1 mmol) Kupfer und 328 mg (3.3 mmol) Kupfer(I)-chlorid gegeben. Anschließend wurde 1 h auf 70 °C erhitzt. Nach Abkühlen auf RT wurde durch Kieselgel filtriert und das Filtrat wurde im Vakuum eingeengt. Durch SC (DCE/EtOH/konz. aq. $NH_4OH$-Lsg. 4:4:0.25) mit dem Rückstand wurden 1.05 g (6.6 mmol, 33%) N'-Methyl-N'-(1,3,4-thiadiazol-2-yl)ethan-1,2-diamin erhalten.

**b) Synthese von 3-(3-Chlorphenyl)-N-(2-(methyl(1,3,4-thiadiazol-2-yl)amino)ethyl)propiolamid**

Zu einer Lösung von 317 mg (2.0 mmol) $N^1$-Methyl-$N^1$-(1,3,4-thiadiazol-2-yl)ethan-1,2-diamin und 542 mg (3.0 mmol) 3-(3-Chlor-phenyl)propiolsäure in DCM (20 ml) wurden 3.25 g (≅ 4.0 mmol) PS-Carbodiimid gegeben und die Reaktionslösung wurde 16 h bei RT geschüttelt. Anschließend wurde das Harz abfiltriert, mit DCM und Ethanol nachgewaschen und das Filtrat im Vakuum eingeengt. Mit dem Rückstand wurde eine SC (Chloroform) durchgeführt, wobei 165 mg (0.51 mmol, 26%) 3-(3-Chlorphenyl)-N-(2-(methyl(1,3,4-thiadiazol-2-yl)amino)ethyl)propiolamid erhalten wurden.
MS: [$MH^+$] 321.0

**Beispiel 7:**

**3-(3-Chlorphenyl)-N-(2-(methyl(thiazol-5-yl)amino)ethyl)propiolamid**

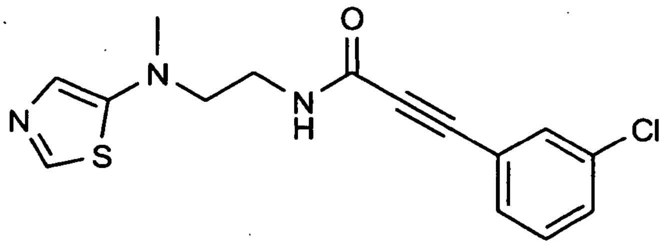

**a) Synthese von $N^1$-Methyl-$N^1$-(thiazol-5-yl)ethan-1,2-diamin**

Zu einer Lösung aus 1.15 ml (10.5 mmol) 5-Bromthiazol und 5.6 ml (63.0 mmol) N-Methyl-ethylen-diamin in Isopropanol (6 ml) wurden 37 mg (0.6 mmol) Kupfer und 172 mg (1.7 mmol) Kupfer(I)-chlorid gegeben. Anschließend wurde 1 h auf 70 °C erhitzt. Anschließend wurde im Vakuum eingeengt. Der Rückstand wurde mit Brine (40 ml) und DCM (20 ml) aufgenommen und die Phasen wurden getrennt. Die organische Phase wurde über $MgSO_4$ getrocknet, filtriert und im Vakuum eingeengt. Mit dem Rückstand wurde eine SC (DCE/EtOH/konz.$NH_4OH$ 5:1:0.06) durchgeführt, wobei 104 mg (0.7 mmol, 6%) $N^1$-Methyl-$N^1$-(thiazol-5-yl)ethan-1,2-diamin erhalten wurden.

**b) Synthese von 3-(3-Chlorphenyl)-N-(2-(methyl(thiazol-5-yl)amino)ethyl)propiolamid**

Zu einer Lösung von 146 mg (0.93 mmol) $N^1$-Methyl-$N^1$-(thiazol-5-yl)ethan-1,2-diamin und 252 mg (1.39 mmol) 3-(3-Chlor-phenyl)propiolsäure in DCM (10 ml) wurden 1.5 g (≅ 1.8 mmol) PS-Carbodiimid gegeben und die Reaktionslösung wurde 16 h bei RT geschüttelt. Anschließend wurde das Harz abfiltriert, mit DCM und Ethanol nach gewaschen und das Filtrat im Vakuum eingeengt. Mit dem Rückstand wurde eine SC (DCE/EtOH 10:1) durchgeführt, wobei 63 mg (0.20 mmol, 21%) 3-(3-Chlorphenyl)-N-(2-(methyl(thiazol-5-yl)amino)ethyl)propiolamid erhalten wurden.
MS: $[MH^+]$ 320.1

**Beispiel 8:**

**3-(3-Chlorphenyl)-N-(2-(methyl(1-methyl-1H-imidazol-2-yl)amino)ethyl)-propiolamid**

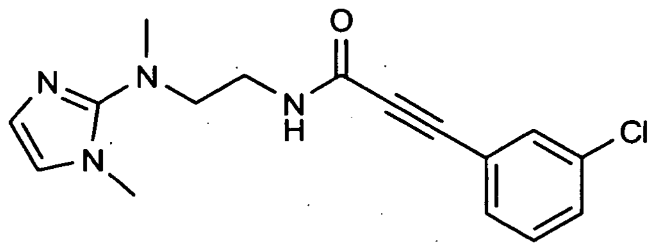

**a) Synthese von 2-Iod-1-methyl-imidazol**

7.97 ml (100.0 mmol) 1-Methyl-imidazol wurden in THF (100 ml) gelöst und auf- 78 °C abgekühlt. Bei dieser Temperatur wurden 40 ml (2.5 M in Hexan, 100 mmol) BuLi zugetropft. Nach beendeter Zugabe wurde für 5 min auf 0 °C erwärmt und anschließen sofort wieder auf -78 °C abgekühlt. Bei dieser Temperatur wurde eine Lösung von 27.9 g (110.0 mmol) Iod ($I_2$) in THF (60 ml) zugetropft und es wurde 20 min bei RT nachgerührt. Anschließend wurde mit eine 5%-igen aq. $Na_2S_2O_3$-Lsg (120 ml) gequencht und mit DCM (4 x 50 ml) extrahiert. Die gesammelten organischen Phasen wurden über $MgSO_4$ getrocknet, filtriert und im Vakuum eingeengt. Durch Kristallisation des Rückstands aus Diethylether wurden 11.5 g (55.2 mmol, 55%) 2-Iod-1-methyl-imidazol erhalten.

**b) Synthese von $N^1$-Methyl-$N^1$-(1-methyl-1H-imidazol-2-yl)ethan-1,2-diamin**

Eine Mischung aus 4.8 g (23.0 mmol) 2-Iod-1-methyl-imidazol, 12.2 ml (138.0 mmol) N-Methyl-ethylen-diamin, 80 mg (1.3 mmol) Kupfer und 374 mg (3.8 mmol) Kupfer(I)-chlorid wurde 1 h auf 70 °C erhitzt. Nach Abkülen auf RT wurde mit Brine und DCM aufgenommen und die Phasen wurden getrennt. Die organische Phase wurde über $MgSO_4$ getrocknet, filtriert und im Vakuum eingeengt. Mit dem Rückstand wurde eine SC (DCE/EtOH/konz. aq. $NH_4OH$-Lsg. 5: 1:0.06) durchgeführt, wobei 215 mg (1.4 mmol, 6%) $N^1$-Methyl-$N^1$-(1-methyl-1H-imidazol-2-yl)ethan-1,2-diamin erhalten wurden.

**c) Synthese von 3-(3-Chlorphenyl)-N-(2-(methyl(1-methyl-1H-imidazol-2-yl)amino)ethyl)-propiolamid**

Zu einer Lösung von 215 mg (1.4 mmol) $N^1$-Methyl-$N^1$-(1-methyl-1H-imidazol-2-yl)ethan-1,2-diamin und 378 mg (2.1 mmol) 3-(3-Chlor-phenyl)propiolsäure in DCM (30 ml) wurden 2.3 g (≅ 2.8 mmol) PS-Carbodiimid gegeben und die Reaktionslösung wurde 16 h bei RT geschüttelt. Anschließend wurde das Harz abfiltriert, mit DCM und Ethanol nachgewaschen und das Filtrat im Vakuum eingeengt. Mit dem Rückstand wurde eine SC (DCE/EtOH 5:1) durchgeführt, wobei 241 mg (0.8 mmol, 54%) 3-(3-Chlorphenyl)-N-(2-(methyl(1-methyl-1H-imidazol-2-yl)amino)ethyl)-propiolamid erhalten wurden.
MS: $[MH^+]$ 317.1

**Pharmakologische Daten:**

1. Die Affinität der erfindungsgemäßen substituierten Propiolsäureamide der allgemeinen Formel I für den mGluR5-Rezeptor wurde wie vorstehend beschrieben bestimmt (Methode I und II).
Die erfindungsgemäßen substituierten Propiolsäureamide zeigen eine ausgezeichnete Affinität für den mGluR5-Rezeptor.
In der nachfolgenden Tabelle 1 sind die pharmakologischen Daten substituierte Propiolsäureamide wiedergegeben:

| Bsp. | $K_i$ mGluR5 Rezeptor (Mensch)$Ca^{2+}$-Influx [nM] | $IC_{50}$ mGluR5 Rezeptor (Schwein) [$^3H$]-MPEP-Bindung [µM] |
|---|---|---|
| 1 | | 1,890 |
| 2 | | 0,630 |
| 3 | | 4,840 |
| 4 | 0,0033 | 0,016 |
| 5 | 0,0048 | 0,027 |
| 6 | | 0,0210 |
| 7 | 0,0008 | 0,0005 |
| 8 | | 0,3500 |

2. Die erfindungsgemäßen substituierten Propiolsäureamide zeigen eine wirkungsvolle Hemmung der Schmerzreaktion im Formalin-Test an der Ratte bei einer i.v. Applikation von 21.5 mg/kg.

| Bsp. | Formalintest (Ratte) i.v. Reduzierung des nozizeptiven Verhaltens gegenüber Kontrolle bei 10 mg/kg [%] |
|---|---|
| 4 | 75 |

**Patentansprüche**

**1.** Substituierte Propiolsäureamide der allgemeinen Formel I,

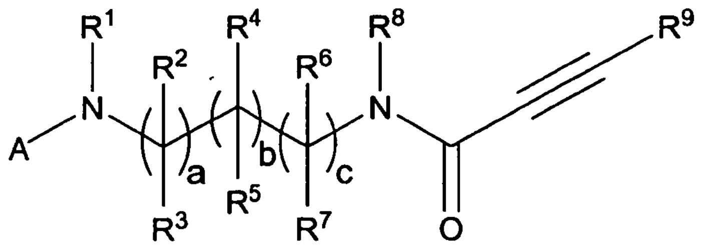

I,

worin

a, b und c, unabhängig voneinander, jeweils für 0 oder 1 stehen, wobei die Summe aus a, b und c gleich 1, 2 oder 3 ist;
A für einen der folgenden Reste

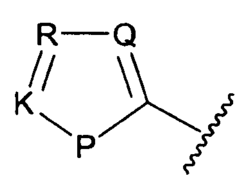

oder

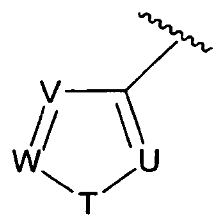

steht;

Q und U jeweils für $CR^{10}$ oder N stehen;
R und V jeweils für $CR^{11}$ oder N stehen;
K und W jeweils für $CR^{12}$ oder N stehen;
P und T jeweils für O, S oder $NR^{13}$ stehen;

mit der Maßgabe, dass Verbindungen ausgeschlossen sind, worin P für S steht, Q für N steht, R für $CR^{11}$ steht und K für $CR^{12}$ oder N steht;
$R^{11}$ und $R^8$, unabhängig voneinander, jeweils für H; -C(=O)-$R^{28}$; -C(=O)-O-$R^{29}$; - C(=O)-$NH_2$; -C(=O)-NH-$R^{30}$; -C (=O)-$NR^{31}R^{32}$; -S(=O)-$R^{33}$; -$S(=O)_2$-$R^{34}$; unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl, -(Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes -(Heteroalkylen)-Cycloalkyl, -(Heteroalkenylen)-Cycloalkyl, - (Heteroalkylen)-Cycloalkenyl oder -(Heteroalkenylen)- Cycloalkenyl; unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl, - (Alkenylen)-Heterocycloalkenyl oder -(Alkinylen)-Heterocycloalkenyl; unsubstituiertes oder substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder substituiertes -(Alkylen)-Heteroaryl, -(Alkenylen)- Heteroaryl, -(Alkinylen)-Heteroaryl, -(Heteroalkylen)-Heteroaryl oder -(Heteroalkenylen)-Heteroaryl stehen;
$R^2$, $R^3$, $R^4$ $R^5$, $R^6$, $R^7$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^2$, $R^3$, $R^4$, $R^5$, $R^6$, $R^7$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ und $R^{27}$, unabhängig voneinander, jeweils für H; F; Cl; Br; I; -$NO_2$; -CN;-$NH_2$; -OH; -SH; -C(=O)-OH; -C(=O)-H; -NH-C(=O)-H; -C(=O)-$R^{28}$; -C(=O)-O-$R^{29}$; -C(=O)-$NH_2$; -C (=O)-NH-$R^{30}$; -C(=O)-$NR^{30}R^{32}$; -S(=O)-$R^{33}$; -$S(=O)_2$-$R^{34}$;-NH-$R^{35}$; -$NR^{36}R^{37}$; -O-C(=O)-$R^{38}$; -NH-C(=O)-$R^{39}$; -$NR^{40}$-C(=O)-$R^{41}$; -O-$R^{42}$; -S-$R^{43}$; -NH-C(=O)-NH-$R^{44}$; -NH-C(=S)-NH-$R^{45}$; -NH-$S(=O)_2$-$R^{46}$; -$NR^{47}$-$S(=O)_2$-$R^{48}$;

unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes -(Alkylen)-Cycloalkyl,-(Alkenylen)-Cycloalkyl, -(Alkinylen)-Cycloalkyl, -(Alkylen)-Cycloalkenyl, - (Alkenylen)-Cycloalkenyl oder -(Alkinylen)-Cycloalkenyl; unsubstituiertes oder wenigstens einfach substituiertes -(Heteroalkylen)-Cycloalkyl, - (Heteroalkenylen)-Cycloalkyl, -(Heteroalkylen)-Cycloalkenyl oder - (Heteroalkenylen)-Cycloalkenyl; unsubstituiertes oder wenigstens einfach substituiertes -(Alkylen)-Heterocycloalkyl, -(Alkenylen)-Heterocycloalkyl, - (Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder wenigstens einfach substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder wenigstens einfach substituiertes -(Alkylen)-Heteroaryl, -(Alkenylen)-Heteroaryl, -(Alkinylen)-Heteroaryl, -(Heteroalkylen)-Heteroaryl oder - (Heteroalkenylen)-Heteroaryl stehen;
oder $R^2$ und $R^3$ oder $R^4$ und $R^5$ oder $R^6$ und $R^7$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$, unabhängig voneinander, zusammen jeweils für einen Rest ausgewählt aus der Gruppe bestehend aus einer Oxo-Gruppe (=O) und einer Thioxo-Gruppe (=S) stehen;
oder $R^1$ und $R^8$ zusammen mit der sie verbindenden $-N-CR^2R^3-(CR^4R^5)_b-(CR^6R^7)_c$-Gruppe einen Rest der allgemeinen Formel B,

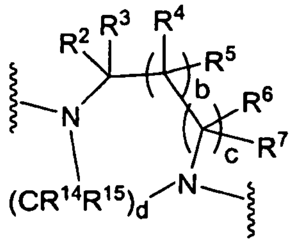

B,

bilden; wobei d für 1, 2 oder 3 steht und b für 0 oder 1 steht und c für 0 oder 1 steht;
oder $R^1$ und $R^2$ zusammen mit der sie verbindenden $-N-CR^3$-Gruppe einen Rest der allgemeinen Formel C,

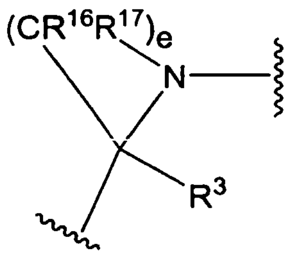

C,

bilden; wobei e für 1, 2, 3 oder 4 steht und, in diesem Fall, b für 0 oder 1 steht und c für 0 steht;
oder $R^1$ und $R^4$ zusammen mit der sie verbindenden $-N-CR^2R^3-CR^5$-Gruppe einen Rest der allgemeinen Formel D,

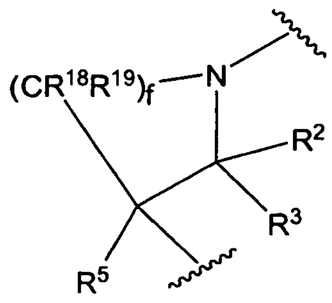

D,

bilden; wobei f für 1, 2, 3 oder 4 steht, und, in diesem Fall, c für 0 steht;
oder $R^1$ und $R^6$ zusammen mit der sie verbindenden $-N-CR^2R^3-CR^4R^5-CR^7$-Gruppe einen Rest der allgemeinen Formel E,

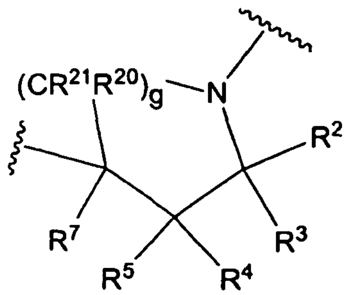

E,

bilden; wobei g für 1, 2 oder 3 steht;
oder $R^6$ und $R^8$ zusammen mit der sie verbindenden $-N-CR^7$-Gruppe einen Rest der allgemeinen Formel F

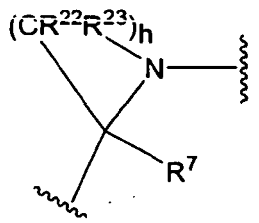

F,

bilden; wobei h für 1, 2 , 3 oder 4 steht und, in diesem Fall, b für 0 oder 1 steht und a für 0 steht;
oder $R^4$ und $R^8$ zusammen mit der sie verbindenden $-N-CR^6R^7-CR^5$-Gruppe einen Rest der allgemeinen Formel G,

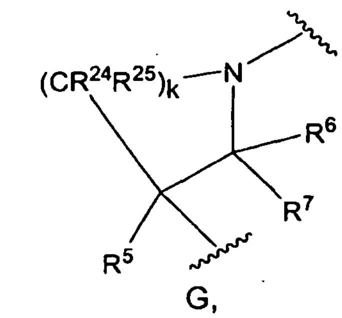

G,

bilden; wobei k für 1, 2, 3 oder 4 steht, und, in diesem, Fall a für 0 steht;
oder $R^2$ und $R^8$ zusammen mit der sie verbindenden -N-$CR^6R^7$-$CR^4R^5$-$CR^3$-Gruppe einen Rest der allgemeinen Formel H,

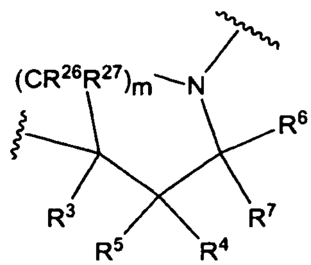

H,

bilden; wobei m für 1, 2 oder 3 steht;
$R^9$ für unsubstituiertes oder substituiertes Aryl oder unsubstituiertes oder substituiertes Heteroaryl steht;
$R^{10}$ $R^{11}$ und $R^{12}$, unabhängig voneinander, jeweils für H; F; Cl; Br; I; -$NO_2$:-CN; -$NH_2$; -OH; -SH; -C(=O)-OH; -C (=O)-H; -NH-C(=O)-H; -C(=O)-$R^{28}$; -C(=O)-O-$R^{29}$; -C(=O)-$NH_2$; -C(=O)-NH-$R^{30}$; -C(=O)-$NR^{31}R^{32}$; -S(=O)-$R^{33}$; -S (=O)$_2$-$R^{34}$; -NH-$R^{35}$; -$NR^{36}R^{37}$; -O-C(=O)-$R^{38}$; -NH-C(=O)-$R^{39}$; -$NR^{40}$-C(=O)-$R^{41}$; -O-$R^{42}$;-S-$R^{43}$; -NH-C(=O)-NH-$R^{44}$; -NH-C(=S)-NH-$R^{45}$; -NH-S(=O)$_2$-$R^{46}$; -$NR^{47}$-S(=O)$_2$-$R^{48}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes -(Alkylen)-Cycloalkyl, - (Alkenylen)-Cycloalkyl, -(Alkinylen)-Cycloalkyl, -(Alkylen)-Cycloalkenyl, - (Alkenylen)-Cycloalkenyl oder -(Alkinylen)-Cycloalkenyl; unsubstituiertes oder wenigstens einfach substituiertes -(Heteroalkylen)-Cycloalkyl,-(Heteroalkenylen)-Cycloalkyl, -(Heteroalkylen)-Cycloalkenyl oder - (Heteroalkenylen)-Cycloalkenyl; unsubstituiertes oder wenigstens einfach substituiertes -(Alkylen)-Heterocycloalkyl, -(Alkenylen)-Heterocycloalkyl, - (Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder wenigstens einfach substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder wenigstens einfach substituiertes -(Alkylen)-Heteroaryl, -(Alkenylen)-Heteroaryl, -(Alkinylen)-Heteroaryl, -(Heteroalkylen)-Heteroaryl oder-(Heteroalkenylen)-Heteroaryl stehen;
$R^{13}$ für H; -C(=O)-OH; -C(=O)-H; -C(=O)-$R^{28}$; -C(=O)-O-$R^{29}$; -C(=O)-$NH_2$; - C(=O)-NH-$R^{30}$; -C(=O)-$NR^{31}R^{32}$; -S (=O)-$R^{33}$; -S(=O)$_2$-$R^{34}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes -(Alkylen)-Cycloalkyl, - (Alkenylen)-Cycloalkyl, -(Alkinylen)-Cycloalkyl, -(Alkylen)-Cycloalkenyl, - (Alkenylen)-Cycloalkenyl oder -(Alkinylen)-Cycloalkenyl; unsubstituiertes oder wenigstens einfach substituiertes -(Heteroalkylen)-Cycloalkyl, - (Heteroalkenylen)-Cycloalkyl, -(Heteroalkylen)-Cycloalkenyl oder - (Heteroalkenylen)-Cycloalkenyl; unsubstituiertes oder wenigstens einfach substituiertes -(Alkylen)-Heterocycloalkyl, -(Alkenylen)-Heterocycloalkyl, -

(Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder wenigstens einfach substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder wenigstens einfach substituiertes -(Alkylen)-Heteroaryl, -(Alkenylen)-Heteroaryl,- (Alkinylen)-Heteroaryl, -(Heteroalkylen)-Heteroaryl oder - (Heteroalkenylen)-Heteroaryl steht;

und $R^{28}$, $R^{29}$, $R^{30}$ , $R^{31}$, $R^{32}$, $R^{33}$, $R^{34}$, $R^{35}$, $R^{36}$ , $R^{37}$ , $R^{38}$, $R^{39}$, $R^{40}$, $R^{41}$, $R^{42}$, $R^{43}$, $R^{44}$ , $R^{45}$, $R^{46}$, $R^{47}$ und $R^{48}$, unabhängig voneinander, jeweils für unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl, -(Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes -(Heteroalkylen)-Cycloalkyl, -(Heteroalkenylen)-Cycloalkyl, - (Heteroalkylen)-Cycloalkenyl oder -(Heteroalkenylen)-Cycloalkenyl; unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl,-(Alkenylen)-Heterocycloalkenyl oder -(Alkinylen)-Heterocycloalkenyl; unsubstituiertes oder substituiertes -(Heteroalkylen)-Heterocycloalkyl, - (Heteroalkenylen)-Heterocycloalkyl, -(Heteroalkylen)-Heterocycloalkenyl; oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder substituiertes -(Alkylen)-Heteroaryl, -(Alkenylen)-Heteroaryl, -(Alkinylen)-Heteroaryl, -(Heteroalkylen)-Heteroaryl oder -(Heteroalkenylen)-Heteroaryl stehen;

wobei "substituiert" im Zusammenhang mit den Resten "Alkyl", "Alkenyl", "Alkinyl", "Heteroalkyl", "Heteroalkenyl" und "Heteroalkinyl" für einen entsprechenden Rest steht, der einfach oder mehrfach mit Substituenten unabhängig voneinander ausgewählt aus der Gruppe bestehend aus F, Cl, Br, I, $-NO_2$, -CN, -OH, -SH, $-NH_2$, $-N(C_{1-5}$-Alkyl$)_2$, $-N(C_{1-5}$-Alkyl)(Phenyl), $-N(C_{1-5}$-Alkyl)($CH_2$-Phenyl), $-N(C_{1-5}$-Alkyl)($CH_2$-$CH_2$-Phenyl), -C(=O)-H, -C(=O)-$C_{1-5}$-Alkyl, -C(=O)-Phenyl, -C(=S)-$C_{1-5}$-Alkyl, -C(=S)-Phenyl, -C(=O)-OH, -C(=O)-O-$C_{1-5}$-Alkyl,- C(=O)-O-Phenyl, -C(=O)-$NH_2$, -C(=O)-NH-$C_{1-5}$-Alkyl, -C(=O)- N($C_{1-5}$-Alkyl$)_2$, -S(=O)-$C_{1-5}$-Alkyl, -S(=O)-Phenyl, $-S(=O)_2$-$C_{1-5}$-Alkyl, $-S(=O)_2$-Phenyl, $-S(=O)_2$-$NH_2$ und $-SO_3H$ substituiert ist, wobei die vorstehend genannten $C_{1-5}$-Alkyl-Reste jeweils linear oder verzweigt sein können und die vorstehend genannten Phenyl-Reste mit 1, 2, 3, 4 oder 5 Substituenten unabhängig voneinander ausgewählt aus der Gruppe bestehend aus F, Cl, Br, I, -CN, $-CF_3$, -OH, $-NH_2$, $-O-CF_3$, -SH, $-O-CH_3$, $-O-C_2H_5$, $-O-C_3H_7$, Methyl, Ethyl, n-Propyl, Isopropyl, n-Butyl, 2-Butyl, Isobutyl und tert-Butyl substituiert sein können,

wobei "substituiert" im Zusammenhang mit den Resten "Cycloalkyl", "Heterocycloalkyl", "Cycloalkenyl" und "Heterocycloalkenyl" für einen entsprechenden Rest steht, der einfach oder mehrfach mit Substituenten unabhängig voneinander ausgewählt aus der Gruppe bestehend aus F, Cl, Br, I, -CN, $-CF_3$, -OH, $-NH_2$, $-O-CF_3$, -SH, -O-$C_{1-5}$-Alkyl, -O-Phenyl, -O-$CH_2$-Phenyl, -($CH_2$)-O-$C_{1-5}$-Alkyl, -S-$C_{1-5}$-Alkyl, -S-Phenyl, -S-$CH_2$-Phenyl, -$C_{1-5}$-Alkyl, -$C_{2-5}$-Alkenyl, -$C_{2-5}$-Alkinyl, -C≡C-Si$(CH_3)_3$, -C≡C-Si$(C_2H_5)_3$, -C(=O)-O-$C_{1-5}$-Alkyl, -C(=O)-$CF_3$, $-S(=O)_2$-$C_{1-5}$-Alkyl, -S(=O)-$C_{1-5}$-Alkyl, $-S(=O)_2$-Phenyl, Oxo (=O), Thioxo (=S), -N($C_{1-5}$-Alkyl$)_2$, -N(H)($C_{1-5}$-Alkyl), $-NO_2$, $-S-CF_3$, -C (=O)-OH, -NH-$S(=O)_2$-$C_{1-5}$-Alkyl, -NH-C(=O)-$C_{1-5}$-Alkyl, -C(=O)-H, -C(=O)-$C_{1-5}$-Alkyl, - C(=O)-$NH_2$, -C(=O)-N ($C_{1-5}$-Alkyl$)_2$, -C(=O)-N(H)($C_{1-5}$-Alkyl) und Phenyl substituiert ist, wobei die vorstehend genannten $C_{1-5}$-Alkyl-Reste jeweils linear oder verzweigt sein können und die Phenyl-Reste jeweils unsubstituiert oder mit 1, 2, 3, 4 oder 5 unabhängig voneinander ausgewählt aus der Gruppe bestehend aus F, Cl, Br, I, -CN, $-CF_3$, -OH, $-NH_2$, $-O-CF_3$, -SH, -O-$C_{1-5}$-Alkyl, - O-Phenyl, -O-$CH_2$-Phenyl, -($CH_2$)-O-$C_{1-5}$-Alkyl, -S-$C_{1-5}$-Alkyl, -S-Phenyl, -S-$CH_2$-Phenyl, -$C_{1-5}$-Alkyl, -$C_{2-5}$-Alkenyl, -$C_{2-5}$-Alkinyl, -C≡C-Si$(CH_3)_3$, -C=C-Si$(C_2H_5)_3$, -C(=O)-O-$C_{1-5}$-Alkyl und -C(=O)-$CF_3$ substituiert sein könnten,

wobei "substituiert" im Zusammenhang mit den Resten "Aryl" und "Heteroaryl" für einen entsprechenden Rest steht, der einfach oder mehrfach mit substituenten unabhängig voneinander ausgewählt aus der Gruppe bestehend aus F, Cl, Br, I, -CN, $-NO_2$, -OH, -SH, $-NH_2$, -C(=O)-OH, -$C_{1-5}$-Alkyl, -($CH_2$)-O-$C_{1-5}$-Alkyl, -$C_{2-5}$-Alkenyl, -$C_{2-5}$-Alkinyl, -C≡C-Si$(CH_3)_3$, -C≡C-Si$(C_2H_5)_3$,- S-$C_{1-5}$-Alkyl, -S-Phenyl, -S-$CH_2$-Phenyl, -O-$C_{1-5}$-Alkyl, -O-Phenyl,-O-$CH_2$-Phenyl, $-CF_3$, $-CHF_2$, $-CH_2F$, $-O-CF_3$, $-O-CHF_2$, $-O-CH_2F$, -C(=O)-$CF_3$, - $S-CF_3$, $-S-CHF_2$, $-S-CH_2F$, $-S(=O)_2$-Phenyl, $-S(=O)_2$-$C_{1-5}$-Alkyl, -S(=O)-$C_{1-5}$-Alkyl, -NH-$C_{1-5}$-Alkyl, N($C_{1-5}$Alkyl$)_2$, -C(=O)-O-$C_{1-5}$-Alkyl, -C(=O)-O-Phenyl, - C (=O)-H; -C(=O)-$C_{1-5}$-Alkyl, -$CH_2$-O-C(=O)-Phenyl, -O-C(=O)-$C_{1-5}$-Alkyl, -O-C(=O)-Phenyl, -NH-$S(=O)_2$-$C_{1-5}$-Alkyl, -NH-C(=O)-$C_{1-5}$-Alkyl, -C(=O)-$NH_2$, - C(=O)-NH-$C_{1-5}$-Alkyl, -C(=O)-N($C_{1-5}$-Alkyl$)_2$, -C(=O)-N($C_{1-5}$-Alkyl)(Phenyl), - C(=O)-NH-Phenyl, Cyclopropyl, Cyclobutyl, Cyclopentyl, Cyclohexyl, Pyrazolyl, Phenyl, Furyl (Furanyl), Thiazolyl, Thiadiazolyl, Thiophenyl (Thienyl), Benzyl und Phenethyl substituiert ist, wobei die vorstehend genannten $C_{1-5}$-Alkyl-Reste jeweils linear oder verzweigt sein können und die zyklischen Substituenten bzw. die zyklischen Reste dieser Substituenten selbst mit ggf. 1, 2, 3, 4 oder 5 Substituenten unabhängig voneinander ausgewählt aus der Gruppe

bestehend aus F, Cl, Br, I, -CN, $-NO_2$, -OH, -SH, - $NH_2$, -C(=O)-OH, $-C_{1-5}$-Alkyl, $-(CH_2)-O-C_{1-5}$-Alkyl, $-C_{2-5}$-Alkenyl, $-C_{2-5}$-Alkinyl, - $C{\equiv}C-Si(CH_3)_3$, $-C{\equiv}C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ und $-S-CH_2F$ substituiert sein können,

wobei "substituiert" im Zusammenhang mit den Resten "Alkylen", "Alkenylen", "Alkinylen", "Heteroalkylen" und "Heteroalkenylen" für einen entsprechenden Rest steht, der einfach oder mehrfach mit Substituenten unabhängig voneinander ausgewählt aus der Gruppe bestehend aus Phenyl, F, Cl, Br, I, - $NO_2$, -CN, -OH, -O-Phenyl, -O-$CH_2$-Phenyl, -SH, -S-Phenyl, $-S-CH_2$-Phenyl, - $NH_2$, $-N(C_{1-5}\text{-Alkyl})_2$, -NH-Phenyl, $-N(C_{1-5}$-Alkyl)(Phenyl), $-N(C_{1-5}$-Alkyl)($CH_2$-Phenyl), $-N(C_{1-5}$-Alkyl)($CH_2$-$CH_2$-Phenyl), -C(=O)-H, $-C(=O)-C_{1-5}$-Alkyl, -C(=O)-Phenyl, $-C(=S)-C_{1-5}$-Alkyl, -C(=S)-Phenyl, -C(=O)-OH, $-C(=O)-O-C_{1-5}$-Alkyl, - C(=O)-O-Phenyl, $-C(=O)-NH_2$, $-C(=O)-NH-C_{1-5}$-Alkyl, -C(=O)-N$(C_{1-5}\text{-Alkyl})_2$, - $S(=O)-C_{1-5}$-Alkyl, -S(=O)-Phenyl, $-S(=O)_2-C_{1-5}$-Alkyl, $-S(=O)_2$-Phenyl, $-S(=O)_2-NH_2$ und $-SO_3H$ substituiert ist, wobei die vorstehend genannten $C_{1-5}$-Alkyl-Reste jeweils linear oder verzweigt sein können und die vorstehend genannten Phenyl-Reste mit 1, 2, 3, 4 oder 5 Substituenten unabhängig voneinander ausgewählt aus der Gruppe bestehend aus F, Cl, Br, I, -CN, $-NO_2$, -OH, -SH, - $NH_2$, -C(=O)-OH, $-C_{1-5}$-Alkyl, $-(CH_2)-O-C_{1-5}$-Alkyl, $-C_{2-5}$-Alkenyl, $-C_{2-5}$-Alkinyl, - $C{\equiv}C-Si(CH_3)_3$, $-C{\equiv}C-Si(C_2H_5)_3$. $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ und $-S-CH_2F$ substituiert sein können,

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Verbindungen gemäß Anspruch 1, **dadurch gekennzeichnet, dass**
a, b und c, unabhängig voneinander, jeweils für 0 oder 1 stehen, wobei die Summe aus a, b und c gleich 1, 2 oder 3 ist;
A für einen der folgenden Reste

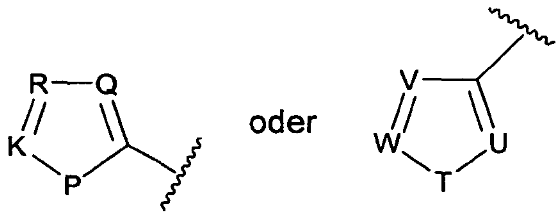

steht;

Q und U jeweils für $CR^{10}$ oder N stehen;
R und V jeweils für $CR^{11}$ oder N stehen;
K und W jeweils für $CR^{12}$ oder N stehen;
P und T jeweils für O, S oder $NR^{13}$ stehen;

mit der Maßgabe, dass Verbindungen ausgeschlossen sind, worin P für S steht, Q für N steht, R für $CR^{11}$ steht und K für $CR^{12}$ oder N steht;

$R^1$ und $R^8$, unabhängig voneinander, jeweils für H; $-C(=O)-R^{28}$; $-C(=O)-O-R^{29}$; - $C(=O)-NH_2$; $-C(=O)-NH-R^{30}$; -C(=O)-$NR^{31}R^{32}$; $-S(=O)-R^{33}$; $-S(=O)_2-R^{34}$; unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl, -(Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes -(Heteroalkylen)-Cycloalkyl, -(Heteroalkenylen)-Cycloalkyl, - (Heteroalkylen)-Cycloalkenyl oder -(Heteroalkenylen)- Cycloalkenyl;

unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl, - (Alkenylen)-Heterocycloalkenyl oder -(Alkinylen)-Heterocycloalkenyl; unsubstituiertes oder substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder substituiertes -(Alkylen)-Heteroaryl, -(Alkenylen)- Heteroaryl, -(Alkinylen)-Heteroaryl, -(Heteroalkylen)-Heteroaryl oder -(Heteroalkenylen)-Heteroaryl stehen;

$R^2$, $R^3$, $R^4$, $R^5$, $R^6$, $R^7$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ und $R^{27}$, unabhängig voneinander, jeweils für H; F; Cl; Br; I; $-NO_2$: -CN; - $NH_2$; -OH; -SH; -C(=O)-OH; -C(=O)-H; -NH-C(=O)-H; -C(=O)-$R^{28}$; -C(=O)-O-$R^{29}$; -C(=O)-$NH_2$; -C(=O)-NH-$R^{30}$; -C(=O)-N$R^{31}R^{32}$; -S(=O)-$R^{33}$; -$S(=O)_2$-$R^{34}$;-NH-$R^{35}$; -N$R^{36}R^{37}$; -O-C(=O)-$R^{38}$; -NH-C(=O)-$R^{39}$; -N$R^{40}$-C(=O)-$R^{41}$; -O-$R^{42}$; -S-$R^{43}$; -NH-C(=O)-NH-$R^{44}$; -NH-C(=S)-NH-$R^{45}$; -NH-$S(=O)_2$-$R^{46}$; -N$R^{47}$-$S(=O)_2$-$R^{48}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes -(Alkylen)-Cycloalkyl, - (Alkenylen)-Cycloalkyl, -(Alkinylen)-Cycloalkyl, -(Alkylen)-Cycloalkenyl, - (Alkenylen)-Cycloalkenyl oder -(Alkinylen)-Cycloalkenyl; unsubstituiertes oder wenigstens einfach substituiertes -(Heteroalkylen)-Cycloalkyl, - (Heteroalkenylen)-Cycloalkyl, -(Heteroalkylen)-Cycloalkenyl oder - (Heteroalkenylen)-Cycloalkenyl; unsubstituiertes oder wenigstens einfach substituiertes -(Alkylen)-Heterocycloalkyl, -(Alkenylen)-Heterocycloalkyl, -(Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder wenigstens einfach substituiertes -(Heteroalkylen)-Heterocycloalkyl, -(Heteroalkenylen)-Heterocycloalkyl, -(Heteroalkylen)-Heterocycloalkenyl oder - (Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder wenigstens einfach substituiertes -(Alkylen)-Heteroaryl, -(Alkenylen)-Heteroaryl, -(Alkinylen)-Heteroaryl, -(Heteroalkylen)-Heteroaryl oder - (Heteroalkenylen)-Heteroaryl stehen;

oder $R^2$ und $R^3$ oder $R^4$ und $R^5$ oder $R^6$ und $R^7$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$, unabhängig voneinander, zusammen jeweils für einen Rest ausgewählt aus der Gruppe bestehend aus einer Oxo-Gruppe (=O) und einer Thioxo-Gruppe (=S) stehen;

oder $R^1$ und $R^8$ zusammen mit der sie verbindenden -N-C$R^2R^3$-$(CR^4R^5)_b$-$(CR^6R^7)_c$-Gruppe einen Rest der allgemeinen Formel B,

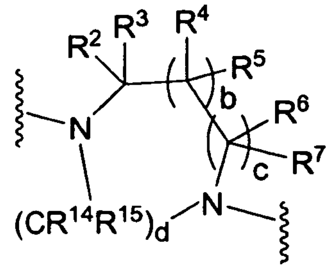

B,

bilden; wobei d für 1, 2 oder 3 steht und b für 0 oder 1 steht und c für 0 oder 1 steht;

oder $R^1$ und $R^2$ zusammen mit der sie verbindenden -N-C$R^3$-Gruppe einen Rest der allgemeinen Formel C,

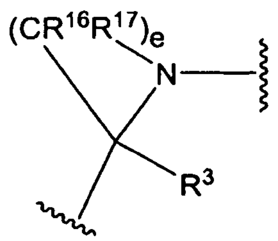

C,

bilden; wobei e für 1, 2 , 3 oder 4 steht und, in diesem Fall, b für 0 oder 1 steht und c für 0 steht; oder $R^1$ und $R^4$ zusammen mit der sie verbindenden $-N-CR^2R^3-CR^5$-Gruppe einen Rest der allgemeinen Formel D,

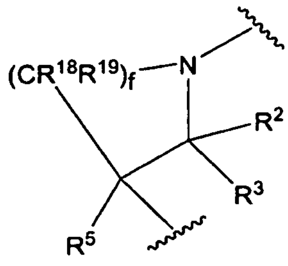

D,

bilden; wobei f für 1, 2, 3 oder 4 steht, und, in diesem Fall, c für 0 steht; oder $R^1$ und $R^6$ zusammen mit der sie verbindenden $-N-CR^2R^3-CR^4R^5-CR^7$-Gruppe einen Rest der allgemeinen Formel E,

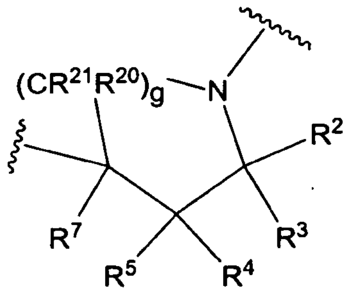

E,

bilden; wobei g für 1, 2 oder 3 steht; oder $R^6$ und $R^8$ zusammen mit der sie verbindenden $-N-CR^7$-Gruppe einen Rest der allgemeinen Formel F

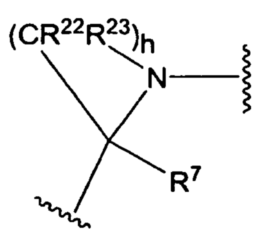

F,

bilden; wobei h für 1, 2 , 3 oder 4 steht und, in diesem Fall, b für 0 oder 1 steht und a für 0 steht; oder $R^4$ und $R^8$ zusammen mit der sie verbindenden $-N-CR^6R^7-CR^5$-Gruppe einen Rest der allgemeinen Formel G,

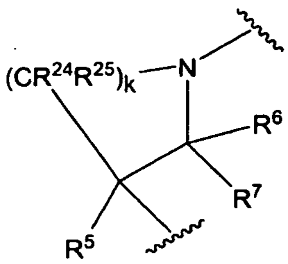

G,

bilden; wobei k für 1, 2, 3 oder 4 steht, und, in diesem, Fall a für 0 steht;

oder $R^2$ und $R^8$ zusammen mit der sie verbindenden -N-$CR^6R^7$-$CR^4R^5$-$CR^3$-Gruppe einen Rest der allgemeinen Formel H

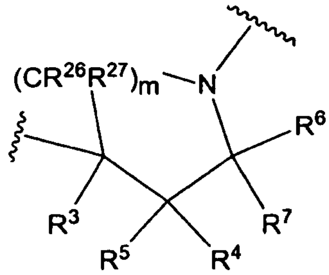

H,

bilden; wobei m für 1, 2 oder 3 steht;

$R^9$ für unsubstituiertes oder substituiertes Aryl oder unsubstituiertes oder substituiertes Heteroaryl steht;

$R^{10}$, $R^{11}$ und $R^{12}$, unabhängig voneinander, jeweils für H; F; Cl; Br; I; -$NO_2$; - CN; -$NH_2$; -OH; -SH; -C(=O)-OH; -C(=O)-H; -NH-C(=O)-H; -C(=O)-$R^{28}$; -C(=O)-O-$R^{29}$; -C(=O)-$NH_2$; -C(=O)-NH-$R^{30}$; -C(=O)-$NR^{31}R^{32}$; -S(=O)-$R^{33}$; -S(=O)$_2$-$R^{34}$; -NH-$R^{35}$ ; -$NR^{36}R^{37}$; -O-C(=O)-$R^{38}$; -NH-C(=O)-$R^{39}$; -$NR^{40}$-C(=O)-$R^{41}$ -O-$R^{42}$; - S-$R^{43}$ ; -NH-C(=O)-NH-$R^{44}$; -NH-C(=S)-NH-$R^{45}$; -NH-S(=O)$_2$-$R^{46}$; -$NR^{47}$-S(=O)$_2$-$R^{48}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes -(Alkylen)-Cycloalkyl, - (Alkenylen)-Cycloalkyl, -(Alkinylen)-Cycloalkyl, -(Alkylen)-Cycloalkenyl, - (Alkenylen)-Cycloalkenyl oder -(Alkinylen)-Cycloalkenyl; unsubstituiertes oder wenigstens einfach substituiertes -(Heteroalkylen)-Cycloalkyl, - (Heteroalkenylen)-Cycloalkyl, -(Heteroalkylen)-Cycloalkenyl oder - (Heteroalkenylen)-Cycloalkenyl; unsubstituiertes oder wenigstens einfach substituiertes -(Alkylen)-Heterocycloalkyl, -(Alkenylen)-Heterocycloalkyl, - (Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder wenigstens einfach substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder wenigstens einfach substituiertes -(Alkylen)-Heteroaryl, -(Alkenylen)-Heteroaryl, -(Alkinylen)-Heteroaryl, -(Heteroalkylen)-Heteroaryl oder - (Heteroalkenylen)-Heteroaryl stehen;

$R^{13}$ für H; -C(=O)-OH; -C(=O)-H; -C(=O)-$R^{28}$; -C(=O)-O-$R^{29}$; -C(=O)-$NH_2$; - C(=O)-NH-$R^{30}$; -C(=O)-$NR^{31}R^{32}$; -S(=O)-$R^{33}$; -S(=O)$_2$-$R^{34}$; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes -(Alkylen)-Cycloalkyl, - (Alkenylen)-Cycloalkyl, -(Alkinylen)-Cycloalkyl, -(Alkylen)-Cycloalkenyl, - (Alkenylen)-Cycloalkenyl oder -(Alkinylen)-Cycloalkenyl; unsubstituiertes oder wenigstens einfach substituiertes -(Heteroalkylen)-Cycloalkyl, -

(Heteroalkenylen)-Cycloalkyl, -(Heteroalkylen)-Cycloalkenyl oder - (Heteroalkenylen)-Cycloalkenyl; unsubstituiertes oder wenigstens einfach substituiertes -(Alkylen)-Heterocycloalkyl, -(Alkenylen)-Heterocycloalkyl, - (Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder wenigstens einfach substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder wenigstens einfach substituiertes -(Alkylen)-Heteroaryl, -(Alkenylen)-Heteroaryl, -(Alkinylen)-Heteroaryl, -(Heteroalkylen)-Heteroaryl oder - (Heteroalkenylen)-Heteroaryl steht;

und $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$ und $R^{48}$, unabhängig voneinander, jeweils für unsubstituiertes oder substituiertes Alkyl, Alkenyl oder Alkinyl; unsubstituiertes oder substituiertes Heteroalkyl, Heteroalkenyl oder Heteroalkinyl; unsubstituiertes oder substituiertes Cycloalkyl oder Cycloalkenyl; unsubstituiertes oder substituiertes Heterocycloalkyl oder Heterocycloalkenyl; unsubstituiertes oder substituiertes -(Alkylen)-Cycloalkyl, -(Alkenylen)-Cycloalkyl, -(Alkinylen)-Cycloalkyl, -(Alkylen)-Cycloalkenyl, -(Alkenylen)-Cycloalkenyl oder -(Alkinylen)-Cycloalkenyl; unsubstituiertes oder substituiertes -(Heteroalkylen)-Cycloalkyl, -(Heteroalkenylen)-Cycloalkyl, - (Heteroalkylen)-Cycloalkenyl oder -(Heteroalkenylen)-Cycloalkenyl; unsubstituiertes oder substituiertes -(Alkylen)-Heterocycloalkyl, -(Alkenylen)-Heterocycloalkyl, -(Alkinylen)-Heterocycloalkyl, -(Alkylen)-Heterocycloalkenyl, - (Alkenylen)-Heterocycloalkenyl oder -(Alkinylen)-Heterocycloalkenyl; unsubstituiertes oder substituiertes -(Heteroalkylen)-Heterocycloalkyl, - (Heteroalkenylen)-Heterocycloalkyl, -(Heteroalkylen)-Heterocycloalkenyl; oder -(Heteroalkenylen)-Heterocycloalkenyl; unsubstituiertes oder substituiertes Aryl; unsubstituiertes oder substituiertes Heteroaryl; unsubstituiertes oder substituiertes -(Alkylen)-Aryl, -(Alkenylen)-Aryl, -(Alkinylen)-Aryl, - (Heteroalkylen)-Aryl oder -(Heteroalkenylen)-Aryl; oder unsubstituiertes oder substituiertes -(Alkylen)-Heteroaryl, -(Alkenylen)-Heteroaryl, -(Alkinylen)-Heteroaryl, -(Heteroalkylen)-Heteroaryl oder -(Heteroalkenylen)-Heteroaryl stehen;

wobei

die vorstehend genannten Alkyl-Reste jeweils verzweigt oder geradkettig sind und 1, 2, 3, 4, 5, 6, 7, 8, 9, 10, 11 oder 12 Kohlenstoffatome als Kettenglieder aufweisen;

die vorstehend genannten Alkenyl-Reste jeweils verzweigt oder geradkettig sind und 2, 3, 4, 5, 6, 7, 8, 9, 10, 11 oder 12 Kohlenstoffatome als Kettenglieder aufweisen;

die vorstehend genannten Alkinyl-Reste jeweils verzweigt oder geradkettig sind und 2, 3, 4, 5, 6, 7, 8, 9, 10, 11 oder 12 Kohlenstoffatome als Kettenglieder aufweisen;

die vorstehend genannten Heteroalkyl-Reste, Heteroalkenyl-Reste und Heteroalkinyl-Reste jeweils 2-, 3-, 4-, 5-, 6-, 7-, 8-, 9-, 10-, 11- oder 12-gliedrig sind;

die vorstehend genannten Heteroalkyl-Reste, Heteroalkenyl-Reste und Heteroalkinyl-Reste jeweils ggf. 1, 2 oder 3 Heteroatom(e) unabhängig voneinander ausgewählt aus der Gruppe bestehend aus Sauerstoff, Schwefel und Stickstoff als Kettenglied(er) aufweisen;

die vorstehend genannten Alkyl-Reste, Alkenyl-Reste, Alkinyl-Reste, Heteroalkyl-Reste, Heteroalkenyl-Reste und Heteroalkinyl-Reste jeweils mit ggf. 1, 2, 3, 4 oder 5 Substituenten unabhängig voneinander ausgewählt aus der Gruppe bestehend aus F, Cl, Br, I, $-NO_2$, -CN, -OH, -SH, $-NH_2$, $-N(C_{1-5}\text{-Alkyl})_2$, $-N(C_{1-5}$-Alkyl)(Phenyl), $-N(C_{1-5}$-Alkyl)($CH_2$-Phenyl), $-N(C_{1-5}$-Alkyl)($CH_2$-$CH_2$-Phenyl),- C(=O)-H, -C(=O)-$C_{1-5}$-Alkyl, -C(=O)-Phenyl, -C(=S)-$C_{1-5}$-Alkyl, -C(=S)-Phenyl, -C(=O)-OH, -C(=O)-O-$C_{1-5}$-Alkyl, -C(=O)-O-Phenyl, -C(=O)-$NH_2$, -C(=O)-NH-$C_{1-5}$-Alkyl, -C(=O)-N($C_{1-5}$-Alkyl)$_2$, -S(=O)-$C_{1-5}$-Alkyl, -S(=O)-Phenyl, -S(=O)$_2$-$C_{1-5}$-Alkyl, -S(=O)$_2$-Phenyl, -S(=O)$_2$-$NH_2$ und $-SO_3H$ substituiert sein können, wobei die Phenyl-Reste mit 1, 2, 3, 4 oder 5 Substituenten unabhängig voneinander ausgewählt aus der Gruppe bestehend aus F, Cl, Br, I, -CN, $-CF_3$, -OH, $-NH_2$, $-O-CF_3$, -SH, $-O-CH_3$, $-O-C_2H_5$, $-O-C_3H_7$, Methyl, Ethyl, n-Propyl, Isopropyl, n-Butyl, 2-Butyl, Isobutyl und tert-Butyl substituiert sein können;

die vorstehend genannten Cycloalkyl-Reste jeweils 3, 4, 5, 6, 7, 8 oder 9 Kohlenstoffatome als Ringglieder aufweisen;

die vorstehend genannten Cycloalkenyl-Reste jeweils 3, 4, 5, 6, 7, 8 oder 9 Kohlenstoffatome als Ringglieder aufweisen;

die vorstehend genannten Heterocycloalkyl-Reste jeweils 3-, 4-, 5-, 6-, 7-, 8-oder 9-gliedrig sind;

die vorstehend genannten Heterocycloalkenyl-Reste jeweils 4-, 5-, 6-, 7-, 8-oder 9-gliedrig sind;

die vorstehend genannten Heterocycloalkyl-Reste und Heterocycloalkenyl-Resten jeweils ggf. 1, 2 oder 3 Heteroatom(e) unabhängig voneinander ausgewählt aus der Gruppe bestehend aus Sauerstoff, Schwefel und Stickstoff (NH) als Ringglied(er) aufweisen;

die vorstehend genannten Cycloalkyl-Reste, Heterocycloalkyl-Reste, Cycloalkenyl-Reste oder Heterocycloalkenyl-Reste jeweils mit ggf. 1, 2, 3, 4 oder 5 Substituenten unabhängig voneinander ausgewählt aus der Gruppe bestehend aus F, Cl, Br, I, -CN, $-CF_3$, -OH, $-NH_2$, $-O-CF_3$, -SH, -O-$C_{1-5}$-Alkyl, - O-Phenyl, -O-$CH_2$-Phenyl, -($CH_2$)-O-$C_{1-5}$-Alkyl, -S-$C_{1-5}$-Alkyl, -S-Phenyl, -S-$CH_2$-Phenyl, -$C_{1-5}$-Alkyl, -$C_{2-5}$-Alkenyl, -$C_{2-5}$-Alkinyl, $-C\equiv C-Si(CH_3)_3$, $-C\equiv C-Si(C_2H_5)_3$, -C(=O)-O-$C_{1-5}$-Alkyl, -C(=O)-$CF_3$, -S(=O)$_2$-$C_{1-5}$-Alkyl, -S(=O)-$C_{1-5}$-Alkyl,- S(=O)$_2$-Phenyl, Oxo (=O), Thioxo (=S),

$-N(C_{1-5}$-Alkyl$)_2$, -N(H)($C_{1-5}$-Alkyl), $-NO_2$, $-S-CF_3$, -C(=O)-OH, -NH-C(=O)-$C_{1-5}$-Alkyl, -C(=O)-H, -C(=O)-$C_{1-5}$-Alkyl, -C(=O)-$NH_2$, -C(=O)-N($C_{1-5}$-Alkyl$)_2$, -C(=O)-N(H)($C_{1-5}$-Alkyl) und Phenyl substituiert sein können, wobei die Phenyl-Reste jeweils unsubstituiert oder mit 1, 2, 3, 4 oder 5 Substituenten unabhängig voneinander ausgewählt aus der Gruppe bestehend aus F, Cl, Br, I, -CN, $-CF_3$, -OH, $-NH_2$, $-O-CF_3$, -SH, -O-$C_{1-5}$-Alkyl, -O-Phenyl, -O-$CH_2$-Phenyl, -($CH_2$)-O-$C_{1-5}$-Alkyl, -S-$C_{1-5}$-Alkyl, -S-Phenyl, -S-$CH_2$-Phenyl, -$C_{1-5}$-Alkyl, -$C_{2-5}$-Alkenyl, -$C_{2-5}$-Alkinyl, -C≡C-Si($CH_3)_3$, -C≡C-Si($C_2H_5)_3$, -C(=O)-O-$C_{1-5}$-Alkyl und -C(=O)-$CF_3$ substituiert sein können, wobei die vorstehend genannten Phenyl-Reste mit 1, 2, 3, 4 oder 5 Substituenten unabhängig voneinander ausgewählt aus der Gruppe bestehend aus F, Cl, Br, I, -CN, $-CF_3$, -OH, $-NH_2$, $-O-CF_3$, -SH, $-O-CH_3$, $-O-C_2H_5$, $-O-C_3H_7$, Methyl, Ethyl, n-Propyl, Isopropyl, n-Butyl, 2-Butyl, Isobutyl und tert-Butyl substituiert sein können;

die vorstehend genannten Alkylen-Reste jeweils verzweigt oder geradkettig sind und 1, 2, 3, 4, 5, 6, 7, 8, 9, 10, 11 oder 12 Kohlenstoffatome als Kettenglieder aufweisen;

die vorstehend genannten Alkenylen-Reste jeweils verzweigt oder geradkettig sind und 2, 3, 4, 5, 6, 7, 8, 9, 10, 11 oder 12 Kohlenstoffatome als Kettenglieder aufweisen;

die vorstehend genannten Alkinylen-Reste jeweils verzweigt oder geradkettig sind und 2, 3, 4, 5, 6, 7, 8, 9, 10, 11 oder 12 Kohlenstoffatome als Kettenglieder aufweisen;

die vorstehend genannten Heteroalkylen-Reste und Heteroalkenylen-Reste jeweils 2-, 3-, 4-, 5-, 6-, 7-, 8-, 9-, 10-, 11- oder 12-gliedrig sind;

die vorstehend genannten Heteroalkylen- und Heteroalkenylen-Gruppen jeweils ggf. 1, 2 oder 3 Heteroatom(e) unabhängig voneinander ausgewählt aus der Gruppe bestehend aus Sauerstoff, Schwefel und Stickstoff (NH) als Kettenglied(er) aufweisen;

die vorstehend genannten Alkylen-, Alkenylen-, Alkinylen-, Heteroalkylen- oder Heteroalkenylen-Gruppe jeweils unsubstituiert oder mit ggf. 1, 2, 3, 4 oder 5 Substituenten unabhängig voneinander ausgewählt aus der Gruppe bestehend aus Phenyl, F, Cl, Br, I, $-NO_2$, -CN, -OH, -O-Phenyl, -O-$CH_2$-Phenyl, -SH, -S-Phenyl, -S-$CH_2$-Phenyl, $NH_2$, -N($C_{1-5}$-Alkyl$)_2$, -NH-Phenyl, - N($C_{1-5}$-Alkyl)(Phenyl), -N($C_{1-5}$-Alkyl)($CH_2$-Phenyl), -N($C_{1-5}$-Alkyl)($CH_2$-$CH_2$-Phenyl), -C(=O)-H, -C(=O)-$C_{1-5}$-Alkyl, -C(=O)-Phenyl, -C(=S)-$C_{1-5}$-Alkyl, - C(=S)-Phenyl, -C(=O)-OH, -C(=O)-O-$C_{1-5}$-Alkyl, -C(=O)-O-Phenyl, -C(=O)-$NH_2$, -C(=O)-NH-$C_{1-5}$-Alkyl, -C(=O)-N($C_{1-5}$-Alkyl$)_2$, -S(=O)-$C_{1-5}$-Alkyl, -S(=O)-Phenyl, -S(=O$)_2$-$C_{1-5}$-Alkyl, -S(=O$)_2$Phenyl, -S(=O$)_2$-$NH_2$ und $-SO_3H$ substituiert sein können, wobei die Phenyl-Reste mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$, -C(=O)-OH, -$C_{1-5}$-Alkyl, -($CH_2$)-O-$C_{1-5}$-Alkyl, -$C_{2-5}$-Alkenyl, -$C_{2-5}$-Alkinyl, -C≡C-Si($CH_3)_3$, -C≡C-Si($C_2H_5)_3$, -S-$C_{1-5}$-Alkyl, -S-Phenyl, -S-$CH_2$-Phenyl, -O-$C_{1-5}$-Alkyl, -O-Phenyl, -O-$CH_2$-Phenyl, $-CF_3$, $-CHF_2$, $-CH_2F$, $-O-CF_3$, $-O-CHF_2$, $-O-CH_2F$, -C(=O)-$CF_3$, $-S-CF_3$, $-S-CHF_2$ und $-S-CH_2F$ substituiert sein können;

die vorstehend genannten Aryl-Reste mono- oder bizyklisch sind und 6, 10 oder 14-Kohlenstoffatome aufweisen;

die vorstehend genannten Heteroaryl-Reste mono-, bi- oder trizyklisch und 5-, 6-, 7-, 8-, 9-, 10-, 11-, 12-, 13- oder 14-gliedrig sind;

die vorstehend genannten 5- bis 14-gliedriges Heteroaryl-Reste ggf. 1, 2, 3, 4 oder 5 Heteroatom(e) unabhängig voneinander ausgewählt aus der Gruppe bestehend aus Sauerstoff, Schwefel und Stickstoff (NH) als Ringglied(er) aufweisen;

und die vorstehend genannten Aryl-Reste und Heteroaryl-Reste jeweils mit ggf. 1, 2, 3, 4 oder 5 Substituenten unabhängig voneinander ausgewählt aus der Gruppe bestehend aus F, Cl, Br, I, -CN, $-NO_2$, -OH, -SH, $-NH_2$, -C(=O)-OH, -$C_{1-5}$-Alkyl, -($CH_2$)-O-$C_{1-5}$-Alkyl, -$C_{2-5}$-Alkenyl, -$C_{2-5}$-Alkinyl, -C≡C-Si($CH_3)_3$,- C≡C-Si($C_2H_5)_3$, -S-$C_{1-5}$-Alkyl, -S-Phenyl, -S-$CH_2$-Phenyl, -O-$C_{1-5}$-Alkyl, -O-Phenyl, -O-$CH_2$-Phenyl, $-CF_3$, $-CHF_2$, $-CH_2F$, $-O-CF_3$, $-O-CHF_2$, $-O-CH_2F$, - C(=O)-$CF_3$, $-S-CF_3$, $-S-CHF_2$, $-S-CH_2F$, -S(=O$)_2$-Phenyl, -S(=O$)_2$-$C_{1-5}$-Alkyl, - S(=O)-$C_{1-5}$-Alkyl, -NH-$C_{1-5}$-Alkyl, N($C_{1-5}$Alkyl$)_2$, -C(=O)-O-$C_{1-5}$-Alkyl, -C(=O)-O-Phenyl, -C(=O)-H, -C(=O)-$C_{1-5}$-Alkyl, -$CH_2$-O-C(=O)-Phenyl, -O-C(=O)-Phenyl, -O-C(=O)-$C_{1-5}$-Alkyl, -NH-C(=O)-$C_{1-5}$-Alkyl, -C(=O)-$NH_2$, -C(=O)-NH-$C_{1-5}$-Alkyl, -C(=O)-N($C_{1-5}$-Alkyl)2, -C(=O)-N($C_{1-5}$-Alkyl)(Phenyl), -C(=O)-NH-Phenyl, Cyclopropyl, Cyclobutyl, Cyclopentyl, Cyclohexyl, Pyrazolyl, Phenyl, Furyl (Furanyl), Thiazolyl, Thiadiazolyl, Thiophenyl (Thienyl) , Benzyl und Phenethyl substituiert sein können, wobei die zyklischen Substituenten bzw. die zyklischen Reste dieser Substituenten selbst mit ggf. 1, 2, 3, 4 oder 5, Substituenten unabhängig voneinander ausgewählt aus der Gruppe bestehend aus F, Cl, Br, I, -CN, $-NO_2$, -OH, -SH, $-NH_2$, -C(=O)-OH. -$C_{1-5}$-Alkyl, -($CH_2$)-O-$C_{1-5}$-Alkyl, -$C_{2-5}$-Alkenyl, -$C_{2-5}$-Alkinyl, -C≡C-Si($CH_3)_3$, -C≡C-Si($C_2H_5)_3$, -S-$C_{1-5}$-Alkyl, -S-Phenyl, -S-$CH_2$-Phenyl, -O-$C_{1-5}$-Alkyl, -O-Phenyl, - O-$CH_2$-Phenyl, $-CF_3$, $-CHF_2$, $-CH_2F$, $-O-CF_3$, $-O-CHF_2$, $-O-CH_2F$, -C(=O)-$CF_3$, - $S-CF_3$, $-S-CHF_2$ und $-S-CH_2F$ substituiert sein können;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

3. Verbindungen gemäß Anspruch 1 oder 2, **dadurch gekennzeichnet, dass**

a, b und c, unabhängig voneinander, jeweils für 0 oder 1 stehen, wobei die Summe aus a, b und c gleich 1, 2 oder 3 ist; A für einen Rest ausgewählt aus der Gruppe bestehend aus

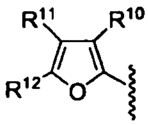, 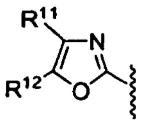, 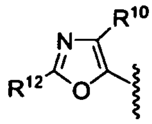, 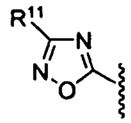,

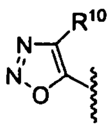, 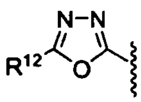, 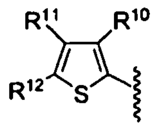, 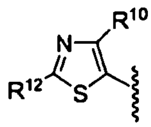,

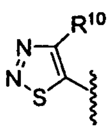, 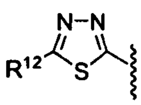, 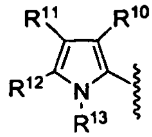, 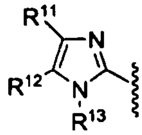,

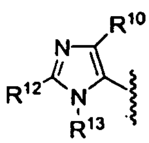, 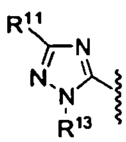, 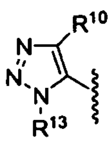, 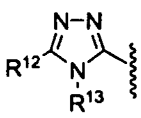, 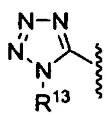,

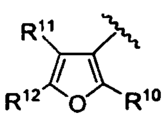, 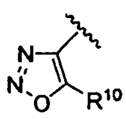, 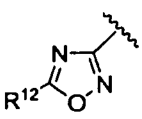, 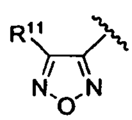, 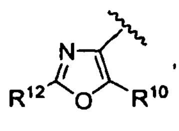,

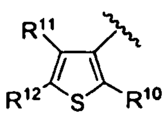, 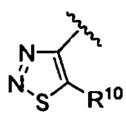, 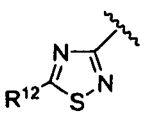, 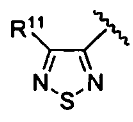, 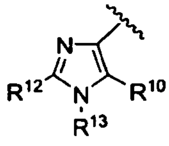,

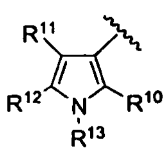, 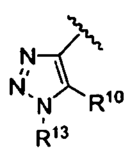, 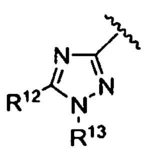, 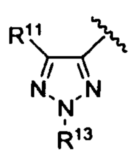, 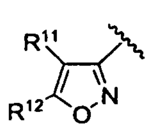,

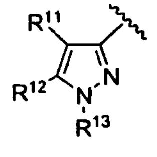 , 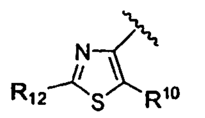 und 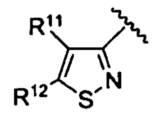

steht;

$R^1$ und $R^8$, unabhängig voneinander, jeweils für H; $-C(=O)-R^{28}$; $-C(=O)-O-R^{29}$; $-C(=O)-NH_2$; $-C(=O)-NH-R^{30}$); $-C(=O)-NR^{31}R^{32}$; $-S(=O)-R^{33}$; $-S(=O)_2-R^{34}$; $C_{1-6}$ Alkyl, das unsubstituiert oder mit ggf. 1, 2, 3, 4 oder 5 Substituenten unabhängig voneinander ausgewählt aus der Gruppe bestehend aus F, Cl, Br, I, $-NO_2$, -CN, -OH, -SH und $-NH_2$ substituiert ist;

$C_{3-8}$-Cycloalkyl, das unsubstituiert oder mit ggf. 1, 2, 3, 4 oder 5 Substituenten unabhängig voneinander ausgewählt aus der Gruppe bestehend aus F, Cl, Br, I, $-NO_2$, -CN, -OH, -SH und $-NH_2$ substituiert ist;

oder für einen Phenyl-Rest stehen, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist;

$R^2$, $R^3$, $R^4$, $R^5$, $R^6$, $R^7$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ und $R^{27}$, unabhängig voneinander, jeweils für H; F; Cl; Br; I; $-NO_2$; -CN; $-NH_2$; -OH; -SH; -C(=O)-OH; $-C(=O)-R^{28}$; $-C(=O)-O-R^{29}$; $-NH-R^{35}$; $-NR^{36}R^{37}$ ; $-O-R^{42}$; $-S-R^{43}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, $-NO_2$, -CN, -OH, -SH und $-NH_2$ substituiert ist; $C_{3-7}$-Cycloalkyl, $C_{5-6}$-Cycloalkenyl, 5- bis 7-gliedriges Heterocycloalkyl oder 5- bis 7-gliedriges Heterocycloalkenyl, das jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH, $-S-CH_3$ und $-S-C_2H_5$ substituiert ist; oder für einen Rest ausgewählt aus der Gruppe bestehend aus Phenyl, Naphthyl, Anthracenyl, Pyrrolyl, Indolyl, Furanyl, Benzo[b]furanyl, Thiophenyl, Benz[b]thiophenyl, Benzo[d]thiazolyl, Pyrazolyl, Imidazolyl, Thiazolyl, Thiadiazolyl, Triazolyl, Oxazolyl, Oxadiazolyl, Isoxazolyl, Pyridinyl, Pyridazinyl, Pyrimidinyl, Pyrazinyl, Pyranyl, Indazolyl, Chinolinyl, Isochinolinyl und Chinazolinyl stehen, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist;

oder $R^2$ und $R^3$ oder $R^4$ und $R^5$ oder $R^6$ und $R^7$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$, unabhängig voneinander, zusammen jeweils für einen Rest ausgewählt aus der Gruppe bestehend aus einer Oxo-Gruppe (=O) und einer Thioxo-Gruppe (=S) stehen;

oder $R^1$ und $R^8$ zusammen mit der sie verbindenden $-N-CR^2R^3-(CR^4R^5)_b-(CR^6R^7)_c$-Gruppe einen Rest ausgewählt aus der Gruppe bestehend aus

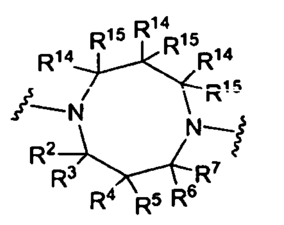 , 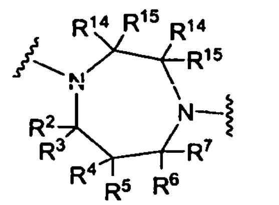 , 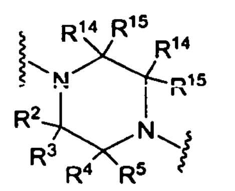 ,

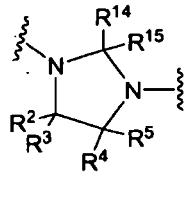 und 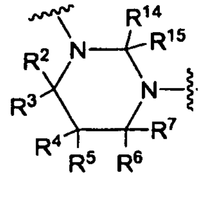

bilden;
oder $R^1$ und $R^2$ zusammen mit der sie verbindenden -N-$CR^3$-Gruppe einen Rest ausgewählt aus der Gruppe bestehend aus

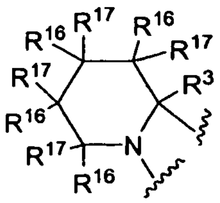
,
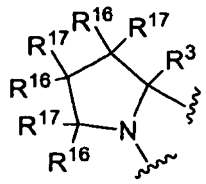
und
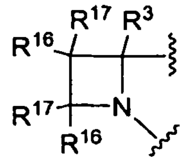

bilden, wobei in diesem Fall b für 1 steht und c für 0 steht;
oder $R^1$ und $R^4$ zusammen mit der sie verbindenden -N-$CR^2R^3$-$CR^5$-Gruppe einen Rest ausgewählt aus der Gruppe bestehend aus

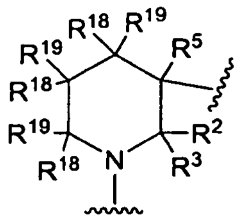
,
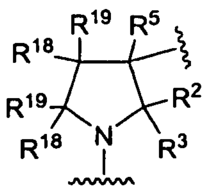
und
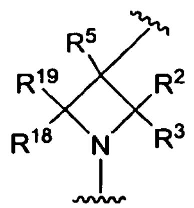

bilden, wobei in diesem Fall c für 0 steht;
$R^1$ und $R^6$ zusammen mit der sie verbindenden -N-$CR^2R^3$-$CR^4R^5$-$CR^7$-Gruppe einen Rest ausgewählt aus der Gruppe bestehend aus

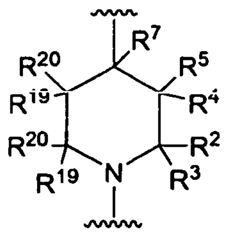
und
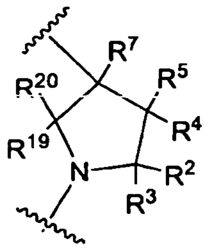

bilden;
oder $R^6$ und $R^8$ zusammen mit der sie verbindenden -N-$CR^7$-Gruppe einen Rest ausgewählt aus der Gruppe bestehend aus

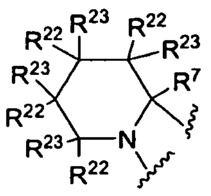
,
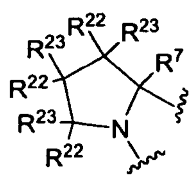
und
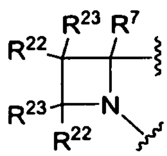

bilden, wobei in diesem Fall b für 1 steht und a für 0 steht;
oder $R^4$ und $R^8$ zusammen mit der sie verbindenden -N-$CR^6R^7$-$CR^5$-Gruppe einen Rest ausgewählt aus der Gruppe bestehend aus

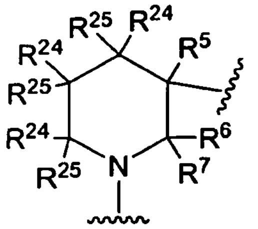
,
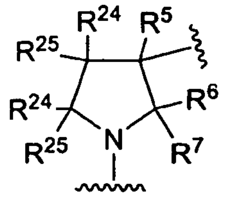
und
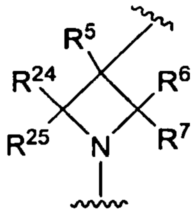

bilden, wobei in diesem Fall a für 0 steht;
oder $R^2$ und $R^8$ zusammen mit der sie verbindenden $-N-CR^6R^7-CR^4R^5-CR^3$-Gruppe einen Rest ausgewählt aus der Gruppe bestehend aus

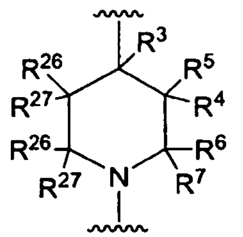
und
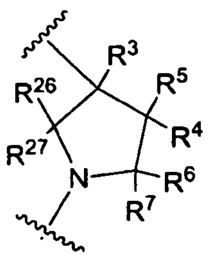

bilden;
$R^9$ für einen Rest ausgewählt aus der Gruppe bestehend aus Phenyl, Naphthyl, Anthracenyl, Furyl, Thienyl, Pyrazolyl, Pyrazinyl, Pyrimidinyl, Pyridazinyl, Pyridinyl, Pyrrolyl, Oxazolyl, Isoxazolyl, Thiazolyl, Thiadiazolyl, Oxadiazolyl, Triazolyl, Imidazolyl, Indolyl, Benz[b]thiophenyl, Benzo[d]thiazolyl, Benzo[b]furanyl, Chinolinyl, Isochinolinyl und Chinazolinyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, Cyclopropyl, Cyclobutyl, Cyclopentyl, $-C\equiv C-Si(CH_3)_3$, - $C\equiv C-Si(C_2H_5)_3$, $-CH_2-O-CH_3$, $-CH_2-O-C_2H_5$, -OH, $-O-CH_3$, $-O-C_2H_5$, $-O-C_3H_7$, - $S-CH_3$, $-S-C_2H_5$, $-S(=O)-CH_3$, $-S(=O)_2-CH_3$, -S (=O)-$C_2H_5$, $-S(=O)_2-C_2H_5$, $-NH_2$, $-N(CH_3)_2$, $-N(C_2H_5)_2$, $-NH-CH_3$, $-NH-C_2H_5$, $-NO_2$, $-CF_3$, $-CH_2F$, $-CHF_2$, $-O-CF_3$, - $S-CF_3$, -SH, $-NH-S(=O)_2-CH_3$, -C(=O)-OH, -C(=O)-H; $-C(=O)-CH_3$, $-C(=O)-C_2H_5$, $-C(=O)-NH_2$, $-C(=O)-N(CH_3)_2$, -C (=O)-$NH-CH_3$, $-NH-C(=O)-CH_3$, $-NH-C(=O)-C_2H_5$, $-C(=O)-O-CH_3$, $-C(=O)-O-C_2H_5$, $-C(=O)-O-C(CH_3)_3$ und Phenyl substituiert ist;
$R^{10}$, $R^{11}$ und $R^{12}$, unabhängig voneinander, jeweils für H; F; Cl; Br; I; $-NO_2$; - CN; $-NH_2$; -OH; -SH; -C(=O)-OH; -C (=O)-H; -NH-C(=O)-H; $-C(=O)-R^{28}$; $-C(=O)-O-R^{29}$; $-C(=O)-NH_2$; $-C(=O)-NH-R^{30}$; $-C(=O)-NR^{31}R^{32}$; $-S(=O)-R^{33}$; -S $(=O)_2-R^{34}$; $-NH-R^{35}$ ; $-NR^{36}R^{37}$; $-O-C(=O)-R^{38}$; $-NH-C(=O)-R^{39}$; $-NR^{40}-C(=O)-R^{41}$; $-O-R^{42}$ ; - $S-R^{43}$-NH-C(=O)-NH-$R^{44}$; $-NH-C(=S)-NH-R^{41}$; $-NH-S(=O)_2-R^{46}$; $-NR^{47}-S(=O)_2-R^{48}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, $-NO_2$, -CN, -OH, -SH und $-NH_2$ substituiert ist; $C_{2-6}$-Alkenyl; $C_{2-6}$-Alkinyl; $C_{3-7}$-Cycloalkyl, $C_{5-6}$-Cycloalkenyl, 5- bis 7-gliedriges Heterocycloalkyl oder 5- bis 7-gliedriges Heterocycloalkenyl, das jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, $-O-CH_3$, $-O-C_2H_5$, $-O-C_3H_7$, $-NH_2$, $-N(CH_3)_2$, -N $(C_2H_5)_2$, $-NH-CH_3$, $-NH-C_2H_5$, $-NO_2$, $-CF_3$, $-O-CF_3$, $-S-CF_3$, -SH, $-S-CH_3$ und $-S-C_2H_5$ substituiert ist; oder für einen Rest ausgewählt aus der Gruppe bestehend aus Phenyl, Naphthyl, Anthracenyl, Thienyl, Furyl, Pyridinyl, Thiazolyl, Thiadiazolyl, Oxazolyl, Oxadiazolyl und Isoxazolyl stehen, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl, - OH, -SH, $-NH_2$, -C(=O)-OH, $-S-CH_3$, $-S-C_2H_5$, $-S(=O)-CH_3$, -S $(=O)_2-CH_3$, - $S(=O)-C_2H_5$, $-S(=O)_2-C_2H_5$, $-O-CH_3$, $-O-C_2H_5$, $-O-C_3H_7$, $-CF_3$, $-CHF_2$, $-CH_2F$ und $-O-CF_3$ substituiert ist;
$R^{13}$ für H; $-C(=O)-R^{28}$; -C(=O)-H; $-C(=O)-O-R^{29}$; $-C(=O)-NH_2$; $-C(=O)-NH-R^{30}$; - $C(=O)-NR^{31}R^{32}$; $-S(=O)-R^{33}$; -S $(=O)_2-R^{34}$; $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, $-NO_2$, -CN, -OH, -SH und $-NH_2$ substituiert ist; $C_{3-7}$-Cycloalkyl, $C_{5-6}$-Cycloalkenyl, 5- bis 7-gliedriges Heterocycloalkyl oder 5- bis 7-gliedriges Heterocycloalkenyl, das jeweils über

eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, - OH, Oxo, Thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH, $-S-CH_3$ und $-S-C_2H_5$ substituiert ist; oder für einen Rest ausgewählt aus der Gruppe bestehend aus Phenyl, Naphthyl, Anthracenyl, Thienyl, Furyl, Pyridinyl, Thiazolyl, Thiadiazolyl, Oxazolyl, Oxadiazolyl und Isoxazolyl steht, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl, - OH, -SH, $-NH_2$, -C(=O)-OH, $-S-CH_3$, $-S-C_2H_5$, $-S(=O)-CH_3$, $-S(=O)_2-CH_3$, - $S(=O)-C_2H_5$, $-S(=O)_2-C_2H_5$, $-O-CH_3$, $-O-C_2H_5$, $-O-C_3H_7$, $-CF_3$, $-CHF_2$, $-CH_2F$ und $-O-CF_3$ substituiert ist;

und $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$ und $R^{48}$, unabhängig voneinander, jeweils für $C_{1-6}$-Alkyl, das unsubstituiert oder mit ggf. 1, 2, 3, 4 oder 5 Substituenten unabhängig voneinander ausgewählt aus der Gruppe bestehend aus F, Cl, Br, I, $-NO_2$, - CN, -OH, -SH und $-NH_2$ substituiert ist; $C_{3-7}$-Cycloalkyl, $C_{5-6}$-Cycloalkenyl, 5-bis 7-gliedriges Heterocycloalkyl oder 5- bis 7-gliedriges Heterocycloalkenyl, das jeweils über eine $C_{1-3}$-A!ky!en-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH, - $S-CH_3$ und $-S-C_2H_5$ substituiert ist; oder für einen Rest ausgewählt aus der Gruppe bestehend aus Phenyl, Naphthyl, Anthracenyl, Pyrrolyl, Indolyl, Furanyl, Benzo[b]furanyl, Thiophenyl, Benz[b]thiophenyl, Benzo[d]thiazolyl, Pyrazolyl, Imidazolyl, Thiazolyl, Thiadiazolyl, Triazolyl, Oxazolyl, Oxadiazolyl, Isoxazolyl, Pyridinyl, Pyridazinyl, Pyrimidinyl, Pyrazinyl, Pyranyl, Indazolyl, Chinolinyl, Isochinolinyl und Chinazolinyl stehen, der jeweils über eine $C_{1-3}$-Alkylen-, $C_{2-3}$-Alkenylen- oder $C_{2-3}$-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, - OH, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH, $-NH-S(=O)_2-CH_3$, -C(=O)-OH, $-C(=O)-CH_3$, $-C(=O)-C_2H_5$, $-C(=O)-N(CH_3)_2$, $-C(=O)-NH-CH_3$, $-NH-C(=O)-CH_3$, $-NH-C(=O)-C_2H_5$, $-C(=O)-O-CH_3$ und $-C(=O)-O-C_2H_5$ substituiert ist;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsvefiältnis, oder jeweils in Form entsprechender Salze, oder jeweils in Form entsprechender Solvate.

**4.** Verbindungen gemäß einem oder mehreren der Ansprüche 1 bis 3, **dadurch gekennzeichnet, dass**
a, b und c, unabhängig voneinander, jeweils für 0 oder 1 stehen, wobei die Summe aus a, b und c gleich 1, 2 oder 3 ist;
A für einen Rest ausgewählt aus der Gruppe bestehend aus

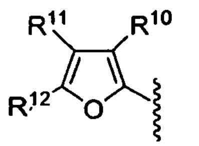 , 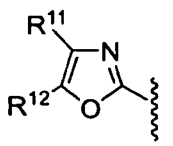 , 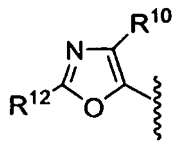 , 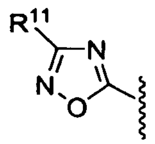 ,

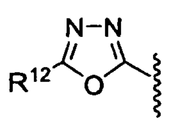 , 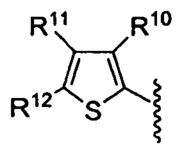 , 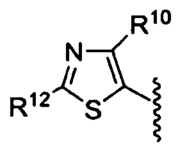 , 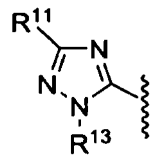 ,

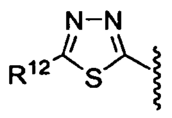, 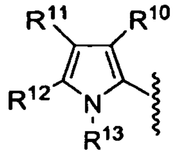, 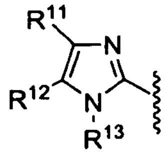, 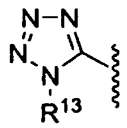,

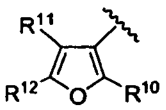, 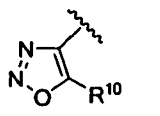, 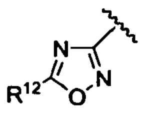, 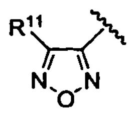, 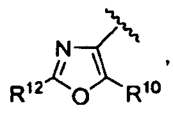,

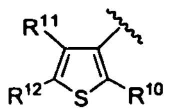, 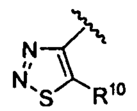, 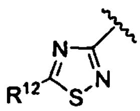, 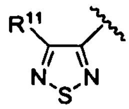, 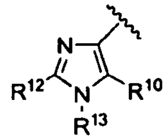,

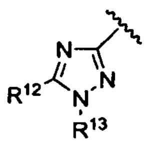, 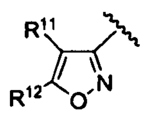, 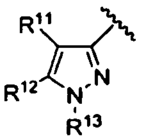, 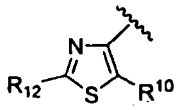 und 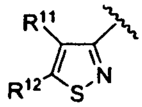

steht;

$R^1$ und $R^8$, unabhängig voneinander, jeweils für H; -C(=O)-$R^{28}$; -C(=O)-O-$R^{29}$; S(=O)-$R^{33}$; -$S(=O)_2$-$R^{34}$; einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; einen Cycloalkyl-Rest ausgewählt aus der Gruppe bestehend aus Cyclopropyl, Cyclobutyl, Cyclopentyl und Cyclohexyl; oder für einen Benzyl- oder Phenethyl-Rest stehen, der unsubstituiert oder mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, -O-$CH_3$, -O-$C_2H_5$ und -O-$C_3H_7$ substituiert ist;

$R^2$, $R^3$, $R^4$, $R^5$, $R^6$, $R^7$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ und $R^{27}$, unabhängig voneinander, jeweils für H; F; Cl; Br; I; -$NH_2$; -OH; - SH; -CN; -$NO_2$; -$CF_3$; -NH-$R^{35}$; -$NR^{36}R^{37}$; -O-$R^{42}$; -S-$R^{43}$; für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; oder für einen Rest ausgewählt aus der Gruppe bestehend aus Cyclopropyl, Cyclobutyl, Cyclopentyl und Cyclohexyl stehen;

oder $R^2$ und $R^3$ oder $R^4$ und $R^5$ oder $R^6$ und $R^7$ oder $R^{14}$ und $R^{15}$ oder $R^{16}$ und $R^{17}$ oder $R^{18}$ und $R^{19}$ oder $R^{20}$ und $R^{21}$ oder $R^{22}$ und $R^{23}$ oder $R^{24}$ und $R^{25}$ oder $R^{26}$ und $R^{27}$, unabhängig voneinander, zusammen jeweils für einen Rest ausgewählt aus der Gruppe bestehend aus einer Oxo-Gruppe (=O) und einer Thioxo-Gruppe (=S) stehen;

oder $R^1$ und $R^3$ zusammen mit der sie verbindenden -N-$CR^2R^3$-$(CR^4R^5)_b$-$(CR^6R^7)_c$-Gruppe einen Rest ausgewählt aus der Gruppe bestehend aus

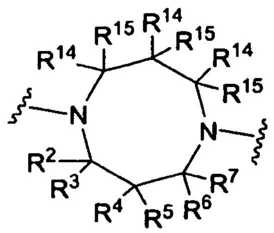 , 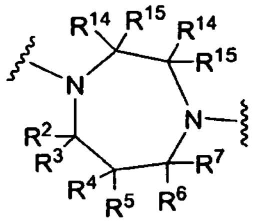 , 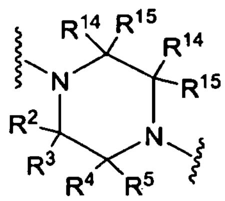 ,

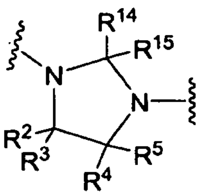 und 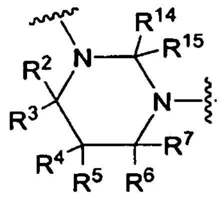

bilden;
oder $R^1$ und $R^2$ zusammen mit der sie verbindenden -N-CR$^3$-Gruppe einen Rest ausgewählt aus der Gruppe bestehend aus

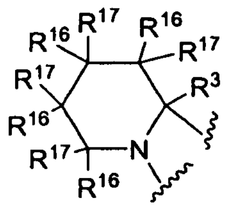 , 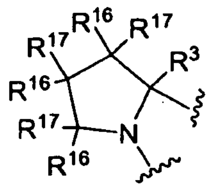 und 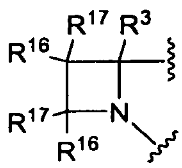

bilden, wobei in diesem Fall b für 1 steht und c für 0 steht;
oder $R^1$ und $R^4$ zusammen mit der sie verbindenden -N-CR$^2$R$^3$-CR$^5$-Gruppe einen Rest ausgewählt aus der Gruppe bestehend aus

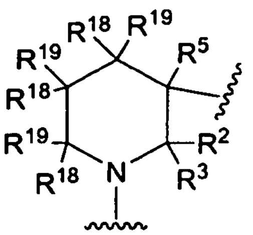 , 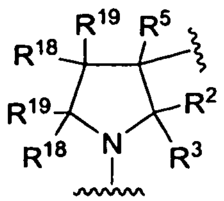 und 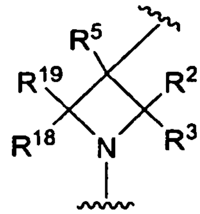

bilden, wobei in diesem Fall c für 0 steht;
$R^1$ und $R^6$ zusammen mit der sie verbindenden -N-CR$^2$R$^3$-CR$^4$R$^5$-CR$^7$-Gruppe einen Rest ausgewählt aus der Gruppe bestehend aus

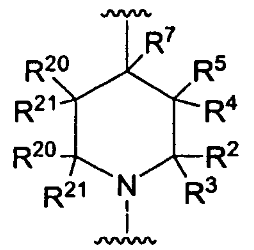 und 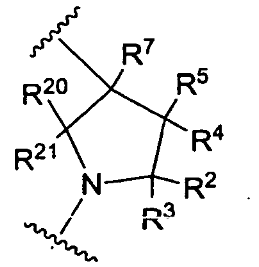

bilden;
oder $R^6$ und $R^8$ zusammen mit der sie verbindenden -N-CR$^7$-Gruppe einen Rest ausgewählt aus der Gruppe bestehend aus

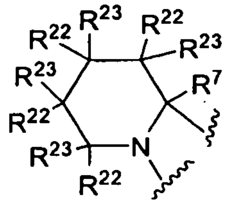 , 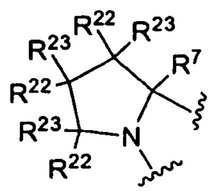 und 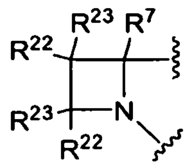

bilden, wobei in diesem Fall b für 1 steht und a für 0 steht;
oder $R^4$ und $R^8$ zusammen mit der sie verbindenden -N-CR$^6$R$^7$-CR$^5$-Gruppe einen Rest ausgewählt aus der Gruppe bestehend aus

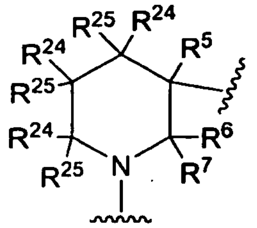 , 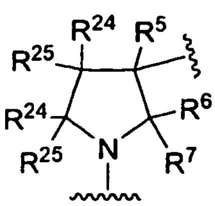 und 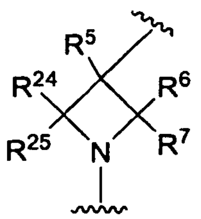

bilden, wobei in diesem Fall a für 0 steht;
oder $R^2$ und $R^8$ zusammen mit der sie verbindenden -N-CR$^6$R$^7$-CR$^4$R$^5$-CR$^3$-Gruppe einen Rest ausgewählt aus der Gruppe bestehend aus

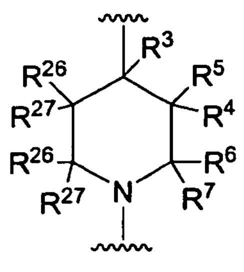 und 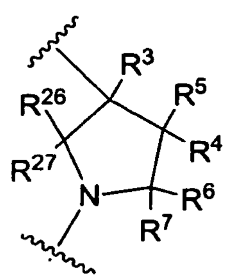

bilden;
$R^9$ für einen Rest ausgewählt aus der Gruppe bestehend aus Phenyl, Furyl, Thienyl, Pyrazolyl, Pyrazinyl, Pyridazinyl,

Pyrimidinyl, Pyridinyl, Pyrrolyl, Oxazolyl, Isoxazolyl, Thiazolyl, Thiadiazolyl, Oxadiazolyl, Triazolyl und Imidazolyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, Cyclopropyl, Cyclobutyl, $-C\equiv C-Si(CH_3)_3$, $-C\equiv C-Si(C_2H_5)_3$, $-CH_2-O-CH_3$, $-CH_2-O-C_2H_5$, -OH, - $O-CH_3$, $-O-C_2H_5$, $-O-C_3H_7$, $-S-CH_3$, $-S-C_2H_5$, $-S(=O)-CH_3$, $-S(=O)_2-CH_3$, $-S(=O)-C_2H_5$, -S $(=O)_2-C_2H_5$, $-NH_2$, $-N(CH_3)_2$, $-N(C_2H_5)_2$, $-NH-CH_3$, $-NH-C_2H_5$, $-NO_2$, - $CF_3$, $-CH_2F$, $-CHF_2$, $-O-CF_3$, $-S-CF_3$, -SH, $-NH-S(=O)_2-CH_3$, -C(=O)-OH, - C(=O)-H; $-C(=O)-CH_3$, $-C(=O)-C_2H_5$, $-C(=O)-NH_2$, $-C(=O)-N(CH_3)_2$, $-C(=O)-NH-CH_3$, $-NH-C(=O)-CH_3$, $-NH-C(=O)-C_2H_5$, $-C(=O)-O-CH_3$ , $-C(=O)-O-C_2H_5$, - $C(=O)-O-C(CH_3)_3$ und Phenyl substituiert ist;

$R^{10}$, $R^{11}$ und $R^{12}$, unabhängig voneinander, jeweils für H; F; Cl; Br; I; $-CF_3$; - $NO_2$; -CN; -C(=O)-OH; $-C(=O)-O-R^{29}$; $-C(=O)-NH_2$; $-C(=O)-NH-R^{30}$; $-C(=O)-NR^{31}R^{32}$ ; $-S(=O)-R^{33}$; $-S(=O)_2-R^{34}$; $-O-R^{42}$; $-S-R^{43}$; einen Alkyl-Rest ausgewählt aus der Gruppe bestehend aus Methyl, Ethyl, n-Propyl, Isopropyl, n-Butyl, 2-Butyl, Isobutyl und tert-Butyl; einen Rest ausgewählt aus der Gruppe bestehend aus Ethenyl, Ethinyl, Allyl und Propinyl; für einen Rest ausgewählt aus der Gruppe bestehend aus Cyclopropyl, Cyclobutyl und Cyclopentyl; oder für einen Rest ausgewählt aus der Gruppe bestehend aus Phenyl, Benzyl,
Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist, stehen;

$R^{13}$ für H; $-C(=O)-R^{28}$; -C(=O)-H; $-C(=O)-O-R^{29}$; $-S(=O)-R^{33}$; $-S(=O)_2-R^{34}$; einen Alkyl-Rest ausgewählt aus der Gruppe bestehend aus Methyl, Ethyl, n-Propyl, Isopropyl, n-Butyl, 2-Butyl, Isobutyl und tert-Butyl; einen Rest ausgewählt aus der Gruppe bestehend Cyclopropyl, Cyclobutyl, Cyclopentyl und Cyclohexyl; oder für einen Rest ausgewählt aus der Gruppe bestehend aus Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils über eine $C_{1-3}$-Alkylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist, steht;

und $R^{28}$, $R^{29}$, $R^{30}$, $R^{31}$, $R^{32}$, $R^{33}$, $R^{34}$, $R^{35}$, $R^{36}$, $R^{37}$, $R^{42}$ und $R^{43}$, unabhängig voneinander, jeweils für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; $-CF_3$; $-C_2F_5$; $-CH_2-CF_3$; oder für einen Phenyl-, Benzyl- oder Phenethyl-Rest stehen, der unsubstituiert oder mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, $-O-CH_3$, $-O-C_2H_5$ und $-O-C_3H_7$ substituiert ist;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

5. Verbindungen gemäß einem oder mehreren der Ansprüche 1 bis 4, **dadurch gekennzeichnet, dass**
a, b und c, unabhängig voneinander, jeweils für 0 oder 1 stehen, wobei die Summe aus a, b und c gleich 1, 2 oder 3 ist;
A für einen Rest ausgewählt aus der Gruppe bestehend aus

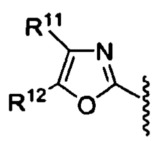 , 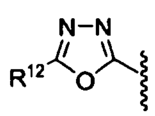 , 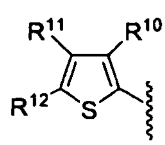 , 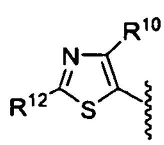 ,

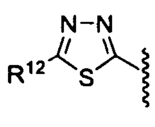 , 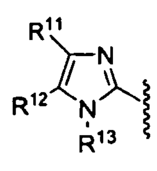 , 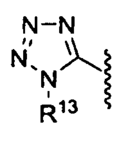 , 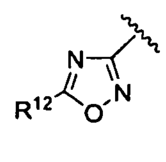 ,

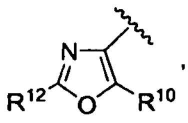, 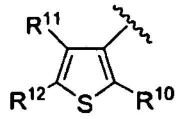, 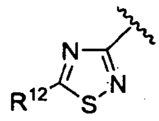, 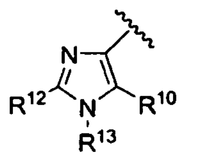,

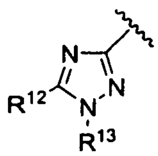, 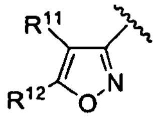, 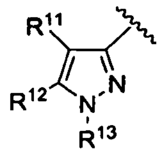 und 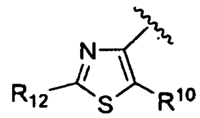

steht;

$R^1$ und $R^8$, unabhängig voneinander, jeweils für H; -C(=O)-$R^{28}$; -C(=O)-O-$R^{29}$; für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; oder für einen Cyclopropyl-Rest; stehen;

$R^2$, $R^3$, $R^4$, $R^5$, $R^6$, $R^7$, $R^{14}$, $R^{15}$, $R^{16}$, $R^{17}$, $R^{18}$, $R^{19}$, $R^{22}$, $R^{23}$, $R^{24}$ und $R^{25}$, unabhängig voneinander, jeweils für H; F; Cl; Br; I; -$NH_2$; -OH; -SH; -CN; - $NO_2$; -$CF_3$; -NH-$R^{35}$; -$NR^{36}R^{37}$; -O-$R^{42}$; -S-$R^{43}$; für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl oder für einen Cyclopropyl-Rest stehen;

oder $R^1$ und $R^8$ zusammen mit der sie verbindenden -N-$CR^2R^3$-$(CR^4R^5)_b$-$(CR^6R^7)_c$-Gruppe einen Rest ausgewählt aus der Gruppe bestehend aus

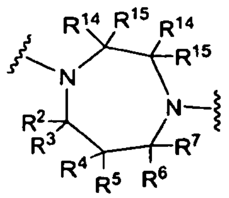, 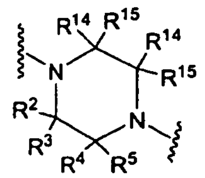, 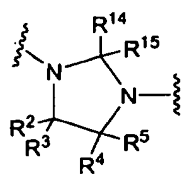 und 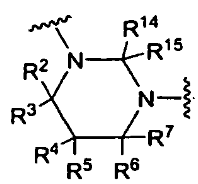

bilden;

oder $R^1$ und $R^2$ zusammen mit der sie verbindenden -N-$CR^3$-Gruppe einen Rest ausgewählt aus der Gruppe bestehend aus

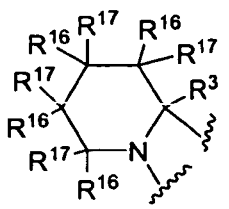, 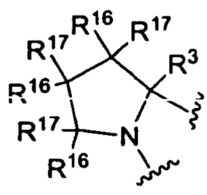 und 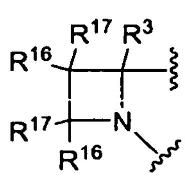

bilden, wobei in diesem Fall b für 1 steht und c für 0 steht;

oder $R^1$ und $R^4$ zusammen mit der sie verbindenden -N-$CR^2R^3$-$CR^5$-Gruppe einen Rest ausgewählt aus der Gruppe bestehend aus

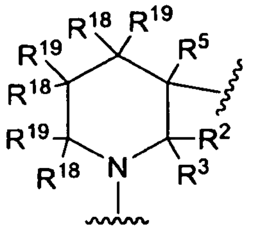 , 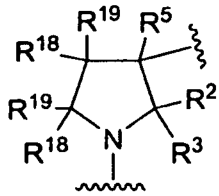 und 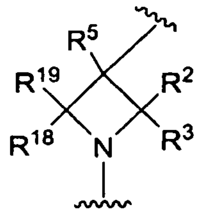

bilden, wobei in diesem Fall c für 0 steht;
oder $R^6$ und $R^8$ zusammen mit der sie verbindenden -N-$CR^7$-Gruppe einen Rest ausgewählt aus der Gruppe bestehend aus

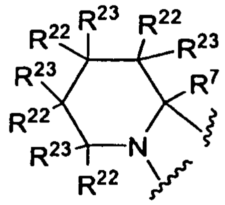 , 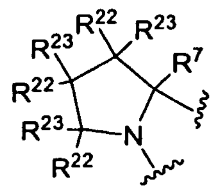 und 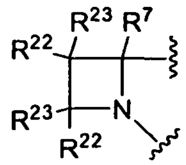

bilden, wobei in diesem Fall b für 1 steht und a für 0 steht;
oder $R^4$ und $R^8$ zusammen mit der sie verbindenden -N-$CR^6R^7$-$CR^5$-Gruppe einen Rest ausgewählt aus der Gruppe bestehend aus

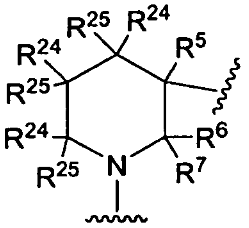 , 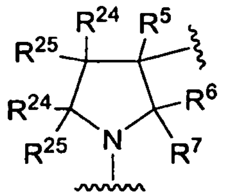 und 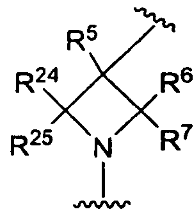

bilden, wobei in diesem Fall a für 0 steht;
$R^9$ für einen Rest ausgewählt aus der Gruppe bestehend aus Phenyl, Thienyl, Pyrazolyl, Pyrazinyl, Pyridazinyl, Pyrimidinyl, Pyridinyl, Thiazolyl und Thiadiazolyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, Cyclopropyl, Cyclobutyl, -C≡C-Si$(CH_3)_3$, -C=C-Si$(C_2H_5)_3$. -$CH_2$-O-$CH_3$, -$CH_2$-O-$C_2H_5$, -OH, - O-$CH_3$, -O-$C_2H_5$, -O-$C_3H_7$, -S-$CH_3$, -S-$C_2H_5$, -S(=O)-$CH_3$, -S$(=O)_2$-$CH_3$, -S(=O)-$C_2H_5$, -S$(=O)_2$-$C_2H_5$, -$NH_2$, -N$(CH_3)_2$, -N$(C_2H_5)_2$, -NH-$CH_3$, -NH-$C_2H_5$, -$NO_2$, -$CF_3$, -$CH_2F$, -$CHF_2$, -O-$CF_3$, -S-$CF_3$, -SH, -NH-S$(=O)_2$-$CH_3$, -C(=O)-OH,-C(=O)-H; -C(=O)-$CH_3$, -C(=O)-$C_2H_5$, -C(=O)-$NH_2$, -C(=O)-N$(CH_3)_2$, -C(=O)-NH-$CH_3$, -NH-C(=O)-$CH_3$, -NH-C(=O)-$C_2H_5$, -C(=O)-O-$CH_3$, -C(=O)-O-$C_2H_5$, - C(=O)-O-C$(CH_3)_3$ und Phenyl substituiert ist;
$R^{10}$, $R^{11}$ und $R^{12}$, unabhängig voneinander, jeweils für H; F; Cl; Br; I; -$CF_3$; - $NO_2$; -CN; -C(=O)-OH; -C(=O)-O-$R^{29}$; -C(=O)-$NH_2$; -O-$R^{42}$; -S-$R^{43}$; Ethenyl;
Ethinyl; Cyclopropyl oder für einen Alkyl-Rest ausgewählt aus der Gruppe bestehend aus Methyl, Ethyl, n-Propyl, Isopropyl, n-Butyl, 2-Butyl, Isobutyl und tert-Butyl stehen;
$R^{13}$ für H; -C(=O)-$R^{28}$; -C(=O)-H; -C(=O)-O-$R^{29}$; -S(=O)-$R^{33}$; -S$(=O)_2$-$R^{34}$; Cyclopropyl; Cyclobutyl; oder für einen Alkyl-Rest ausgewählt aus der Gruppe bestehend aus Methyl, Ethyl, n-Propyl, Isopropyl, n-Butyl, 2-Butyl, Isobutyl und tert-Butyl steht;
und $R^{28}$, $R^{29}$, $R^{33}$, $R^{34}$, $R^{35}$, $R^{36}$, $R^{37}$, $R^{42}$ und $R^{43}$, unabhängig voneinander, jeweils für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und

n-Hexyl; $-CF_3$; $-C_2F_5$; $-CH_2-CF_3$; oder für einen Phenyl-, Benzyl- oder Phenethyl-Rest stehen, der unsubstituiert oder mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, 2-Butyl, Isobutyl, tert-Butyl, -OH, - $O-CH_3$, $-O-C_2H_5$ und $-O-C_3H_7$ substituiert ist;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

**6.** Verbindungen gemäß einem oder mehreren der Ansprüche 1 bis 5, **dadurch gekennzeichnet, dass**
a, b und c, unabhängig voneinander, jeweils für 0 oder 1 stehen, wobei die Summe aus a, b und c gleich 1 oder 2 ist;
A für einen Rest ausgewählt aus der Gruppe bestehend aus

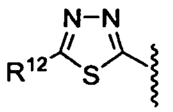 , 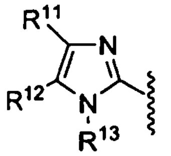 , 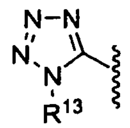 , 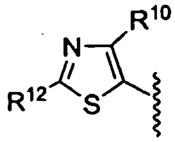 ,

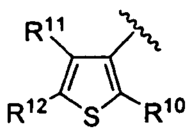 und 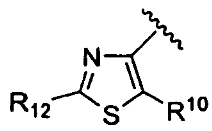

steht;
$R^1$ und $R^8$, unabhängig voneinander, jeweils für H; für $-C(=O)-R^{28}$; für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl; oder für einen Cyclopropyl-Rest stehen;
$R^2$, $R^3$, $R^4$, $R^5$, $R^6$, $R^7$, $R^{14}$, $R^{15}$, $R^{16}$, $R^{17}$, $R^{22}$, $R^{23}$, $R^{24}$ und $R^{25}$, unabhängig voneinander, jeweils für H; F; Cl; Br; I; -CN; $-NO_2$; $-CF_3$; $-O-R^{42}$; für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl oder für einen Cyclopropyl-Rest stehen;
oder $R^1$ und $R^8$ zusammen mit der sie verbindenden $-N-CR^2R^3-CR^4R^5$ -Gruppe den folgenden Rest

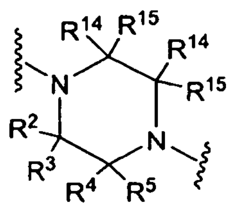

bilden;
oder $R^1$ und $R^2$ zusammen mit der sie verbindenden $-N-CR^3$-Gruppe einen Rest ausgewählt aus der Gruppe bestehend aus

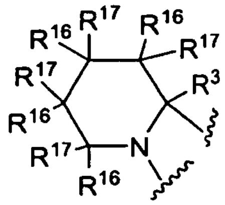 , 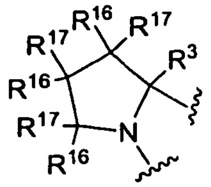 und 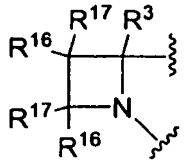

bilden, wobei in diesem Fall b für 1 steht und c für 0 steht;
oder $R^6$ und $R^8$ zusammen mit der sie verbindenden -N-$CR^7$-Gruppe einen Rest ausgewählt aus der Gruppe bestehend aus

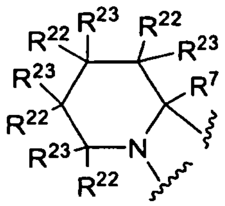 , 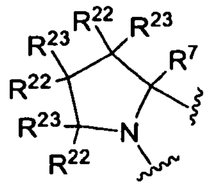 und 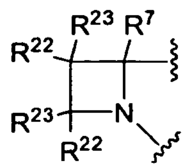

bilden, wobei in diesem Fall b für 1 steht und a für 0 steht;
$R^9$ für einen Rest ausgewählt aus der Gruppe bestehend aus Phenyl und Thienyl steht, der jeweils unsubstituiert oder mit 1 oder 2 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Ethinyl, Cyclopropyl, -O-$CH_3$, -O-$C_2H_5$, -O-$C_3H_7$,-$NO_2$, -$CF_3$ und -0-$CF_3$ substituiert ist;
$R^{10}$, $R^{11}$ und $R^{12}$, unabhängig voneinander, jeweils für H; F; Cl; Br; -$CF_3$; -$NO_2$;-CN; -O-$R^{42}$; für einen Alkyl-Rest ausgewählt aus der Gruppe bestehend aus Methyl, Ethyl, n-Propyl, Isopropyl, Isobutyl und tert-Butyl; Ethenyl; Ethinyl oder Cyclopropyl stehen;
$R^{13}$ für H; -C(=O)-H; -C(=O)-$R^{28}$; Cyclopropyl; oder für einen Alkyl-Rest ausgewählt aus der Gruppe bestehend aus Methyl, Ethyl, n-Propyl, Isopropyl, n-Butyl, 2-Butyl, Isobutyl und tert-Butyl steht;
und $R^{28}$ und $R^{42}$, unabhängig voneinander, jeweils für einen Alkyl-Rest ausgewählt aus der Gruppe bestehend aus Methyl, Ethyl, n-Propyl, Isopropyl, n-Butyl, 2-Butyl, Isobutyl und tert-Butyl; oder -$CF_3$ stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

7. Verbindungen gemäß einem oder mehreren der Ansprüche 1 bis 6, **dadurch gekennzeichnet, dass**
a, b und c, unabhängig voneinander, jeweils für 0 oder 1 stehen, wobei die Summe aus a, b und c gleich 2 ist;
A für einen Rest ausgewählt aus der Gruppe bestehend aus

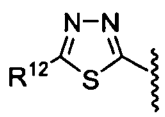 , 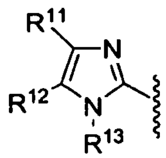 , 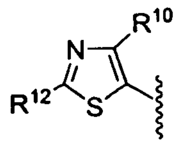 ,

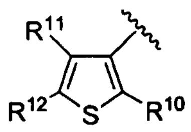 und 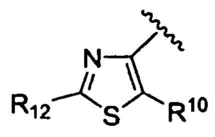

steht;
$R^1$ und $R^8$, unabhängig voneinander, jeweils für H, -C(=O)-$CH_3$, Methyl, Isopropyl oder Cyclopropyl stehen; $R^2$, $R^3$, $R^4$, $R^5$, $R^6$, $R^7$, $R^{14}$, $R^{15}$, $R^{16}$, $R^{17}$, $R^{22}$ und $R^{23}$, unabhängig voneinander, jeweils für H oder für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl und n-Hexyl stehen;
oder $R^1$ und $R^8$ zusammen mit der sie verbindenden -N-$CR^2R^3$-$CR^4R^5$ -Gruppe den folgenden Rest

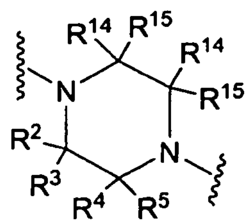

bilden;
oder $R^1$ und $R^2$ zusammen mit der sie verbindenden -N-$CR^3$-Gruppe den folgenden Rest

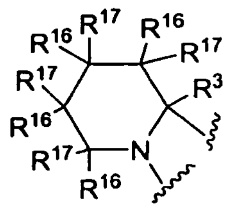

bilden, wobei in diesem Fall b für 1 steht und c für 0 steht;
oder $R^6$ und $R^8$ zusammen mit der sie verbindenden -N-$CR^7$-Gruppe den folgenden Rest

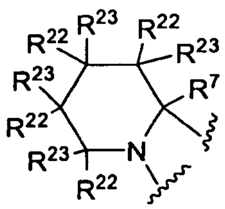

bilden, wobei in diesem Fall b für 1 steht und a für 0 steht;
$R^9$ für einen Phenyl-Rest, der jeweils unsubstituiert oder mit 1 oder 2 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Ethinyl, Cyclopropyl, -O-$CH_3$, -O-$C_2H_5$, -0-$C_3H_7$,-$NO_2$, -$CF_3$ und -O-$CF_3$ substituiert ist;
$R^{10}$, $R^{11}$ und $R^{12}$, unabhängig voneinander, jeweils für H; F; Cl, Br; -$CF_3$; -$NO_2$;-CN; -O-$CF_3$; für einen Alkyl-Rest ausgewählt aus der Gruppe bestehend aus Methyl, Ethyl, n-Propyl, Isopropyl, Isobutyl und tert-Butyl; Ethenyl; Ethinyl oder Cyclopropyl stehen;
und $R^{13}$ für H oder für einen Alkyl-Rest ausgewählt aus der Gruppe bestehend aus Methyl, Ethyl, n-Propyl, Isopropyl,

n-Butyl, 2-Butyl, Isobutyl und tert-Butyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

**8.** Verbindungen gemäß einem oder mehreren der Ansprüche 1 bis 7, **dadurch gekennzeichnet, dass**
a, b und c, unabhängig voneinander, jeweils für 0 oder 1 stehen, wobei die Summe aus a, b und c gleich 2 ist;
A für einen Rest ausgewählt aus der Gruppe bestehend aus

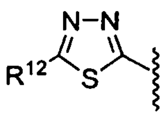 , 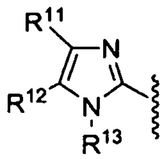 , 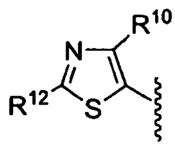 ,

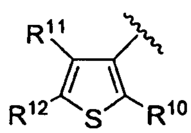 und 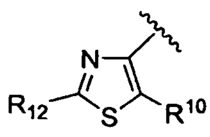

steht;
$R^1$ und $R^8$, unabhängig voneinander, jeweils für H, Methyl oder Isopropyl stehen;
$R^2$, $R^3$, $R^4$, $R^5$, $R^6$, $R^7$, $R^{14}$ und $R^{15}$, unabhängig voneinander, jeweils für H oder für einen Alkyl-Rest ausgewählt aus der Gruppe bestehend aus Methyl, und Ethyl stehen;
oder $R^1$ und $R^8$ zusammen mit der sie verbindenden -N-$CR^2R^3$-$CR^4R^5$ -Gruppe den folgenden Rest

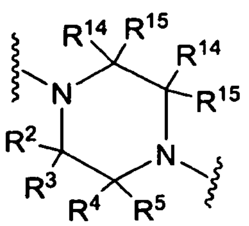

bilden;
$R^9$ für einen Phenyl-Rest, der jeweils unsubstituiert oder mit 1 oder 2 Substituenten unabhängig voneinander ausgewählt aus der Gruppe bestehend aus F, Cl; Br; I und -CN substituiert ist;
$R^{10}$, $R^{11}$ und $R^{12}$, unabhängig voneinander, jeweils für H; oder für einen Alkyl-Rest ausgewählt aus der Gruppe bestehend aus Methyl, Ethyl, n-Propyl, Isopropyl, Isobutyl und tert-Butyl;
und $R^{13}$ für H oder für einen Alkyl-Rest ausgewählt aus der Gruppe bestehend aus Methyl, Ethyl, n-Propyl, Isopropyl, n-Butyl, 2-Butyl, Isobutyl und tert-Butyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

**9.** Verbindungen gemäß einem oder mehreren der Ansprüche 1 bis 8 ausgewählt aus der Gruppe bestehend aus

[1] 1-(3-Phenyl-propiolyl)-4-(thiophen-3-yl)-piperazin,
[2] 1-(3-Phenyl-propiolyl)-4-(thiazol-4-yl)-piperazin,

[3] 1-(3-Phenyl-propiolyl)-4-(1-methyl-imidazol-2-yl)-piperazin,
[4] (R)-2-Methyl-1-(3-(3-chloro-phenyl)-propiolyl)-4-(thiazol-4-yl)-piperazin,
[5] 2-Methyl-1-(3-(3-chloro-phenyl)-propiolyl)-4-(1,3,4-thiadiazol-2-yl)-piperazin,
[6] 3-(3-Chlorphenyl)-N-(2-(methyl(1,3,4-thiadiazol-2-yl)amino)ethyl)propiolamid,
[7] 3-(3-Chlorphenyl)-N-(2-(methyl(thiazol-5-yl)amino)ethyl)propiolamid und
[8] 3-(3-Chlorphenyl)-N-(2-(methyl(1-methyl-1H-imidazol-2-yl)amino)ethyl)-propiolamid

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

**10.** Arzneimittel enthaltend wenigstens eine Verbindung gemäß einem oder mehreren der Ansprüche 1 bis 9 und ggf. einen oder mehrere physiologisch verträgliche Hilfsstoffe.

**11.** Verbindungen gemäß einem oder mehreren der Ansprüche 1 bis 9 zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Erkrankungen, vorzugsweise kognitiven Mangelzuständen, besonders bevorzugt des Aufmerksamkeit-Defizit-Syndroms (ADS); Angstzuständen; Panikattacken; Epilepsie; Husten; Harninkontinenz; Diarrhöe; Pruritus; Schizophrenie; cerebralen Ischämien; Muskelspasmen; Krämpfen; Lungenkrankheiten, vorzugsweise ausgewählt aus der Gruppe bestehend aus Asthma und Pseudokrupp; Regurgitation (Erbrechen); Schlaganfall; Dyskinesie; Retinopathie; Antriebslosigkeit; Kehlkopfentzündung (Laryngitis); Störungen der Nahrungsaufnahme, vorzugsweise ausgewählt aus der Gruppe bestehend aus Bulimie, Kachexie, Anorexie und Fettleibigkeit; Alkoholabhängigkeit; Medikamentenabhängigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen- (insbesondere Nikotin- und/oder Kokain-)abhängigkeit; Toleranzentwicklung gegenüber Medikamenten, insbesondere gegenüber natürlichen oder synthetischen Opioiden; Magen-Ösaphagus-Reflux-Syndrom; gastroösophageale Refluxkrankheit; Reizdarmsyndrom; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie.

**12.** Verbindungen gemäß Anspruch 11 zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz; Angstzuständen; Panikattacken; Alkoholabhängigkeit; Medikamentenabhängigkeit; kognitiven Erkrankungen, vorzugsweise kognitiven Mangelzuständen, besonders bevorzugt des Aufmerksamkeit-Defizit-Syndroms (ADS); Störungen der Nahrungsaufnahme, vorzugsweise ausgewählt aus der Gruppe bestehend aus Bulimie, Kachexie, Anorexie und Fettleibigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen-(insbesondere Nikotin- und/oder Kokain-)abhängigkeit; Toleranzentwicklung gegenüber Medikamenten und/oder Drogen, insbesondere gegenüber natürlichen oder synthetischen Opioiden; Magen-Ösaphagus-Reflux-Syndrom, gastroösophageale Refluxkrankheit und Reizdarmsyndrom.

**13.** Verbindungen gemäß Anspruch 11 oder 12 zur Behandlung von Schmerz, vorzugsweise von Schmerz ausgewählt aus der Gruppe bestehend aus akutem Schmerz, chronischem Schmerz, neuropathischem Schmerz und visceralem Schmerz.

**14.** Verbindungen gemäß Anspruch 11 oder 12 zur Behandlung von Angstzuständen oder Panikattacken.

## Claims

**1.** Substituted propiolic acid amides of the general formula I,

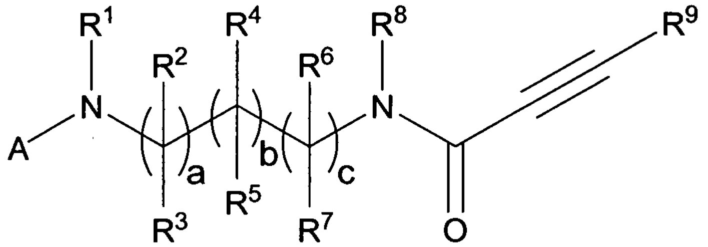

I,

in which

a, b and c, mutually independently, in each case denote 0 or 1, whereby the sum of a, b and c is equal to 1, 2 or 3;

A denotes one of the following residues

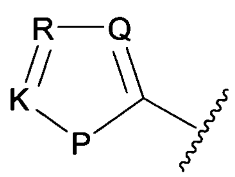

or

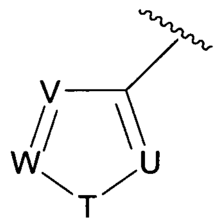

Q and U respectively denote $CR^{10}$ or N;

R and V respectively denote $CR^{11}$ or N;

K and W respectively denote $CR^{12}$ or N;

P and T respectively denote O, S or $NR^{13}$;

with the proviso that compounds are excluded in which P denotes S, Q denotes N, R denotes $CR^{11}$ and K denotes $CR^{12}$ or N;

$R^1$ and $R^8$, mutually independently, in each case denote H; $-C(=O)-R^{28}$; $-C(=O)-O-R2^{9;}$ $-C(=O)-NH_2$; $-C(=O)-NH-R^{30}$; $-C(=O)-NR^{31}R_{32}$; $-S(=O)-R^{33}$; $-S(=O)_2-R^{34}$; unsubstituted or substituted alkyl, alkenyl or alkynyl; unsubstituted or substituted heteroalkyl, heteroalkenyl or heteroalkynyl; unsubstituted or substituted cycloalkyl or cycloalkenyl; unsubstituted or substituted heterocycloalkyl or heterocycloalkenyl; unsubstituted or substituted - (alkylene)-cycloalkyl, -(alkenylene)-cycloalkyl, -(alkynylene)-cycloalkyl, - (alkylene)-cycloalkenyl, -(alkenylene)-cycloalkenyl or -(alkynylene)-cycloalkenyl; unsubstituted or substituted -(heteroalkylene)-cycloalkyl, - (heteroalkenylene)-cycloalkyl, -(heteroalkylene)-cycloalkenyl or - (heteroalkenylene)- cycloalkenyl; unsubstituted or substituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or substituted -(heteroalkylene)-heterocycloalkyl, -(heteroalkenylene)-heterocycloalkyl, -(heteroalkylene)-heterocycloalkenyl or -(heteroalkenylene)-heterocycloalkenyl; unsubstituted or substituted aryl; unsubstituted or substituted heteroaryl; unsubstituted or substituted -(alkylene)-aryl, - (alkenylene)-aryl, -(alkynylene)-aryl, -(heteroalkylene)-aryl or - (heteroalkenylene)-aryl; or unsubstituted or substituted -(alkylene)-heteroaryl, - (alkenylene)-heteroaryl, -(alkynylene)-heteroaryl, -(heteroalkylene)-heteroaryl or -(heteroalkenylene)-heteroaryl;

$R^2$, $R^3$, $R^4$, $R^5$, $R^6$, $R^7$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ and $R^{27}$, mutually independently, in each case denote H; F; Cl; Br; I; $-NO_2$; -CN; $-NH_2$; -OH; -SH; -C(=O)-OH; -C(=O)-H; -NH-C(=O)-H; $-C(=O)-R^{28}$; $-C(=O)-O-R^{29}$; $-C(=O)-NH_2$; $-C(=O)-NH-R^{30}$; $-C(=O)-NR^{31}R^{32}$; $-S(=O)-R^{33}$;$-S(=O)_2-R^{34}$; $-NH-R^{35}$ ; $-NR^{36}R^{37}$; $-O-C(=O)-R^{38}$; $-NH-C(=O)-R^{39}$; $-NR^{40}-C(=O)-R^{41}$; $-O-R^{42}$; $-S-R^{43}$; $-NH-C(=O)-NH-R^{44}$; $-NH-C(=S)-NH-R^{45}$; $-NH-S(=O)2-R^{46}$;$-NR^{47}-S(=O)_2-R^{48}$; unsubstituted or at least monosubstituted alkyl, alkenyl or alkynyl; unsubstituted or at least monosubstituted heteroalkyl, heteroalkenyl or heteroalkynyl; unsubstituted or at least monosubstituted cycloalkyl or cycloalkenyl; unsubstituted or at least monosubstituted heterocycloalkyl or heterocycloalkenyl; unsubstituted or

at least monosubstituted -(alkylene)-cycloalkyl, -(alkenylene)-cycloalkyl, -(alkynylene)-cycloalkyl, -(alkylene)-cycloalkenyl, -(alkenylene)-cycloalkenyl or -(alkynylene)-cycloalkenyl;

unsubstituted or at least monosubstituted -(heteroalkylene)-cycloalkyl, - (heteroalkenylene)-cycloalkyl, -(heteroalkylene)-cycloalkenyl or - (heteroalkenylene)-cycloalkenyl; unsubstituted or at least monosubstituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or at least monosubstituted -(heteroalkylene)-heterocycloalkyl, -(heteroalkenylene)-heterocycloalkyl, -(heteroalkylene)-heterocycloalkenyl or -(heteroalkenylene)-heterocycloalkenyl; unsubstituted or at least monosubstituted aryl; unsubstituted or at least monosubstituted heteroaryl; unsubstituted or at least monosubstituted -(alkylene)-aryl, -(alkenylene)-aryl, -(alkynylene)-aryl, - (heteroalkylene)-aryl or -(heteroalkenylene)-aryl; or unsubstituted or at least monosubstituted -(alkylene)-heteroaryl, -(alkenylene)-heteroaryl, -(alkynylene)-heteroaryl, -(heteroalkylene)-heteroaryl or -(heteroalkenylene)-heteroaryl;

or $R^2$ and $R^3$ or $R^4$ and $R^5$ or $R^6$ and $R^7$ or $R^{14}$ and $R^{15}$ or $R^{16}$ and $R^{17}$ or $R^{18}$ and $R^{19}$ or $R^{20}$ and $R^{21}$ or $R^{22}$ and $R^{23}$ or $R^{24}$ and $R^{25}$ or $R^{26}$ and $R^{27}$, mutually independently, jointly denote a residue selected from the group comprising an oxo group (=O) and a thioxo group (=S);

or $R^1$ and $R^8$ together with the $-N-CR^2R^3-(CR^4R^5)_b-(CR^6R^7)_c$ group joining them together form a residue of the general formula B,

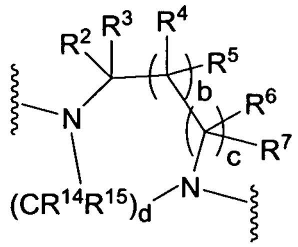

B,

whereby d denotes 1, 2 or 3 and b denotes 0 or 1 and c denotes 0 or 1; or $R^1$ and R2 together with the $-N-CR^3$ group joining them together form a residue of the general formula C,

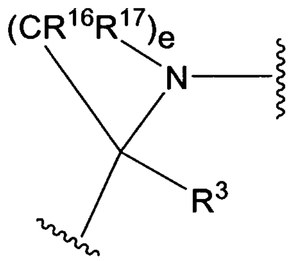

C,

whereby e denotes 1, 2 , 3 or 4 and, in this case, b denotes 0 or 1 and c denotes 0;

or $R^1$ and $R^4$ together with the $-N-CR^2R^3-CR^5$ group joining them together form a residue of the general formula D,

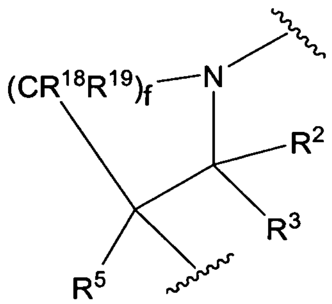

D,

whereby f denotes 1, 2, 3 or 4, and, in this case, c denotes 0; or $R^1$ and $R^6$ together with the $-N-CR^2R^3-CR^4R^5-CR^7$ group joining them together form a residue of the general formula E,

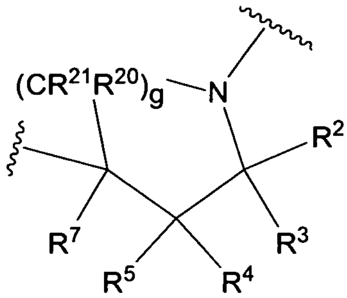

E,

whereby g denotes 1, 2 or 3; or $R^6$ and $R^8$ together with the $-N-CR^7$ group joining them together form a residue of the general formula F

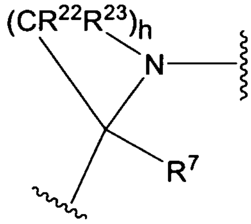

F,

whereby h denotes 1, 2 , 3 or 4 and, in this case, b denotes 0 or 1 and a denotes 0; or $R^4$ and $R^8$ together with the $-N-CR^6R^7-CR^5$ group joining them together form a residue of the general formula G,

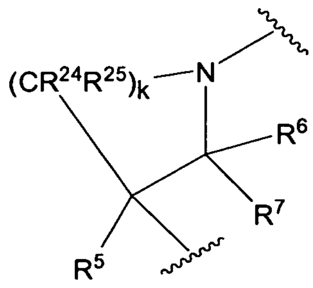

G,

whereby k denotes 1, 2, 3 or 4, and, in this case, a denotes 0;
or $R^2$ and $R^8$ together with the -N-$CR^6R^7$-$CR^4R^5$-$CR^3$ group joining them together form a residue of the general formula H,

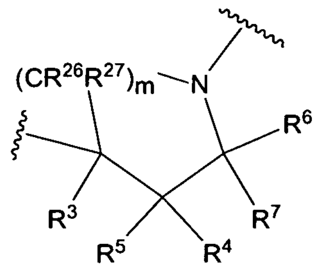

H,

whereby m denotes 1, 2 or 3;
$R^9$ denotes unsubstituted or substituted aryl or unsubstituted or substituted heteroaryl;
$R^{10}$, $R^{11}$ and $R^{12}$, mutually independently, in each case denote H; F; Cl; Br; I; - $NO_2$; -CN; -$NH_2$; -OH; -SH; -C(=O)-OH; -C(=O)-H; -NH-C(=O)-H; -C(=O)-$R^{28}$; - C(=O)-O-$R^{29}$; -C(=O)-$NH_2$; -C(=O)-NH-$R^{30}$; -C(=O)-$NR^{31}R^{32}$; -S(=O)-$R^{33}$; - S$(=O)_2$-$R^{34}$; -NH-$R^{35}$; -$NR^{36}R^{37}$; -O-C(=O)-$R^{38}$; -NH-C(=O)-$R^{39}$; -$NR^{40}$-C(=O)-$R^{41}$; -O-$R^{42}$; -S-$R^{43}$; -NH-C(=O)-NH-$R^{44}$; -NH-C(=S)-NH-$R^{45}$; -NH-S$(=O)_2$-$R^{46}$;-$NR^{47}$-S$(=O)_2$-$R^{48}$; unsubstituted or at least monosubstituted alkyl, alkenyl or alkynyl; unsubstituted or at least monosubstituted heteroalkyl, heteroalkenyl or heteroalkynyl; unsubstituted or at least monosubstituted cycloalkyl or cycloalkenyl; unsubstituted or at least monosubstituted heterocycloalkyl or heterocycloalkenyl; unsubstituted or at least monosubstituted -(alkylene)-cycloalkyl, -(alkenylene)-cycloalkyl, -(alkynylene)-cycloalkyl, -(alkylene)-cycloalkenyl, -(alkenylene)-cycloalkenyl or -(alkynylene)-cycloalkenyl; unsubstituted or at least monosubstituted -(heteroalkylene)-cycloalkyl, - (heteroalkenylene)-cycloalkyl, -(heteroalkylene)-cycloalkenyl or - (heteroalkenylene)-cycloalkenyl; unsubstituted or at least monosubstituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or at least monosubstituted -(heteroalkylene)-heterocycloalkyl, -(heteroalkenylene)-heterocycloalkyl, -(heteroalkylene)-heterocycloalkenyl or -(heteroalkenylene)-heterocycloalkenyl; unsubstituted or at least monosubstituted aryl; unsubstituted or at least monosubstituted heteroaryl; unsubstituted or at least monosubstituted -(alkylene)-aryl, -(alkenylene)-aryl, -(alkynylene)-aryl, - (heteroalkylene)-aryl or -(heteroalkenylene)-aryl; or unsubstituted or at least monosubstituted -(alkylene)-heteroaryl, -(alkenylene)-heteroaryl, -(alkynylene)-heteroaryl, -(heteroalkylene)-heteroaryl or -(heteroalkenylene)-heteroaryl;
$R^{13}$ denotes H; -C(=O)-OH; -C(=O)-H; -C(=O)-$R^{28}$; -C(=O)-O-$R^{29}$; -C(=O)-$NH_2$; -C(=O)-NH-$R^{30}$; -C(=O)-$NR^{31}R^{32}$; -S(=O)-$R^{33}$; -S$(=O)_2$-$R^{34}$; unsubstituted or at least monosubstituted alkyl, alkenyl or alkynyl; unsubstituted or at least monosubstituted heteroalkyl, heteroalkenyl or heteroalkynyl; unsubstituted or at least monosubstituted cycloalkyl or cycloalkenyl; unsubstituted or at least monosubstituted heterocycloalkyl or heterocycloalkenyl; unsubstituted or at least monosubstituted -(alkylene)-cycloalkyl, -(alkenylene)-cycloalkyl, - (alkynylene)-cycloalkyl, -(alkylene)-cycloalkenyl, -(alkenylene)-cycloalkenyl or - (alkynylene)-cycloalkenyl; unsubstituted or at least monosubstituted -(heteroalkylene)-cycloalkyl, -(heteroalkenylene)-cycloalkyl, -(heteroalkylene)-cycloalkenyl or -(heteroalkenylene)-cycloalkenyl; unsubstituted or at least monosubstituted -(alkylene)-heterocycloalkyl, -(alkenylene)-heterocycloalkyl, - (alkynylene)-heterocycloalkyl, -(alkylene)-heterocycloalkenyl, -(alkenylene)-heterocycloalkenyl or

-(alkynylene)-heterocycloalkenyl; unsubstituted or at least monosubstituted -(heteroalkylene)-heterocycloalkyl, -(heteroalkenylene)-heterocycloalkyl, -(heteroalkylene)-heterocycloalkenyl or -(heteroalkenylene)-heterocycloalkenyl; unsubstituted or at least monosubstituted aryl;

unsubstituted or at least monosubstituted heteroaryl; unsubstituted or at least monosubstituted -(alkylene)-aryl, -(alkenylene)-aryl, -(alkynylene)-aryl, - (heteroalkylene)-aryl or -(heteroalkenylene)-aryl; or unsubstituted or at least monosubstituted -(alkylene)-heteroaryl, -(alkenylene)-heteroaryl, -(alkynylene)-heteroaryl, -(heteroalkylene)-heteroaryl or -(heteroalkenylene)-heteroaryl;

and $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$ and $R^{48}$, mutually independently, in each case denote unsubstituted or substituted alkyl, alkenyl or alkynyl; unsubstituted or substituted heteroalkyl, heteroalkenyl or heteroalkynyl; unsubstituted or substituted cycloalkyl or cycloalkenyl; unsubstituted or substituted heterocycloalkyl or heterocycloalkenyl; unsubstituted or substituted - (alkylene)-cycloalkyl, -(alkenylene)-cycloalkyl, -(alkynylene)-cycloalkyl, - (alkylene)-cycloalkenyl, -(alkenylene)-cycloalkenyl or -(alkynylene)-cycloalkenyl; unsubstituted or substituted -(heteroalkylene)-cycloalkyl, - (heteroalkenylene)-cycloalkyl, -(heteroalkylene)-cycloalkenyl or - (heteroalkenylene)-cycloalkenyl; unsubstituted or substituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or substituted -(heteroalkylene)-heterocycloalkyl, -(heteroalkenylene)-heterocycloalkyl, -(heteroalkylene)-heterocycloalkenyl; or -(heteroalkenylene)-heterocycloalkenyl; unsubstituted or substituted aryl; unsubstituted or substituted heteroaryl; unsubstituted or substituted -(alkylene)-aryl, - (alkenylene)-aryl, -(alkynylene)-aryl, -(heteroalkylene)-aryl or - (heteroalkenylene)-aryl; or unsubstituted or substituted -(alkylene)-heteroaryl, - (alkenylene)-heteroaryl, -(alkynylene)-heteroaryl, -(heteroalkylene)-heteroaryl or -(heteroalkenylene)-heteroaryl;

whereby "substituted" in the context of the residues "alkyl", "alkenyl", "alkynyl", "heteroalkyl", "heteroalkenyl" and "heteroalkynyl" denotes a corresponding residue, which is monosubstituted or multiply substituted with substituents mutually independently selected from the group comprising F, Cl, Br, I, $-NO_2$, - CN, -OH, -SH, $-NH_2$, $-N(C_{1-5}\text{-alkyl})_2$, $-N(C_{1-5}\text{-alkyl})(\text{phenyl})$, $-N(C_{1-5}\text{-alkyl})(CH_2\text{-phenyl})$, $-N(C_{1-5}\text{-alkyl})(CH_2\text{-}CH_2\text{-phenyl})$, -C(=O)-H, $-C(=O)\text{-}C_{1-5}\text{-alkyl}$, -C(=O)-phenyl, $-C(=S)\text{-}C_{1-5}\text{-alkyl}$, -C(=S)-phenyl, C(=O)-OH, $-C(=O)\text{-}O\text{-}C_{1-5}\text{-alkyl}$, - C(=O)-O-phenyl, $-C(=O)\text{-}NH_2$, $-C(=O)\text{-}NH\text{-}C_{1-5}\text{-alkyl}$, $-C(=O)\text{-}N(C_{1-5}\text{-alkyl})_2$, - $S(=O)\text{-}C_{1-5}\text{-alkyl}$, -S(=O)-phenyl, $-S(=O)_2\text{-}C_{1-5}\text{-alkyl}$, $S(=O)_2\text{-phenyl}$, $S(=O)_2\text{-}NH_2$ and $-SO_3H$, whereby the above-mentioned $C_{1-5}$-alkyl residues can in each case be linear or branched and the above-mentioned phenyl residues can be substituted with 1, 2, 3, 4 or 5 substituents mutually independently selected from the group comprising F, Cl, Br, I, -CN, $-CF_3$, -OH, $-NH_2$, $-O\text{-}CF_3$, -SH, $\text{-}0\text{-}CH_3$, $-O\text{-}C_2H_5$, $-O\text{-}C_3H_7$, methyl, ethyl, n-propyl, isopropyl, n-butyl, 2-butyl, isobutyl and tert-butyl,

whereby "substituted" in the context of the residues "cycloalkyl", "heterocycloalkyl", "cycloalkenyl", "heterocycloalkenyl" denotes a corresponding residue, which is monosubstituted or multiply substituted with substituents mutually independently selected from the group comprising F, Cl, Br, I, -CN, $-CF_3$, -OH, $-NH_2$, $-O\text{-}CF_3$, -SH, $-O\text{-}C_{1-5}\text{-alkyl}$, -O-phenyl, $-O\text{-}CH_2\text{-phenyl}$, $-(CH_2)\text{-}O\text{-}C_{1-5}\text{-alkyl}$, $-S\text{-}C_{1-5}\text{-alkyl}$, -S-phenyl, $-S\text{-}CH_2\text{-phenyl}$, $-C_{1-5}\text{-alkyl}$, $-C_{2-5}\text{-alkenyl}$, $-C_{2-5}\text{-alkynyl}$, $-C{=}C\text{-}Si(CH_3)_3$, $-C{=}C\text{-}Si(C_2H_5)_3$, $-C(=O)\text{-}O\text{-}C_{1-5}\text{-alkyl}$, $-C(=O)\text{-}CF_3$, $-S(=O)_2\text{-}C_{1-5}\text{-alkyl}$, -S (=O)-$C_{1-5}$-alkyl, $-S(=O)_2\text{-phenyl}$, oxo (=O), thioxo (=S), $-N(C_{1-5}\text{-alkyl})_2$, $-N(H)(C_{1-5}\text{-alkyl})$, $-NO_2$, $-S\text{-}CF_3$, -C(=O)-OH, - $NH\text{-}S(=O)_2\text{-}C_{1-5}\text{-alkyl}$, $-NH\text{-}C(=O)\text{-}C_{1-5}\text{-alkyl}$, -C(=O)-H, $-C(=O)\text{-}C_{1-5}\text{-alkyl}$, - $C(=O)\text{-}NH_2$, $-C(=O)\text{-}N(C_{1-5}\text{-alkyl})_2$, -C (=O)-N(H)($C_{1-5}$-alkyl) and phenyl, whereby the above-mentioned $C_{1-5}$-alkyl residues can in each case be linear or branched and the phenyl residues can be respectively unsubstituted or substituted with 1, 2, 3, 4 or 5 substituents mutually independently selected from the group comprising F, Cl, Br, I, -CN, $-CF_3$, -OH, $-NH_2$, $-O\text{-}CF_3$, -SH, -0-$C_{1-5}$-alkyl, -O-phenyl, $-O\text{-}CH_2\text{-phenyl}$, $-(CH_2)\text{-}O\text{-}C_{1-5}\text{-alkyl}$, $-S\text{-}C_{1-5}\text{-alkyl}$, -S-phenyl, $-S\text{-}CH_2\text{-phenyl}$, $-C_{1-5}\text{-alkyl}$, $-C_{2-5}\text{-alkenyl}$, $-C_{2-5}\text{-alkynyl}$, $-C{=}C\text{-}Si(CH_3)_3$, - $C{=}C\text{-}Si(C_2H_5)_3$, $-C(=O)\text{-}O\text{-}C_{1-5}\text{-alkyl}$ and $-C(=O)\text{-}CF_3$,

whereby "substituted" in the context of the residues "aryl" and "heteroaryl" denotes a corresponding residue, which is monosubstituted or multiply substituted with substituents mutually independently selected from the group comprising F, Cl, Br, I, -CN, $-NO_2$, -OH, -SH, $-NH_2$, -C(=O)-OH, $-C_{1-5}\text{-alkyl}$, - $(CH_2)\text{-}O\text{-}C_{1-5}\text{-alkyl}$, $-C_{2-5}\text{-alkenyl}$, $-C_{2-5}\text{-alkynyl}$ , $-C{=}\text{-}C\text{-}Si(CH_3)_3$, $-C{=}C\text{-}Si(C_2H_5)_3$, $-S\text{-}C_{1-5}\text{-alkyl}$, -S-phenyl, $-S\text{-}CH_2\text{-phenyl}$, $-O\text{-}C_{1-5}\text{-alkyl}$, -O-phenyl, $-O\text{-}CH_2\text{-phenyl}$, $-CF_3$, $-CHF_2$, $-CH_2F$, $-O\text{-}CF_3$, $-O\text{-}CHF_2$, $-O\text{-}CH_2F$, $-C(=O)\text{-}CF_3$, $-S\text{-}CF_3$, $-S\text{-}CHF_2$, $-S\text{-}CH_2F$, -S (=O)$_2$-phenyl, $-S(=O)_2\text{-}C_{1-5}\text{-alkyl}$, $-S(=O)\text{-}C_{1-5}\text{-alkyl}$, $-NH\text{-}C_{1-5}\text{-alkyl}$, $N(C_{1-5}\text{-alkyl})_2$, $-C(=O)\text{-}O\text{-}C_{1-5}\text{-alkyl}$, -C(=O)-O-phenyl, -C(=O)-H; - $C(=O)\text{-}C_{1-5}\text{-alkyl}$, $-CH_2\text{-}O\text{-}C(=O)\text{-phenyl}$, $-O\text{-}C(=O)\text{-}C_{1-5}\text{-alkyl}$, -O-C(=O)-phenyl, -NH-S (=O)$_2$-$C_{1-5}$-alkyl, $-NH\text{-}C(=O)\text{-}C_{1-5}\text{-alkyl}$, $-C(=O)\text{-}NH_2$, $-C(=O)\text{-}NH\text{-}C_{1-5}\text{-alkyl}$, $-C(=O)\text{-}N(C_{1-5}\text{-alkyl})_2$, -C(=O)-N ($C_{1-5}$-alkyl)(phenyl), -C(=O)-NH-phenyl, cyclopropyl, cyclobutyl, cyclopentyl, cyclohexyl, pyrazolyl, phenyl, furyl (furanyl), thiazolyl, thiadiazolyl, thiophenyl (thienyl), benzyl and phenethyl, whereby the above-mentioned $C_{1-5}$-alkyl residues can in each case be linear or branched and the cyclic substituents or the cyclic residues of these substituents themselves can be substituted with optionally 1, 2, 3, 4 or 5, mutually independently selected from the group comprising F, Cl, Br, I, -CN, - $NO_2$, -OH, -SH, $-NH_2$, -C(=O)-OH, $-C_{1-5}\text{-alkyl}$, $-(CH_2)\text{-}O\text{-}C_{1-5}\text{-alkyl}$, $-C_{2-5}\text{-alkenyl}$, $-C_{2-5}\text{-alkynyl}$ , $-C{\equiv}C\text{-}Si(CH_3)_3$, $-C{\equiv}\text{-}C\text{-}Si(C_2H_5)_3$, $-S\text{-}C_{1-5}\text{-alkyl}$, -S-phenyl, $-S\text{-}CH_2\text{-phenyl}$, $-O\text{-}C_{1-5}\text{-alkyl}$, -O-phenyl, $-O\text{-}CH_2\text{-phenyl}$, $-CF_3$, $-CHF_2$, $-CH_2F$, $-O\text{-}CF_3$, - $O\text{-}CHF_2$, $-O\text{-}CH_2F$, $-C(=O)\text{-}CF_3$, $-S\text{-}CF_3$, $-S\text{-}CHF_2$ and $-S\text{-}CH_2F$,

whereby "substituted" in the context of the residues "alkylene", "alkenylene", "alkynylene", "heteroalkylene" or "heteroalkenylene" denotes a corresponding residue, which is monosubstituted or multiply substituted with substituents mutually independently selected from the group comprising phenyl, F, Cl, Br, I, $-NO_2$, -CN, -OH, -O-phenyl, $-O-CH_2$-phenyl, -SH, -S-phenyl, $-S-CH_2$-phenyl, - $NH_2$, $-N(C_{1-5}\text{-alkyl})_2$, -NH-phenyl, $-N(C_{1-5}$-alkyl)(phenyl), $-N(C_{1-5}$-alkyl)($CH_2$-phenyl), $-N(C_{1-5}$-alkyl)($CH_2$-$CH_2$-phenyl), -C(=O)-H, $-C(=O)-C_{1-5}$-alkyl, -C(=O)-phenyl, $-C(=S)-C_{1-5}$-alkyl, -C(=S)-phenyl, -C(=O)-OH, $-C(=O)-O-C_{1-5}$-alkyl, - C(=O)-O-phenyl, $-C(=O)-NH_2$, $-C(=O)-NH-C_{1-5}$-alkyl, $-C(=O)-N(C_{1-5}\text{-alkyl})_2$, - $S(=O)-C_{1-5}$-alkyl, -S(=O)-phenyl, $-S(=O)_2-C_{1-5}$-alkyl, $-S(=O)_2$-phenyl, $-S(=O)_2-NH_2$ and $-SO_3H$, whereby the above-mentioned $C_{1-5}$-alkyl residues can in each case be linear or branched and the above-mentioned phenyl residues can be substituted with 1, 2, 3, 4 or 5 substituents mutually independently selected from the group comprising F, Cl, Br, I, -CN, $-NO_2$, -OH, -SH, $-NH_2$, -C(=O)-OH, $-C_{1-5}$-alkyl, $-(CH_2)-O-C_{1-5}$-alkyl, $-C_{2-5}$-alkenyl, $-C_{2-5}$-alkynyl, $-C{\equiv}C-Si(CH_3)_3$, - $C{\equiv}C-Si(C_2H_5)_3$, $-S-C_{1-5}$-alkyl, -S-phenyl, $-S-CH_2$-phenyl, $-O-C_{1-5}$-alkyl, -O-phenyl, $-O-CH_2$-phenyl, $-CF_3$, $-CHF_2$, $-CH_2F$, $-O-CF_3$, $-O-CHF_2$, $-O-CH_2F$, - $C(=O)-CF_3$, $-S-CF_3$, $-S-CHF_2$ and $-S-CH_2F$,
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

**2.** Compounds according to Claim 1, **characterised in that**
a, b and c, mutually independently, in each case denote 0 or 1, whereby the sum of a, b and c is equal to 1, 2 or 3;
A denotes one of the following residues

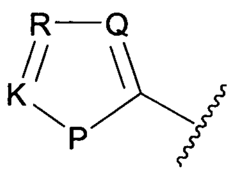

or

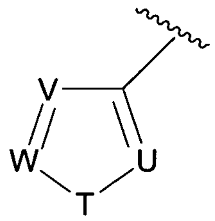

Q and U respectively denote $CR^{10}$ or N;
R and V respectively denote $CR^{11}$ or N;
K and W respectively denote $CR^{12}$ or N;
P and T respectively denote O, S or $NR^{13}$;

with the proviso that compounds are excluded in which P denotes S, Q denotes N, R denotes $CR^{11}$ and K denotes $CR^{12}$ or N;
$R^1$ and $R^8$, mutually independently, in each case denote H; $-C(=O)-R^{28}$; - $C(=O)-O-R^{29}$; $-C(=O)-NH_2$; $-C(=O)-NH-R^{30}$; $-C(=O)-NR^{31}R^{32}$; $-S(=O)-R^{33}$;$-S(=O)_2-R^{34}$; unsubstituted or substituted alkyl, alkenyl or alkynyl; unsubstituted or substituted heteroalkyl, heteroalkenyl or heteroalkynyl; unsubstituted or substituted cycloalkyl or cycloalkenyl; unsubstituted or substituted heterocycloalkyl or heterocycloalkenyl; unsubstituted or substituted - (alkylene)-cycloalkyl, -(alkenylene)-cycloalkyl, -(alkynylene)-cycloalkyl, - (alkylene)-cycloalkenyl, -(alkenylene)-cycloalkenyl or -(alkynylene)-cycloalkenyl; unsubstituted or substituted -(heteroalkylene)-cycloalkyl, - (heteroalkenylene)-cycloalkyl, -(heteroalkylene)-cycloalkenyl or - (heteroalkenylene)- cycloalkenyl; unsubstituted or substituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or substituted -(heteroalkylene)-heterocycloalkyl, -(heteroalkenylene)-heterocycloalkyl, -(heteroalkylene)-heterocycloalkenyl or -(heteroalkenylene)-heterocycloalkenyl; unsubstituted or substituted aryl; unsubstituted or substituted heteroaryl; unsubstituted or substituted -(alkylene)-aryl, - (alkenylene)-aryl, -(alkynylene)-aryl, -(heteroalkylene)-aryl or - (heteroalkenylene)-aryl; or unsubstituted or substituted -(alkylene)-heteroaryl, - (alkenylene)-heteroaryl, -(alkynylene)-heteroaryl, -(heteroalkylene)-heteroaryl or -(heteroalkenylene)-heteroaryl;
$R^2$ $R^3$, $R^4$, $R^5$, $R^6$, $R^7$, $R^{14}$, $R^{15}$, $R^{16}$, $R^{17}$, $R^{18}$, $R^{19}$ $R^{20}$, $R^{21}$, $R^{22}$, $R^{23}$, $R^{24}$, $R^{25}$, $R^{26}$ and $R^{27}$, mutually independently, in each case denote H; F; Cl; Br; I; $-NO_2$; -CN; $-NH_2$; -OH; -SH; -C(=O)-OH; -C(=O)-H; -NH-C(=O)-H; $-C(=O)-R^{28}$; - $C(=O)-O-R^{29}$; $-C(=O)-NH_2$; $-C(=O)-NH-R^{30}$; $-C(=O)-NR^{31}R^{32}$; $-S(=O)-R^{33}$; - $S(=O)_2-R^{34}$; $-NH-R^{35}$; $-NR^{36}R^{37}$; $-O-C(=O)-R^{38}$; $-NH-C(=O)-R^{39}$; $-NR^{40}-C(=O)-R^{41}$; $-O-R^{42}$; $-S-R^{43}$; $-NH-C(=O)-NH-R^{44}$; $-NH-C(=S)-NH-R^{45}$; -NH-S

$(=O)_2-R^{46}$; - $NR^{47}-S(=O)_2-R^{48}$; unsubstituted or at least monosubstituted alkyl, alkenyl or alkynyl; unsubstituted or at least monosubstituted heteroalkyl, heteroalkenyl or heteroalkynyl; unsubstituted or at least monosubstituted cycloalkyl or cycloalkenyl; unsubstituted or at least monosubstituted heterocycloalkyl or heterocycloalkenyl; unsubstituted or at least monosubstituted -(alkylene)-cycloalkyl, -(alkenylene)-cycloalkyl, -(alkynylene)-cycloalkyl, -(alkylene)-cycloalkenyl, -(alkenylene)-cycloalkenyl or -(alkynylene)-cycloalkenyl; unsubstituted or at least monosubstituted -(heteroalkylene)-cycloalkyl, - (heteroalkenylene)-cycloalkyl, -(heteroalkylene)-cycloalkenyl or - (heteroalkenylene)-cycloalkenyl; unsubstituted or at least monosubstituted - (alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or at least monosubstituted -(heteroalkylene)-heterocycloalkyl, -(heteroalkenylene)-heterocycloalkyl, -(heteroalkylene)-heterocycloalkenyl or -(heteroalkenylene)-heterocycloalkenyl; unsubstituted or at least monosubstituted aryl; unsubstituted or at least monosubstituted heteroaryl; unsubstituted or at least monosubstituted -(alkylene)-aryl, -(alkenylene)-aryl, -(alkynylene)-aryl, - (heteroalkylene)-aryl or -(heteroalkenylene)-aryl; or unsubstituted or at least monosubstituted -(alkylene)-heteroaryl, -(alkenylene)-heteroaryl, -(alkynylene)-heteroaryl, -(heteroalkylene)-heteroaryl or -(heteroalkenylene)-heteroaryl;
or $R^{2a}$ and $R^3$ or $R^4$ and $R^5$ or $R^6$ and $R^7$ or $R^{14}$ and $R^{15}$ or $R^{16}$ and $R^{17}$ or $R^{18}$ and $R^{19}$ or $R^{20}$ and $R^{21}$ or $R^{22}$ and $R^{23}$ or $R^{24}$ and $R^{25}$ or $R^{26}$ and $R^{27}$, mutually independently, jointly denote a residue selected from the group comprising an oxo group (=O) and a thioxo group (=S);
or R' and $R^8$ together with the $-N-CR^2R^3-(CR^4R^5)_b-(CR^6R^7)_c$ group joining them together form a residue of the general formula B,

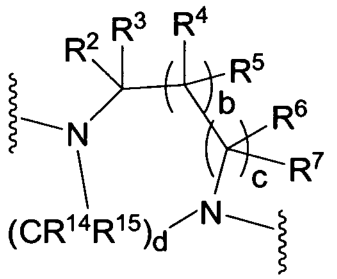

B,

whereby d denotes 1, 2 or 3 and b denotes 0 or 1 and c denotes 0 or 1;
or $R^1$ and $R^2$ together with the $-N-CR^3$ group joining them together form a residue of the general formula C,

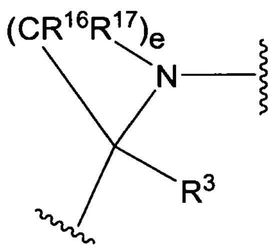

C,

whereby e denotes 1, 2 , 3 or 4 and, in this case, b denotes 0 or 1 and c denotes 0;
or $R^1$ and $R^4$ together with the $-N-CR^2R^3-CR^5$ group joining them together form a residue of the general formula D,

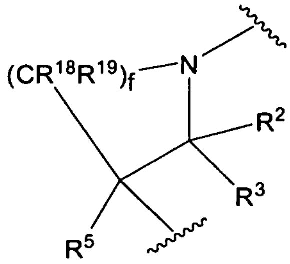

D,

whereby f denotes 1, 2, 3 or 4, and, in this case, c denotes 0;
or $R^1$ and $R^6$ together with the -N-$CR^2R^3$-$CR^4R^5$-$CR^7$ group joining them together form a residue of the general formula E,

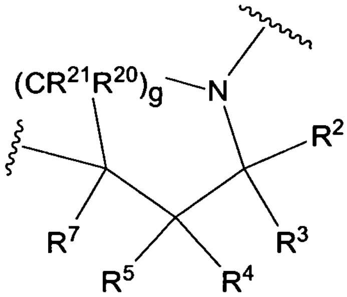

E,

whereby g denotes 1, 2 or 3;
or $R^6$ and $R^8$ together with the -N-$CR^7$ group joining them together form a residue of the general formula F

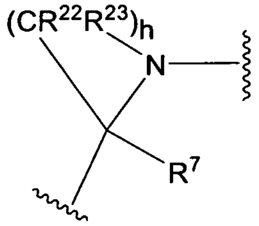

F,

whereby h denotes 1, 2 , 3 or 4 and, in this case, b denotes 0 or 1 and a denotes 0;
or $R^4$ and $R^8$ together with the -N-$CR^6R^7$-$CR^5$ group joining them together form a residue of the general formula G,

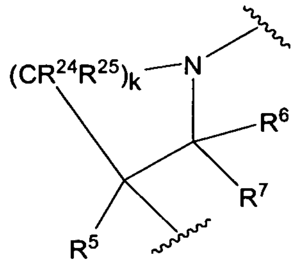

G,

whereby k denotes 1, 2, 3 or 4, and, in this case, a denotes 0;

or $R^2$ and $R^8$ together with the -N-$CR^6R^7$-$CR^4R^5$-$CR^3$ group joining them together form a residue of the general formula H,

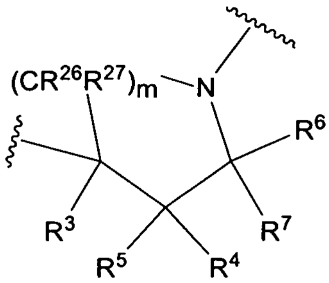

H,

whereby m denotes 1, 2 or 3;

$R^9$ denotes unsubstituted or substituted aryl or unsubstituted or substituted heteroaryl;

$R^{10}$, $R^{11}$ and $R^{12}$, mutually independently, in each case denote H; F; Cl; Br; I; - $NO_2$; -CN; -$NH_2$; -OH; -SH; -C (=O)-OH; -C(=O)-H; -NH-C(=O)-H; -C(=O)-$R^{28}$; - C(=O)-O-$R^{29}$; -C(=O)-$NH_2$; -C(=O)-NH-$R^{30}$; -C(=O)-$NR^{31}R^{32}$; -S (=O)-$R^{33}$; S(=O)$_2$-$R^{34}$; -NH-$R^{35}$; -$NR^{36}R^{37}$; -O-C(=O)-$R^{38}$; -NH-C(=O)-$R^{39}$; -$NR^{40}$-C(=O)-$R^{41}$ ; -O-$R^{42}$; -S-$R^{43}$; -NH-C(=O)-NH-$R^{44}$; -NH-C(=S)-NH-$R^{45}$; -NH-S(=O)$_2$-$R^{46}$; $NR^{47}$-S(=O)$_2$-$R^{48}$; unsubstituted or at least monosubstituted alkyl, alkenyl or alkynyl; unsubstituted or at least monosubstituted heteroalkyl, heteroalkenyl or heteroalkynyl; unsubstituted or at least monosubstituted cycloalkyl or cycloalkenyl; unsubstituted or at least monosubstituted heterocycloalkyl or heterocycloalkenyl; unsubstituted or at least monosubstituted -(alkylene)-cycloalkyl, -(alkenylene)-cycloalkyl, -(alkynylene)-cycloalkyl, -(alkylene)-cycloalkenyl,- (alkenylene)-cycloalkenyl or -(alkynylene)-cycloalkenyl; unsubstituted or at least monosubstituted -(heteroalkylene)-cycloalkyl, - (heteroalkenylene)-cycloalkyl, -(heteroalkylene)-cycloalkenyl or - (heteroalkenylene)-cycloalkenyl; unsubstituted or at least monosubstituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or at least monosubstituted -(heteroalkylene)-heterocycloalkyl, -(heteroalkenylene)-heterocycloalkyl, -(heteroalkylene)-heterocycloalkenyl or -(heteroalkenylene)-heterocycloalkenyl; unsubstituted or at least monosubstituted aryl; unsubstituted or at least monosubstituted heteroaryl; unsubstituted or at least monosubstituted -(alkylene)-aryl, -(alkenylene)-aryl, -(alkynylene)-aryl, - (heteroalkylene)-aryl or -(heteroalkenylene)-aryl; or unsubstituted or at least monosubstituted -(alkylene)-heteroaryl, -(alkenylene)-heteroaryl, -(alkynylene)-heteroaryl, -(heteroalkylene)-heteroaryl or -(heteroalkenylene)-heteroaryl;

$R^{13}$ denotes H; -C(=O)-OH; -C(=O)-H; -C(=O)-$R^{28}$; -C(=O)-O-$R^{29}$; -C(=O)-$NH_2$; -C(=O)-NH-$R^{30}$; -C(=O)-$NR^{31}R^{32}$; -S(=O)-$R^{33}$; -S(=O)$_2$-$R^{34}$; unsubstituted or at least monosubstituted alkyl, alkenyl or alkynyl; unsubstituted or at least monosubstituted heteroalkyl, heteroalkenyl or heteroalkynyl; unsubstituted or at least monosubstituted cycloalkyl or cycloalkenyl; unsubstituted or at least monosubstituted heterocycloalkyl or heterocycloalkenyl; unsubstituted or at least monosubstituted -(alkylene)-cycloalkyl, -(alkenylene)-cycloalkyl, - (alkynylene)-cycloalkyl, -(alkylene)-cycloalkenyl, -(alkenylene)-cycloalkenyl or - (alkynylene)-cycloalkenyl; unsubstituted or at least monosubstituted -(heteroalkylene)-cycloalkyl, -(heteroalkenylene)-cycloalkyl, -(heteroalkylene)-cycloalkenyl or -(heteroalkenylene)-cycloalkenyl; unsubstituted or at least monosubstituted -(alkylene)-heterocycloalkyl, -(alkenylene)-heterocycloalkyl, - (alkynylene)-heterocycloalkyl, -(alkylene)-heterocycloalkenyl, -(alkenylene)-heterocycloalkenyl or -(alkynylene)-heterocycloalkenyl; unsubstituted or at least monosubstituted -(heteroalkylene)-heterocycloalkyl, -(heteroalkenylene)-heterocycloalkyl, -(heteroalkylene)-heterocycloalkenyl or -(heteroalkenylene)-heterocycloalkenyl; unsubstituted or at least monosubstituted aryl; unsubstituted or at least monosubstituted heteroaryl; unsubstituted or at least monosubstituted -(alkylene)-aryl, -(alkenylene)-aryl, -(alkynylene)-aryl, - (heteroalkylene)-aryl or -(heteroalkenylene)-aryl; or unsubstituted or at least monosubstituted -(alkylene)-heteroaryl, -(alkenylene)-heteroaryl, -(alkynylene)-heteroaryl, -(heteroalkylene)-heteroaryl or -(heteroalkenylene)-heteroaryl;

and $R^{28}$, $R^{29}$, $R^{30}$, $R^{31}$, $R^{32}$, $R^{33}$, $R^{34}$, $R^{35}$, $R^{36}$ , $R^{37}$, $R^{38}$, $R^{39}$, $R^{40}$, $R^{24}$, $R^{42}$, $R^{43}$, $R^{44}$, $R^{45}$, $R^{46}$, $R^{47}$ and $R^{48}$, mutually independently, in each case denote unsubstituted or substituted alkyl, alkenyl or alkynyl; unsubstituted or substituted heteroalkyl, heteroalkenyl or heteroalkynyl; unsubstituted or substituted cycloalkyl or cycloalkenyl; unsubstituted or substituted heterocycloalkyl or heterocycloalkenyl; unsubstituted or substituted -(alkylene)-cycloalkyl, -(alkenylene)-cycloalkyl, -(alkynylene)-cycloalkyl, -(alkylene)-cycloalkenyl, -(alkenylene)-cycloalkenyl or -(alkynylene)-cycloalkenyl; unsubstituted or substituted -(heteroalkylene)-cycloalkyl, -(heteroalkenylene)-cycloalkyl, -(heteroalkylene)-cycloalkenyl or -(heteroalkenylene)-cycloalkenyl; unsubstituted or substituted -(alkylene)-heterocycloalkyl, -(alkenylene)-heterocycloalkyl, -(alkynylene)-heterocycloalkyl, -(alkylene)-heterocycloalkenyl, -(alkenylene)-heterocycloalkenyl or -(alkynylene)-heterocycloalkenyl; unsubstituted or substituted -(heteroalkylene)-heterocycloalkyl, -(heteroalkenylene)-heterocycloalkyl, -(heteroalkylene)-heterocycloalkenyl; or -(heteroalkenylene)-heterocycloalkenyl; unsubstituted or substituted aryl; unsubstituted or substituted heteroaryl; unsubstituted or substituted -(alkylene)-aryl, -(alkenylene)-aryl, -(alkynylene)-aryl, - (heteroalkylene)-aryl or -(heteroalkenylene)-aryl; or unsubstituted or substituted -(alkylene)-heteroaryl, -(alkenylene)-heteroaryl, -(alkynylene)-heteroaryl, -(heteroalkylene)-heteroaryl or -(heteroalkenylene)-heteroaryl;

whereby

the above-mentioned alkyl residues are in each case branched or straight-chained and have 1, 2, 3, 4, 5, 6, 7, 8, 9, 10, 11 or 12 carbon atoms as chain members;

the above-mentioned alkenyl residues are in each case branched or straight-chained and have 2, 3, 4, 5, 6, 7, 8, 9, 10, 11 or 12 carbon atoms as chain members;

the above-mentioned alkynyl residues are in each case branched or straight-chained and have 2, 3, 4, 5, 6, 7, 8, 9, 10, 11 or 12 carbon atoms as chain members;

the above-mentioned heteroalkyl residues, heteroalkenyl residues and heteroalkynyl residues are in each case 2-, 3-, 4-, 5-, 6-, 7-, 8-, 9-, 10-, 11- or 12-membered;

the above-mentioned heteroalkyl residues, heteroalkenyl residues and heteroalkynyl residues in each case have optionally 1, 2 or 3 heteroatom(s) mutually independently selected from the group comprising oxygen, sulphur and nitrogen as the chain member(s);

the above-mentioned alkyl residues, alkenyl residues, alkynyl residues, heteroalkyl residues, heteroalkenyl residues and heteroalkynyl residues can in each case be substituted with optionally 1, 2, 3, 4 or 5 substituents mutually independently selected from the group comprising F, Cl, Br, I, $-NO_2$, -CN, -OH, -SH, $-NH_2$, $-N(C_{1-5}$-alkyl$)_2$, -N ($C_{1-5}$-alkyl)(phenyl), $-N(C_{1-5}$-alkyl)($CH_2$-phenyl), - $N(C_{1-5}$-alkyl)($CH_2$-$CH_2$-phenyl), -C(=O)-H, -C(=O)-$C_{1-5}$-alkyl, -C (=O)-phenyl, - C(=S)-$C_{1-5}$-alkyl, -C(=S)-phenyl, -C(=O)-OH, -C(=O)-O-$C_{1-5}$-alkyl, -C(=O)-O-phenyl, -C(=O)-$NH_2$, -C (=O)-NH-$C_{1-5}$-alkyl, -C(=O)-$N(C_{1-5}$-alkyl$)_2$, -S(=O)-$C_{1-5}$-alkyl, -S(=O)-phenyl, $-S(=O)_2$-$C_{1-5}$-alkyl, $-S(=O)_2$-phenyl, $-S(=O)_2$-$NH_2$ and - $SO_3H$, whereby the phenyl residues can be substituted with 1, 2, 3, 4 or 5 substituents mutually independently selected from the group comprising F, Cl, Br, I, -CN, $-CF_3$, -OH, $-NH_2$, -O-$CF_3$, -SH, -O-$CH_3$, -O-$C_2H_5$, -O-$C_3H_7$, methyl, ethyl, n-propyl, isopropyl, n-butyl, 2-butyl, isobutyl and tert-butyl;

the above-mentioned cycloalkyl residues in each case have 3, 4, 5, 6, 7, 8 or 9 carbon atoms as ring members;

the above-mentioned cycloalkenyl residues in each case have 3, 4, 5, 6, 7, 8 or 9 carbon atoms as ring members;

the above-mentioned heterocycloalkyl residues are in each case 3-, 4-, 5-, 6-, 7-, 8- or 9-membered;

the above-mentioned heterocycloalkenyl residues are in each case 4-, 5-, 6-, 7-, 8- or 9-membered;

the above-mentioned heterocycloalkyl residues and heterocycloalkenyl residues in each case have optionally 1, 2 or 3 heteroatom(s) mutually independently selected from the group comprising oxygen, sulphur and nitrogen (NH) as the ring member(s);

the above-mentioned cycloalkyl residues, heterocycloalkyl residues, cycloalkenyl residues or heterocycloalkenyl residues can in each case be substituted with optionally 1, 2, 3, 4 or 5 substituents mutually independently selected from the group comprising F, Cl, Br, I, -CN, $-CF_3$, -OH, $-NH_2$, -O-$CF_3$, -SH, -O-$C_{1-5}$-alkyl, -O-phenyl, -O-$CH_2$-phenyl, -($CH_2$)-O-$C_{1-5}$-alkyl, -S-$C_{1-5}$-alkyl, -S-phenyl, -S-$CH_2$-phenyl, -$C_{1-5}$-alkyl, -$C_{2-5}$-alkenyl, -$C_{2-5}$-alkynyl, -C=C-Si $(CH_3)_3$, -C≡C-Si$(C_2H_5)_3$, -C(=O)-O-$C_{1-5}$-alkyl, -C(=O)-$CF_3$, $-S(=O)_2$-$C_{1-5}$-alkyl, -S(=O)-$C_{1-5}$-alkyl, $-S(=O)_2$-phenyl, oxo (=O), thioxo (=S), $-N(C_{1-5}$-alkyl$)_2$, - N(H)($C_{1-5}$-alkyl), $-NO_2$, -S-$CF_3$, -C(=O)-OH, -NH-C(=O)-$C_{1-5}$-alkyl, -C(=O)-H, - C(=O)-$C_{1-5}$-alkyl, -C(=O)-$NH_2$, -C(=O)-$N(C_{1-5}$-alkyl$)_2$, -C(=O)-N(H)($C_{1-5}$-alkyl) and phenyl, whereby the phenyl residues can respectively be unsubstituted or substituted with 1, 2, 3, 4 or 5 substituents mutually independently selected from the group comprising F, Cl, Br, I, -CN, $-CF_3$, -OH, $-NH_2$, -O-$CF_3$, -SH, -O-$C_{1-5}$-alkyl, -O-phenyl, -O-

$CH_2$-phenyl, -($CH_2$)-O-$C_{1-5}$-alkyl, -S-$C_{1-5}$-alkyl, -S-phenyl, -S-$CH_2$-phenyl, -$C_{1-5}$-alkyl, -$C_{2-5}$-alkenyl, -$C_{2-5}$-alkynyl, -C≡C-Si($CH_3$)$_3$, -C≡C-Si($C_2H_5$)$_3$, -C(=O)-O-$C_{1-5}$-alkyl and -C(=O)-$CF_3$, whereby the above-mentioned phenyl residues can preferably be substituted with 1, 2, 3, 4 or 5 substituents mutually independently selected from the group comprising F, Cl, Br, I, -CN, -$CF_3$, -OH, -$NH_2$, -O-$CF_3$, -SH, -O-$CH_3$, -O-$C_2H_5$, -O-$C_3H_7$, methyl, ethyl, n-propyl, isopropyl, n-butyl, 2-butyl, isobutyl and tert-butyl;

the above-mentioned alkylene residues are in each case branched or straight-chained and have 1, 2, 3, 4, 5, 6, 7, 8, 9, 10, 11 or 12 carbon atoms as chain members;

the above-mentioned alkenylene residues are in each case branched or straight-chained and have 2, 3, 4, 5, 6, 7, 8, 9, 10, 11 or 12 carbon atoms as chain members;

the above-mentioned alkynylene residues are in each case branched or straight-chained and have 2, 3, 4, 5, 6, 7, 8, 9, 10, 11 or 12 carbon atoms as chain members;

the above-mentioned heteroalkylene residues and heteroalkenylene residues are in each case 2-, 3-, 4-, 5-, 6-, 7-, 8-, 9-, 10-, 11- or 12-membered;

the above-mentioned heteroalkylene and heteroalkenylene groups have in each case optionally 1, 2 or 3 heteroatom (s) mutually independently selected from the group comprising oxygen, sulphur and nitrogen (NH) as the chain member(s);

the above-mentioned alkylene, alkenylene, alkynylene, heteroalkylene or heteroalkenylene group can in each case be unsubstituted or substituted with optionally 1, 2, 3, 4 or 5 substituents mutually independently selected from the group comprising phenyl, F, Cl, Br, I, -$NO_2$, -CN, -OH, -O-phenyl, -O-$CH_2$-phenyl, -SH, -S-phenyl, -S-$CH_2$-phenyl, $NH_2$, -N($C_{1-5}$-alkyl)$_2$, -NH-phenyl, - N($C_{1-5}$-alkyl)(phenyl), -N($C_{1-5}$-alkyl)($CH_2$-phenyl), -N($C_{1-5}$-alkyl)($CH_2$-$CH_2$-phenyl), -C(=O)-H, -C(=O)-$C_{1-5}$-alkyl, -C(=O)-phenyl, -C(=S)-$C_{1-5}$-alkyl, -C(=S)-phenyl, -C(=O)-OH, -C(=O)-O-$C_{1-5}$-alkyl, -C(=O)-O-phenyl, -C(=O)-$NH_2$, - C(=O)-NH-$C_{1-5}$-alkyl, -C(=O)-N($C_{1-5}$-alkyl)$_2$, -S(=O)-$C_{1-5}$-alkyl, -S (=O)-phenyl, - S(=O)$_2$-$C_{1-5}$-alkyl, -S(=O)$_2$-phenyl, -S(=O)$_2$-$NH_2$ and -$SO_3H$, whereby the phenyl residues can be substituted with 1, 2, 3, 4 or 5 substituents mutually independently selected from the group comprising F, Cl, Br, I, -CN, -$NO_2$, -OH, -SH, -$NH_2$, -C(=O)-OH, -$C_{1-5}$-alkyl, -($CH_2$)-O-$C_{1-5}$-alkyl, -$C_{2-5}$-alkenyl, -$C_{2-5}$-alkynyl,- C=C-Si($CH_3$)$_3$, -C=C-Si($C_2H_5$)$_3$, -S-$C_{1-5}$-alkyl, -S-phenyl, -S-$CH_2$-phenyl, -O-$C_{1-5}$-alkyl, -O-phenyl, -O-$CH_2$-phenyl, -$CF_3$, -$CHF_2$, -$CH_2F$, -O-$CF_3$, - O-$CHF_2$, -O-$CH_2F$, -C(=O)-$CF_3$, -S-$CF_3$, -S-$CHF_2$ and -S-$CH_2F$;

the above-mentioned aryl residues are mono- or bicyclic and have 6, 10 or 14 carbon atoms;

the above-mentioned heteroaryl residues are mono-, bi- or tricyclic and 5-, 6-, 7-, 8-, 9-, 10-, 11-, 12-, 13- or 14-membered;

the above-mentioned 5- to 14-membered heteroaryl residues in each case have optionally 1, 2, 3, 4 or 5 heteroatom (s) mutually independently selected from the group comprising oxygen, sulphur and nitrogen (NH) as the ring member (s);

and the above-mentioned aryl residues and heteroaryl residues can in each case be substituted with optionally 1, 2, 3, 4 or 5 substituents mutually independently selected from the group comprising F, Cl, Br, I, -CN, -$NO_2$, -OH, -SH, -$NH_2$, -C(=O)-OH, -$C_{1-5}$-alkyl, -($CH_2$)-O-$C_{1-5}$-alkyl, -$C_{2-5}$-alkenyl, -$C_{2-5}$-alkynyl , -C≡C-Si($CH_3$)$_3$, -C=C-Si ($C_2H_5$)$_3$, -S-$C_{1-5}$-alkyl, -S-phenyl, -S-$CH_2$-phenyl, -O-$C_{1-5}$-alkyl, -O-phenyl, -O-$CH_2$-phenyl, -$CF_3$, -$CHF_2$, -$CH_2F$, -O-$CF_3$, - O-$CHF_2$, -O-$CH_2F$, -C(=O)-$CF_3$, -S-$CF_3$, -S-$CHF_2$, -S-$CH_2F$, -S(=O)$_2$-phenyl, - S(=O)$_2$-$C_{1-5}$-alkyl, -S (=O)-$C_{1-5}$-alkyl, -NH-$C_{1-5}$-alkyl, N($C_{1-5}$-alkyl)$_2$, -C(=O)-O-$C_{1-5-5}$-alkyl, -C(=O)-O-phenyl, -C(=O)-H, -C (=O)-$C_{1-5}$-alkyl, -$CH_2$-O-C(=O)-phenyl, - O-C(=O)-phenyl, -O-C(=O)-$C_{1-5}$-alkyl, -NH-C(=O)-$C_{1-5}$-alkyl, -C(=O)-$NH_2$, - C(=O)-NH-$C_{1-5}$-alkyl, -C(=O)-N($C_{1-5}$-alkyl)$_2$, -C(=O)-N($C_{1-5}$-alkyl)(phenyl), - C(=O)-NH-phenyl, cyclopropyl, cyclobutyl, cyclopentyl, cyclohexyl, pyrazolyl, phenyl, furyl (furanyl), thiazolyl, thiadiazolyl, thiophenyl (thienyl), benzyl and phenethyl, whereby the cyclic substituents or the cyclic residues of these substituents themselves can be substituted with optionally 1, 2, 3, 4 or 5 substituents mutually independently selected from the group comprising F, Cl, Br, I, -CN, -$NO_2$, -OH, -SH, -$NH_2$, -C(=O)-OH, -$C_{1-5}$-alkyl, -($CH_2$)-O-$C_{1-5}$-alkyl, - $C_{2-5}$-alkenyl, -$C_{2-5}$-alkynyl, -C≡C-Si($CH_3$)$_3$, -C≡C-Si($C_2H_5$)$_3$, -S-$C_{1-5}$-alkyl, -S-phenyl,- S-$CH_2$-phenyl, -O-$C_{1-5}$-alkyl, -O-phenyl, -O-$CH_2$-phenyl, -$CF_3$, -$CHF_2$, - $CH_2F$, -O-$CF_3$, -O-$CHF_2$, -O-$CH_2F$, -C(=O)-$CF_3$, -S-$CF_3$, -S-$CHF_2$ and -S-$CH_2F$;

in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

3. Compounds according to Claim 1 or 2, **characterised in that**
a, b and c, mutually independently, in each case denote 0 or 1, whereby the sum of a, b and c is equal to 1, 2 or 3;
A denotes a residue selected from the group comprising

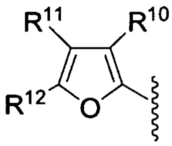 , 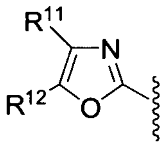 , 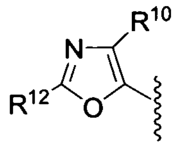 , 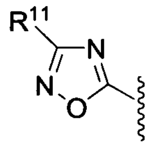 ,

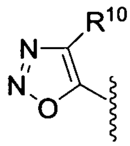 , 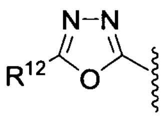 , 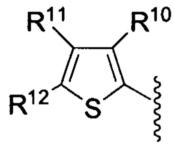 , 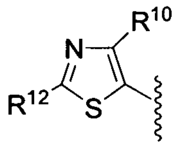 ,

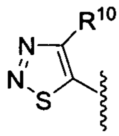 , 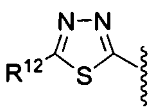 , 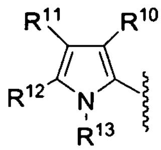 , 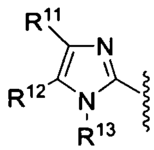 ,

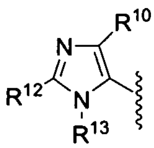 , 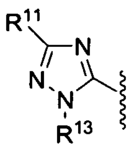 , 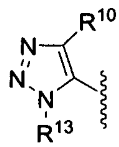 , 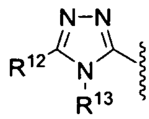 , 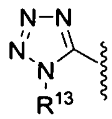 ,

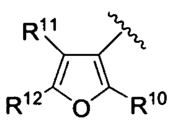 , 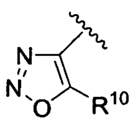 , 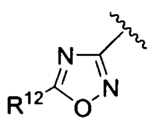 , 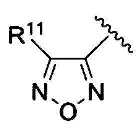 , 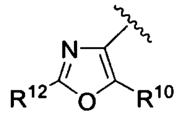 ,

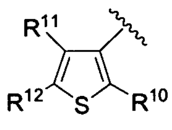 , 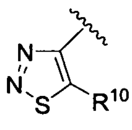 , 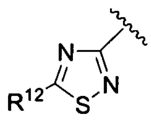 , 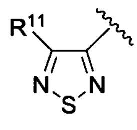 , 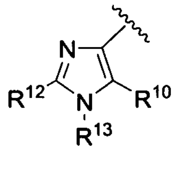 ,

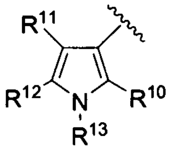 , 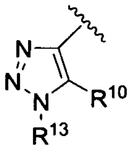 , 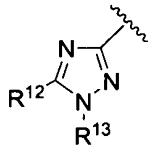 , 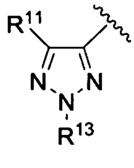 , 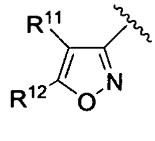 ,

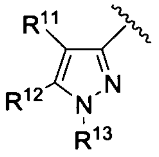,
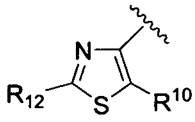
and
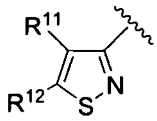

$R^1$ and $R^8$, mutually independently, in each case denote H; $-C(=O)-R^{28}$; $-C(=O)-O-R^{29}$; $-C(=O)-NH_2$; $-C(=O)-NH-R^{30}$; $-C(=O)-NR^{31}R^{32}$; $-S(=O)-R^{33}$; $-S(=O)_2-R^{34}$; $C_{1-6}$-alkyl, which is unsubstituted or substituted with optionally 1, 2, 3, 4 or 5 substituents mutually independently selected from the group comprising F, Cl, Br, I, $-NO_2$, -CN, -OH, -SH and $-NH_2$;

$C_{3-8}$-cycloalkyl, which is unsubstituted or substituted with optionally 1, 2, 3, 4 or 5 substituents mutually independently selected from the group comprising F, Cl, Br, I, $-NO_2$, -CN, -OH, -SH and $-NH_2$;

or a phenyl residue, which can be bound in each case via a $C_{1-3}$-alkylene-, $C_{2-3}$-alkenylene- or $C_{2-3}$-alkynylene group and/or is unsubstituted or substituted with 1, 2, 3, 4 or 5 substituents mutually independently selected from the group comprising F, Cl, Br, I, -CN, methyl, ethyl, n-propyl, isopropyl, n-butyl, 2-butyl, isobutyl, tert-butyl, -OH, $-O-CH_3$, $-O-C_2H_5$ and $-O-C_3H_7$.

$R^2$, $R^3$, $R^4$, $R^5$, $R^6$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ and $R^{27}$, mutually independently, in each case denote H; F; Cl; Br; I; $-NO_2$; -CN; $-NH_2$; -OH; -SH; -C(=O)-OH; $-C(=O)-R^{28}$; $-C(=O)-O-R^{29}$; $-NH-R^{35}$; $-NR^{36}R^{37}$; $-O-R^{42}$; $-S-R^{43}$; $C_{1-6}$-alkyl, which is unsubstituted or substituted with optionally 1, 2, 3, 4 or 5 substituents mutually independently selected from the group comprising F, Cl, Br, I, $-NO_2$, -CN, -OH, -SH and $-NH_2$; $C_{3-7}$-cycloalkyl, $C_{5-6}$-cycloalkenyl, 5- to 7-membered heterocycloalkyl or 5- to 7-membered heterocycloalkenyl, which can be bound in each case via a $C_{1-3}$-alkylene-, $C_{2-3}$-alkenylene- or $C_{2-3}$-alkynylene group and/or unsubstituted or substituted with optionally 1, 2, 3, 4 or 5 substituents mutually independently selected from the group comprising F, Cl, Br, I, -CN, methyl, ethyl, n-propyl, isopropyl, n-butyl, 2-butyl, isobutyl, tert-butyl, -OH, oxo, thioxo, $-O-CH_3$, $-O-C_2H_5$, $-O-C_3H_7$, $-NH_2$; $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH, $-S-CH_3$ and $-S-C_2H_5$; or denote a residue selected from the group comprising phenyl, naphthyl, anthracenyl, pyrrolyl, indolyl, furanyl, benzo[b]furanyl, thiophenyl, benzo[b]thiophenyl, benzo[d]thiazolyl, pyrazolyl, imidazolyl, thiazolyl, thiadiazolyl, triazolyl, oxazolyl, oxadiazolyl, isoxazolyl, pyridinyl, pyridazinyl, pyrimidinyl, pyrazinyl, pyranyl, indazolyl, chinolinyl, isochinolinyl and chinazolinyl, which can be bound in each case via a $C_{1-3}$-alkylene, $C_{2-3}$-alkenylene or $C_{2-3}$ alkynylene group and/or is unsubstituted or substituted with optionally 1, 2, 3, 4 or 5 substituents mutually independently selected from the group comprising F, Cl, Br, I, -CN, methyl, ethyl, n-propyl, isopropyl, n-butyl, 2-butyl, isobutyl, tert-butyl, -OH, $-O-CH_3$, $-O-C_2H_5$ and $-O-C_3H_7$;

or $R^2$ and $R^3$or $R^4$ and $R^5$ or $R^6$ and $R^7$ or $R^{14}$ and $R^{15}$ or $R^{16}$ and $R^{17}$ or $R^{18}$ and $R^{19}$ or $R^{20}$ and $R^{21}$ or $R^{22}$ and $R^{23}$ or $R^{24}$ and $R^{25}$ or $R^{26}$ and $R^{27}$, mutually independently, jointly in each case denote a residue selected from the group comprising an oxo group (=O) and a thioxo group (=S).

or $R^1$ and $R^8$ together with the $-N-CR^2R^3-(CR^4R^5)_b-(CR^6R^7)_c$ group joining them together form a residue selected from the group comprising

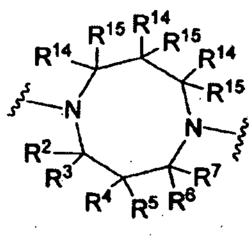,
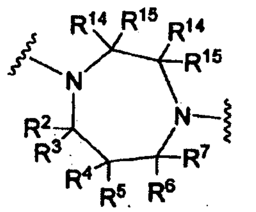,
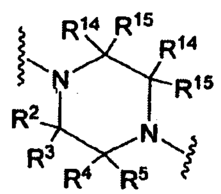,

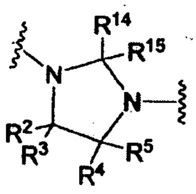

and

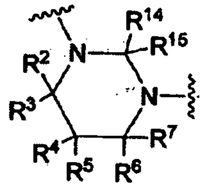

or $R^1$ and $R^2$ together with the -N-$CR^3$ group joining them together form a residue selected from the group comprising

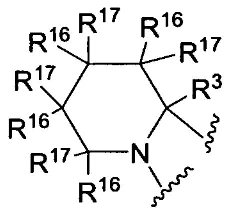

,

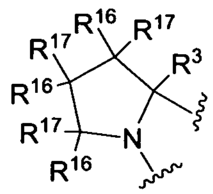

and

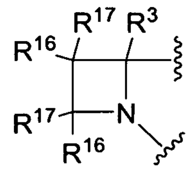

whereby, in this case, b denotes 1 and c denotes 0;
or $R^1$ and $R^4$ together with the -N-$CR^2R^3$-$CR^5$ group joining them together form a residue selected from the group comprising

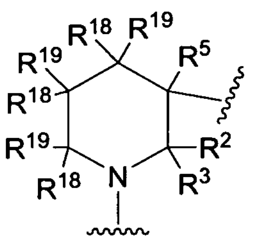

,

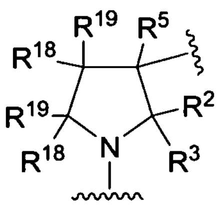

and

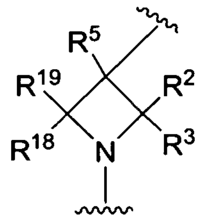

whereby, in this case, c denotes 0;
$R^1$ and $R^6$ together with the -N-$CR^2R^3$-$CR^4R^5$-$CR^7$ group joining them together form a residue selected from the group comprising

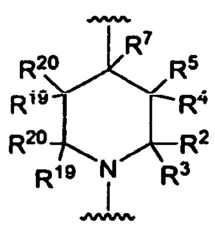

and

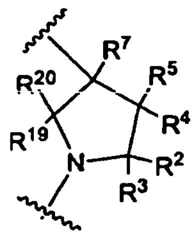

or $R^6$ and $R^8$ together with the -N-$CR^7$ group joining them together form a residue selected from the group comprising

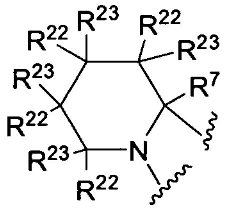 , 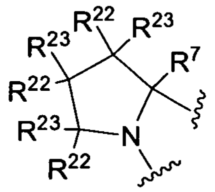 and 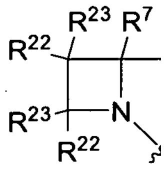

whereby, in this case, b denotes 1 and a denotes 0.
or $R^4$ and $R^8$ together with the -N-$CR^6R^7$-$CR^5$ group joining them together form a residue selected from the group comprising

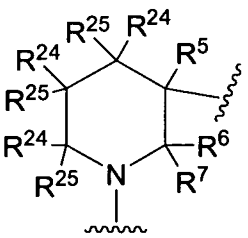 , 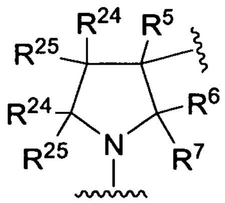 and 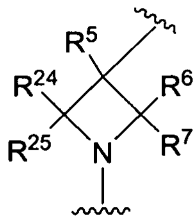

whereby, in this case, a denotes 0.
or $R^2$ and $R^8$ together with the -N-$CR^6R^7$-$CR^4R^5$-$CR^3$ group joining them together form a residue selected from the group comprising

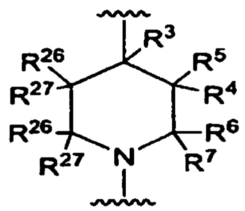 and 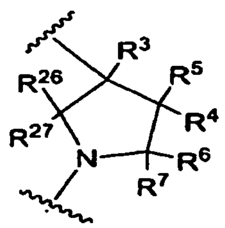

$R^9$ denotes a residue selected from the group comprising phenyl, naphthyl, anthracenyl, furyl, thienyl, pyrazolyl, pyrazinyl, pyrimidinyl, pyridazinyl, pyridinyl, pyrrolyl, oxazolyl, isoxazolyl, thiazolyl, thiadiazolyl, oxadiazolyl, triazolyl, imidazolyl, indolyl, benzo[b]thiophenyl, benzo[d]thiazolyl, benzo[b]furanyl, chinolinyl, isochinolinyl and chinazolinyl, which is in each case unsubstituted or substituted with optionally 1, 2, 3, 4 or 5 substituents mutually independently selected from the group comprising F, Cl, Br, I, -CN, methyl, ethyl, n-propyl, isopropyl, n-butyl, 2-butyl, isobutyl, tert-butyl, ethenyl, allyl, ethynyl, propynyl, cyclopropyl, cyclobutyl, cyclopentyl, -C≡C-$Si(CH_3)_3$, - C≡C-$Si(C_2H_5)_3$, -$CH_2$-O-$CH_3$, -$CH_2$-O-$C_2H_5$, -OH, -O-$CH_3$, -O-$C_2H_5$, -O-$C_3H_7$, - S-$CH_3$, -S-$C_2H_5$, -S(=O)-$CH_3$, -S$(=O)_2$-$CH_3$, -S(=O)-$C_2H_5$, -S$(=O)_2$-$C_2H_5$, -$NH_2$, -$N(CH_3)_2$, -$N(C_2H_5)_2$, -NH-$CH_3$, -NH-$C_2H_5$, -$NO_2$, -$CF_3$, -$CH_2F$, -$CHF_2$, -O-$CF_3$, - S-$CF_3$, -SH, -NH-S$(=O)_2$-$CH_3$, -C(=O)-OH, -C(=O)-H; -C(=O)-$CH_3$, -C(=O)-$C_2H_5$, -C(=O)-$NH_2$, -C(=O)-$N(CH_3)_2$, -C(=O)-NH-$CH_3$, -NH-C(=O)-$CH_3$, -NH-C(=O)-$C_2H_5$, -C(=O)-O-$CH_3$, -C(=O)-O-$C_2H_5$, -C(=O)-O-$C(CH_3)_3$ and phenyl;
$R^{10}$, $R^{11}$ and $R^{12}$, mutually independently, in each case denote H; F; Cl; Br; I; - $NO_2$; -CN; -$NH_2$; -OH; -SH; -C(=O)-OH; -C(=O)-H; -NH-C(=O)-H; -C(=O)-$R^{28}$; - C(=O)-O-$R^{29}$; -C(=O)-$NH_2$; -C(=O)-NH-$R^{30}$; -C(=O)-$NR^{31}R^{32}$; -S(=O)-$R^{33}$; - S$(=O)_2$-$R^{34}$; -NH-$R^{35}$; -$NR^{36}R^{37}$; -O-C(=O)-$R^{38}$; -NH-C(=O)-$R^{39}$; -$NR^{40}$-C(=O)-$R^{41}$; -O-$R^{42}$; -S-$R^{43}$-NH-C(=O)-NH-$R^{44}$; -NH-C(=S)-NH-$R^{45}$; -NH-S$(=O)_2$-$R^{46}$; - $NR^{47}$-S$(=O)_2$-$R^{48}$; $C_{1-6}$-alkyl, which is unsubstituted or substituted with optionally 1, 2, 3, 4 or 5 substituents mutually independently selected from the group comprising F, Cl, Br, I, -$NO_2$, -CN, -OH, -SH and -$NH_2$; $C_{2-6}$-alkenyl; $C_{2-6}$-alkynyl; $C_{3-7}$-cycloalkyl, $C_{5-6}$-cydoalkenyl, 5- to 7-membered heterocycloalkyl or 5- to 7-membered heterocycloalkenyl, which can in each case be bound via a $C_{1-3}$-alkylene, $C_{2-3}$-alkenylene or $C_{2-3}$-alkynylene group and/or is unsubstituted or substituted with optionally 1, 2, 3, 4 or 5 substituents mutually independently selected from the group comprising F, Cl, Br, I, -CN, methyl, ethyl, n-propyl, isopropyl, n-butyl, 2-butyl, isobutyl, tert-butyl, -OH, oxo, thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, - $NO_2$, $-CF_3$, $-O-CF_3$, $-S-CF_3$, -SH, $-S-CH_3$ and $-S-C_2H_5$; or denote a residue selected from the group comprising phenyl, naphthyl, anthracenyl, thienyl,
furyl, pyridinyl, thiazolyl, thiadiazolyl, oxazolyl, oxadiazolyl and isoxazolyl, which in each case can be bound via a $C_{1-3}$-alkylene-, $C_{2-3}$-alkenylene- or $C_{2-3}$-alkynylene group and/or is unsubstituted or substituted with optionally 1, 2, 3, 4 or 5 substituents mutually independently selected from the group comprising F, Cl, Br, I, -CN, methyl, ethyl, n-propyl, isopropyl, n-butyl, isobutyl, 2-butyl, tert-butyl, -OH, -SH, $-NH_2$, -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-CF_3$, $-CHF_2$, - $CH_2F$ and $-O-CF_3$;
$R^{13}$ denotes H; $-C(=O)-R^{28}$; -C(=O)-H; $-C(=O)-O-R^{29}$; $-C(=O)-NH_2$; $-C(=O)-NH-R^{30}$; $-C(=O)-NR^{31}R^{32}$; $-S(=O)-R^{33}$; $-S(=O)_2-R^{34}$; $C_{1-6}$-alkyl, which is unsubstituted or substituted with optionally 1, 2, 3, 4 or 5 substituents mutually independently selected from the group comprising F, Cl, Br, I, $-NO_2$, -CN, -OH, -SH and $-NH_2$; $C_{3-7}$-cycloalkyl, $C_{5-6}$-cycloalkenyl, 5- to 7-membered heterocycloalkyl or 5- to 7-membered heterocycloalkenyl, which in each case can be bound via a $C_{1-3}$-alkylene, $C_{2-3}$-alkenylene or $C_{2-3}$-alkynylene group and/or is unsubstituted or is substituted with optionally 1, 2, 3, 4 or 5 substituents mutually independently selected from the group comprising F, Cl, Br, I, -CN, methyl, ethyl, n-propyl, isopropyl, n-butyl, 2-butyl, isobutyl, tert-butyl, -OH, oxo, thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH, $-S-CH_3$ and $-S-C_2H_5$; or denotes a residue selected from the group comprising phenyl, naphthyl, anthracenyl, thienyl, furyl, pyridinyl, thiazolyl, thiadiazolyl, oxazolyl, oxadiazolyl and isoxazolyl, which in each case can be bound via a $C_{1-3}$-alkylene, $C_{2-3}$-alkenylene or $C_{2-3}$-alkynylene group and/or is unsubstituted or substituted with optionally 1, 2, 3, 4 or 5 substituents mutually independently selected from the group comprising F, Cl, Br, I, -CN, methyl, ethyl, n-propyl, isopropyl, n-butyl, isobutyl, 2-butyl, tert-butyl, -OH, -SH, $-NH_2$, -C(=O)-OH, $-S-CH_3$, $-S-C_2H_5$, - $S(=O)-CH_3$, $-S(=O)_2-CH_3$, -S (=O)-$C_2H_5$, $-S(=O)_2-C_2H_5$, $-O-CH_3$, $-O-C_2H_5$, $-0-C_3H_7$, $-CF_3$, $-CHF_2$, $-CH_2F$ and $-O-CF_3$.
and $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{36}$,$R^{47}$ and $R^{48}$, mutually independently, in each case denote $C_{1-6}$-alkyl, which is unsubstituted or substituted with optionally 1, 2, 3, 4 or 5 substituents mutually independently selected from the group comprising F, Cl, Br, I, $-NO_2$, -CN, -OH, -SH and $-NH_2$; $C_{3-7}$-cycloalkyl, $C_{5-6}$-cycloalkenyl, 5- to 7-membered heterocycloalkyl or 5- to 7-membered heterocycloalkenyl, which can in each case be bound via a $C_{1-3}$-alkylene, $C_{2-3}$-alkenylene or $C_{2-3}$-alkynylene group and/or is unsubstituted or substituted with optionally 1, 2, 3, 4 or 5 substituents mutually independently selected from the group comprising F, Cl, Br, I, -CN, methyl, ethyl, n-propyl, isopropyl, n-butyl, 2-butyl, isobutyl, tert-butyl, -OH, oxo, thioxo, $-O-CH_3$, $-O-C_2H_5$, $-O-C_3H_7$, $-NH_2$, $-N(CH_3)_2$, - $N(C_2H_5)_2$, $-NH-CH_3$, $-NH-C_2H_5$, $-NO_2$, $-CF_3$, $-O-CF_3$, $-S-CF_3$, -SH, $-S-CH_3$ and - $S-C_2H_5$; or denote a residue selected from the group comprising phenyl, naphthyl, anthracenyl, pyrrolyl, indolyl, furanyl, benzo[b]furanyl, thiophenyl, benzo[b]thiophenyl, benzo[d]thiazolyl, pyrazolyl, imidazolyl, thiazolyl, thiadiazolyl, triazolyl, oxazolyl, oxadiazolyl, isoxazolyl, pyridinyl, pyridazinyl, pyrimidinyl, pyrazinyl, pyranyl, indazolyl, chinolinyl, isochinolinyl and chinazolinyl, which in each case can be bound via a $C_{1-3}$-alkylene, $C_{2-3}$-alkenylene or $C_{2-3}$-alkynylene group and/or is unsubstituted or substituted with optionally 1, 2, 3, 4 or 5 substituents mutually independently selected from the group comprising F, Cl, Br, I, -CN, methyl, ethyl, n-propyl, isopropyl, n-butyl, 2-butyl, isobutyl, tert-butyl, -OH, $-O-CH_3$, $-O-C_2H_5$, $-O-C_3H_7$, $-NH_2$, $-N(CH_3)_2$, - $N(C_2H_5)_2$, $-NH-CH_3$, $-NH-C_2H_5$, $-NO_2$, $-CF_3$, $-O-CF_3$, $-S-CF_3$, -SH, $-NH-S(=O)_2-CH_3$, -C(=O)-OH, $-C(=O)-CH_3$, $-C(=O)-C_2H_5$, $-C(=O)-N(CH_3)_2$, $-C(=O)-NH-CH_3$, $-NH-C(=O)-CH_3$, $-NH-C(=O)-C_2H_5$, $-C(=O)-O-CH_3$ and $-C(=O)-O-C_2H_5$;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

**4.** Compounds according to one or more of Claims 1 to 3, **characterised in that**
a, b and c, mutually independently, in each case denote 0 or 1, whereby the sum of a, b and c is equal to 1, 2 or 3;
A denotes a residue selected from the group comprising

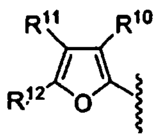 , 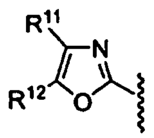 , 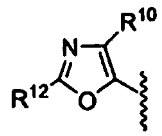 , 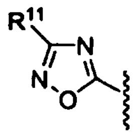 ,

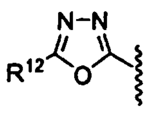 , 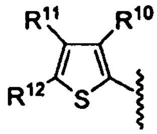 , 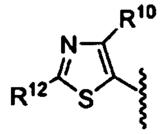 , 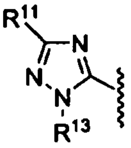 ,

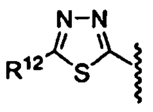 , 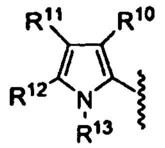 , 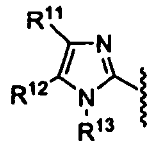 , 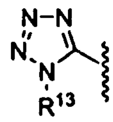 ,

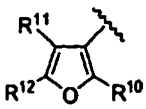 , 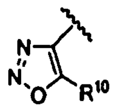 , 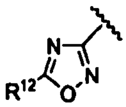 , 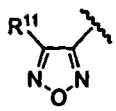 , 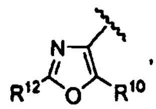 ,

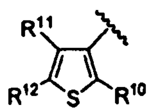 , 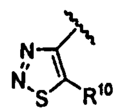 , 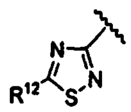 , 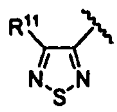 , 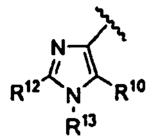 ,

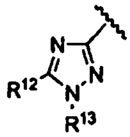 , 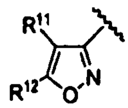 , 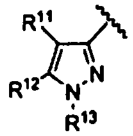 , 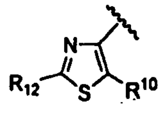 and 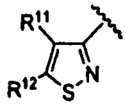 ;

$R^1$ and $R^8$, mutually independently, in each case denote H; -C(=O)-$R^{28}$; - C(=O)-O-$R^{29}$; -S(=O)-$R^{33}$; -S(=O)$_2$-$R^{34}$; an alkyl residue selected from the group comprising methyl, ethyl, n-propyl, isopropyl, n-butyl, 2-butyl, isobutyl, tert-butyl, n-pentyl and n-hexyl; a cycloalkyl residue selected from the group comprising cyclopropyl, cyclobutyl, cyclopentyl and cyclohexyl; or a benzyl or phenethyl residue which is unsubstituted or substituted with 1, 2, 3, 4 or 5 substituents mutually independently selected from the group comprising F, Cl, Br, methyl, ethyl, n-propyl, isopropyl, n-butyl, 2-butyl, isobutyl, tert-butyl, -OH, - O-$CH_3$, O-$C_2H_5$ and O-$C_3H_7$;

$R^2$, $R^3$, $R^4$, $R^5$, $R^6$, $R^7$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ and $R^{27}$, mutually independently, in each case denote H; F; Cl; Br; I; -$NH_2$; -OH; -SH; -CN; -$NO_2$; -$CF_3$; -NH-$R^{35}$; -$NR^{36}R^{37}$; -O-$R^{42}$; -S-$R^{43}$; an alkyl residue selected from the group comprising methyl, ethyl, n-propyl, isopropyl, n-butyl, 2-butyl, isobutyl, tert-butyl, n-pentyl and n-hexyl; or a residue selected from the group comprising cyclopropyl, cyclobutyl, cyclopentyl and cyclohexyl;

or$R^2$ and $R^3$ or $R^4$ and $R^5$ or $R^6$ and $R^7$ or $R^{14}$ and $R^{15}$ or $R^{16}$ and $R^{17}$ or $R^{18}$ and $R^{19}$ or $R^{20}$ and $R^{21}$ or $R^{22}$ and $R^{23}$ or $R^{24}$ and $R^{25}$ or $R^{26}$ and $R^{27}$, mutually independently, jointly in each case denote a residue selected from the group comprising an oxo group (=O) and a thioxo group (=S);

or $R^1$ and $R^8$ together with the -N-$CR^2R^3$-$(CR^4R^5)_b$-$(CR^6R^7)_c$ group joining them together form a residue selected from the group comprising

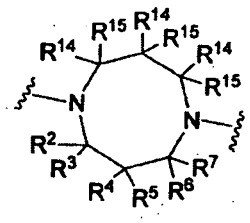,
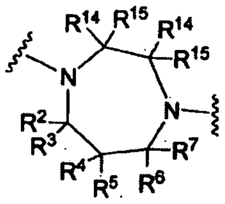,
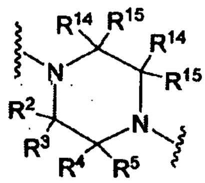

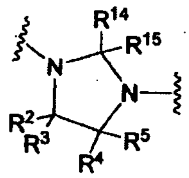 and 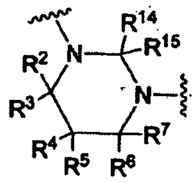

or $R^1$ and $R^2$ together with the -N-$CR^3$ group joining them together form a residue selected from the group comprising

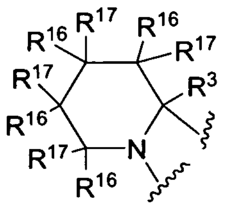,
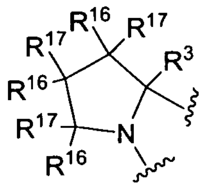 and
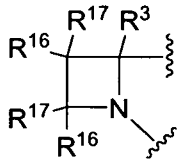

whereby, in this case, b denotes 1 and c denotes 0;
or $R^1$ and $R^4$ together with the -N-$CR^2R^3$-$CR^5$ group joining them together form a residue selected from the group comprising

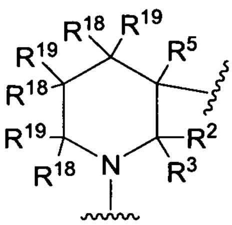,
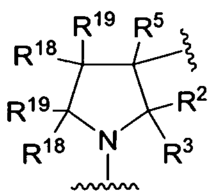 and
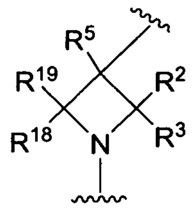

whereby, in this case, c denotes 0;
$R^1$ and $R^6$ together with the -N-$CR^2R^3$-$CR^4R^5$-$CR^7$ group joining them together form a residue selected from the group comprising

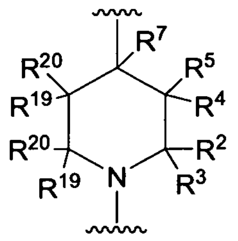

and

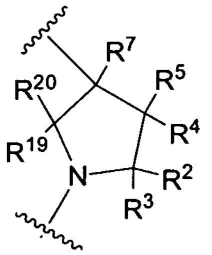

or $R^6$ and $R^8$ together with the -N-$CR^7$ group joining them together form a residue selected from the group comprising

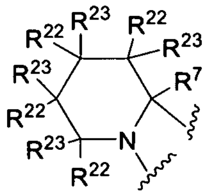

,

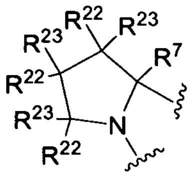

and

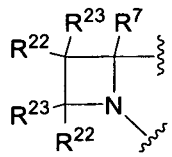

whereby, in this case, b denotes 1 and a denotes 0;
or $R^4$ and $R^8$ together with the -N-$CR^6R^7$-$CR^5$ group joining them together form a residue selected from the group comprising

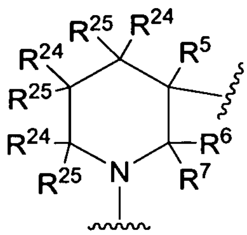

,

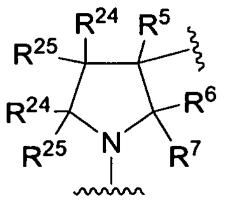

and

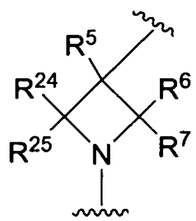

whereby, in this case, a denotes 0;
or $R^2$ and $R^8$ together with the -N-$CR^6R^7$-$CR^4R^5$-$CR^3$ group joining them together form a residue selected from the group comprising

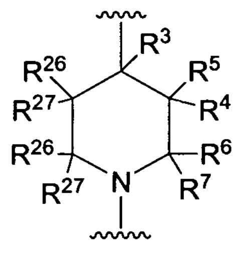

and

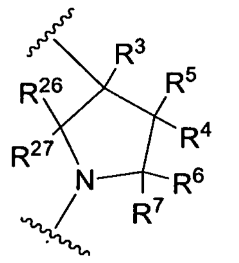

$R^9$ denotes a residue selected from the group comprising phenyl, furyl, thienyl, pyrazolyl, pyrazinyl, pyridazinyl, pyrimidinyl, pyridinyl, pyrrolyl, oxazolyl, isoxazolyl, thiazolyl, thiadiazolyl, oxadiazolyl, triazolyl and imidazolyl, which is in each case unsubstituted or substituted with optionally 1, 2, 3, 4 or 5 substituents mutually independently selected from the group comprising F, Cl, Br, I, -CN, methyl, ethyl, n-propyl, isopropyl, n-butyl, 2-butyl, isobutyl, tert-butyl, ethenyl, allyl, ethynyl, propynyl, cyclopropyl, cyclobutyl, -C≡C-Si$(CH_3)_3$, -C=C-Si$(C_2H_5)_3$, -$CH_2$-O-$CH_3$, -$CH_2$-O-$C_2H_5$, -OH, -O-$CH_3$, -O-$C_2H_5$, -O-$C_3H_7$, -S-$CH_3$, -S-$C_2H_5$, -S(=O)-$CH_3$, -S$(=O)_2$-$CH_3$, -S(=O)-$C_2H_5$, -S$(=O)_2$-$C_2H_5$,

$-NH_2$, $-N(CH_3)_2$, $-N(C_2H_5)_2$, $-NH-CH_3$, $-NH-C_2H_5$, $-NO_2$, $-CF_3$, $-CH_2F$, $-CHF_2$, $-O-CF_3$, $-S-CF_3$, -SH, $-NH-S(=O)_2-CH_3$, -C(=O)-OH, -C(=O)-H; $-C(=O)-CH_3$, $-C(=O)-C_2H_5$, $-C(=O)-NH_2$, $-C(=O)-N(CH_3)_2$, $-C(=O)-NH-CH_3$, $-NH-C(=O)-CH_3$, $-NH-C(=O)-C_2H_5$, $-C(=O)-O-CH_3$, $-C(=O)-O-C_2H_5$, $-C(=O)-O-C(CH_3)_3$ and phenyl;

$R^{10}$, $R^{11}$ and $R^{12}$, mutually independently, in each case denote H; F; Cl; Br; I; $-CF_3$; $-NO_2$; -CN; -C(=O)-OH; $-C(=O)-O-R^{29}$; $-C(=O)-NH_2$; $-C(=O)-NH-R^{30}$; $-C(=O)-NR^{31}R^{32}$; $-S(=O)-R^{33}$; $-S(=O)2-R^{34}$; $-O-R^{42}$; $-S-R^{43}$; an alkyl residue selected from the group comprising methyl, ethyl, n-propyl, isopropyl, n-butyl, 2-butyl, isobutyl, tert-butyl; a residue selected from the group comprising ethenyl, ethynyl, allyl and propynyl; a residue selected from the group comprising cyclopropyl, cyclobutyl and cyclopentyl; or a residue selected from the group comprising phenyl, benzyl, phenethyl, oxadiazolyl, 2-pyridyl, 3-pyridyl, 4-pyridyl, 2-thienyl, 3-thienyl, 2-furyl and 3-furyl, which in each case is unsubstituted or substituted with optionally 1, 2, 3, 4 or 5 substituents mutually independently selected from the group comprising F, Cl, Br, methyl, ethyl, n-propyl, isopropyl, n-butyl, 2-butyl, isobutyl, tert-butyl, -OH, $-O-CH_3$, $-O-C_2H_5$ and $-O-C_3H_7$;

$R^{13}$ denotes H; $-C(=O)-R^{28}$; -C(=O)-H; $-C(=O)-O-R^{29}$; $-S(=O)-R^{33}$; $-S(=O)_2-R^{34}$; an alkyl residue selected from the group comprising methyl, ethyl, n-propyl, isopropyl, n-butyl, 2-butyl, isobutyl and tert-butyl; a residue selected from the group comprising cyclopropyl, cyclobutyl, cyclopentyl and cyclohexyl; or a residue selected from the group comprising phenyl, benzyl, phenethyl, oxadiazolyl, 2-pyridyl, 3-pyridyl, 4-pyridyl, 2-thienyl, 3-thienyl, 2-furyl and 3-furyl, which in each case can be bound via a $C_{1-3}$-alkylene group and/or is unsubstituted or substituted with optionally 1, 2, 3, 4 or 5 substituents mutually independently selected from the group comprising F, Cl, Br, methyl, ethyl, n-propyl, isopropyl, n-butyl, 2-butyl, isobutyl, tert-butyl, -OH, $-O-CH_3$, $-O-C_2H_5$ and $-O-C_3H_7$;

and $R^{28}$, $R^{29}$, $R^{30}$, $R^{31}$, $R^{32}$, $R^{33}$, $R^{34}$, $R^{35}$, $R^{36}$, $R^{37}$, $R^{42}$ and $R^{43}$, mutually independently, in each case denote an alkyl residue selected from the group comprising methyl, ethyl, n-propyl, isopropyl, n-butyl, 2-butyl, isobutyl, tert-butyl, n-pentyl and n-hexyl; $-CF_3$; $-C_2F_5$; $CH_2-CF_3$; or a phenyl, benzyl or phenethyl residue which is unsubstituted or substituted with 1, 2, 3, 4 or 5 substituents mutually independently selected from the group comprising F, Cl, Br, methyl, ethyl, n-propyl, isopropyl, n-butyl, 2-butyl, isobutyl, tert-butyl, -OH, $-O-CH_3$, $-O-C_2H_5$ and $-O-C_3H_7$;

in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

**5.** Compounds according to one or more of Claims 1 to 4, **characterised in that**
a, b and c, mutually independently, in each case denote 0 or 1, whereby the sum of a, b and c is equal to 1, 2 or 3;
A denotes a residue selected from the group comprising

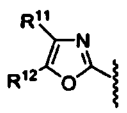 , 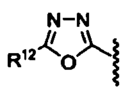 , 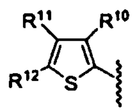 , 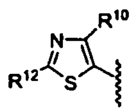 ,

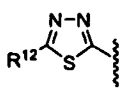 , 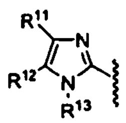 , 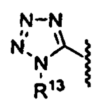 , 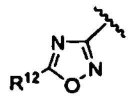 ,

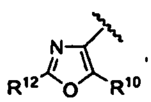 , 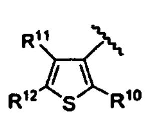 , 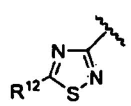 , 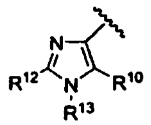 ,

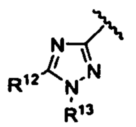 , 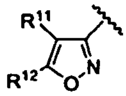 , 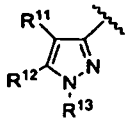 and 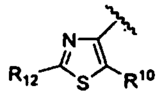 ;

$R^1$ and $R^8$, mutually independently, in each case denote H; -C(=O)-$R^{28}$; - C(=O)-O-$R^{29}$; an alkyl residue selected from the group comprising methyl, ethyl, n-propyl, isopropyl, n-butyl, 2-butyl, isobutyl, tert-butyl, n-pentyl and n-hexyl; or a cyclopropyl residue;

$R^2$, $R^3$, $R^4$, $R^5$, $R^6$, $R^7$, $R^{14}$, $R^{15}$, $R^{16}$, $R^{17}$, $R^{18}$, $R^{19}$, $R^{22}$, $R^{23}$, $R^{24}$ and $R^{25}$, mutually independently, in each case denote H; F; Cl; Br; I; $-NH_2$; -OH; -SH; - CN; $-NO_2$; $-CF_3$; -NH-$R^{35}$; -$NR^{36}R^{37}$; -O-$R^{42}$; -S-$R^{43}$; an alkyl residue selected from the group comprising methyl, ethyl, n-propyl, isopropyl, n-butyl, 2-butyl, isobutyl, tert-butyl, n-pentyl and n-hexyl; or a cyclopropyl residue;

or $R^1$ and $R^8$ together with the $-N-CR^2R^3-(CR^4R^5)_b-(CR^6R^7)_c$ group joining them together form a residue selected from the group comprising

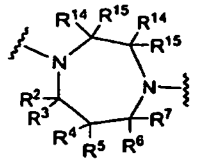 , 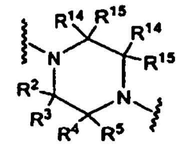 , 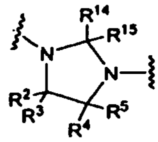 and 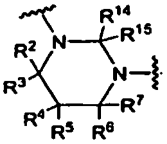

or $R^1$ and $R^2$ together with the $-N-CR^3$ group joining them together form a residue selected from the group comprising

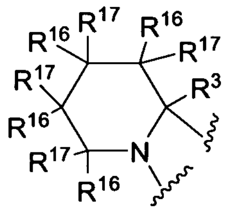 , 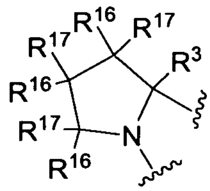 and 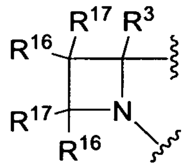

whereby, in this case, b denotes 1 and c denotes 0;

or $R^1$ and $R^4$ together with the $-N-CR^2R^3-CR^5$ group joining them together form a residue selected from the group comprising

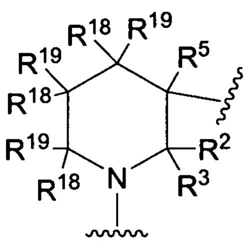 , 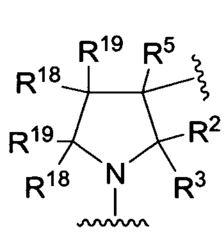 and 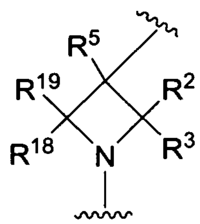

whereby, in this case, c denotes 0;

or $R^6$ and $R^8$ together with the $-N-CR^7$ group joining them together form a residue selected from the group comprising

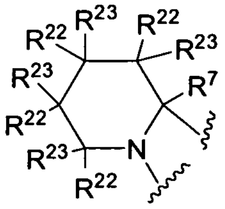 , 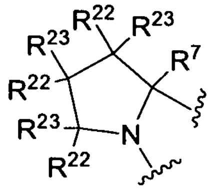 and 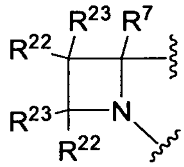

whereby, in this case, b denotes 1 and a denotes 0;
or $R^4$ and $R^8$ together with the $-N-CR^6R^7-CR^5$ group joining them together form a residue selected from the group comprising

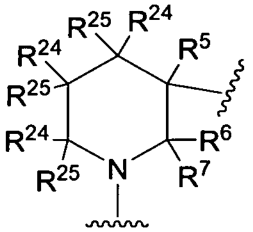 , 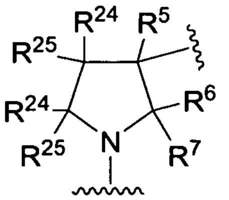 and 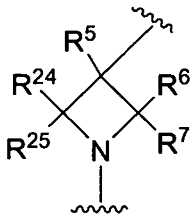

whereby, in this case, a denotes 0;
$R^9$ denotes a residue selected from the group comprising phenyl, thienyl, pyrazolyl, pyrazinyl, pyridazinyl, pyrimidinyl, pyridinyl, thiazolyl and thiadiazolyl, which in each case is unsubstituted or substituted with optionally 1, 2, 3, 4 or 5 substituents mutually independently selected from the group comprising F, Cl, Br, I, -CN, methyl, ethyl, n-propyl, isopropyl, n-butyl, 2-butyl, isobutyl, tert-butyl, ethenyl, allyl, ethynyl, propynyl, cyclopropyl, cyclobutyl, $-C\equiv C-Si(CH_3)_3$, $-C=C-Si(C_2H_5)_3$, $-CH_2-O-CH_3$, $-CH_2-O-C_2H_5$, -OH, $-O-CH_3$, $-O-C_2H_5$, $-O-C_3H_7$, $-S-CH_3$, $-S-C_2H_5$, $-S(=O)-CH_3$, $-S(=O)_2-CH_3$, $-S(=O)-C_2H_5$, $-S(=O)_2-C_2H_5$, $-NH_2$, $-N(CH_3)_2$, $-N(C_2H_5)_2$, $-NH-CH_3$, $-NH-C_2H_5$, $-NO_2$, $-CF_3$, $-CH_2F$, $-CHF_2$, $-O-CF_3$, $-S-CF_3$, -SH, $-NH-S(=O)_2-CH_3$, -C(=O)-OH, -C(=O)-H; $-C(=O)-CH_3$, $-C(=O)-C_2H_5$, $-C(=O)-NH_2$, $-C(=O)-N(CH_3)_2$, $-C(=O)-NH-CH_3$, $-NH-C(=O)-CH_3$, $-NH-C(=O)-C_2H_5$, $-C(=O)-O-CH_3$, $-C(=O)-O-C_2H_5$, $-C(=O)-O-C(CH_3)_3$ and phenyl;
$R^{10}$, $R^{11}$ and $R^{12}$, mutually independently, in each case denote H; F; Cl; Br; I; $-CF_3$; $-NO_2$; -CN; -C(=O)-OH; $-C(=O)-O-R^{29}$; $-C(=O)-NH_2$; $-O-R^{42}$; $-S-R^{43}$; ethenyl; ethynyl; cyclopropyl or an an alkyl residue selected from the group comprising methyl, ethyl, n-propyl, isopropyl, n-butyl, 2-butyl, isobutyl and tert-butyl;
$R^{13}$ denotes H; $-C(=O)-R^{28}$; -C(=O)-H; $-C(=O)-O-R^{29}$; $-S(=O)-R^{33}$; $-S(=O)_2-R^{34}$; cyclopropyl; cyclobutyl; or an alkyl residue selected from the group comprising methyl, ethyl, n-propyl, isopropyl, n-butyl, 2-butyl, isobutyl and tert-butyl;
and $R^{28}$, $R^{29}$, $R^{33}$, $R^{34}$, $R^{35}$, $R^{36}$, $R^{37}$, $R^{42}$ and $R^{43}$, mutually independently, in each case denote an alkyl residue selected from the group comprising methyl, ethyl, n-propyl, isopropyl, n-butyl, 2-butyl, isobutyl, tert-butyl, n-pentyl and n-hexyl; $-CF_3$; $-C_2F_5$; $-CH_2-CF_3$; or a phenyl, benzyl or phenethyl residue, which is unsubstituted or substituted with 1, 2, 3, 4 or 5 substituents mutually independently selected from the group comprising F, Cl, Br, methyl, ethyl, n-propyl, isopropyl, n-butyl, 2-butyl, isobutyl, tert-butyl, -OH, $-O-CH_3$, $-O-C_2H_5$ and $-O-C_3H_7$;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

6. Compounds according to one or more of Claims 1 to 5, **characterised in that**
a, b and c, mutually independently, in each case denote 0 or 1, whereby the sum of a, b and c is equal to 1 or 2;
A denotes a residue selected from the group comprising

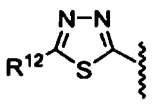 , 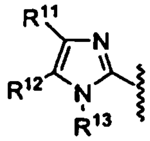 , 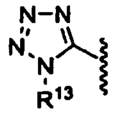 , 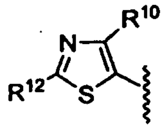 ,

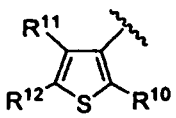 and 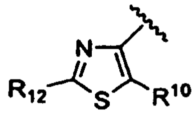 ;

$R^1$ and $R^8$, mutually independently, in each case denote H; -C(=O)-$R^{28}$; an alkyl residue selected from the group comprising methyl, ethyl, n-propyl, isopropyl, n-butyl, 2-butyl, isobutyl, tert-butyl, n-pentyl and n-hexyl; or a cyclopropyl residue;

$R^2$, $R^3$, $R^4$, $R^5$, $R^6$, $R^7$, $R^{14}$, $R^{15}$, $R^{16}$, $R^{17}$, $R^{22}$, $R^{23}$, $R^{24}$ and $R^{25}$, mutually independently, in each case denote H; F; Cl; Br; I; -CN; -$NO_2$; -$CF_3$; -O-$R^{42}$; an alkyl residue selected from the group comprising methyl, ethyl, n-propyl, isopropyl, n-butyl, 2-butyl, isobutyl, tert-butyl, n-pentyl and n-hexyl or a cyclopropyl residue;

or $R^1$ and $R^8$ together with the -N-C$R^2R^3$-C$R^4R^5$ group joining them together form the following residue

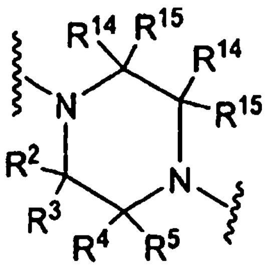

or $R^1$ and $R^2$ together with the -N-C$R^3$ group joining them together form a residue selected from the group comprising

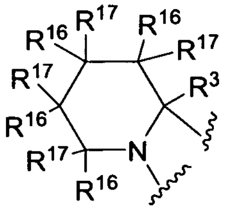 , 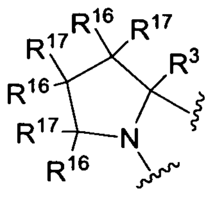 and 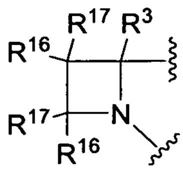

whereby, in this case, b denotes 1 and c denotes 0;

or $R^6$ and $R^8$ together with the -N-C$R^7$ group joining them together form a residue selected from the group comprising

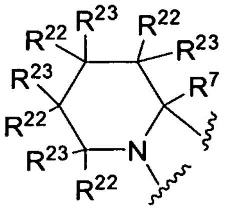 , 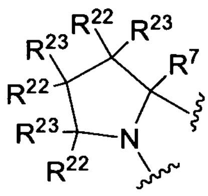 and 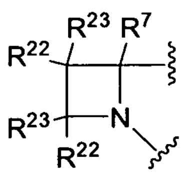

whereby, in this case, b denotes 1 and a denotes 0;
$R^9$ denotes a residue selected from the group comprising phenyl and thienyl, which in each case is unsubstituted or substituted with optionally 1 or 2 substituents mutually independently selected from the group comprising F, Cl, Br, I, -CN, methyl, ethyl, n-propyl, isopropyl, n-butyl, 2-butyl, isobutyl, tert-butyl, ethenyl, ethynyl, cyclopropyl, O-$CH_3$, -O-$C_2H_5$, -O-$C_3H_7$, -$NO_2$, -$CF_3$, and O-$CF_3$;
$R^{10}$, $R^{11}$ and $R^{12}$, mutually independently, in each case denote H; F; Cl; Br; - $CF_3$; -$NO_2$; -CN; -O-$R^{42}$; an alkyl residue selected from the group comprising methyl, ethyl, n-propyl, isopropyl, isobutyl and tert-butyl; ethenyl; ethynyl or cyclopropyl;
$R^{13}$ denotes H; -C(=O)-H; -C(=O)-$R^{28}$; cyclopropyl; or an alkyl residue selected from the group comprising methyl, ethyl, n-propyl, isopropyl, n-butyl, 2-butyl, isobutyl and tert-butyl;
and $R^{28}$ and $R^{42}$, mutually independently, in each case denote an alkyl residue selected from the group comprising methyl, ethyl, n-propyl, isopropyl, n-butyl, 2-butyl, isobutyl and tert-butyl; or -$CF_3$;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

7. Compounds according to one or more of Claims 1 to 6, **characterised in that**
a, b and c, mutually independently, in each case denote 0 or 1, whereby the sum of a, b and c is equal to 2;
A denotes a residue selected from the group comprising

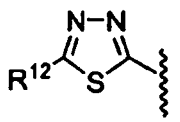 , 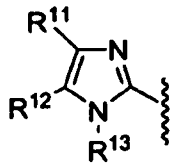 , 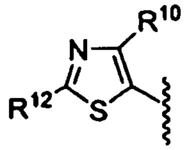 ,

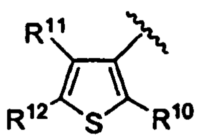 and 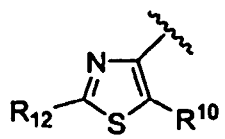 ;

$R^1$ and $R^8$, mutually independently, in each case denote H; -C(=O)-$CH_3$, methyl, isopropyl or cyclopropyl;
$R^2$, $R^3$, $R^4$, $R^5$, $R^6$, $R^7$, $R^{14}$, $R^{15}$, $R^{16}$, $R^{17}$, $R^{22}$ and $R^{23}$, mutually independently, in each case denote H or an an alkyl residue selected from the group comprising methyl, ethyl, n-propyl, isopropyl, n-butyl, 2-butyl, isobutyl, tert-butyl, n-pentyl and n-hexyl;
or $R^1$ and $R^8$ together with the -N-$CR^2R^3$-$CR^4R^5$ group joining them together form the following residue

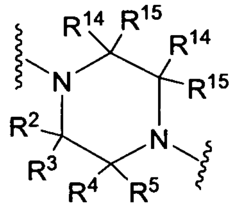

or $R^1$ and $R^2$ together with the -N-$CR^3$ group joining them together form the following residue

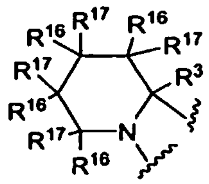

whereby, in this case, b denotes 1 and c denotes 0;
or $R^6$ and $R^8$ together with the -N-$CR^7$ group joining them together form the following residue

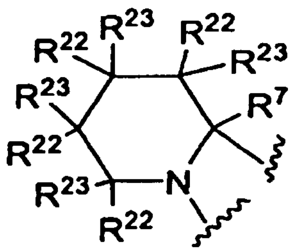

whereby, in this case, b denotes 1 and a denotes 0;
$R^9$ denotes a phenyl residue, which in each case is unsubstituted or substituted with 1 or 2 substituents mutually independently selected from the group comprising F, Cl, Br, I, -CN, methyl, ethyl, n-propyl, isopropyl, n-butyl, 2-butyl, isobutyl, tert-butyl, ethenyl, ethynyl, cyclopropyl, -O-$CH_3$, -O-$C_2H_5$, -0-$C_3H_7$,-$NO_2$, -$CF_3$ and -O-$CF_3$;
$R^{10}$, $R^{11}$ and $R^{12}$, mutually independently, in each case denote H; F; Cl; Br; - $CF_3$; -$NO_2$; -CN; -0- $CF_3$; an alkyl residue selected from the group comprising methyl, ethyl, n-propyl, isopropyl, isobutyl and tert-butyl; ethenyl; ethynyl or cyclopropyl;
$R^{13}$ denotes H or an alkyl residue selected from the group comprising methyl, ethyl, n-propyl, isopropyl, n-butyl, 2-butyl, isobutyl and tert-butyl;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

8. Compounds according to one or more of Claims 1 to 7, **characterised in that** a, b and c, mutually independently, in each case denote 0 or 1, whereby the sum of a, b and c is equal to 2;
A denotes a residue selected from the group comprising

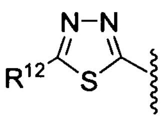 , 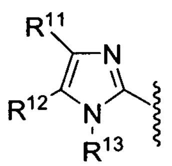 , 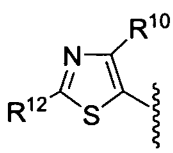 ,

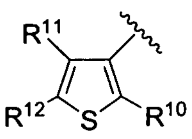 and 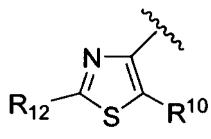

$R^1$ and $R^8$, mutually independently, in each case denote H, methyl or isopropyl;
$R^2$, $R^3$, $R^4$, $R^5$, $R^6$, $R^7$, $R^{14}$ and $R^{15}$, mutually independently, in each case denote H or an alkyl residue selected

from the group comprising methyl and ethyl;
or $R^1$ and $R^8$ together with the -N-$CR^2R^3$-$CR^4R^5$ group joining them together form the following residue

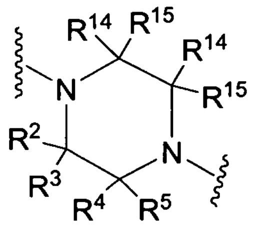

$R^9$ denotes a phenyl residue which in each case is unsubstituted or substituted with 1 or 2 substituents mutually independently selected from the group comprising F, Cl, Br, I and -CN;
$R^{10}$, $R^{11}$ and $R^{12}$, mutually independently, in each case denote H; or an alkyl residue selected from the group comprising methyl, ethyl, n-propyl, isopropyl, isobutyl and tert-butyl;
and $R^{13}$ denotes H or an alkyl residue selected from the group comprising methyl, ethyl, n-propyl, isopropyl, n-butyl, 2-butyl, isobutyl and tert-butyl;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

**9.** Compounds according to one or more of Claims 1 to 8 selected from the group comprising

[1] 1-(3-phenyl-propiolyl)-4-(thiophen-3-yl)-piperazine,
[2] 1-(3-phenyl-propiolyl)-4-(thiazol-4-yl)-piperazine,
[3] 1-(3-phenyl-propiolyl)-4-(1-methyl-imidazol-2-yl)-piperazine,
[4] (R)-2-methyl-1-(3-(3-chloro-phenyl)-propiolyl)-4-(thiazol-4-yl)-piperazine,
[5] 2-methyl-1-(3-(3-chloro-phenyl)-propiolyl)-4-(1,3,4-thiadiazol-2-yl)-piperazine,
[6] 3-(3-chlorophenyl)-N-(2-(methyl(1,3,4-thiadiazol-2-yl)amino)ethyl)propiolamide,
[7] 3-(3-chlorophenyl)-N-(2-(methyl(thiazol-5-yl)amino)ethyl)propiolamide and
[8] 3-(3-chlorophenyl)-N-(2-(methyl(1-methyl-1H-imidazol-2-yl)amino)ethyl)-propiolamide;

in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

**10.** Medicament containing at least one compound according to one or more of Claims 1 to 9 and optionally one or more physiologically acceptable auxiliary substances.

**11.** Compounds according to one or more of Claims 1 to 9 for the treatment and/or prevention of pain, preferably of pain selected from the group consisting of acute pain, chronic pain, neuropathic pain and visceral pain; migraine; depression; neurodegenerative diseases, preferably selected from the group consisting of multiple sclerosis, Alzheimer's disease, Parkinson's disease and Huntington's chorea; cognitive dysfunction, preferably cognitive deficiency states, particularly preferably Attention Deficit Disorder (ADD); anxiety states; panic attacks; epilepsy; coughing; urinary incontinence; diarrhoea; pruritus; schizophrenia; cerebral ischaemia; muscle spasms; cramps; lung illnesses, preferably selected from the group comprising asthma and pseudo-croup; regurgitation (vomiting); stroke; dyskinesia; retinopathy; listlessness; laryngitis; disorders of food intake, preferably selected from the group consisting of bulimia, cachexia, anorexia and obesity; dependency on alcohol; dependency on medicines; dependency on drugs, preferably dependency on nicotine and/or cocaine; alcohol abuse; abuse of medication; drug abuse; preferably nicotine and/or cocaine abuse; withdrawal symptoms associated with dependency on alcohol, medications and/or drugs (in particular nicotine and/or cocaine); development of tolerance to medications, preferably to natural or synthetic opioids; stomach-esophagus-reflux-syndrome; gastroesophagal reflux; irritable bowel syndrome; for diuresis; for antinatriuresis; for influencing the cardiovascular system; for increasing vigilance; for increasing libido; for modulating locomotor activity or for local anaesthesia.

**12.** Compounds according to Claim 11 for the treatment and/or prevention of pain, preferably of pain selected from the group consisting of acute pain, chronic pain, neuropathic pain and visceral pain; anxiety states; panic attacks; dependency on alcohol; dependency on medicines; cognitive dysfunction, preferably cognitive deficiency states, particularly preferably Attention Deficit Disorder (ADD); disorders of food intake, preferably selected from the group consisting of bulimia, cachexia, anorexia and obesity; dependency on drugs, preferably dependency on nicotine and/or cocaine; alcohol abuse; abuse of medication; drug abuse; preferably nicotine and/or cocaine abuse; withdrawal symptoms associated with dependency on alcohol, medications and/or drugs (in particular nicotine and/or cocaine); development of tolerance to medications and/or drugs, preferably to natural or synthetic opioids; stomach-esophagus-reflux-syndrome, gastroesophagal reflux and irritable bowel syndrome.

**13.** Compounds according to Claim 11 or 12 for the treatment of pain, preferably of pain selected from the group consisting of acute pain, chronic pain, neuropathic pain and visceral pain.

**14.** Compounds according to Claim 11 or 12 for the treatment of anxiety states or panic attacks.

**Revendications**

**1.** Propiolamides substitués de formule générale I,

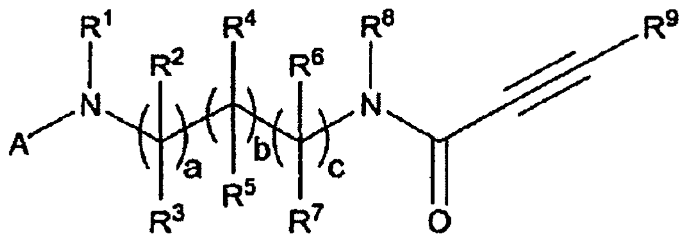

I,

dans laquelle
a, b et c représentent, chacun indépendamment, 0 ou 1, la somme de a, b et c étant égale à 1, 2 ou 3 ;
A représente l'un des radicaux suivants

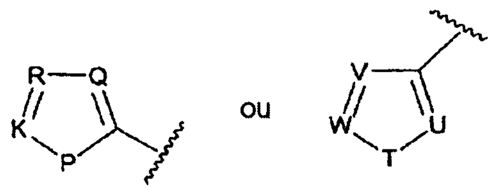

Q et U représentent chacun $CR^{10}$ ou N ;
R et V représentent chacun $CR^{11}$ ou N ;
K et W représentent chacun $CR^{12}$ ou N ;
P et T représentent chacun O, S ou $NR^{13}$ ;
étant entendu que sont exclus les composés dans lesquels P représente S, Q représente N, R représente $CR^{11}$ et K représente $CR^{12}$ ou N ;
$R^1$ et $R^8$, indépendamment l'un de l'autre, représentent chacun H ; $-C(=O)-R^{28}$ ; $-C(=O)-O-R^{29}$ ; $-C(=O)-NH_2$ ; $-C(=O)-NH-R^{30}$ ; $-C(=O)-NR^{31}R^{32}$ ; $-S(=O)-R^{33}$ ; $-S(=O)_2-R^{34}$ ; un groupe alkyle, alcényle ou alcynyle substitué ou non substitué ; hétéroalkyle, hétéroalcényle ou hétéroalcynyle substitué ou non substitué; cycloalkyle ou cycloalcényle substitué ou non substitué ; hétérocycloalkyle ou hétérocycloalcényle substitué ou non substitué ; -(alkylène)-cycloalkyle, -(alcénylène)-cycloalkyle, -(alcynylène)-cycloalkyle, -(alkylène)-cycloalcényle, -(alcénylène)-cycloalcényle ou -(alcynylène)-cycloalcényle substitué ou non substitué ; -(hétéroalkylène)-cycloalkyle, -(hétéroalcénylène)-cycloalkyle, -(hétéroalkylène)-cycloalcényle ou -(hétéroalcénylène)-cycloalcényle substitué ou non substitué ; -(alkylène)-hétérocycloalkyle, -(alcénylène)-hétérocycloalkyle, -(alcynylène)-hétérocycloalkyle, -(alkylène)-hétérocycloalcényle, -(alcénylène)-hétérocycloalcényle ou -(alcynylène)-hétérocycloalcényle substitué ou non substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle substitué ou non substitué ; aryle substitué ou non substitué ; hétéroaryle substitué ou non substitué ; -(alkylène)-aryle, -(alcénylène)-aryle, -(alcynylène)-aryle, -(hétéroalkylène)-aryle ou -(hétéroalcénylène)-aryle substitué ou non substitué ; ou -(alkylène)-hétéroaryle, -(alcénylène)-hétéroaryle, -(alcynylène)-hétéroaryle, -(hétéroalkylène)-hétéroaryle ou -(hétéroalcénylène)-hétéroaryle substitué ou non substitué ;

$R^2$, $R^3$, $R^4$, $R^5$, $R^6$, $R^7$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ et $R^{27}$, indépendamment les uns des autres, représentent chacun H ; F ; Cl ; Br ; I ; $-NO_2$ ; -CN ; $-NH_2$ ; -OH ; -SH ; -C(=O)-OH ; -C(=O)-H ; -NH-C(=O)-H ; $-C(=O)-R^{28}$ ; $-C(=O)-O-R^{29}$ ; $-C(=O)-NH_2$ ; $-C(=O)-NH-R^{30}$ ; $-C(=O)-NR^{31}R^{32}$ ; $-S(=O)-R^{33}$ ; $-S(=O)_2-R^{34}$ ; $-NH-R^{35}$ ; $-NR^{36}R^{37}$ ; $-O-C(=O)-R^{38}$ ; $-NH-C(=O)-R^{39}$ ; $-NR^{40}-C(=O)-R^{41}$ ; $-O-R^{42}$ ; $-S-R^{43}$ ; $-NH-C(=O)-NH-R^{44}$ ; -NH-C(=S)-NH-R ; $-NH-S(=O)_2-R^{46}$ ; $-NR^{47}-S(=O)_2-R^{48}$ ; un groupe alkyle, alcényle ou alcynyle non substitué ou au moins une fois substitué ; hétéroalkyle, hétéroalcényle ou hétéroalcynyle non substitué ou au moins une fois substitué ; cycloalkyle ou cycloalcényle non substitué ou au moins une fois substitué ; hétérocycloalkyle ou hétérocycloalcényle non substitué ou au moins une fois substitué ; -(alkylène)-cycloalkyle, -(alcénylène)-cycloalkyle, -(alcynylène)-cycloalkyle, -(alkylène)-cycloalcényle, -(alcénylène)-cycloalcényle ou -(alcynylène)-cycloalcényle non substitué ou au moins une fois substitué ; -(hétéroalkylène)-cycloalkyle, -(hétéroalcénylène)-cycloalkyle, -(hétéroalkylène)-cycloalcényle ou -(hétéroalcénylène)-cycloalcényle non substitué ou au moins une fois substitué ; -(alkylène)-hétérocycloalkyle, -(alcénylène)-hétérocycloalkyle, -(alcynylène)-hétérocycloalkyle, -(alkylène)-hétérocycloalcényle, -(alcénylène)-hétérocycloalcényle ou -(alcynylène)-hétérocycloalcényle non substitué ou au moins une fois substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle non substitué ou au moins une fois substitué ; aryle non substitué ou au moins une fois substitué ; hétéroaryle non substitué ou au moins une fois substitué; -(alkylène)-aryle, -(alcénylène)-aryle, -(alcynylène)-aryle, -(hétéroalkylène)-aryle ou- (hétéroalcénylène)-aryle non substitué ou au moins une fois substitué ; ou -(alkylène)-hétéroaryle, -(alcénylène)-hétéroaryle, -(alcynylène)-hétéroaryle, -(hétéroalkylène)-hétéroaryle ou -(hétéroalcénylène)-hétéroaryle non substitué ou au moins une fois substitué ;

ou $R^2$ et $R^3$ ou $R^4$ et $R^5$ ou $R^6$ et $R^7$ ou $R^{14}$ et $R^{15}$ ou $R^{16}$ et $R^{17}$ ou $R^{18}$ et $R^{19}$ ou $R^{20}$ et $R^{21}$ ou $R^{22}$ et $R^{23}$ ou $R^{24}$ et $R^{25}$ ou $R^{26}$ et $R^{27}$ indépendamment les uns des autres, représentent ensemble chaque fois un radical choisi dans l'ensemble constitué par un groupe oxo (=O) et un groupe thio (=S) ;

ou $R^1$ et $R^8$ forment ensemble avec le groupe $-N-CR^2R^3-(CR^4R^5)_b-(CR^6R^7)_c$ qui les relie un radical de formule B,

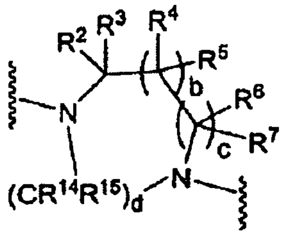

B ;

d représentant 1, 2 ou 3 et b représentant 0 ou 1 et c représentant 0 ou 1 ;

ou $R^1$ et $R^2$ forment ensemble avec le groupe $-N-CR^3$ qui les relie un radical de formule générale C,

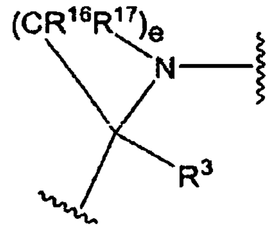

C ;

e représentant 1, 2, 3 ou 4 et, dans ce cas, b représentant 0 ou 1 et c représentant 0 ;
ou $R^1$ et $R^4$ forment ensemble avec le groupe -N-$CR^2R^3$-$CR^5$ qui les relie un radical de formule générale D,

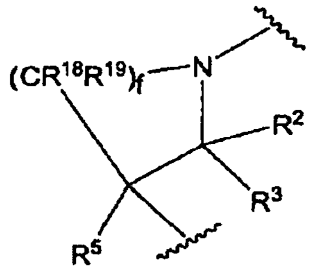

D ;

f représentant 1, 2, 3 ou 4, et, dans ce cas, c représentant 0 ;
ou $R^1$ et $R^6$ forment ensemble avec le groupe -N-$CR^2R^3$-$CR^4R^5$-$CR^7$ qui les relie un radical de formule générale E,

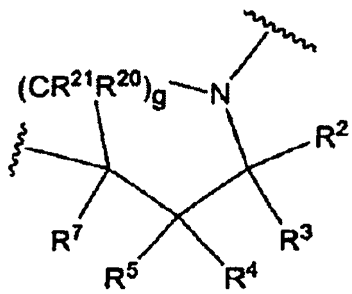

E ;

g représentant 1, 2 ou 3 ;
ou $R^6$ et $R^8$ forment ensemble avec le groupe -N-$CR^7$ qui les relie un radical de formule générale F

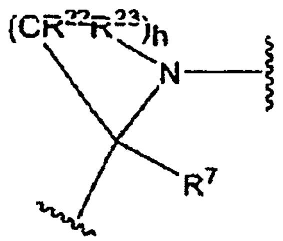

F ;

h représentant 1, 2, 3 ou 4 et, dans ce cas, b représentant 0 ou 1 et a représentant 0 ;
ou $R^4$ et $R^8$ forment ensemble avec le groupe -N-$CR^6R^7$-$CR^5$ qui les relie un radical de formule générale G,

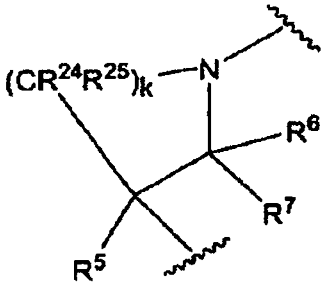

G ;

k représentant 1, 2, 3 ou 4, et, dans ce cas, a représentant 0 ;
ou $R^2$ et $R^8$ forment ensemble avec le groupe -N-$CR^6R^7$-$CR^4R^5$-$CR^3$ qui les relie un radical de formule générale H,

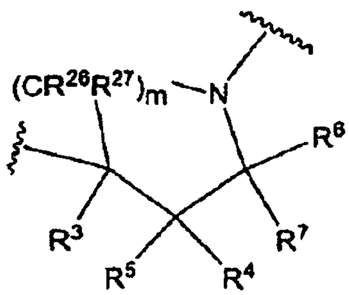

H ;

m représentant 1, 2 ou 3 ;
$R^9$ représente un groupe aryle substitué ou non substitué ou un groupe hétéroaryle substitué ou non substitué ;
$R^{10}$, $R^{11}$ et $R^{12}$ représentent chacun indépendamment H ; F ; Cl ; Br ; I ; -$NO_2$ ; -CN ; -$NH_2$ ; -OH ; -SH ; -C(=O)-OH ; -C(=O)-H ; -NH-C(=O)-H ; -C(=O)-$R^{28}$ ; -C(=O)-O-$R^{29}$; -C(=O)-$NH_2$ ; -C(=O)-NH-$R^{30}$ ; -C(=O)-$NR^{31}R^{32}$ ; -S (=O)-$R^{33}$ ; -S $(=O)_2$-$R^{34}$ ; -NH-$R^{35}$ -$NR^{36}R^{37}$ ; -O-C(=O)-$R^{38}$ ; -NH-C(=O)-$R^{39}$ ; -$NR^{40}$-C(=O)-$R^{41}$ ; -O-$R^{42}$ ; -S-$R^{43}$ -NH-C(=O)-NH-$R^{44}$ ; -NH-C(=S)-NH-$R^{45}$ ; -NH-$S(=O)_2$-$R^{46}$ ; -$NR^{47}$-$S(=O)_2$-$R^{48}$ ; un groupe alkyle, alcényle ou alcynyle non substitué ou au moins une fois substitué ; hétéroalkyle, hétéroalcényle ou hétéroalcynyle non substitué ou au moins une fois substitué ; cycloalkyle ou cycloalcényle non substitué ou au moins une fois substitué ; hétérocycloalkyle ou hétérocycloalcényle non substitué ou au moins une fois substitué ; -(alkylène)-cycloalkyle, -(alcénylène)-cycloalkyle, -(alcynylène)-cycloalkyle, -(alkylène)-cycloalcényle, -(alcénylène)-cycloalcényle ou -(alcynylène)-cycloalcényle non substitué ou au moins une fois substitué ; -(hétéroalkylène)-cycloalkyle, -(hétéroalcénylène)-cycloalkyle, -(hétéroalkylène)-cycloalcényle ou -(hétéroalcénylène)-cycloalcényle non substitué ou au moins une fois substitué ; -(alkylène)-hétérocycloalkyle, -(alcénylène)-hétérocycloalkyle, -(alcynylène)-hétérocycloalkyle, -(alkylène)-hétérocycloalcényle, -(alcénylène)-hétérocycloalcényle ou -(alcynylène)-hétérocycloalcényle non substitué ou au moins une fois substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle non substitué ou au moins une fois substitué ; aryle non substitué ou au moins une fois substitué ; hétéroaryle non substitué ou au moins une fois substitué ; -(alkylène)-aryle, -(alcénylène)-aryle,- (alcynylène)-aryle, -(hétéroalkylène)-aryle ou -(hétéroalcénylène)-aryle non substitué ou au moins une fois substitué ; ou -(alkylène)-hétéroaryle, -(alcénylène)-hétéroaryle, -(alcynylène)-hétéroaryle, -(hétéroalkylène)-hétéroaryle ou -(hétéroalcénylène)-hétéroaryle non substitué ou au moins une fois substitué ;

$R^{13}$ représente H ; -C(=O)-OH ; -C(=O)-H ; -C(=O)-$R^{28}$ ; -C(=O)-O-$R^{29}$ ; -C(=O)-$NH_2$ ; -C(=O)-NH-$R^{30}$ ; -C(=O)-$NR^{31}R^{32}$ ; -S(=O)-$R^{33}$ ; -S(=O)$_2$-$R_{34}$ ; un groupe alkyle, alcényle ou alcynyle non substitué ou au moins une fois substitué ; hétéroalkyle, hétéroalcényle ou hétéroalcynyle non substitué ou au moins une fois substitué ; cycloalkyle ou cycloalcényle non substitué ou au moins une fois substitué ; hétérocycloalkyle ou hétérocycloalcényle non substitué ou au moins une fois substitué ; -(alkylène)-cycloalkyle, -(alcénylène)-cycloalkyle, -(alcynylène)-cycloalkyle, -(alkylène)-cycloalcényle, -(alcénylène)-cycloalcényle ou -(alcynylène)-cycloalcényle non substitué ou au moins une fois substitué ; -(hétéroalkylène)-cycloalkyle, -(hétéroalcénylène)-cycloalkyle, -(hétéroalkylène)-cycloalcényle ou -(hétéroalcénylène)-cycloalcényle non substitué ou au moins une fois substitué ; -(alkylène)-hétérocycloalkyle, -(alcénylène)-hétérocycloalkyle, -(alcynylène)-hétérocycloalkyle, -(alkylène)-hétérocycloalcényle, -(alcénylène)-hétérocycloalcényle ou -(alcynylène)-hétérocycloalcényle non substitué ou au moins une fois substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle non substitué ou au moins une fois substitué ; aryle non substitué ou au moins une fois substitué ; hétéroaryle non substitué ou au moins une fois substitué ; -(alkylène)-aryle, -(alcénylène)-aryle, -(alcynylène)-aryle, -(hétéroalkylène)-aryle ou- (hétéroalcénylène)-aryle non substitué ou au moins une fois substitué ; ou -(alkylène)-hétéroaryle, -(alcénylène)-hétéroaryle, -(alcynylène)-hétéroaryle, -(hétéroalkylène)-hétéroaryle ou -(hétéroalcénylène)-hétéroaryle non substitué ou au moins une fois substitué ;

et $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$ et $R^{48}$, indépendamment les uns des autres, représentent chacun un groupe alkyle, alcényle ou alcynyle substitué ou non substitué ; hétéroalkyle, hétéroalcényle ou hétéroalcynyle substitué ou non substitué; cycloalkyle ou cycloalcényle substitué ou non substitué ; hétérocycloalkyle ou hétérocycloalcényle substitué ou non substitué ; -(alkylène)-cycloalkyle, -(alcénylène)-cycloalkyle, -(alcynylène)-cycloalkyle, -(alkylène)-cycloalcényle, -(alcénylène)-cycloalcényle ou -(alcynylène)-cycloalcényle substitué ou non substitué ; -(hétéroalkylène)-cycloalkyle, -(hétéroalcénylène)-cycloalkyle, -(hétéroalkylène)-cycloalcényle ou -(hétéroalcénylène)-cycloalcényle substitué ou non substitué ; -(alkylène)-hétérocycloalkyle, -(alcénylène)-hétérocycloalkyle, -(alcynylène)-hétérocycloalkyle, -(alkylène)-hétérocycloalcényle, -(alcénylène)-hétérocycloalcényle ou -(alcynylène)-hétérocycloalcényle substitué ou non substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle substitué ou non substitué ; aryle substitué ou non substitué ; hétéroaryle substitué ou non substitué ; -(alkylène)-aryle, -(alcénylène)-aryle, -(alcynylène)-aryle, -(hétéroalkylène)-aryle ou -(hétéroalcénylène)-aryle substitué ou non substitué ; ou -(alkylène)-hétéroaryle, -(alcénylène)-hétéroaryle, -(alcynylène)-hétéroaryle, -(hétéroalkylène)-hétéroaryle ou- (hétéroalcénylène)-hétéroaryle substitué ou non substitué ;

« substitué » en relation avec les radicaux « alkyle », « alcényle », « alcynyle », « hétéroalkyle », « hétéroalcényle » et « hétéroalcynyle » signifiant un radical correspondant qui porte un ou plusieurs substituants choisis, indépendamment les uns des autres, dans l'ensemble constitué par F, Cl, Br, I, -$NO_2$, -CN, -OH, -SH, -$NH_2$, -N[alkyle ($C_1$-$C_5$)]$_2$, -N[alkyle ($C_1$-$C_5$)](phényle), -N[alkyle ($C_1$-$C_5$)]($CH_2$-phényle), -N[alkyle ($C_1$-$C_5$)]($CH_2$-$CH_2$-phényle), -C(=O)-H, -C(=O)-alkyle($C_1$-$C_5$), -C(=O)-phényle, -C(=S)-alkyle($C_1$-$C_5$), -C(=S)-phényle, -C(=O)-OH, -C(=O)-O-alkyle ($C_1$-$C_5$) -C(=O)-O-phényle, -C(=O)-$NH_2$, -C(=O)-NH-alkyle($C_1$-$C_5$) -C(=O)-N[alkyle($C_1$-$C_5$)]$_2$, -S(=O)-alkyle($C_1$-$C_5$), -S(=O)-phényle, -S(=O)$_2$-alkyle($C_1$-$C_5$), -S(=O)$_2$-phényle, -S(=O)$_2$-$NH_2$ et -$SO_3H$, les radicaux alkyle en $C_1$-$C_5$ nommés précédemment pouvant être chacun linéaire ou ramifié et les radicaux phényle nommés précédemment pouvant porter 1, 2, 3, 4 ou 5 substituants choisis indépendamment les uns des autres dans l'ensemble constitué par F, Cl, Br, I, -CN, -$CF_3$, -OH, -$NH_2$, -O-$CF_3$, -SH, -O-$CH_3$, -O-$C_2H_5$, -O-$C_3H_7$, méthyle, éthyle, n-propyle, isopropyle, n-butyle, 2-butyle, isobutyle et tert-butyle,

« substitué » en relation avec les radicaux « cycloalkyle », « hétérocycloalkyle », « cycloalcényle » et « hétérocycloalcényle » signifiant un radical correspondant qui porte un ou plusieurs substituants choisis, indépendamment les uns des autres, dans l'ensemble constitué par F, Cl, Br, I, -CN, -$CF_3$, -OH, -$NH_2$, -O-$CF_3$, -SH, -O-alkyle ($C_1$-$C_5$) , -O-phényle, -O-$CH_2$-phényle, -($CH_2$)-O-alkyle($C_1$-$C_5$), -S-alkyle($C_1$-$C_5$), -S-phényle, -S-$CH_2$-phényle, -alkyle en $C_1$-$C_5$, -alcényle en $C_2$-$C_5$, -alcynyle en $C_2$-$C_5$,- C≡C-Si($CH_3$)$_3$, -C≡C-Si ($C_2H_5$)$_3$, -C(=O)-O-alkyle

$(C_1-C_5)$, -C(=O)-$CF_3$, -S(=O)$_2$-alkyle$(C_1-C_5)$, -S(=O)-alkyle$(C_1-C_5)$, -S(=O)2-phényle, oxo (=O), thioxo (=S), -N[alkyle$(C_1-C_5)_2$, -N(H)[alkyle$(C_1-C_5)$], -$NO_2$, -S-$CF_3$, -C(=O)-OH, -NH-S(=O)$_2$-alkyle$(C_1-C_5)$, -NH-C(=O)-alkyle $(C_1-C_5)$, -C(=O)-H, -C(=O)-alkyle$(C_1-C_5)$, -C(=O)-$NH_2$, -C(=O)-N[alkyle$(C_1-C_5)$]$_2$, -C=O)-N(H)[alkyle$(C_1-C_5)$] et phényle, les radicaux alkyle en $C_1$-$C_5$ nommés précédemment pouvant être chacun linéaire ou ramifié et les radicaux phényle pouvant dans chaque cas être non substitués ou porter 1, 2, 3, 4 ou 5 substituants choisis indépendamment les uns des autres dans l'ensemble constitué par F, Cl, Br, I, -CN, -$CF_3$, -OH, -$NH_2$, -O-$CF_3$, -SH, -O-alkyle$(C_1-C_5)$, -O-phényle, -O-$CH_2$-phényle, -($CH_2$)-O-alkyle$(C_1-C_5)$, -S-alkyle$(C_1-C_5)$, -S-phényle, -S-$CH_2$-phényle, -alkyle en $C_1$-$C_5$, -alcényle en $C_2$-$C_5$, -alcynyle en $C_2$-$C_5$, -C≡C-Si$(CH_3)_3$, -C≡C-Si$(C_2H_5)_3$, -C(=O)-O-alkyle $(C_1-C_5)$ et - C(=O)-$CF_3$, « substitué » en relation avec les radicaux « aryle » et « hétéroaryle » signifiant un radical correspondant qui porte un ou plusieurs substituants choisis, indépendamment les uns des autres, dans l'ensemble constitué par F, Cl, Br, I, -CN, -$NO_2$, -OH, -SH, -$NH_2$, -C(=O)-OH, -alkyle en $C_1$-$C_5$, -($CH_2$)-O-alkyle$(C_1-C_5)$, -alcényle en $C_2$-$C_5$, -alcynyle en $C_2$-$C_5$, -C≡C-Si$(CH_3)_3$, -C≡C-Si$(C_2H_5)_3$, -S-alkyle$(C_1-C_5)$, -S-phényle, -S-$CH_2$-phényle, -O-alkyle$(C_1-C_5)$, -O-phényle, -O-$CH_2$-phényle, -$CF_3$, -$CHF_2$, -$CH_2F$, -O-$CF_3$, -O-$CHF_2$, -O-$CH_2F$, -C(=O)-$CF_3$, -S-$CF_3$, -S-$CHF_2$, -S-$CH_2F$, -S(=O)$_2$-phényle, -S(=O)$_2$-alkyle$(C_1-C_5)$, -S(=O)-alkyle$(C_1-C_5)$, -NH-alkyle$(C_1-C_5)$, N[alkyle$(C_1-C_5)$]$_2$, -C(=O)-O-alkyle$(C_1-C_5)$, -C(=O)-O-phényle, -C(=O)-H; -C(=O)-alkyle$(C_1-C_5)$, -$CH_2$-O-C(=O)-phényle, -O-C(=O)-alkyle$(C_1-C_5)$, -O-C(=O)-phényle, -NH-S(=O)$_2$-alkyle$(C_1-C_5)$, -NH-C(=O)-alkyle$(C_1-C_5)$, -C(=O)-$NH_2$,- C(=O)-NH-alkyle$(C_1-C_5)$, -C(=O)-N[alkyle $(C_1-C_5)$]$_2$, -C(=O)-N[alkyle $(C_1-C_5)$ (phényle), -C(=O)-NH-phényle, cyclopropyle, cyclobutyle, cyclopentyle, cyclohexyle, pyrazolyle, phényle, furyle (furannyle), thiazolyle, thiadiazolyle, thiophényle (thiényle), benzyle et phenéthyle, les radicaux alkyle en $C_1$-$C_5$ nommés précédemment pouvant être chacun linéaire ou ramifié et les substituants cycliques ou les radicaux cycliques de ces substituants pouvant eux-mêmes porter éventuellement 1, 2, 3, 4 ou 5 substituants choisis, indépendamment les uns des autres, dans l'ensemble constitué par F, Cl, Br, I, -CN, -$NO_2$, -OH, -SH, -$NH_2$, -C(=O)-OH, -alkyle en $C_1$-$C_5$, -($CH_2$)-O-alkyle$(C_1-C_5)$, -alcényle en $C_2$-$C_5$, -alcynyle en $C_2$-$C_5$, -C≡C-Si$(CH_3)_3$, -C≡C-Si$(C_2H_5)_3$, -S-alkyle$(C_1-C_5)$, -S-phényle, -S-$CH_2$-phényle, -O-alkyle $(C_1-C_5)$, -O-phényle, -O-$CH_2$-phényle, -$CF_3$, -$CHF_2$, -$CH_2F$, -O-$CF_3$, -O-$CHF_2$, -O-$CH_2F$, -C(=O)-$CF_3$, -S-$CF_3$, -S-$CHF_2$ et -S-$CH_2F$,

« substitué » en relation avec les radicaux « alkylène », « alcénylène », « alcynylène », « hétéroalkylène » et « hétéroalcénylène » signifiant un radical correspondant qui porte un ou plusieurs substituants choisis, indépendamment les uns des autres, dans l'ensemble constitué par F, Cl, Br, I, -$NO_2$, -CN, -OH, -O-phényle, -O-$CH_2$-phényle, -SH, -S-phényle, -S-$CH_2$-phényle, -$NH_2$, -N[alkyle $(C_1-C_5)$]$_2$, -NH-phényle, -N[alkyle$(C_1-C_5)$](phényle), -N[alkyle $(C_1-C_5)$]($CH_2$-phényle), -N[alkyle $(C_1-C_5)$]($CH_2$-$CH_2$-phényle), -C(=O)-H, -C(=O)-alkyle$(C_1-C_5)$ , -C(=O)-phényle, -C(=S)-alkyle $(C_1-C_5)$, -C(=S)-phényle, -C(=O)-OH, -C(=O)-O-alkyle$(C_1-C_5)$, -C(=O)-O-phényle, -C(=O)-$NH_2$, -C(=O)-NH-alkyle$(C_1-C_5)$ , -C(=O)-N [alkyle $(C_1-C_5)$]$_2$, -S(=O)-alkyle$(C_1-C_5)$ , -S(=O)-phényle, -S(=O)$_2$-alkyle$(C_1-C_5)$, -S(=O)$_2$-phényle, -S(=O)$_2$-$NH_2$ et -$SO_3H$, les radicaux alkyle en $C_1$-$C_5$ nommés précédemment pouvant être chacun linéaire ou ramifié et les radicaux phényle nommés précédemment pouvant porter 1, 2, 3, 4 ou 5 substituants choisis indépendamment les uns des autres dans l'ensemble constitué par F, Cl, Br, I, -CN, -$NO_2$, -OH, -SH, -$NH_2$, -C(=O)-OH, -alkyle en $C_1$-$C_5$, -($CH_2$)-O-alkyle$(C_1-C_5)$, -alcényle en $C_2$-$C_5$, -alcynyle en $C_2$-$C_5$, -C≡C-Si$(CH_3)_3$, -C≡C-Si$(C_2H_5)_3$, -S-alkyle $(C_1-C_5)$, -S-phényle, -S-$CH_2$-phényle, -O-alkyle$(C_1-C_5)$, -O-phényle, -O-$CH_2$-phényle, -$CF_3$, -$CHF_2$, -$CH_2F$, -O-$CF_3$, -O-$CHF_2$, -O-$CH_2F$, -C(=O)-$CF_3$, -S-$CF_3$, -S-$CHF_2$ et -S-$CH_2F$,

chaque fois éventuellement sous forme d'un de leurs stéréoisomères purs, en particulier énantiomères ou diastéréoisomères, de leurs racémates ou sous forme d'un mélange de stéréoisomères, en particulier des énantiomères et/ou diastéréoisomères, en un rapport quelconque de mélange, ou chaque fois sous forme de sels correspondants, ou chaque fois sous forme de produits de solvatation correspondants.

**2.** Composés selon la revendication 1, **caractérisés en ce que**
a, b et c représentent, chacun indépendamment, 0 ou 1, la somme de a, b et c étant égale à 1, 2 ou 3 ;
A représente l'un des radicaux suivants

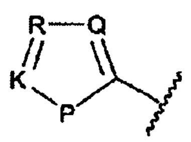
ou
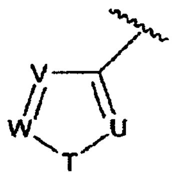
;

Q et U représentent chacun $CR^{10}$ ou N ;
R et V représentent chacun $CR^{11}$ ou N ;

K et W représentent chacun $CR^{12}$ ou N ;

P et T représentent chacun O, S ou $NR^{13}$ ;

étant entendu que sont exclus les composés dans lesquels P représente S, Q représente N, R représente $CR^{11}$ et K représente $CR^{12}$ ou N ;

$R^{11}$ et $R^8$, indépendamment l'un de l'autre, représentent chacun H ; -C(=O)-$R^{28}$ ; -C(=O)-O-$R^{29}$ ; -C(=O)-$NH_2$ ; -C(=O)-NH-$R^{30}$; -C(=O)-$NR^{31}R^{32}$ ; -S(=O)-$R^{33}$ ; -$S(=O)_2$-$R^{34}$; un groupe alkyle, alcényle ou alcynyle substitué ou non substitué ; hétéroalkyle, hétéroalcényle ou hétéroalcynyle substitué ou non substitué; cycloalkyle ou cycloalcényle substitué ou non substitué ; hétérocycloalkyle ou hétérocycloalcényle substitué ou non substitué ; -(alkylène)-cycloalkyle, -(alcénylène)-cycloalkyle, -(alcynylène)-cycloalkyle, -(alkylène)-cycloalcényle, -(alcénylène)-cycloalcényle ou -(alcynylène)-cycloalcényle substitué ou non substitué ; -(hétéroalkylène)-cycloalkyle, -(hétéroalcénylène)-cycloalkyle, -(hétéroalkylène)-cycloalcényle ou -(hétéroalcénylène)-cycloalcényle substitué ou non substitué ; -(alkylène)-hétérocycloalkyle, -(alcénylène)-hétérocycloalkyle, -(alcynylène)-hétérocycloalkyle, -(alkylène)-hétérocycloalcényle, -(alcénylène)-hétérocycloalcényle ou -(alcynylène)-hétérocycloalcényle substitué ou non substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle substitué ou non substitué ; aryle substitué ou non substitué ; hétéroaryle substitué ou non substitué ; -(alkylène)-aryle, -(alcénylène)-aryle, -(alcynylène)-aryle, -(hétéroalkylène)-aryle ou -(hétéroalcénylène)-aryle substitué ou non substitué ; ou -(alkylène)-hétéroaryle, -(alcénylène)-hétéroaryle, -(alcynylène)-hétéroaryle, -(hétéroalkylène)-hétéroaryle ou -(hétéroalcénylène)-hétéroaryle substitué ou non substitué ;

$R^2$, $R^3$, $R^4$, $R^5$, $R^6$, $R^7$, $R^{14}$, $R^{15}$,$R^{16}$, $R^{17}$, $R^{18}$, $R^{19}$, $R^{20}$, $R^{21}$, $R^{22}$, $R^{23}$, $R^{24}$, $R^{25}$, $R^{26}$ et $R^{27}$ indépendamment les uns des autres, représentent chacun H ; F ; Cl ; Br ; I ; -$NO_2$ ; -CN ; -$NH_2$ ; -OH ; -SH ; -C(=O)-OH ; -C(=O)-H ; -NH-C(=O)-H ; -C(=O)-$R^{28}$ ; -C(=O)-O-$R^{29}$ ; -C(=O)-$NH_2$ ; -C(=O)-NH-$R^{30}$ ; -C(=O)-$NR^{31}R^{32}$ ; -S(=O)-$R^{33}$ -$S(=O)_2$-$R^{34}$ ; -NH-$R^{35}$; -$NR^{36}R^{37}$ -O-C(=O)-$R^{38}$ -NH-C(=O)-R ; -$NR^{40}$-C(=O)-$R^{41}$ ; -O-$R^{42}$ ; -S-$R^{43}$ -NH-C(=O)-NH-$R^{44}$ ; -NH-C(=S)-NH-$R^{45}$ ; -NH-$S(=O)_2$-$R^{46}$ ; -$NR^{47}$-$S(=O)_2$-$R^{48}$ ; un groupe alkyle, alcényle ou alcynyle non substitué ou au moins une fois substitué ; hétéroalkyle, hétéroalcényle ou hétéroalcynyle non substitué ou au moins une fois substitué ; cycloalkyle ou cycloalcényle non substitué ou au moins une fois substitué ; hétérocycloalkyle ou hétérocycloalcényle non substitué ou au moins une fois substitué ; -(alkylène)-cycloalkyle, -(alcénylène)-cycloalkyle, -(alcynylène)-cycloalkyle, -(alkylène)-cycloalcényle, -(alcénylène)-cycloalcényle ou -(alcynylène)-cycloalcényle non substitué ou au moins une fois substitué ; -(hétéroalkylène)-cycloalkyle, -(hétéroalcénylène)-cycloalkyle, -(hétéroalkylène)-cycloalcényle ou -(hétéroalcénylène)-cycloalcényle non substitué ou au moins une fois substitué ; -(alkylène)-hétérocycloalkyle, -(alcénylène)-hétérocycloalkyle, -(alcynylène)-hétérocycloalkyle, -(alkylène)-hétérocycloalcényle, -(alcénylène)-hétérocycloalcényle ou -(alcynylène)-hétérocycloalcényle non substitué ou au moins une fois substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle non substitué ou au moins une fois substitué ; aryle non substitué ou au moins une fois substitué ; hétéroaryle non substitué ou au moins une fois substitué; -(alkylène)-aryle, -(alcénylène)-aryle, -(alcynylène)-aryle, -(hétéroalkylène)-aryle ou -(hétéroalcénylène)-aryle non substitué ou au moins une fois substitué ; ou -(alkylène)-hétéroaryle, -(alcénylène)-hétéroaryle, -(alcynylène)-hétéroaryle, -(hétéroalkylène)-hétéroaryle ou -(hétéroalcénylène)-hétéroaryle non substitué ou au moins une fois substitué ;

ou $R^2$ et $R^3$ ou $R^4$ et $R^5$ ou $R^6$ et $R^7$ ou $R^{14}$ et $R^{15}$ ou $R^{16}$ et $R^{17}$ ou $R^{18}$ et $R^{19}$ ou $R^{20}$ et $R^{21}$ ou $R^{22}$ et $R^{23}$ ou $R^{24}$ et $R^{25}$ ou $R^{26}$ et $R^{27}$, indépendamment les uns des autres, représentent ensemble chaque fois un radical choisi dans l'ensemble constitué par un groupe oxo (=O) et un groupe thio (=S) ;

ou $R^1$ et $R^8$ forment ensemble avec le groupe -N-$CR^2R^3$-$(CR^4R^5)_b$-$(CR^6R^7)_c$ qui les relie un radical de formule B,

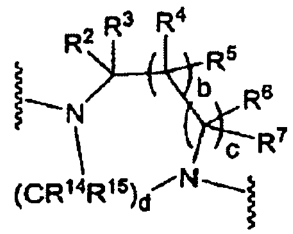

B ;

d représentant 1, 2 ou 3 et b représentant 0 ou 1 et c représentant 0 ou 1 ;

ou $R^1$ et $R^2$ forment ensemble avec le groupe -N-$CR^3$ qui les relie un radical de formule générale C,

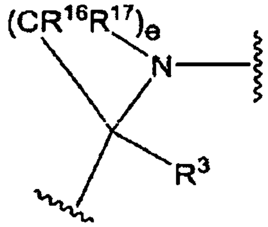

C ;

e représentant 1, 2, 3 ou 4 et, dans ce cas, b représentant 0 ou 1 et c représentant O ;
ou $R^1$ et $R^4$ forment ensemble avec le groupe $-N-CR^2R^3-CR^5$ qui les relie un radical de formule générale D,

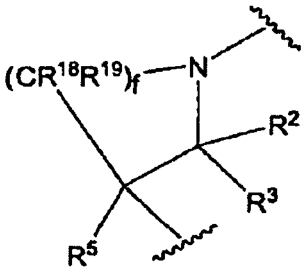

D ;

f représentant 1, 2, 3 ou 4, et, dans ce cas, c représentant 0 ;
ou $R^1$ et $R^6$ forment ensemble avec le groupe $-N-CR^2R^3-CR^4R^5-CR^7$ qui les relie un radical de formule générale E,

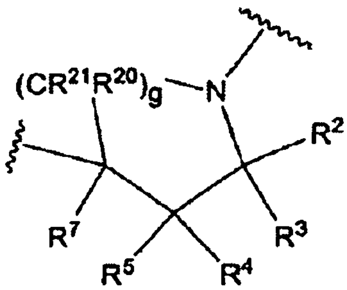

E ;

g représentant 1, 2 ou 3 ;
ou $R^6$ et $R^8$ forment ensemble avec le groupe $-N-CR^7$ qui les relie un radical de formule générale F

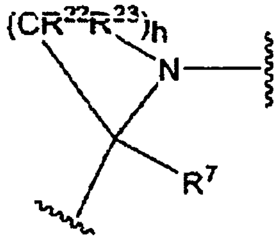

F ;

h représentant 1, 2, 3 ou 4 et, dans ce cas, b représentant 0 ou 1 et a représentant 0 ;

ou $R^4$ et $R^8$ forment ensemble avec le groupe -N-$CR^6R^7$-$CR^5$ qui les relie un radical de formule générale G,

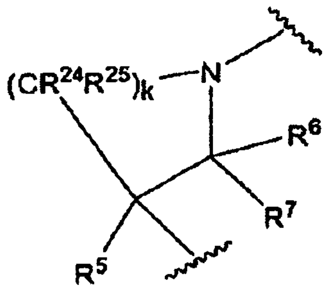

G ;

k représentant 1, 2, 3 ou 4, et, dans ce cas, a représentant 0 ;

ou $R^2$ et $R^8$ forment ensemble avec le groupe -N-$CR^6R^7$-$CR^4R^5$-$CR^3$ qui les relie un radical de formule générale H,

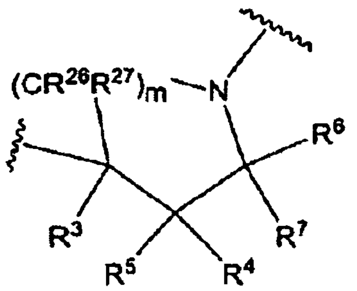

H ;

m représentant 1, 2 ou 3 ;

$R^9$ représente un groupe aryle substitué ou non substitué ou un groupe hétéroaryle substitué ou non substitué ;

$R^{10}$ $R^{11}$ et $R^{12}$ représentent chacun indépendamment H ; F ; Cl ; Br ; I ; $-NO_2$ ; -CN ; $-NH_2$ ; -OH ; -SH ; -C(=O)-OH ; -C(=O)-H; -NH-C(=O)-H ; -C(=O)-$R^{28}$ ; -C(=O)-O-$R^{29}$ ; -C(=O)-$NH_2$ ; -C(=O)-NH-$R^{30}$; -C(=O)-$NR^{31}R^{32}$ ; -S(=O)-$R^{33}$ ; -S(=O)$_2$-$R^{31}$ ; -NH-$R^{35}$ ; -$NR^{36}R^{37}$ ; -O-C(=O)-$R^{38}$; -NH-C(=O)-$R^{39}$; -$NR^{40}$-C(=O)-$R^{41}$ ; -O-$R^{42}$ ; -S-$R^{43}$ ; -NH-C(=O)-NH-$R^{44}$ ; -NH-C(=S)-NH-$R^{45}$ ; -NH-S(=O)$_2$-$R^{46}$ ; -NR-S(=O)$_2$-$R^{48}$ ; un groupe alkyle, alcényle ou alcynyle non substitué ou au moins une fois substitué ; hétéroalkyle, hétéroalcényle ou hétéroalcynyle non substitué ou au moins une fois substitué ; cycloalkyle ou cycloalcényle non substitué ou au moins une fois substitué ; hétérocycloalkyle ou hétérocycloalcényle non substitué ou au moins une fois substitué ; -(alkylène)-cycloalkyle, -(alcénylène)-cycloalkyle, -(alcynylène)-cycloalkyle, -(alkylène)-cycloalcényle, -(alcénylène)-cycloalcényle ou -(alcynylène)-cycloalcényle non substitué ou au moins une fois substitué ; -(hétéroalkylène)-cycloalkyle, -(hétéroalcénylène)-cycloalkyle, -(hétéroalkylène)-cycloalcényle ou -(hétéroalcénylène)-cycloalcényle non substitué ou au moins une fois substitué ; -(alkylène)-hétérocycloalkyle, -(alcénylène)-hétérocycloalkyle, -(alcynylène)-hétérocycloalkyle, -(alkylène)-hétérocycloalcényle, -(alcénylène)-hétérocycloalcényle ou -(alcynylène)-hétérocycoalcényle non substitué ou au moins une fois substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle non substitué ou au moins une fois substitué ; aryle non substitué ou au moins une fois substitué ; hétéroaryle non substitué ou au moins une fois substitué; -(alkylène)-aryle, -(alcénylène)-aryle, -(alcynylène)-aryle, -(hétéroalkylène)-aryle ou -(hétéroalcénylène)-aryle non substitué ou au moins une fois substitué ; ou -(alkylène)-hétéroaryle, -(alcénylène)-hétéroaryle, -(alcynylène)-hétéroaryle, -(hétéroalkylène)-hétéroaryle ou -(hétéroalcénylène)-hétéroaryle non substitué ou au moins une fois substitué ;

$R^{13}$ représente H ; -C(=O)-OH ; -C(=O)-H ; -C(=O)-$R^{28}$ ; -C(=O)-O-$R^{29}$ ; -C(=O)-$NH_2$ ; -C(=O)-NH-$R^{30}$ ; -C(=O)-$NR^{31}R^{32}$ ; -S(=O)-$R^{33}$ ; -S(=O)$_2$-$R^{34}$ ; un groupe alkyle, alcényle ou alcynyle non substitué ou au moins une fois substitué ; hétéroalkyle, hétéroalcényle ou hétéroalcynyle non substitué ou au moins une fois substitué ; cycloalkyle ou cycloalcényle non substitué ou au moins une fois substitué ; hétérocycloalkyle ou hétérocycloalcényle non substitué ou au moins une fois substitué ; -(alkylène)-cycloalkyle, -(alcénylène)-cycloalkyle, -(alcynylène)-cycloalkyle, -(alkylène)-cycloalcényle, -(alcénylène)-cycloalcényle ou -(alcynylène)-cycloalcényle non substitué ou au

moins une fois substitué ; -(hétéroalkylène)-cycloalkyle, -(hétéroalcénylène)-cycloalkyle, -(hétéroalkylène)-cycloalcényle ou -(hétéroalcénylène)-cycloalcényle non substitué ou au moins une fois substitué ; -(alkylène)-hétérocycloalkyle, -(alcénylène)-hétérocycloalkyle, -(alcynylène)-hétérocycloalkyle, -(alkylène)-hétérocycloalcényle, -(alcénylène)-hétérocycloalcényle ou -(alcynylène)-hétérocycloalcényle non substitué ou au moins une fois substitué ; -(hétéroalkylène)-hétérocycloalkyle, -(hétéroalcénylène)-hétérocycloalkyle, -(hétéroalkylène)-hétérocycloalcényle ou -(hétéroalcénylène)-hétérocycloalcényle non substitué ou au moins une fois substitué ; aryle non substitué ou au moins une fois substitué ; hétéroaryle non substitué ou au moins une fois substitué; -(alkylène)-aryle, -(alcénylène)-aryle, -(alcynylène)-aryle, -(hétéroalkylène)-aryle ou -(hétéroalcénylène)-aryle non substitué ou au moins une fois substitué ; ou -(alkylène)-hétéroaryle, -(alcénylène)-hétéroaryle, -(alcynylène)-hétéroaryle, -(hétéroalkylène)-hétéroaryle ou -(hétéroalcénylène)-hétéroaryle non substitué ou au moins une fois substitué ;

et $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$ et $R^{48}$, indépendamment les uns des autres, représentent chacun un groupe alkyle, alcényle ou alcynyle substitué ou non substitué ; hétéroalkyle, hétéroalcényle ou hétéroalcynyle substitué ou non substitué; cycloalkyle ou cycloalcényle substitué ou non substitué ; hétérocycloalkyle ou hétérocycloalcényle substitué ou non substitué ; -(alkylène)-cycloalkyle, -(alcénylène)-cycloalkyle, -(alcynylène)-cycloalkyle, -(alkylène)-cycloalcényle, -(alcénylène)-cycloalcényle ou -(alcynylène)-cycloalcényle substitué ou non substitué ; -(hétéroalkylène)-cycloalkyle, -(hétéroalcénylène)-cycloalkyle, -(hétéroalkylène)-cycloalcényle ou -(hétéroalcénylène)-cycloalcényle substitué ou non substitué ; -(alkylène)-hétérocycloalkyle, -(alcénylène)-hétérocycloalkyle, -(alcynylène)-hétérocycloalkyle, -(alkylène)-hétérocycloalcényle, -(alcénylène)-hétérocycloalcényle ou -(alcynylène)-hétérocycloalcényle substitué ou non substitué ; -(hétéroalkylène)-hétérocycloalkyle, -(hétéroalcénylène)-hétérocycloalkyle,- (hétéroalkylène)-hétérocycloalcényle ou -(hétéroalcénylène)-hétérocycloalcényle substitué ou non substitué ; aryle substitué ou non substitué ; hétéroaryle substitué ou non substitué; -(alkylène)-aryle, -(alcénylène)-aryle, -(alcynylène)-aryle, -(hétéroalkylène)-aryle ou -(hétéroalcénylène)-aryle substitué ou non substitué ; ou -(alkylène)-hétéroaryle, -(alcénylène)-hétéroaryle, -(alcynylène)-hétéroaryle, -(hétéroalkylène)-hétéroaryle ou -(hétéroalcénylène)-hétéroaryle substitué ou non substitué ;

les radicaux alkyle nommés précédemment étant chacun à chaîne droite ou ramifiée et comportant 1, 2, 3, 4, 5, 6, 7, 8, 9, 10, 11 ou 12 atomes de carbone en tant que chaînons ;

les radicaux alcényle nommés précédemment étant chacun à chaîne droite ou ramifiée et comportant 2, 3, 4, 5, 6, 7, 8, 9, 10, 11 ou 12 atomes de carbone en tant que chaînons ;

les radicaux alcynyle nommés précédemment étant chacun à chaîne droite ou ramifiée et comportant 2, 3, 4, 5, 6, 7, 8, 9, 10, 11 ou 12 atomes de carbone en tant que chaînons;

Les radicaux hétéroalkyle, radicaux hétéroalcényle et radicaux hétéroalcynyle nommés précédemment étant chacun à 2, 3, 4, 5, 6, 7, 8, 9, 10, 11 ou 12 chaînons ;

les radicaux hétéroalkyle, radicaux hétéroalcényle et radicaux hétéroalcynyle nommés précédemment comportant chacun éventuellement 1, 2 ou 3 hétéroatome(s), choisis chacun indépendamment dans l'ensemble constitué par les atomes d'oxygène, de soufre et d'azote, en tant que chaînons(s) ;

les radicaux alkyle, radicaux alcényle, radicaux alcynyle, radicaux hétéroalkyle, radicaux hétéroalcényle et radicaux hétéroalcynyle nommés précédemment pouvant éventuellement porter chacun 1, 2, 3, 4 ou 5 substituants choisis, indépendamment les uns des autres, dans l'ensemble constitué par F, Cl, Br, I, $-NO_2$, -CN, -OH, -SH, $-NH_2$, -N [alkyle($C_1$-$C_5$)]$_2$, -N[alkyle($C_1$-$C_5$)](phényle), -N[alkyle($C_1$-$C_5$)]($CH_2$-phényle), -N[alkyle ($C_1$-$C_5$)]($CH_2$-$CH_2$-phényle), -C(=O)-H, -C(=O)-alkyle($C_1$-$C_5$), -C(=O)-phényle, -C(=S)-alkyle($C_1$-$C_5$), -C(=S)-phényle, -C(=O)-OH, -C(=O)-O-alkyle($C_1$-$C_5$), -C(=O)-O-phényle, -C(=O)-$NH_2$, -C(=O)-NH-alkyle($C_1$-$C_5$), -C(=O)-N [alkyle ($C_1$-$C_5$)]$_2$, -S(=O)-alkyle ($C_1$-$C_5$), -S(=O)-phényle, -S=O)$_2$-alkyle($C_1$-$C_5$), -S(=O)$_2$-phényle, -S(=O)$_2$-$NH_2$ et $-SO_3H$, les radicaux phényle pouvant porter 1, 2, 3, 4 ou 5 substituants choisis, indépendamment les uns des autres, dans l'ensemble constitué par F, Cl, Br, I, -CN, $-CF_3$, -OH, $-NH_2$, $-O-CF_3$, -SH, $-O-CH_3$, $-O-C_2H_5$, $-O-C_3H_7$, méthyle, éthyle, n-Propyle, isopropyle, n-butyle, 2-butyle, isobutyle et tert-butyle ;

les radicaux cycloalkyle nommés précédemment comportant chacun 3, 4, 5, 6, 7, 8 ou 9 atomes de carbone en tant que chaînons formant le cycle;

les radicaux cycloalcényle nommés précédemment comportant chacun 3, 4, 5, 6, 7, 8 ou 9 atomes de carbone en tant que chaînons formant le cycle;

les radicaux hétérocycloalkyle nommés précédemment étant chacun à 3, 4, 5, 6, 7, 8 ou 9 chaînons;

les radicaux hétérocycloalcényle nommés précédemment étant chacun à 4, 5, 6, 7, 8 ou 9 chaînons;

les radicaux hétérocycloalkyle et radicaux hétérocycloalcényle nommés précédemment comportant éventuellement chacun 1, 2 ou 3 hétéroatome(s) choisis, chacun indépendamment, dans l'ensemble constitué par les atomes d'oxygène, de soufre et d'azote (NH), en tant que chaînon(s) formant le cycle ;

les radicaux cycloalkyle, hétérocycloalkyle, cycloalcényle ou hétérocycloalcényle nommés précédemment pouvant éventuellement porter chacun 1, 2, 3, 4 ou 5 substituants choisis, indépendamment les uns des autres, dans l'ensemble constitué par F, Cl, Br, I, -CN, $-CF_3$, -OH, $-NH_2$, $-O-CF_3$, -SH, -O-alkyle($C_1$-$C_5$), -O-phényle, -O-$CH_2$-phényle, -($CH_2$)-O-alkyle($C_1$-$C_5$), -S-alkyle($C_1$-$C_5$), -S-phényle, -S-$CH_2$-phényle, -alkyle en $C_1$-$C_5$, -alcényle en $C_2$-$C_5$, -al-

cynyle en $C_2$-$C_5$, -C≡C-Si($CH_3$)$_3$, -C≡C-Si($C_2H_5$)$_3$, -C(=O)-O-alkyle($C_1$-$C_5$), -C(=O)-$CF_3$, -S(=O)$_2$-alkyle($C_1$-$C_5$), -S (=O)-alkyle ($C_1$-$C_5$), -S(=O)$_2$-phényle, oxo (=O), thioxo (=S), -N[alkyle($C_1$-$C_5$)]$_2$, -N(H)[alkyle($C_1$-$C_5$)], -$NO_2$, -S-$CF_3$, -C(=O)-OH, -NH-C(=O)-alkyle ($C_1$-$C_5$), -C(=O)-H, -C(=O)-alkyle ($C_1$-$C_5$), -C(=O)-$NH_2$, -C(=O)-N[alkyle($C_1$-$C_5$)]$_2$, -C (=O)N(H)[alkyle($C_1$-$C_5$)] et phényle, les radicaux phényle pouvant chacun être non substitués ou porter 1, 2, 3, 4 ou 5 substituants choisis, indépendamment les uns des autres, dans l'ensemble constitué par F, Cl, Br, I, -CN, -$CF_3$, -OH, -$NH_2$, -O-$CF_3$, -SH, -O-alkyle($C_1$-$C_5$), -O-phényle, -O-$CH_2$-phényle,-(CH2)-O-alkyle($C_1$-$C_5$), -S-alkyle($C_1$-$C_5$), -S-phényle, -S-$CH_2$-phényle, -alkyle en $C_1$-$C_5$, -alcényle en $C_2$-$C_5$, -alcynyle en $C_2$-$C_5$, -C≡C-Si($CH_3$)$_3$, -C≡C-Si ($C_2H_5$)$_3$, -C(=O)-O-alkyle($C_1$-$C_5$) et -C(=O)-$CF_3$, les radicaux phényle nommés précédemment pouvant porter 1, 2, 3, 4 ou 5 substituants choisis, indépendamment les uns des autres, dans l'ensemble constitué par F, Cl, Br, I, -CN, -$CF_3$, -OH, -$NH_2$, -O-$CF_3$, -SH, -O-$CH_3$, -O-$C_2H_5$, -O-$C_3H_7$, méthyle, éthyle, n-propyle, isopropyle, n-butyle, 2-butyle, isobutyle et tert-butyle;

les radicaux alkylène nommés précédemment étant chacun à chaîne droite ou ramifiée et comportant 1, 2, 3, 4, 5, 6, 7, 8, 9, 10, 11 ou 12 atomes de carbone en tant que chaînons;

les radicaux alcénylène nommés précédemment étant chacun à chaîne droite ou ramifiée et comportant 2, 3, 4, 5, 6, 7, 8, 9, 10, 11 ou 12 atomes de carbone en tant que chaînons ;

les radicaux alcynylène nommés précédemment étant chacun à chaîne droite ou ramifiée et comportant 2, 3, 4, 5, 6, 7, 8, 9, 10, 11 ou 12 atomes de carbone en tant que chaînons;

les radicaux hétéroalkylène et hétéroalcénylène nommés précédemment étant chacun à 2, 3, 4, 5, 6, 7, 8, 9, 10, 11 ou 12 chaînons ;

les groupes hétéroalkylène et hétéroalcénylène nommés précédemment comportant chacun éventuellement 1, 2 ou 3 hétéroatome(s), choisis indépendamment les uns des autres dans l'ensemble constitué par les atomes d'oxygène, de soufre et d'azote (NH), en tant que chaînons(s) ;

le groupe alkylène, alcénylène, alcynylène, hétéroalkylène ou hétéroalcénylène nommé précédemment chaque fois étant non substitué ou portant éventuellement 1, 2, 3, 4 ou 5 substituants choisis, indépendamment les uns des autres, dans l'ensemble constitué par phényle, F, Cl, Br, I, -$NO_2$, -CN, -OH, -0-phényle, -O-$CH_2$-phényle, -SH, -S-phényle, -S-$CH_2$-phényle, -$NH_2$, -N[alkyle($C_1$-$C_5$)]$_2$, -NH-phényle, -N[alkyle($C_1$-$C_5$)](phényle),- N[alkyle($C_1$-$C_5$)] ($CH_2$-phényle), -N[alkyle($C_1$-$C_5$)]($CH_2$-$CH_2$-phényle), -C(=O)-H, -C(=O)-alkyle($C_1$-$C_5$), -C(=O)-phényle, -C(=S)-alkyle($C_1$-$C_5$), -C(=S)-phényle, -C(=O)-OH, -C(=O)-O-alkyle($C_1$-$C_5$), -C((=O)-O-phényle, -C(=O)-$NH_2$, -C(=O)-NH-alkyle($C_1$-$C_5$), -C(=O)-N[alkyle($C_1$-$C_5$)]$_2$, -S(=O)-alkyle($C_1$-$C_5$), -S(=O)-phényle, -S(=O)$_2$-alkyle($C_1$-$C_5$), -S(=O)$_2$-phényle, -S(=O)$_2$-$NH_2$ et -$SO_3H$, les radicaux phényle pouvant porter 1, 2, 3, 4 ou 5 substituants choisis indépendamment les uns des autres dans l'ensemble constitué par F, Cl, Br, I, -CN, -$NO_2$, -OH, -SH, -$NH_2$, -C(=O)-OH, -alkyle en $C_1$-$C_5$, -($CH_2$)-O-alkyle($C_1$-$C_5$), -alcényle en $C_2$-$C_5$, -alcynyle en $C_2$-$C_5$, -C≡C-Si($CH_3$)$_3$, -C≡C-Si($C_2H_5$)$_3$, -S-alkyle ($C_1$-$C_5$) -S-phényle, -S-$CH_2$-phényle, -O-alkyle($C_1$-$C_5$), -O-phényle, -O-$CH_2$-phényle, -$CF_3$, -$CHF_2$, -$CH_2F$, -O-$CF_3$, -O-$CHF_2$, -O-$CH_2F$, -C(=O)-$CF_3$, -S-$CF_3$, -S-$CHF_2$ et -S-$CH_2F$;

les radicaux aryle nommés précédemment étant mono- ou bicycliques et comportant 6, 10 ou 14 atomes de carbone ;

les radicaux hétéroaryle nommés précédemment étant mono-, bi- ou tricycliques et à 5, 6, 7, 8, 9, 10, 11, 12, 13 ou 14 chaînons;

les radicaux hétéroaryle à 5-14 chaînons nommés précédemment comportant éventuellement 1, 2, 3, 4 ou 5 hétéroatome(s) choisis, indépendamment les uns des autres, dans le groupe constitué par les atomes d'oxygène, de soufre ou d'azote (NH), en tant que chaînon(s) formant le cycle ;

les radicaux aryle et hétéroaryle nommés précédemment pouvant éventuellement porter chacun 1, 2, 3, 4 ou 5 substituants choisis, indépendamment les uns des autres, parmi F, Cl, Br, I, -CN, -$NO_2$, -OH, -SH, -$NH_2$,- C(=O)-OH, -alkyle en $C_1$-$C_5$, -($CH_2$)-O-alkyle($C_1$-$C_5$), -alcényle en $C_2$-$C_5$, -alcynyle en $C_2$-$C_5$, -C≡C-Si($CH_3$)$_3$, -C≡C-Si($C_2H_5$)$_3$, -S-alkyle($C_1$-$C_5$), -S-phényle, -S-$CH_2$-phényle, -O-alkyle($C_1$-$C_5$), -O-phényle, -O-$CH_2$-phényle, -$CF_3$, -$CHF_2$, -$CH_2F$, -O-$CF_3$, -O-$CHF_2$, -O-$CH_2F$, -C(=O)-$CF_3$, -S-$CF_3$, -S-$CHF_2$, -S-$CH_2F$, -S(=O)$_2$-phényle, -S(=O)$_2$-alkyle($C_1$-$C_5$), -S (=O)-alkyle($C_1$-$C_5$), -NH-alkyle($C_1$-$C_5$), N[alkyle($C_1$-$C_5$)]$_2$, -C(=O)-O-alkyle($C_1$-$C_5$), -C(=O)-O-phényle, -C(=O)-H; -C(=O)-alkyle($C_1$-$C_5$), -$CH_2$-O-C(=O)-phényle, -O-C(=O)-phényle, -O-C(=O)-alkyle($C_1$-$C_5$), -NH-C(=O)-alkyle ($C_1$-$C_5$), -C(=O)-$NH_2$, -C(=O)-NH-alkyle($C_1$-$C_5$), -C(=O)-N[alkyle($C_1$-$C_5$)]$_2$, -C(=O)-N[alkyle($C_1$-$C_5$)](phényle), -C (=O)-NH-phényle, cyclopropyle, cyclobutyle, cyclopentyle, cyclohexyle, pyrazolyle, phényle, furyle (furannyle), thiazolyle, thiadiazolyle, thiophényle (thiényle), benzyle et phenéthyle, les substituants cycliques ou les radicaux cycliques de ces substituants pouvant eux-mêmes porter éventuellement 1, 2, 3, 4 ou 5 substituants choisis, indépendamment les uns des autres, dans l'ensemble constitué par F, Cl, Br, I, -CN, -$NO_2$, -OH, -SH, -$NH_2$, -C(=O)-OH, -alkyle en $C_1$-$C_5$, -($CH_2$)-O-alkyle($C_1$-$C_5$), -alcényle en $C_2$-$C_5$, -alcynyle en $C_2$-$C_5$, -C=C-Si($CH_3$)$_3$, -C=C-Si($C_2H_5$)$_3$, -S-alkyle($C_1$-$C_5$), -S-phényle, -S-$CH_2$-phényle, -O-alkyle($C_1$-$C_5$), -O-phényle, -O-$CH_2$-phényle, -$CF_3$, -$CHF_2$, -$CH_2F$, -O-$CF_3$, -O-$CHF_2$, -O-$CH_2F$, -C(=O)-$CF_3$, -S-$CF_3$, -S-$CHF_2$ et -S-$CH_2F$;

chaque fois éventuellement sous forme d'un de leurs stéréoisomères purs, en particulier énantiomères ou diastéréoisomères, de leurs racémates ou sous forme d'un mélange de stéréoisomères, en particulier des énantiomères et/ou diastéréoisomères, en un rapport quelconque de mélange, ou chaque fois sous forme de sels correspondants,

ou chaque fois sous forme de produits de solvatation correspondants.

3. Composés selon la revendication 1 ou 2, **caractérisés en ce que** a, b et c représentent, chacun indépendamment, 0 ou 1, la somme de a, b et c étant égale à 1, 2 ou 3 ; A représente un radical choisi dans l'ensemble constitué par

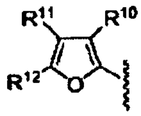 , 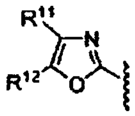 , 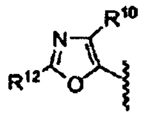 , 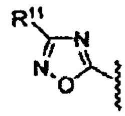 ,

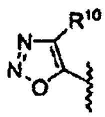 , 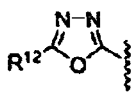 , 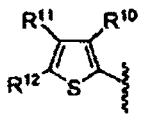 , 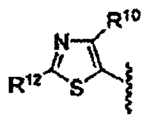 ,

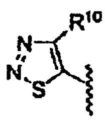 , 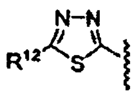 , 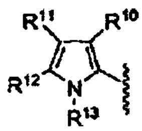 , 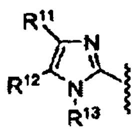 ,

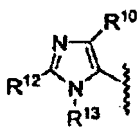 , 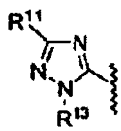 , 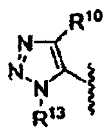 , 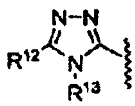 , 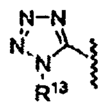 ,

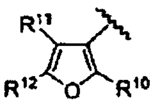 , 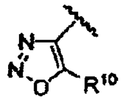 , 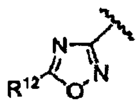 , 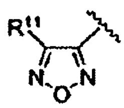 , 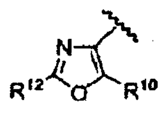 ,

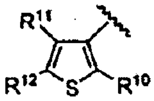 , 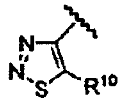 , 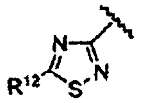 , 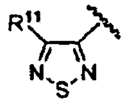 , 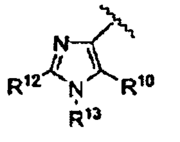 ,

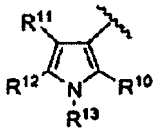 , 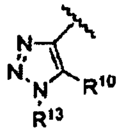 , 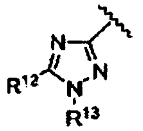 , 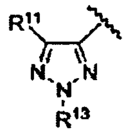 , 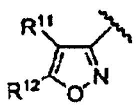 ,

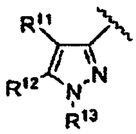 , 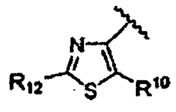 et 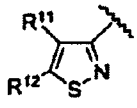 ;

$R^1$ et $R^8$, indépendamment l'un de l'autre, représentent chacun H ; $-C(=O)-R^{28}$; $-C(=O)-O-R^{29}$; $-C(=O)-NH_2$; $-C(=O)-NH-R^{30}$; $-C(=O)-NR^{31}R^{32}$; $-S(=O)-R^{33}$; $-S(=O)_2-R^{34}$; un groupe alkyle en $C_1$-$C_6$ qui est non substitué ou porte éventuellement 1, 2, 3, 4 ou 5 substituants choisis, indépendamment les uns des autres, dans l'ensemble constitué par F, Cl, Br, I, $-NO_2$, -CN, -OH, -SH et $-NH_2$;
un groupe cycloalkyle en $C_3$-$C_8$, qui est non substitué ou porte éventuellement 1, 2, 3, 4 ou 5 substituants choisis, indépendamment les uns des autres, dans l'ensemble constitué par F, Cl, Br, I, $-NO_2$, -CN, -OH, -SH et $-NH_2$ ;
ou représentent un radical phényle, qui dans chaque cas peut être lié par un groupe alkylène en $C_1$-$C_3$, alcénylène en $C_2$-$C_3$ ou alcynylène en $C_2$-$C_3$ et/ou est non substitué ou porte 1, 2, 3, 4 ou 5 substituants choisis, indépendamment les uns des autres, dans l'ensemble constitué par F, Cl, Br, I, -CN, méthyle, éthyle, n-propyle, isopropyle, n-butyle, 2-butyle, isobutyle, tert-butyle, -OH, $-O-CH_3$, $-O-C_2H_5$ et $-O-C_3H_7$ ;
$R^2$, $R^3$, $R^4$, $R^5$, $R^6$, $R^7$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ et $R^{27}$ indépendamment les uns des autres, représentent chacun H ; F; Cl; Br; I ; $-NO_2$; -CN; $-NH_2$; -OH; -SH; -C(=O)-OH; $-C(=O)-R^{28}$; $-C(=O)-O-R^{29}$; $-NH-R^{35}$; $-NR^{36}R^{37}$; $-O-R^{42}$; $-S-R^{43}$; un groupe alkyle en $C_1$-$C_6$, qui est non substitué ou porte éventuellement 1, 2, 3, 4 ou 5 substituants choisis, indépendamment les uns des autres, dans l'ensemble constitué par F, Cl, Br, I, $-NO_2$, -CN, -OH, -SH et $-NH_2$; un groupe cycloalkyle en $C_3$-$C_7$, cycloalcényle en $C_5$-$C_8$, hétérocycloalkyle à 5-7 chaînons ou hétérocycloalcényle à 5-7 chaînons, qui dans chaque cas peut être lié par un groupe alkylène en $C_1$-$C_3$, alcénylène en $C_2$-$C_3$ ou alcynylène en $C_2$-$C_3$ et/ou est non substitué ou porte éventuellement 1, 2, 3, 4 ou 5 substituants choisis, indépendamment les uns des autres, dans l'ensemble constitué par F, Cl, Br, I, - CN, méthyle, éthyle, n-propyle, isopropyle, n-butyle, 2-butyle, isobutyle, tert-butyle, -OH, oxo, thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH, $-S-CH_3$ et $-S-C_2H_5$; ou représente un radical choisi dans l'ensemble constitué par phényle, naphtyle, anthracényle, pyrrolyle, indolyle, furannyle, benzo[b]furannyle, thiophényle, benzo[b]thiophényle, benzo[d]thiazolyle, pyrazolyle, imidazolyle, thiazolyle, thiadiazolyle, triazolyle, oxazolyle, oxadiazolyle, isoxazolyle, pyridinyle, pyridazinyle, pyrimidinyle, pyrazinyle, pyrannyle, indazolyle, quinolinyle, isoquinolinyle et quinazolinyle, qui dans chaque cas peut être lié par un groupe alkylène en $C_1$-$C_3$, alcénylène en $C_2$-$C_3$ ou alcynylène en $C_2$-$C_3$ et/ou est non substitué ou porte éventuellement 1, 2, 3, 4 ou 5 substituants choisis, indépendamment les uns des autres, dans l'ensemble constitué par F, Cl, Br, I, -CN, méthyle, éthyle, n-propyle, isopropyle, n-butyle, 2-butyle, isobutyle, tert-butyle, -OH, $-O-CH_3$, $-O-C_2H_5$ et $-O-C_3H_7$ ;
ou $R^2$ et $R^3$ ou $R^4$ et $R^5$ ou $R^6$ et $R^7$ ou $R^{14}$ et $R^{15}$ ou $R^{16}$ et $R^{17}$ ou $R^{18}$ et $R^{19}$ ou $R^{20}$ et $R^{21}$ ou $R^{22}$ et $R^{23}$ ou $R^{24}$ et $R^{25}$ ou $R^{26}$ et $R^{27}$, indépendamment les uns des autres, forment ensemble chaque fois un radical choisi dans l'ensemble constitué par un groupe oxo (=O) et un groupe thioxo (=S);
ou $R^1$ et $R^8$ forment ensemble avec le groupe $-N-CR^2R^3-(CR^4R^5)_b-(CR^6R^7)_c$ qui les relie un radical choisi dans l'ensemble constitué par

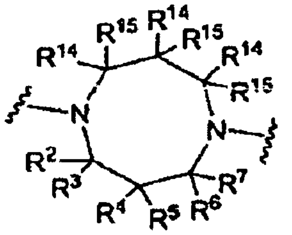 , 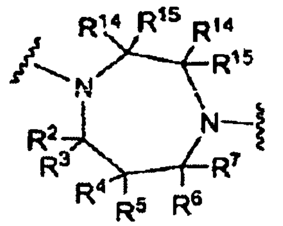 , 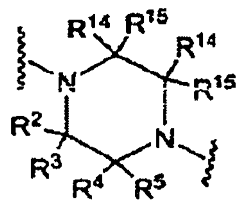 ,

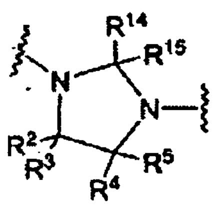 et 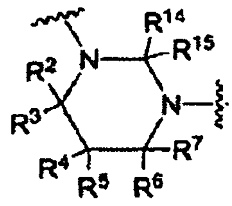 ;

ou $R^1$ et $R^2$ forment ensemble avec le groupe -N-$CR^3$ qui les relie un radical choisi dans l'ensemble constitué par

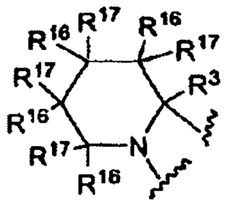 , 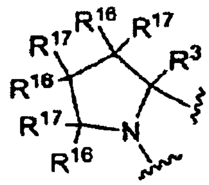 et 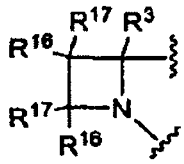 ,

en ce cas b représentant 1 et c représentant 0 ;
ou $R^1$ et $R^4$ forment ensemble avec le groupe -N-$CR^2R^3$-$CR^5$ qui les relie un radical choisi dans l'ensemble constitué par

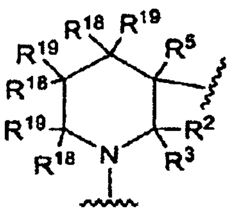 , 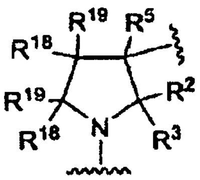 et 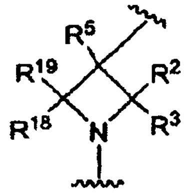 ,

en ce cas c représentant 0 ;
$R^1$ et $R^6$ forment ensemble avec le groupe -N-$CR^2R^3$-$CR^4R^5$-$CR^7$ qui les relie un radical choisi dans l'ensemble constitué par

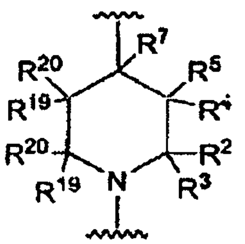 et 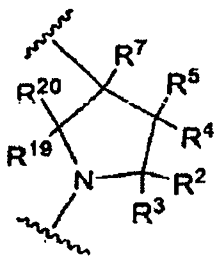 ;

ou $R^6$ et $R^8$ forment ensemble avec le groupe -N-$CR^7$ qui les relie un radical choisi dans l'ensemble constitué par

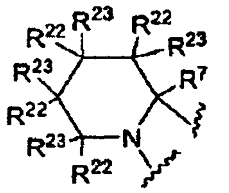 , 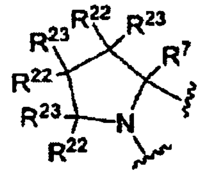 et 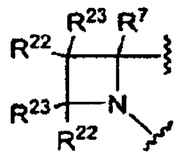 ,

en ce cas b représentant 1 et a représentant 0 ;

ou $R^4$ et $R^8$ forment ensemble avec le groupe $-N-CR^6R^7-CR^5$ qui les relie un radical choisi dans l'ensemble constitué par

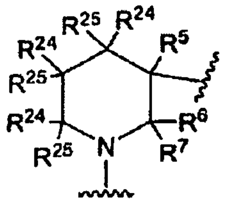 , 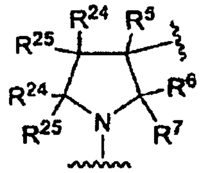 et 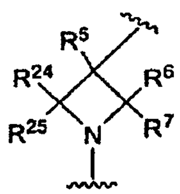 ,

en ce cas a représentant 0 ;

ou $R^2$ et $R^8$ forment ensemble avec le groupe $-N-CR^6R^7-CR^4R^5-CR^3$ qui les relie un radical choisi dans l'ensemble constitué par

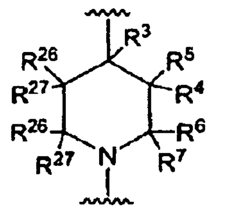 et 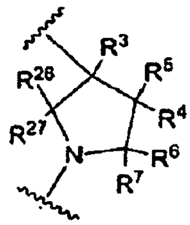 ;

$R^9$ représente un radical choisi dans l'ensemble constitué par les groupes phényle, naphtyle, anthracényle, furyle, thiényle, pyrazolyle, pyrazinyle, pyrimidinyle, pyridazinyle, pyridinyle, pyrrolyle, oxazolyle, isoxazolyle, thiazolyle, thiadiazolyle, oxadiazolyle, triazolyle, imidazolyle, indolyle, benzo[b]thiophényle, benzo[d]thiazolyle, benzo[b]furannyle, quinolinyle, isoquinolinyle et quinazolinyle, qui dans chaque cas est non substitué ou porte éventuellement 1, 2, 3, 4 ou 5 substituants choisis, indépendamment les uns des autres, dans l'ensemble constitué par F, Cl, Br, I, -CN, méthyle, éthyle, n-propyle, isopropyle, n-butyle, 2-butyle, isobutyle, tert-butyle, éthényle, allyle, éthynyle, propynyle, cyclopropyle, cyclobutyle, cyclopentyle, $-C{\equiv}C-Si(CH_3)_3$, $-C{\equiv}C-S(C_2H_5)_3$, $-CH_2-O-CH_3$, $-CH_2-O-C_2H_5$, -OH, $-O-CH_3$, $-O-C_2H_5$, $-O-C_3H_7$, $-S-CH_3$, $-S-C_2H_5$, $-S(=O)-CH_3$, $-S(=O)_2-CH_3$, $-S(=O)-C_2H_5$, $-S(=O)_2-C_2H_5$, $-NH_2$, $-N(CH_3)_2$, $-N(C_2H_5)_2$, $-NH-CH_3$, $-NH-C_2H_5$, $-NO_2$, $-CF_3$, $-CH_2F$, $-CHF_2$, $-O-CF_3$, $-S-CF_3$, -SH, $-NH-S(=O)_2-CH_3$, -C(=O)-OH, -C(=O)-H, $-C(=O)-CH_3$, $-C(=O)-C_2H_5$, $-C(=O)-NH_2$, $-C(=O)-N(CH_3)_2$, $-C(=O)-NH-CH_3$, $-NH-C(=O)-CH_3$, $-NH-C(=O)-C_2H_5$, $-C(=O)-O-CH_3$, $-C(=O)-O-C_2H_5$, $-C(=O)-O-C(CH_3)$ et phényle;

$R^{10}$ $R^{11}$ et $R^{12}$, chacun indépendamment, représentent H ; F ; Cl; Br; I; $-N_2$ ; -CN ; $-NH_2$; -OH; -SH; -C(=O)-OH; -C(=O)-H; -NH-C(=O)-H; $-C(=O)-R^{28}$; $-C(=O)-O-R^{29}$; $-C(=O)-NH_2$; $-C(=O)-NH-R^{30}$; $-C(=O)-NR^{31}R^{32}$; $-S(=O)-R^{33}$; $-S(=O)_2-R^{34}$; $-NH-R^{35}$; $-NR^{36}R^{37}$; $-O-C(=O)-R^{38}$; $-NH-C(=O)-R^{39}$; $-NR^{40}-C(=O)-R^{41}$; $-O-R^{42}$; $-S-R^{43}-NH-C(=O)-NH-R^{44}$;- $NH-C(=S)-NH-R^{45}$; $-NH-S(=O)_2-R^{46}$; $-NR^{47}-S(=O)_2-R^{48}$; un groupe alkyle en $C_1$-$C_6$, qui est non substitué ou porte éventuellement 1, 2, 3, 4 ou 5 substituants choisis, indépendamment les uns des autres, dans l'ensemble constitué par F, Cl, Br, I, $-NO_2$, -CN, -OH, -SH et $-NH_2$; un groupe alcényle en $C_2$-$C_6$ ; un groupe alcynyle en $C_2$-$C_6$ ; un groupe cycloalkyle en $C_3$-$C_7$, cycloalcényle en $C_5$-$C_6$, hétérocycloalkyle à 5-7 chaînons ou hétérocycloalcényle à 5-7 chaînons, qui dans chaque cas peut être lié par un groupe alkylène en $C_1$-$C_3$, alcénylène en $C_2$-$C_3$ ou alcynylène en $C_2$-$C_3$ et/ou est non substitué ou porte éventuellement 1, 2, 3, 4 ou 5 substituants choisis, indépendamment les uns des autres, dans l'ensemble constitué par F, Cl, Br, I, -CN, méthyle, éthyle, n-propyle, isopropyle, n-butyle, 2-butyle, isobutyle, tert-butyle, -OH, oxo, thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH, $-S-CH_3$ et $-S-C_2H_5$ ; ou représentent un radical choisi dans l'ensemble constitué par phényle, naphtyle, anthracényle, thiényle, furyle, pyridinyle, thiazolyle, thiadiazolyle, oxazolyle, oxadiazolyle et isoxazolyle, qui dans chaque cas peut être lié par un groupe alkylène en $C_1$-$C_3$, alcénylène en $C_2$-$C_3$ ou alcynylène en $C_2$-$C_3$ et/ou est non substitué ou porte éventuellement 1, 2, 3, 4 ou 5 substituants choisis, indépendamment les uns des autres, dans l'ensemble constitué par F, Cl, Br, I, -CN, méthyle, éthyle, n-propyle, isopropyle, n-butyle, isobutyle, 2-butyle, tert-butyle, -OH, -SH, $-NH_2$, -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-CF_3$, $-CHF_2$, $-CH_2F$ et $-O-CF_3$ ;

$R^{13}$ représente H; $-C(=O)-R^{28}$; -C(=O)-H; $-C(=O)-O-R^{29}$; $-C(=O)-NH_2$; $-C(=O)-NH-R^{30}$ ; $-C(=O)-NR^{31}R^{32}$; $-S(=O)-R^{33}$; $-S(=O)_2-R^{34}$; un groupe alkyle en $C_1$-$C_6$, qui est non substitué ou porte éventuellement 1, 2, 3, 4 ou 5 substituants choisis, indépendamment les uns des autres, dans l'ensemble constitué par F, Cl, Br, I, $-NO_2$, -CN, -OH, -SH et $-NH_2$; un groupe cycloalkyle en $C_3$-$C_7$, cycloalcényle en $C_5$-$C_6$, hétérocycloalkyle à 5-7 chaînons ou hétérocycloalcényle à 5-7 chaînons, qui dans chaque cas peut être lié par un groupe alkylène en $C_1$-$C_3$, alcénylène en $C_2$-$C_3$ ou alcynylène en $C_2$-$C_3$ et/ou est non substitué ou porte éventuellement 1, 2, 3, 4 ou 5 substituants choisis, indépendamment les uns des autres, dans l'ensemble constitué par F, Cl, Br, I, -CN, méthyle, éthyle, n-propyle, isopropyle, n-butyle, 2-butyle, isobutyle, tert-butyle, -OH, oxo, thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH, $-S-CH_3$ et $-S-C_2H_5$; ou représente un radical choisi dans l'ensemble constitué par phényle, naphtyle, anthracényle, thiényle, furyle, pyridinyle, thiazolyle, thiadiazolyle, oxazolyle, oxadiazolyle et isoxazolyle, qui dans chaque cas peut être lié par un groupe alkylène en $C_1$-$C_3$, alcénylène en $C_2$-$C_3$ ou alcynylène en $C_2$-$C_3$ et/ou est non substitué ou porte éventuellement 1, 2, 3, 4 ou 5 substituants choisis, indépendamment les uns des autres, dans l'ensemble constitué par F, Cl, Br, I, -CN, méthyle, éthyle, n-propyle, isopropyle, n-butyle, isobutyle, 2-butyle, tert-butyle, -OH, -SH, -NH2, -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-CF_3$, $-CHF_2$, $-CH_2F$ et $-O-CF_3$;

et $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$ et $R^{48}$, indépendamment les uns des autres, représentent chacun un groupe alkyle en $C_1$-$C_6$, qui est non substitué ou porte éventuellement 1, 2, 3, 4 ou 5 substituants choisis, indépendamment les uns des autres, dans l'ensemble constitué par F, Cl, Br, I, $-NO_2$, -CN, -OH, -SH et $-NH_2$; un groupe cycloalkyle en $C_3$-$C_7$, cycloalcényle en $C_5$-$C_6$, hétérocycloalkyle à 5-7 chaînons ou hétérocycloalcényle à 5-7 chaînons, qui dans chaque cas peut être lié par un groupe alkylène en $C_1$-$C_3$, alcénylène en $C_2$-$C_3$ ou alcynylène en $C_2$-$C_3$ et/ou est non substitué ou porte éventuellement 1, 2, 3, 4 ou 5 substituants choisis, indépendamment les uns des autres, dans l'ensemble constitué par F, Cl, Br, I, -CN, méthyle, éthyle, n-propyle, isopropyle, n-butyle, 2-butyle, isobutyle, tert-butyle, -OH, oxo, thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH, $-S-CH_3$ et $-S-C_2H_5$ ; ou représente un radical choisi dans l'ensemble constitué par phényle, naphtyle, anthracényle, pyrrolyle, indolyle, furannyle, benzo[b]furannyle, thiophényle, benzo[b]thiophényle, benzo[d]thiazolyle, pyrazolyle, imidazolyle, thiazolyle, thiadiazolyle, triazolyle, oxazolyle, oxadiazolyle, isoxazolyle, pyridinyle, pyridazinyle, pyrimidinyle, pyrazinyle, pyrannyle, indazolyle, quinolinyle, isoquinolinyle et quinazolinyle, qui dans chaque cas peut être lié par un groupe alkylène en $C_1$-$C_3$, alcénylène en $C_2$-$C_3$ ou alcynylène en $C_2$-$C_3$ et/ou est non substitué ou porte éventuellement 1, 2, 3, 4 ou 5 substituants choisis, indépendamment les uns des autres, dans l'ensemble constitué par F, Cl, Br, I, -CN, méthyle, éthyle, n-propyle, isopropyle, n-butyle, 2-butyle, isobutyle, tert-butyle, -OH, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH, $-NH-S(=O)_2-CH_3$, -C(=O)-OH, $-C(=O)-CH_3$, $-C(=O)-C_2H_5$, $-C(=O)-N(CH_3)_2$, $-C(=O)-NH-CH_3$, $-NH-C(=O)-CH_3$, $-NH-C(=O)-C_2H_5$, $-_C(=O)-O-CH_3$ et $-C(=O)-O-C_2H_5$;

chaque fois éventuellement sous forme d'un de leurs stéréoisomères purs, en particulier énantiomères ou diastéréoisomères, de leurs racémates ou sous forme d'un mélange de stéréoisomères, en particulier des énantiomères et/ou diastéréoisomères, en un rapport quelconque de mélange, ou chaque fois sous forme de sels correspondants, ou chaque fois sous forme de produits de solvatation correspondants.

4. Composés selon une ou plusieurs des revendications 1 à 3, **caractérisés en ce que**
a, b et c représentent, chacun indépendamment, 0 ou 1, la somme de a, b et c étant égale à 1, 2 ou 3;
A représente un radical choisi dans l'ensemble constitué par

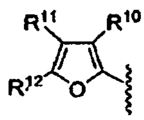 , 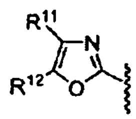 , 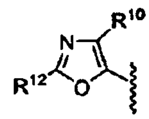 , 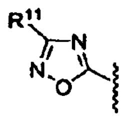 ,

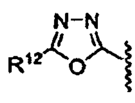 , 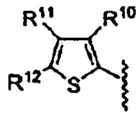 , 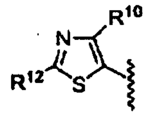 , 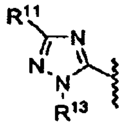 ,

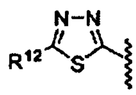 , 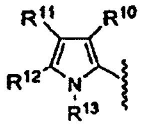 , 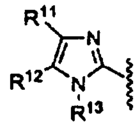 , 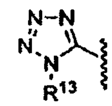 ,

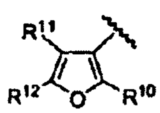 , 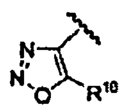 , 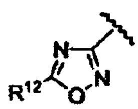 , 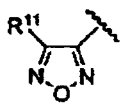 , 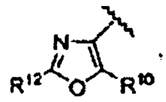 ,

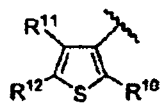 , 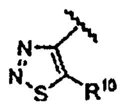 , 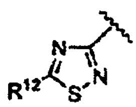 , 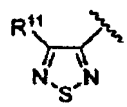 , 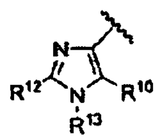 ,

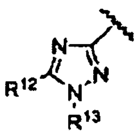 , 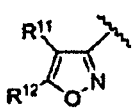 , 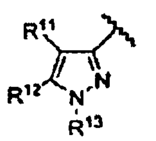 , 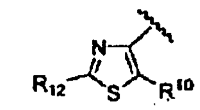 et 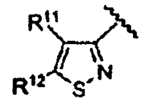 ;

$R^1$ et $R^8$ indépendamment l'un de l'autre, représentent chacun H ; -C(=O)-$R^{28}$; -C (=O)-O-$R^{29}$; -S(=O)-$R^{33}$; -S (=O)$_2$-$R^{34}$ ; un radical alkyle choisi dans l'ensemble constitué par le groupes méthyle, éthyle, n-propyle, isopropyle, n-butyle, 2-butyle, isobutyle, tert-butyle, n-pentyle et n-hexyle ; un radical cycloalkyle choisi dans l'ensemble constitué par les groupes cyclopropyle, cyclobutyle, cyclopentyle et cyclohexyle ; ou représentent un radical benzyle ou phénéthyle qui est non substitué ou porte 1, 2, 3, 4 ou 5 substituants choisis, indépendamment les uns des autres, dans l'ensemble constitué par F, Cl, Br, méthyle, éthyle, n-propyle, isopropyle, n-butyle, 2-butyle, isobutyle, tert-butyle, -OH, -O-$CH_3$, -O-$C_2H_5$ et -O-$C_3H_7$ ;

$R^2$, $R^3$, $R^4$, $R^5$, $R^6$, $R^7$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$ et $R^{27}$ indépendamment les uns des autres, représentent chacun H ; F ; Cl; Br ; I ; -$NH_2$ ; -OH ; -SH ; -CN; -$NO_2$ ; -$CF_3$; -NH-$R^{35}$; -$NR^{36}R^{37}$; -O-$R^{42}$; -S-$R^{43}$; un radical alkyle choisi dans l'ensemble constitué par les groupes méthyle, éthyle, n-propyle, isopropyle, n-butyle, 2-butyle, isobutyle, tert-butyle, n-pentyle et n-hexyle; ou représentent un radical choisi dans l'ensemble constitué par les groupes cyclopropyle, cyclobutyle, cyclopentyle et cyclohexyle;

ou $R^2$ et $R^3$ ou $R^4$ et $R^5$ ou $R^6$ et $R^7$ ou $R^{14}$ et $R^{15}$ ou $R^{16}$ et $R^{17}$ ou $R^{18}$ et $R^{19}$ ou $R^{20}$ et $R^{21}$ ou $R^{22}$ et $R^{23}$ ou $R^{24}$ et $R^{25}$ ou $R^{26}$ et $R^{27}$, indépendamment les uns des autres, représentent dans chaque cas un radical choisi dans l'ensemble constitué par un groupe oxo (=O) et un groupe thioxo (=S);

ou $R^1$ et $R^8$ forment ensemble avec le groupe -N-$CR^2R^3$-$(CR^4R^5)_b(CR^6R^7)$ qui les relie un radical choisi dans l'ensemble constitué par

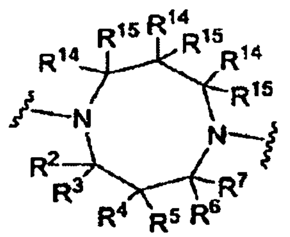 , 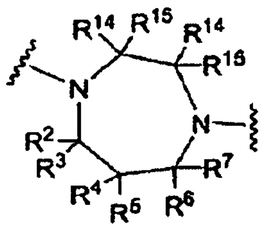 , 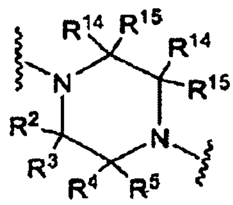 ,

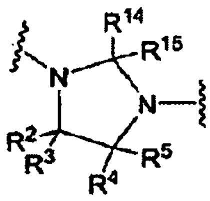 et 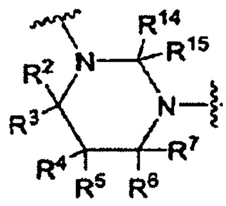 ;

ou $R^1$ et $R^2$ forment ensemble avec le groupe -N-$CR^3$ qui les relie un radical choisi dans l'ensemble constitué par

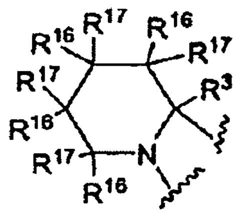 , 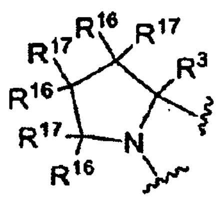 et 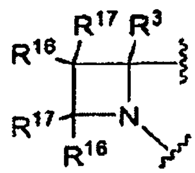 ,

dans ce cas b représentant 1 et c représentant 0 ;
ou $R^1$ et $R^4$ forment ensemble avec le groupe -N-$CR^2R^3$-$CR^5$ qui les relie un radical choisi dans l'ensemble constitué par

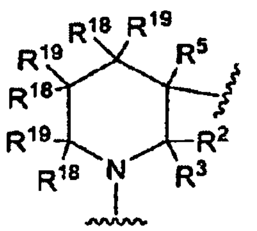 , 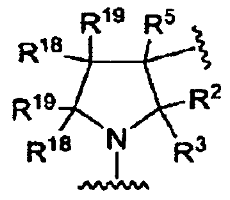 et 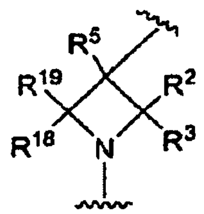 ;

dans ce cas c représentant 0 ;
$R^1$ et $R^6$ forment ensemble avec le groupe -N-$CR^2R^3$-$R^4R^5$-$R^7$ qui les relie un radical choisi dans l'ensemble constitué par

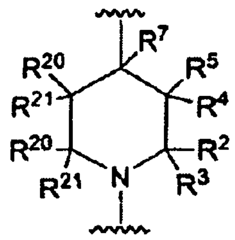 et 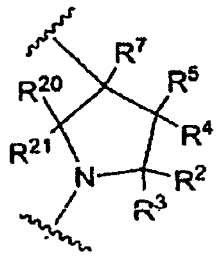 ;

ou $R^6$ et $R^8$ forment ensemble avec le groupe -N-$CR^7$ qui les relie un radical choisi dans l'ensemble constitué par

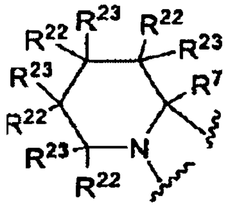 , 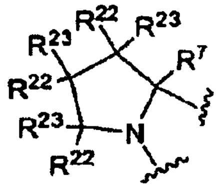 et 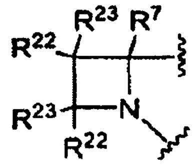 ,

dans ce cas b représentant 1 et a représentant 0 ;
ou $R^4$ et $R^8$ forment ensemble avec le groupe -N-$CR^6R^7$-$CR^5$ qui les relie un radical choisi dans l'ensemble constitué par

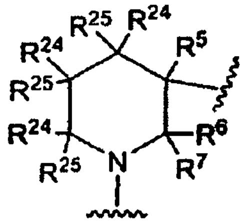 ; 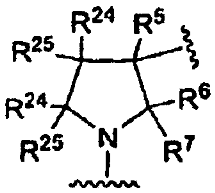 et 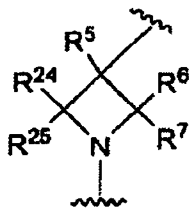 ,

dans ce cas a représentant 0 ;
ou $R^2$ et $R^8$ forment ensemble avec le groupe -N-$CR^6R^7$-$CR^4R^5$-$CR^3$ qui les relie un radical choisi dans l'ensemble constitué par

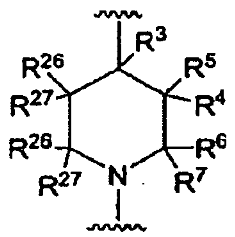 et 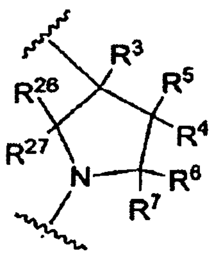 ;

$R^9$ représente un radical choisi dans l'ensemble constitué par les groupes phényle, furyle, thiényle, pyrazolyle, pyrazinyle, pyridazinyle, pyrimidinyle, pyridinyle, pyrrolyle, oxazolyle, isoxazolyle, thiazolyle, thiadiazolyle, oxadiazolyle, triazolyle et imidazolyle, qui dans chaque cas est non substitué ou porte éventuellement 1, 2, 3, 4 ou 5 substituants choisis, indépendamment les uns des autres, dans l'ensemble constitué par F, Cl, Br, I, -CN, méthyle, éthyle, n-propyle, isopropyle, n-butyle, 2-butyle, isobutyle, tert-butyle, éthényle, allyle, éthynyle, propynyle, cyclopropyle, cyclobutyle, -C≡C-Si$(CH_3)_3$, -C≡C-Si$(C_2H_5)_3$, -$CH_2$-O-$CH_3$, -$CH_2$-O-$C_2H_5$, -OH, -O-$CH_3$, -O-$C_2H_5$, -O-$C_3H_7$, -S-$CH_3$, -S-$C_2H_5$, -S(=O)-$CH_3$, -S $(=O)_2$-$CH_3$, -S (=O) -$C_2H_5$, -S $(=O)_2$-$C_2H_5$, -$NH_2$, -N$(CH_3)_2$, -N ($C_2H_5$) 2, -NH-$CH_3$, -NH-$C_2H_5$, -$NO_2$, -$CF_3$, -$CH_2F$, -$CHF_2$, -O-$CF_3$, -S-$CF_3$, -SH, -NH-S$(=O)_2$-$CH_3$, -C(=O)-OH, -C(=O)-H ; -C (=O)-$CH_3$, -C(=O)-$C_2H_5$, -C(=O)-$NH_2$, -C(=O)-N$(CH_3)_2$, -C(=O)-NH-$CH_3$, -NH-C(=O)-$CH_3$, -NH-C(=O)-$C_2H_5$, -C (=O)-O-$CH_3$, -C(=O)-O-$C_2H_5$, -C(=O)-O-C$(CH_3)_3$ et phényle ;

$R^{10}$, $R^{11}$ et $R^{12}$, chacun indépendamment, représentent H ; F ; Cl ; Br ; I ; -$CF_3$ ; -$NO_2$ ; -CN ; -C(=O)-OH ; -C(=O)-O-$R^{29}$ ; -C(=O)-$NH_2$ ; -C(=O)-NH-$R^{30}$ ; -C(=O)-N$R^{31}R^{32}$ ; -S(=O)-$R^{33}$ ; -S$(=O)_2$-$R^{34}$ ; -O-$R^{42}$ ; -S-$R^{43}$ ; un radical alkyle choisi dans l'ensemble constitué par les groupes méthyle, éthyle, n-propyle, isopropyle, n-butyle, 2-butyle, isobutyle et tert-butyle ; un radical choisi dans l'ensemble constitué par les groupes éthényle, éthynyle, allyle et propynyle ; un radical choisi dans l'ensemble constitué par les groupes cyclopropyle, cyclobutyle et cyclopentyle ; ou un radical choisi dans l'ensemble constitué par les groupes phényle, benzyle, phénéthyle, oxadiazolyle, 2-pyridyle, 3-pyridyle, 4-pyridyle, 2-thiényle, 3-thiényle, 2-furyle et 3-furyle, qui dans chaque cas est non substitué ou porte éventuellement 1, 2, 3, 4 ou 5 substituants choisis, indépendamment les uns des autres, dans l'ensemble constitué par F, Cl, Br, méthyle, éthyle, n-propyle, isopropyle, n-butyle, 2-butyle, isobutyle, tert-butyle, -OH, -O-$CH_3$, -O-$C_2H_5$ et -O-$C_3H_7$ ;

$R^{13}$ représente H ; -C(=O)-$R^{28}$ ; -C(=O)-H ; -C(=O)-O-$R^{29}$ ; -S(=O)-$R^{33}$ ; -S$(=O)_2$-$R^{34}$ ; un radical alkyle choisi dans l'ensemble constitué par les groupes méthyle, éthyle, n-propyle, isopropyle, n-butyle, 2-butyle, isobutyle et tert-butyle ; un radical choisi dans l'ensemble constitué par les groupes cyclopropyle, cyclobutyle, cyclopentyle et cyclohexyle ; ou un radical choisi dans l'ensemble constitué par les groupes phényle, benzyle, phénéthyle, oxadiazolyle, 2-pyridyle, 3-pyridyle; 4-pyridyle, 2-thiényle, 3-thiényle, 2-furyle et 3-furyle, qui dans chaque cas peut être lié par un groupe alkylène en $C_1$-$C_3$ et/ou est non substitué ou porte éventuellement 1, 2, 3, 4 ou 5 substituants choisis, indépendamment les uns des autres, dans l'ensemble constitué par F, Cl, Br, méthyle, éthyle, n-propyle, isopropyle, n-butyle, 2-butyle, isobutyle, tert-butyle, -OH, -O-$CH_3$, -O-$C_2H_5$ et -O-$C_3H_7$ ;

et $R^{28}$, $R^{29}$, $R^{30}$, $R^{31}$, $R^{32}$, $R^{33}$, $R^{34}$, $R^{35}$, $R^{36}$, $R^{37}$, $R^{42}$ et $R^{43}$, indépendamment les uns des autres, représentent chacun un radical alkyle choisi dans l'ensemble constitué par les groupes méthyle, éthyle, n-propyle, isopropyle, n-butyle, 2-butyle, isobutyle, tert-butyle, n-pentyle et n-hexyle ; -$CF_3$ ; -$C_2F_5$ ; -$CH_2$-$CF_3$ ; ou un radical phényle, benzyle ou phénéthyle, qui est non substitué ou porte éventuellement 1, 2, 3, 4 ou 5 substituants choisis, indépendamment les uns des autres, dans l'ensemble constitué par F, Cl, Br, méthyle, éthyle, n-propyle, isopropyle, n-butyle, 2-butyle, isobutyle, tert-butyle, -OH, -O-$CH_3$, -O-$C_2H_5$ et -O-$C_3H_7$ ;

chaque fois éventuellement sous forme d'un de leurs stéréoisomères, purs, en particulier énantiomères ou diastéréoisomères, de leurs racémates ou sous forme d'un mélange de stéréoisomères, en particulier des énantiomères et/ou diastéréoisomères, en un rapport quelconque de mélange, ou chaque fois sous forme de sels correspondants, ou chaque fois sous forme de produits de solvatation correspondants.

5. Composés selon une ou plusieurs des revendications 1 à 4, **caractérisés en ce que**
a, b et c représentent, chacun indépendamment, 0 ou 1, la somme de a, b et c étant égale à 1, 2 ou 3 ;
A représente un radical choisi dans l'ensemble constitué par

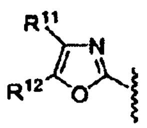 , 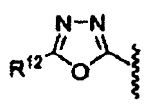 , 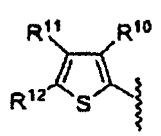 , 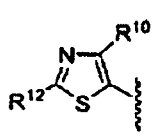 ,

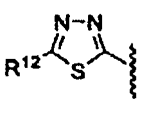 , 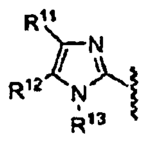 , 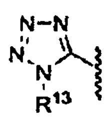 , 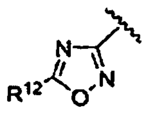 ,

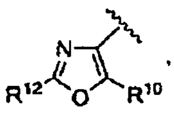 , 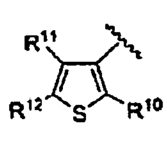 , 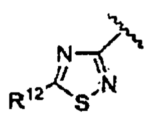 , 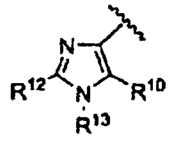 ,

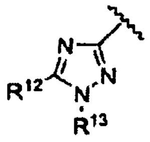 , 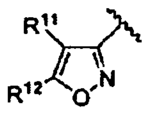 , 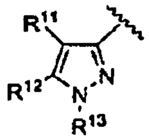 et 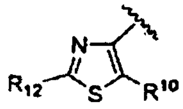 ;

$R^1$ et $R^8$, indépendamment l'un de l'autre, représentent chacun H ; -C(=O)-$R^{28}$ ; -C(=O)-O-$R^{29}$ ; un radical alkyle choisi dans l'ensemble constitué par les groupes méthyle, éthyle, n-propyle, isopropyle, n-butyle, 2-butyle, isobutyle, tert-butyle, n-pentyle et n-hexyle ; ou représentent un radical cyclopropyle ;

$R^2$, $R^3$, $R^4$, $R^5$, $R^6$, $R^7$, $R^{14}$, $R^{15}$, $R^{16}$, $R^{17}$, $R^{18}$, $R^{19}$, $R^{22}$, $R^{23}$, $R^{24}$ et $R^{25}$, indépendamment les uns des autres, représentent chacun H ; F ; Cl ; Br ; I ; -$NH_2$ ; -OH ; -SH ; -CN ; -$NO_2$ ; -$CF_3$ ; -NH-$R^{35}$ ; -$NR^{36}R^{37}$ ; -O-R ; -S-$R^{43}$ ; un radical alkyle choisi dans l'ensemble constitué par les groupes méthyle, éthyle, n-propyle, isopropyle, n-butyle, 2-butyle, isobutyle, tert-butyle, n-pentyle et n-hexyle ou représentent un radical cyclopropyle ;

ou $R^1$ et $R^8$ forment ensemble avec le groupe -N-$CR^2R^3$ - $(CR^4R^5)_b(CR^6R^7)_c$ qui les relie un radical choisi dans l'ensemble constitué par

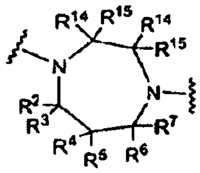 , 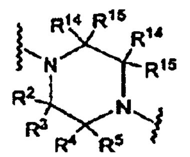 , 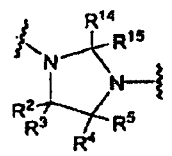 et 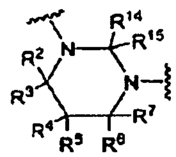 ;

ou $R^1$ et $R^2$ forment ensemble avec le groupe -N-$CR^3$ qui les relie un radical choisi dans l'ensemble constitué par

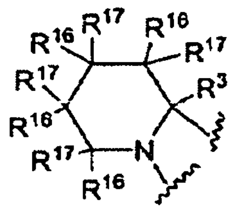 , 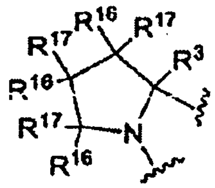 et 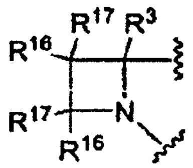 ,

dans ce cas b représentant 1 et c représentant 0 ;

ou $R^1$ et $R^4$ forment ensemble avec le groupe -N-$CR^2R^3$-$CR^5$ qui les relie un radical choisi dans l'ensemble constitué par

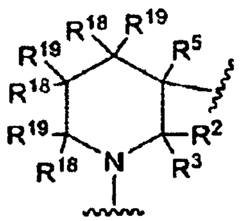 , 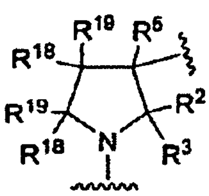 et 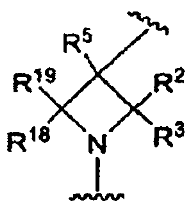 ,

dans ce cas c représentant 0 ;

ou $R^6$ et $R^8$ forment ensemble avec le groupe -N-$CR^7$ qui les relie un radical choisi dans l'ensemble constitué par

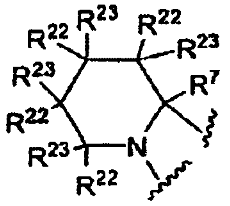 , 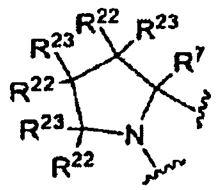 et 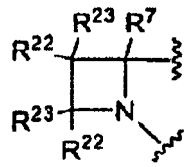 ,

dans ce cas b représentant 1 et a représentant 0 ;
ou $R^4$ et $R^8$ forment ensemble avec le groupe $-N-CR^6R^7-CR^5$ qui les relie un radical choisi dans l'ensemble constitué par

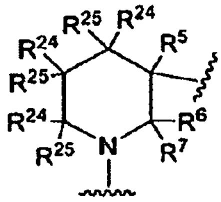 , 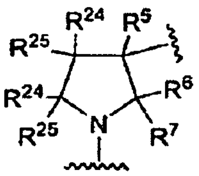 et 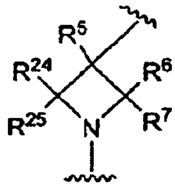 ,

dans ce cas a représentant 0 ;
$R^9$ représente un radical choisi dans l'ensemble constitué par les groupes phényle, thiényle, pyrazolyle, pyrazinyle, pyridazinyle, pyrimidinyle, pyridinyle, thiazolyle et thiadiazolyle, qui dans chaque cas est non substitué ou porte éventuellement 1, 2, 3, 4 ou 5 substituants choisis, indépendamment les uns des autres, dans l'ensemble constitué par F, Cl, Br, I, -CN, méthyle, éthyle, n-propyle, isopropyle, n-butyle, 2-butyle, isobutyle, tert-butyle, éthényle, allyle, éthynyle, propynyle, cyclopropyle, cyclobutyle, $-C\equiv C-Si(CH_3)_3$, $-C\equiv C-Si(C_2H_5)_3$, $-CH_2-O-CH_3$, $-CH_2-O-C_2H_5$, -OH, $-O-CH_3$, $-O-C_2H_5$, $-O-C_3H_7$, $-S-CH_3$, $-S-C_2H_5$, $-S(=O)-CH_3$, $-S(=O)_2-CH_3$, $-S(=O)-C_2H_5$, $-S(=O)_2-C_2H_5$, $-NH_2$, $-N(CH_3)_2$, $-N(C_2H_5)_2$, $-NH-CH_3$, $-NH-C_2H_5$, $-NO_2$, $-CF_3$, $-CH_2F$, $-CHF_2$, $-O-CF_3$, $-S-CF_3$, -SH, $-NH-S(=O)_2-CH_3$, -C (=O)-OH, -C(=O)-H ; $-C(=O)-CH_3$, $-C(=O)-C_2H_5$, $-C(=O)-NH_2$, $-C(=O)-N(CH_3)_2$, $-C(=O)-NH-CH_3$, $-NH-C(=O)-CH_3$, $-NH-C(=O)-C_2H_5$, $-C(=O)-O-CH_3$, $-C(=O)-O-C_2H_5$, $-C (=O) -O-C (CH_3)_3$ et phényle ;
$R^{10}$, $R^{11}$ et $R^{12}$, chacun indépendamment, représentent H ; F ; Cl ; Br ; I ; $-CF_3$ ; $-NO_2$ ; -CN ; -C (=O) -OH ; -C (=O)-O-$R^{29}$ ; $-C(=O)-NH_2$ ; -O-$R^{24}$ ; -S-$R^{43}$ ; éthényle ; éthynyle ; cyclopropyle ou un radical alkyle choisi dans l'ensemble constitué par les groupes méthyle, éthyle, n-propyle, isopropyle, n-butyle 2-butyle, isobutyle et tert-butyle ;
$R^{13}$ représente H ; -C(=O)-$R^{28}$ ; -C(=O)-H ; -C(=O)-O-$R^{29}$ ; -S(=O)-$R^{33}$ ; $-S(=O)_2-R^{34}$ ; le groupe cyclopropyle, cyclobutyle ; ou un radical alkyle choisi dans l'ensemble constitué par les groupes méthyle, éthyle, n-propyle, isopropyle, n-butyle, 2-butyle, isobutyle et tert-butyle ;
et $R^{28}$, $R^{29}$, $R^{33}$, $R^{34}$, $R^{35}$, $R^{36}$, $R^{37}$, $R^{42}$ et $R^{43}$, indépendamment les uns des autres, représentent chacun un radical alkyle choisi dans l'ensemble constitué par les groupes méthyle, éthyle, n-propyle, isopropyle, n-butyle, 2-butyle, isobutyle, tert-butyle, n-pentyle et n-hexyle ; $-CF_3$ ; $-C_2F_5$ ; $-CH_2-CF_3$ ; ou un radical phényle, benzyle ou phénéthyle, qui est non substitué ou porte 1, 2, 3, 4 ou 5 substituants choisis, indépendamment les uns des autres, dans l'ensemble constitué par F, Cl, Br, méthyle, éthyle, n-propyle, isopropyle, n-butyle, 2-butyle, isobutyle, tert-butyle, -OH, $-O-CH_3$, $-O-C_2H_5$ et $-O-C_3H_7$ ;
chaque fois éventuellement sous forme d'un de leurs stéréoisomères purs, en particulier énantiomères ou diastéréoisomères, de leurs racémates ou sous forme d'un mélange de stéréoisomères, en particulier des énantiomères et/ou diastéréoisomères, en un rapport quelconque de mélange, ou chaque fois sous forme de sels correspondants, ou chaque fois sous forme de produits de solvatation correspondants.

**6.** Composés selon une ou plusieurs des revendications 1 à 5, **caractérisés en ce que**
a, b et c représentent, chacun indépendamment, 0 ou 1, la somme de a, b et c étant égale à 1 ou 2 ;
A représente un radical choisi dans l'ensemble constitué par

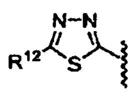 , 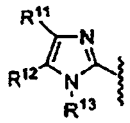 , 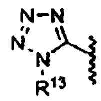 , 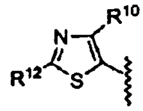 ,

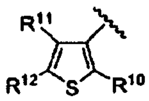 et 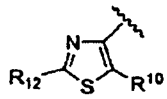 ;

$R^1$ et $R^8$, indépendamment l'un de l'autre, représentent chacun H ; -C(=O)-$R^{28}$ ; un radical alkyle choisi dans l'ensemble constitué par les groupes méthyle, éthyle, n-propyle, isopropyle, n-butyle, 2-butyle, isobutyle, tert-butyle, n-pentyle et n-hexyle ; ou représentent un radical cyclopropyle ;
$R^2$, $R^3$, $R^4$, $R^5$, $R^6$, $R^7$, $R^{14}$, $R^{15}$, $R^{16}$, $R^{17}$, $R^{22}$, $R^{23}$, $R^{24}$ et $R^{25}$, indépendamment les uns des autres, représentent chacun H ; F ; Cl ; Br ; I ; -CN ; $-NO_2$ ; $-CF_3$ ; -O-$R^{42}$ ; un radical alkyle choisi dans l'ensemble constitué par les groupes méthyle, éthyle, n-propyle,
isopropyle, n-butyle, 2-butyle, isobutyle, tert-butyle, n-pentyle et n-hexyle ou représentent un radical cyclopropyle ;
ou $R^1$ et $R^8$ forment ensemble avec le groupe -N-C$R^2R^3$-C$R^4R^5$ qui les relie le radical suivant

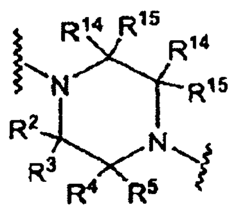 ;

ou $R^1$ et $R^2$ forment ensemble avec le groupe -N-C$R^3$ qui les relie un radical choisi dans l'ensemble constitué par

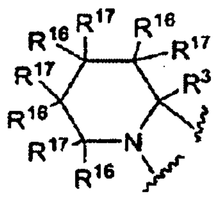 , 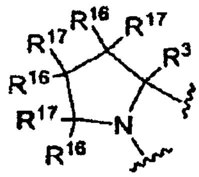 et 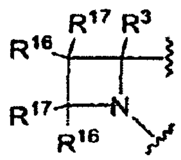 ,

dans ce cas b représentant 1 et c représentant 0 ;
ou $R^6$ et $R^8$ forment ensemble avec le groupe -N-C$R^7$ qui les relie un radical choisi dans l'ensemble constitué par

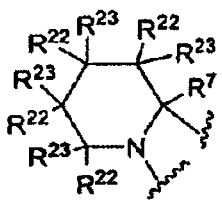 , 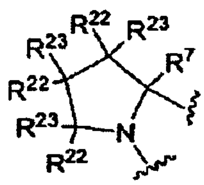 et 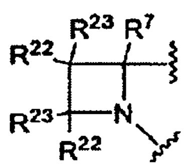 ,

dans ce cas b représentant 1 et a représentant 0 ;
$R^9$ représente un radical choisi dans l'ensemble constitué par les groupes phényle et thiényle, qui dans chaque cas est non substitué ou porte 1 ou 2 substituants choisis, indépendamment l'un de l'autre, dans l'ensemble constitué

par F, Cl, Br, I, -CN, méthyle, éthyle, n-propyle, isopropyle, n-butyle, 2-butyle, isobutyle, tert-butyle, éthényle, éthynyle, cyclopropyle, $-O-CH_3$, $-O-C_2H_5$, $-O-C_3H_7$, $-NO_2$, $-CF_3$ et $-O-CF_3$ ;

$R^{10}$, $R^{11}$ et $R^{12}$, chacun indépendamment, représentent H ; F ; Cl ; Br ; $-CF_3$ ; $-NO_2$ ; -CN ; $-O-R^{42}$ ; un radical alkyle choisi dans l'ensemble constitué par les groupes méthyle, éthyle, n-propyle, isopropyle, isobutyle et tert-butyle ; éthényle ; éthynyle ou cyclopropyle ;

$R^{13}$ représente H ; -C(=O)-H ; $-C(=O)-R^{28}$ ; cyclopropyle ; ou un radical alkyle choisi dans l'ensemble constitué par les groupes méthyle, éthyle, n-propyle, isopropyle, n-butyle, 2-butyle, isobutyle et tert-butyle ;

et $R^{28}$ et $R^{42}$, indépendamment l'un de l'autre, représentent chacun un radical alkyle choisi dans l'ensemble constitué par les groupes méthyle, éthyle, n-propyle, isopropyle, n-butyle, 2-butyle, isobutyle et tert-butyle ; ou $-CF_3$ ;

chaque fois éventuellement sous forme d'un de leurs stéréoisomères purs, en particulier énantiomères ou diastéréoisomères, de leurs racémates ou sous forme d'un mélange de stéréoisomères, en particulier des énantiomères et/ou diastéréoisomères, en un rapport quelconque de mélange, ou chaque fois sous forme de sels correspondants, ou chaque fois sous forme de produits de solvatation correspondants.

**7.** Composés selon une ou plusieurs des revendications 1 à 6, **caractérisés en ce que**

a, b et c représentent, chacun indépendamment, 0 ou 1, la somme de a, b et c étant égale à 2 ;

A représente un radical choisi dans l'ensemble constitué par

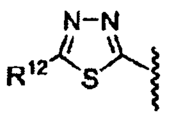 , 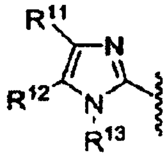 , 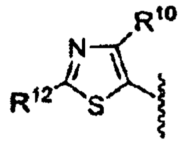 ,

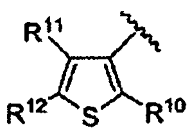 et 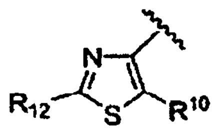 ;

$R^1$ et $R^8$, indépendamment l'un de l'autre, représentent chacun H ; $-C(=O)-CH_3$, le groupe méthyle, isopropyle ou cyclopropyle ;

$R^2$, $R^3$, $R^4$, $R^5$, $R^6$, $R^7$, $R^{14}$, $R^{15}$, $R^{16}$, $R^{17}$, $R^{22}$ et $R^{23}$, indépendamment les uns des autres, représentent chacun H ou un radical alkyle choisi dans l'ensemble constitué par les groupes méthyle, éthyle, n-propyle, isopropyle, n-butyle, 2-butyle, isobutyle, tert-butyle, n-pentyle et n-hexyle ;

ou $R^1$ et $R^8$ forment ensemble avec le groupe $-N-CR^2R^3-CR^4R^5$ qui les relie le radical suivant

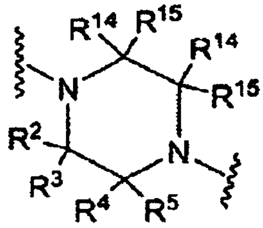 ;

ou $R^1$ et $R^2$ forment ensemble avec le groupe $-N-CR^3$ qui les relie le radical suivant

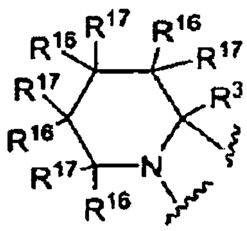,

dans ce cas b représentant 1 et c représentant 0 ;
ou $R^6$ et $R^8$ forment ensemble avec le groupe -N-C$R^7$ qui les relie le radical suivant

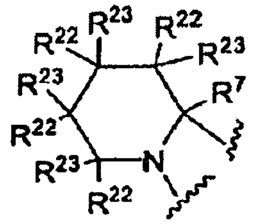,

dans ce cas b représentant 1 et a représentant 0 ;
$R^9$ représente un radical phényle qui est respectivement non substitué ou porte 1 ou 2 substituants choisis, indépendamment l'un de l'autre, dans l'ensemble constitué par F, Cl, Br, I, -CN, méthyle, éthyle, n-propyle, isopropyle, n-butyle, 2-butyle, isobutyle, tert-butyle, éthényle, éthynyle, cyclopropyle, $-O-CH_3$, $-O-C_2H_5$, $-O-C_3H_7$, $-NO_2$, $-CF_3$ et $-O-CF_3$ ;
$R^{10}$, $R^{11}$ et $R^{12}$, chacun indépendamment, représentent H ; F ; Cl ; Br ; $-CF_3$ ; $-NO_2$ ; -CN ; $-O-CF_3$ ; un radical alkyle choisi dans l'ensemble constitué par les groupes méthyle, éthyle, n-propyle, isopropyle, isobutyle et tert-butyle ; éthényle ; éthynyle ou cyclopropyle ;
et $R^{13}$ représente H ou un radical alkyle choisi dans l'ensemble constitué par les groupes méthyle, éthyle, n-propyle, isopropyle, n-butyle, 2-butyle, isobutyle et tert-butyle ;
chaque fois éventuellement sous forme d'un de leurs stéréoisomères purs, en particulier énantiomères ou diastéréoisomères, de leurs racémates ou sous forme d'un mélange de stéréoisomères, en particulier des énantiomères et/ou diastéréoisomères, en un rapport quelconque de mélange, ou chaque fois sous forme de sels correspondants, ou chaque fois sous forme de produits de solvatation correspondants.

8. Composés selon une ou plusieurs des revendications 1 à 7, **caractérisés en ce que**
a, b et c représentent, chacun indépendamment, 0 ou 1, la somme de a, b et c étant égale à 2 ;
A représente un radical choisi dans l'ensemble constitué par

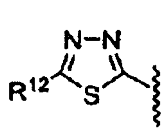, 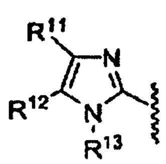, 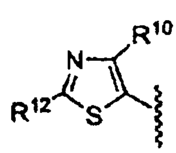,

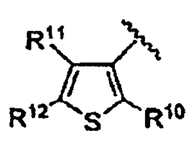 et 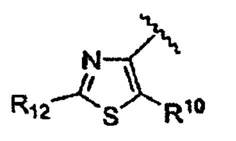 ;

$R^1$ et $R^8$, indépendamment l'un de l'autre, représentent chacun H ; le groupe méthyle ou isopropyle ;

$R^2$, $R^3$, $R^4$, $R^5$, $R^6$, $R^7$, $R^{14}$ et $R^{15}$, indépendamment les uns des autres, représentent chacun H ou un radical alkyle choisi dans l'ensemble constitué par les groupes méthyle et éthyle ;
ou $R^1$ et $R^8$ forment ensemble avec le groupe -N-$CR^2R^3$-$CR^4R^5$ qui les relie le radical suivant

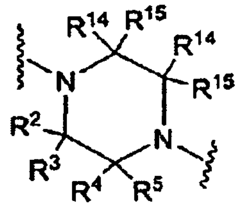

;

$R^9$ représente un radical phényle qui est respectivement non substitué ou porte 1 ou 2 substituants choisis, indépendamment l'un de l'autre, dans l'ensemble constitué par F, Cl, Br, I et -CN ;
$R^{10}$, $R^{11}$ et $R^{12}$, chacun indépendamment, représentent H ; ou un radical alkyle choisi dans l'ensemble constitué par les groupes méthyle, éthyle, n-propyle, isopropyle, isobutyle et tert-butyle ;
et $R^{13}$ représente H ou un radical alkyle choisi dans l'ensemble constitué par les groupes méthyle, éthyle, n-propyle, isopropyle, n-butyle, 2-butyle, isobutyle et tert-butyle ;
chaque fois éventuellement sous forme d'un de leurs stéréoisomères purs, en particulier énantiomères ou diastéréoisomères, de leurs racémates ou sous forme d'un mélange de stéréoisomères, en particulier des énantiomères et/ou diastéréoisomères, en un rapport quelconque de mélange, ou chaque fois sous forme de sels correspondants, ou chaque fois sous forme de produits de solvatation correspondants.

**9.** Composés selon une ou plusieurs des revendications 1 à 8, choisis dans le groupe constitué par les composés suivants

[1] 1-(3-phényl-propiolyl)-4-(thiophén-3-yl)-pipérazine,
[2] 1-(3-phényl-propiolyl)-4-(thiazol-4-yl)-pipérazine,
[3] 1-(3-phényl-propiolyl)-4-(1-méthyl-imidazol-2-yl)-pipérazine,
[4] (R)-2-méthyl-1-(3-(3-chloro-phényl)-propiolyl)-4-(thiazol-4-yl)-pipérazine,
[5] 2-méthyl-1-(3-(3-chloro-phényl)-propiolyl)-4-(1,3,4-thiadiazol-2-yl)pipérazine,
[6] 3-(3-chlorophényl)-N-(2-(méthyl(1,3,4-thiadiazol-2-yl)amino)éthyl)propiolamide,
[7] 3-(3-chlorophényl)-N-(2-(méthyl(thiazol-5-yl)amino)éthyl)propiolamide et
[8] 3-(3-chlorophényl)-N-(2-(méthyl(1-méthyl-1H-imidazol-2-yl)amino)éthyl)propiolamide

chaque fois éventuellement sous forme d'un de leurs stéréoisomères purs, en particulier énantiomères ou diastéréoisomères, de leurs racémates ou sous forme d'un mélange de stéréoisomères, en particulier des énantiomères et/ou diastéréoisomères, en un rapport quelconque de mélange, ou chaque fois sous forme de sels correspondants, ou chaque fois sous forme de produits de solvatation correspondants.

**10.** Médicament contenant au moins un composé selon une ou plusieurs des revendications 1 à 9 et éventuellement un ou plusieurs adjuvants physiologiquement acceptables.

**11.** Composés selon une ou plusieurs des revendications 1 à 9, pour le traitement et/ou la prophylaxie de la douleur, de préférence d'une douleur choisie dans le groupe constitué par la douleur aiguë, la douleur chronique, la douleur neuropathique et la douleur viscérale ; la migraine ; les dépressions ; les maladies neurodégénératives, choisies de préférence dans le groupe constitué par la sclérose en plaques, la maladie d'Alzheimer, le maladie de Parkinson et la chorée de Huntington ; les maladies cognitives, de préférence les états de déficit cognitif, de façon particulièrement préférée le syndrome de déficit de l'attention (SDA) ; les états anxieux ; les attaques de panique ; l'épilepsie ; la toux ; l'incontinence urinaire ; la diarrhée ; le prurit ; la schizophrénie ; les ischémies cérébrales ; les spasmes musculaires ; les crampes ; les maladies pulmonaires, choisies de préférence dans le groupe constitué par l'asthme et le faux croup ; la régurgitation (vomissements) ; l'accident vasculaire cérébral ; la dyskinésie ; la rétinopathie ; la perte de tonus ; l'inflammation du larynx (laryngite) ; les troubles de la prise alimentaire, choisis de préférence dans le groupe constitué par la boulimie, la cachexie, l'anorexie et l'obésité ; la dépendance à l'alcool ; la pharmacodépendance ; la dépendance aux drogues, de préférence la dépendance à la nicotine et/ou à la cocaïne ; l'abus d'alcool ; l'abus de médicaments ; l'abus de drogues ; de préférence l'abus de nicotine et/ou de cocaïne ; les

phénomènes de sevrage dans le cas de dépendance à l'alcool, aux médicaments et/ou aux drogues (en particulier nicotine et/ou cocaïne) ; le développement d'une tolérance à des médicaments, en particulier aux opioïdes naturels ou de synthèse ; le syndrome de reflux gastro-oesophagien ; la maladie du reflux gastro-oesophagien ; le syndrome de l'intestin irritable ; pour la diurèse ; pour l'antinatriurèse ; pour influer sur le système cardiovasculaire ; pour l'augmentation de la vigilance ; pour l'accroissement de la libido ; pour la modulation de l'activité motrice ou pour l'anesthésie locale.

**12.** Composés selon la revendication 11, pour le traitement et/ou la prophylaxie de la douleur, de préférence d'une douleur choisie dans le groupe constitué par la douleur aiguë, la douleur chronique, la douleur neuropathique et la douleur viscérale ; les états anxieux ; les attaques de panique ; la dépendance à l'alcool ; la pharmacodépendance ; les maladies cognitives, de préférence les états de déficit cognitif, de façon particulièrement préférée le syndrome de déficit de l'attention (SDA) ; les troubles de la prise alimentaire, choisis de préférence dans le groupe constitué par la boulimie, la cachexie, l'anorexie et l'obésité ; la dépendance aux drogues, de préférence la dépendance à la nicotine et/ou à la cocaïne ; l'abus d'alcool ; l'abus de médicaments ; l'abus de drogues ; de préférence l'abus de nicotine et/ou de cocaïne ; les phénomènes de sevrage dans le cas de dépendance à l'alcool, aux médicaments et/ou aux drogues (en particulier nicotine et/ou cocaïne) ; le développement d'une tolérance à des médicaments et/ou des drogues, en particulier aux opioïdes naturels ou de synthèse ; le syndrome de reflux gastro-oesophagien ; la maladie du reflux gastro-oesophagien et le syndrome de l'intestin irritable.

**13.** Composés selon la revendication 11 ou 12, pour le traitement de la douleur, de préférence d'une douleur choisie dans le groupe constitué par la douleur aiguë, la douleur chronique, la douleur neuropathique et la douleur viscérale.

**14.** Composés selon la revendication 11 ou 12, pour le traitement d'états anxieux ou d'attaques de panique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2005016323 A
- WO 2004014903 A
- WO 2004029044 A
- WO 03093236 A


### In der Beschreibung aufgeführte Nicht-Patentliteratur


- **T. W. Greene et al.** Monografien Protective Groups in Organic Synthesis. Wiley, 1999
- **P. J. Kocienski.** Protecting Groups. Georg Thieme Verlag, 2004
- Remington's Pharmaceutical Sciences. Mack Publishing Company, 1985
- **Dubuisson, D. ; Dennis, S.G.** *Pain,* 1977, vol. 4, 161-174